# EUROPEAN PATENT APPLICATION

(11) **EP 3 618 133 A1**
(43) Date of publication of application: **04.03.2020**
(21) Application number: 18791146.6
(22) Date of filing: 20.04.2018
(51) Int. Cl.: H01L 51/50, C09K 11/06

(54) **LIGHT-EMITTING ELEMENT**

(30) Priority: 27.04.2017 JP 2017088012
(71) Applicant: Sumitomo Chemical Company Limited, Tokyo 104-8260 (JP)
(72) Inventor: INAKAZU, Shinichi, Tsukuba-shi Ibaraki 300-3294 (JP); USUI, Motoaki, Tsukuba-shi Ibaraki 300-3294 (JP); TANAKA, Masanobu, Tsukuba-shi Ibaraki 300-3294 (JP)
(74) Representative: J A Kemp LLP
(86) International application number: PCT/JP2018/016311
(87) International publication number: WO 2018/198976

(57) **Abstract**

To provide a light emitting device excellent in luminance life.

A light emitting device having
an anode, a cathode,
a first layer containing a compound represented by the formula (C-1), disposed between the anode and the cathode,
a second layer containing at least one selected from a simple substance composed only of an alkali metal element, a simple substance composed only of a group 2 element, a compound containing an alkali metal element and a compound containing a group 2 element (simple substance or compound of second layer), disposed between the first layer and the cathode, and
a third layer containing at least one selected from a simple substance composed only of an alkali metal element, a simple substance composed only of a group 2 element, a compound containing an alkali metal element and a compound containing a group 2 element (simple substance or compound of third layer), disposed between the second layer and the cathode in contact with the cathode,
wherein at least one simple substance or compound of the second layer and at least one simple substance or compound of the third layer are mutually different: 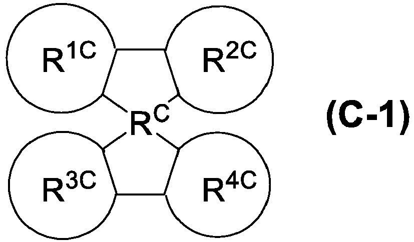 [Ring R^{1C} to Ring R^{4C} represent an aromatic hydrocarbon ring and the like, and R^{C} represents a carbon atom and the like.].

## Description

### Technical Field

The present invention relates to a light emitting device.

### Background Art

Light emitting devices such as organic electroluminescent devices and the like can be suitably used for display and lighting applications. For example, Patent Document 1 describes a light emitting device having a light emitting layer containing a compound (H0-1), an electron transporting layer containing a polymer compound containing a constitutional unit (E0) and an electron injection layer containing sodium fluoride. Patent Document 2 describes a light emitting device having a light emitting layer containing a compound (H0-2) and an electron injection layer containing lithium fluoride.

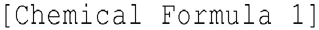

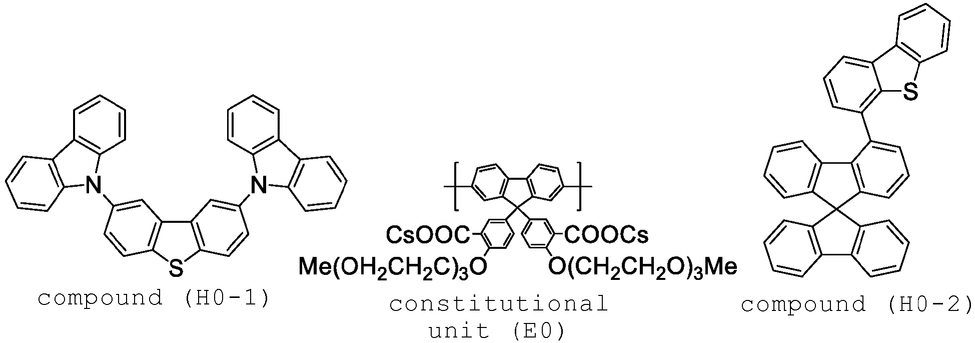

### [Prior Art Document]

### [Patent Document]

Patent Document 1: International Publication WO 2015/159932
Patent Document 2: Chinese Patent Application Publication No. 102911145

### Summary of the Invention

### Problem to be Solved by the Invention

However, this light emitting device was not necessarily sufficient in luminance life. Then, the present invention has an object of providing a light emitting device excellent in luminance life.

### Means for Solving the Problem

The present invention provides the following [1] to [13] .

[1] A light emitting device having an anode, a cathode,
   a first layer containing a compound represented by the formula (C-1), disposed between the anode and the cathode,
   a second layer containing at least one selected from the group consisting of a simple substance composed only of an alkali metal element, a simple substance composed only of a group 2 element, a compound containing an alkali metal element and a compound containing a group 2 element, disposed between the first layer and the cathode, and
   a third layer containing at least one selected from the group consisting of a simple substance composed only of an alkali metal element, a simple substance composed only of a group 2 element, a compound containing an alkali metal element and a compound containing a group 2 element, disposed between the second layer and the cathode in contact with the cathode,
   wherein the at least one selected from the group consisting of a simple substance composed only of an alkali metal element, a simple substance composed only of a group 2 element, a compound containing an alkali metal element and a compound containing a group 2 element contained in the second layer and the at least one selected from the group consisting of a simple substance composed only of an alkali metal element, a simple substance composed only of a group 2 element, a compound containing an alkali metal element and a compound containing a group 2 element contained in the third layer are mutually different: 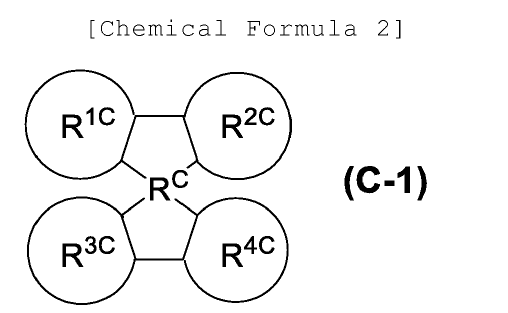 [wherein,
   Ring R^{1C}, Ring R^{2C}, Ring R^{3C} and Ring R^{4C} each independently represent an aromatic hydrocarbon ring or an aromatic hetero ring, and the foregoing rings optionally have a substituent. When a plurality of the substituents are present, they may be combined together to form a ring together with the atoms to which they are attached.
   R^{C} represents a carbon atom, a silicon atom, a germanium atom, a tin atom or a lead atom.].
[2] The light emitting device according to [1], wherein at least one of the above-described Ring R^{1C}, the above-described Ring R^{2C}, the above-described Ring R^{3C} and the above-described Ring R^{4C} has a group represented by the formula (D-1) as a substituent: 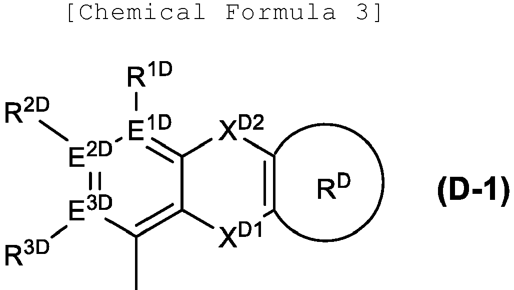 [wherein,
   Ring R^{D} represents an aromatic hydrocarbon ring or an aromatic hetero ring, and the foregoing rings optionally have a substituent. When a plurality of the substituents are present, they may be combined together to form a ring together with the atoms to which they are attached.
   X^{D1} and X^{D2} each independently represent a single bond, an oxygen atom, a sulfur atom, a group represented by -N(R^{XD1})- or a group represented by-C(R^{XD2})₂-. R^{XD1} and R^{XD2} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent. A plurality of R^{XD2} may be the same or different, and may be combined together to form a ring together with the carbon atoms to which they are attached.
   E^{1D}, E^{2D} and E^{3D} each independently represent a nitrogen atom or a carbon atom.
   R^{1D}, R^{2D} and R^{3D} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent.
   When E^{1D} is a nitrogen atom, R^{1D} is absent. When E^{2D} is a nitrogen atom, R^{2D} is absent. When E^{3D} is a nitrogen atom, R^{3D} is absent.
   R^{1D} and R^{2D} may be combined together to form a ring together with the carbon atoms to which they are attached. R^{2D} and R^{3D} may be combined together to form a ring together with the carbon atoms to which they are attached. R^{1D} and R^{XD1} may be combined together to form a ring together with the atoms to which they are attached. R^{1D} and R^{XD2} may be combined together to form a ring together with the carbon atoms to which they are attached. The substituent which Ring R^{D} optionally has and R^{XD1} may be combined together to form a ring together with the atoms to which they are attached. The substituent which Ring R^{D} optionally has and R^{XD2} may be combined together to form a ring together with the carbon atoms to which they are attached.].
[3] The light emitting device according to [2], wherein the above-described group represented by the formula (D-1) is a group represented by the formula (D-2) : 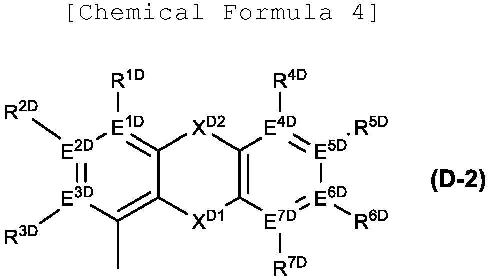 [wherein,
   X^{D1}, X^{D2}, E^{1D}, E^{2D}, E^{3D}, R^{1D}, R^{2D} and R^{3D} represent the same meaning as described above.
   E^{4D}, E^{5D}, E^{6D} and E^{7D} each independently represent a nitrogen atom or a carbon atom.
   R^{4D}, R^{5D}, R^{6D} and R^{7D} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent.
   When E^{4D} is a nitrogen atom, R^{4D} is absent. When E^{5D} is a nitrogen atom, R^{5D} is absent. When E^{6D} is a nitrogen atom, R^{6D} is absent. When E^{7D} is a nitrogen atom, R^{7D} is absent.
   R^{4D} and R^{5D}, R^{5D} and R^{6D}, R^{6D} and R^{7D}, R^{4D} and R^{XD1}, R^{4D} and R^{XD2}, R^{7D} and R^{XD1}, and R^{7D} and R^{XD2} each may be combined together to form a ring together with the atoms to which they are attached.].
[4] The light emitting device according to any one of [1] to [3], wherein the above-described compound represented by the formula (C-1) is a compound represented by the formula (C-2): 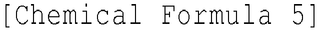 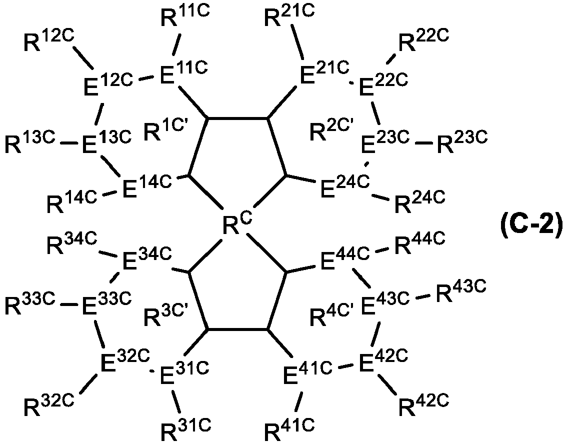 [wherein,
   R^{C} represents the same meaning as described above.
   E^{11C}, E^{12C}, E^{13C}, E^{14C}, E^{21C}, E^{22C}, E^{23C}, E^{24C}, E^{31C}, E^{32C}, E^{33C}, E^{34C}, E^{41C}, E^{42C}, E^{43C} and E^{44C} each independently represent a nitrogen atom or a carbon atom.
   Ring R^{1C'}, Ring R^{2C'}, Ring R^{3C'} and Ring R^{4C'} each independently represent a benzene ring, a pyridine ring or a diazabenzene ring.
   R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent.
   When E^{11C} is a nitrogen atom, R^{11C} is absent. When E^{12C} is a nitrogen atom, R^{12C} is absent. When E^{13C} is a nitrogen atom, R^{13C} is absent. When E^{14C} is a nitrogen atom, R^{14C} is absent. When E^{21C} is a nitrogen atom, R^{21C} is absent. When E^{22C} is a nitrogen atom, R^{22C} is absent. When E^{23C} is a nitrogen atom, R^{23C} is absent. When E^{24C} is a nitrogen atom, R^{24C} is absent. When E^{31C} is a nitrogen atom, R^{31C} is absent. When E^{32C} is a nitrogen atom, R^{32C} is absent. When E^{33C} is a nitrogen atom, R^{33C} is absent. When E^{34C} is a nitrogen atom, R^{34C} is absent. When E^{41C} is a nitrogen atom, R^{41C} is absent. When E^{42C} is a nitrogen atom, R^{42C} is absent. When E^{43C} is a nitrogen atom, R^{43C} is absent. When E^{44C} is a nitrogen atom, R^{44C} is absent.
   R^{11C} and R^{12C}, R^{12C} and R^{13C}, R^{13C} and R^{14C}, R^{14C} and R^{34C}, R^{34C} and R^{33C}, R^{33C} and R^{32C}, R^{32C} and R^{31C}, R^{31C} and R^{41C}, R^{41C} and R^{42C}, R^{42C} and R^{43C}, R^{43C} and R^{44C}, R^{44C} and R^{24C}, R^{24C} and R^{23C}, R^{23C} and R^{22C}, R^{22C} and R^{21C}, and R^{21C} and R^{11C} each may be combined together to form a ring together with the carbon atoms to which they are attached.].
[5] The light emitting device according to [4], wherein the above-described compound represented by the formula (C-2) is a compound represented by the formula (C-3): 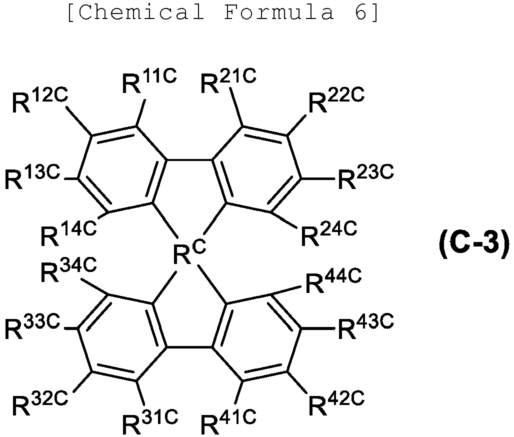 [wherein, R^{C}, R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} represent the same meaning as described above.].
[6] The light emitting device according to [4] or [5], wherein at least one of the above-described R^{11C}, the above-described R^{12C}, the above-described R^{14C}, the above-described R^{21C}, the above-described R^{22C}, the above-described R^{24C}, the above-described R^{31C}, the above-described R^{32C}, the above-described R^{34C}, the above-described R^{41C}, the above-described R^{42C} and the above-described R^{44C} is the above-described group represented by the formula (D-1).
[7] The light emitting device according to any one of [1] to [6], wherein the above-described first layer further contains a phosphorescent compound.
[8] The light emitting device according to [7], wherein the above-described phosphorescent compound is a phosphorescent compound represented by the formula (1) : 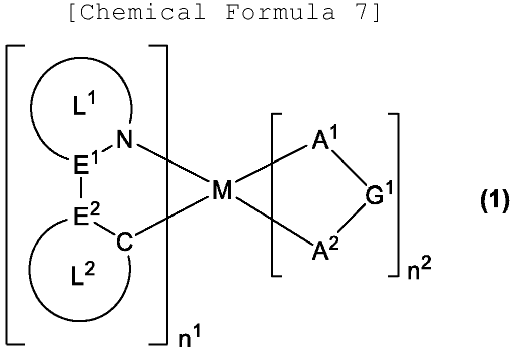 [wherein,
   M represents a ruthenium atom, a rhodium atom, a palladium atom, an iridium atom or a platinum atom.
   n¹ represents an integer of 1 or more, and n² represents an integer of 0 or more. n¹+n² is 3 when M is a ruthenium atom, a rhodium atom or an iridium atom, while n¹+n² is 2 when M is a palladium atom or a platinum atom.
   E¹ and E² each independently represent a carbon atom or a nitrogen atom. At least one of E¹ and E² is a carbon atom. When a plurality of E¹ and E² are present, they may be the same or different at each occurrence.
   Ring L¹ represents an aromatic hetero ring optionally having a substituent. When a plurality of the substituents are present, they may be combined together to form a ring together with the atoms to which they are attached. When a plurality of Ring L¹ are present, they may be the same or different.
   Ring L² represents an aromatic hydrocarbon ring or an aromatic hetero ring, and the foregoing rings optionally have a substituent. When a plurality of the substituents are present, they may be combined together to form a ring together with the atoms to which they are attached. When a plurality of Ring L² are present, they may be the same or different.

   The substituent which Ring L¹ optionally has and the substituent which Ring L² optionally has may be combined together to form a ring together with the atoms to which they are attached.
   A¹-G¹-A² represents an anionic bidentate ligand. A¹ and A² each independently represent a carbon atom, an oxygen atom or a nitrogen atom, and these atoms may be a ring constituent atom. G¹ represents a single bond or an atomic group constituting a bidentate ligand together with A¹ and A². When a plurality of A¹-G¹-A² are present, they may be the same or different.].
[9] The light emitting device according to [8], wherein the above-described phosphorescent compound represented by the formula (1) is a phosphorescent compound represented by the formula (1-A) or a phosphorescent compound represented by the formula (1-B) : 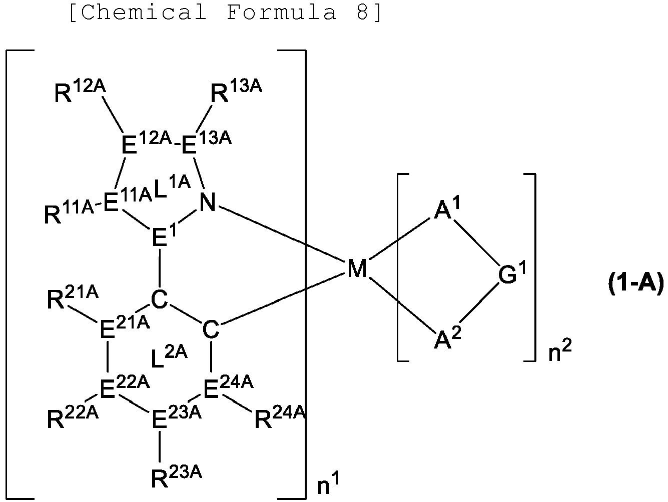 [wherein,
   M, n¹, n², E¹ and A¹-G¹-A² represent the same meaning as described above.
   E^{11A}, E^{12A}, E^{13A}, E^{21A}, E^{22A}, E^{23A} and E^{24A} each independently represent a nitrogen atom or a carbon atom. When a plurality of E^{11A}, E^{12A}, E^{13A}, E^{21A}, E^{22A}, E^{23A} and E^{24A} are present, they may be the same or different at each occurrence. When E^{11A} is a nitrogen atom, R^{11A} may be present or absent. When E^{12A} is a nitrogen atom, R^{12A} may be present or absent. When E^{13A} is a nitrogen atom, R^{13A} may be present or absent. When E^{21A} is a nitrogen atom, R^{21A} is absent. When E^{22A} is a nitrogen atom, R^{22A} is absent. When E^{23A} is a nitrogen atom, R^{23A} is absent. When E^{24A} is a nitrogen atom, R^{24A} is absent.
   R^{11A}, R^{12A}, R^{13A}, R^{21A}, R^{22A}, R^{23A} and R^{24A} each independently represent a hydrogen atom, an alkyl group, a cycloalkyl group, an alkoxy group, a cycloalkoxy group, an aryl group, an aryloxy group, an alkenyl group, a monovalent heterocyclic group, a substituted amino group or a halogen atom, and the foregoing groups optionally have a substituent. When a plurality of R^{11A}, R^{12A}, R^{13A}, R^{21A}, R^{22A}, R^{23A} and R^{24A} are present, they may be the same or different at each occurrence. R^{11A} and R^{12A}, R^{12A} and R^{13A}, R^{11A} and R^{21A}, R^{21A} and R^{22A}, R^{22A} and R^{23A}, and R^{23A} and R^{24A} each may be combined together to form a ring together with the atoms to which they are attached.
   Ring L^{1A} represents a triazole ring or a diazole ring.
   Ring L^{2A} represents a benzene ring, a pyridine ring or a diazabenzene ring.] 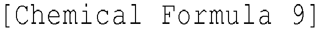 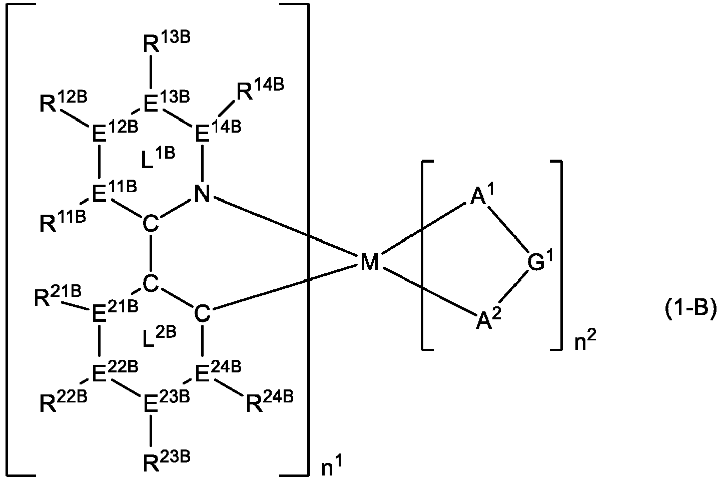 [wherein,
   M, n¹, n² and A¹-G¹-A² represent the same meaning as described above.
   E^{11B}, E^{12B}, E^{13B}, E^{14B}, E^{21B}, E^{22B}, E^{23B} and E^{24B} each independently represent a nitrogen atom or a carbon atom. When a plurality of E^{11B}, E^{12B}, E^{13B}, E^{14B}, E^{21B}, E^{22B}, E^{23B} and E^{24B} are present, they may be the same or different at each occurrence. When E^{11B} is a nitrogen atom, R^{11B} is absent. When E^{12B} is a nitrogen atom, R^{12B} is absent. When E^{13B} is a nitrogen atom, R^{13B} is absent. When E^{14B} is a nitrogen atom, R^{14B} is absent. When E^{21B} is a nitrogen atom, R^{21B} is absent. When E^{22B} is a nitrogen atom, R^{22B} is absent. When E^{23B} is a nitrogen atom, R^{23B} is absent. When E^{24B} is a nitrogen atom, R^{24B} is absent.
   R^{11B}, R^{12B}, R^{13B}, R^{14B}, R^{21B}, R^{22B}, R^{23B} and R^{24B} each independently represent a hydrogen atom, an alkyl group, a cycloalkyl group, an alkoxy group, a cycloalkoxy group, an aryl group, an aryloxy group, an alkenyl group, a monovalent heterocyclic group, a substituted amino group or a halogen atom, and the foregoing groups optionally have a substituent. When a plurality of R^{11B}, R^{12B}, R^{13B}, R^{14B}, R^{21B}, R^{22B}, R^{23B} and R^{24B} are present, they may be the same or different at each occurrence. R^{11B} and R^{12B}, R^{12B} and R^{13B}, R^{13B} and R^{14B}, R^{11B} and R^{21B}, R^{21B} and R^{22B}, R^{22B} and R^{23B}, and R^{23B} and R^{24B} each may be combined together to form a ring together with the carbon atoms to which they are attached.
   Ring L^{1B} represents a pyridine ring or a diazabenzene ring.
   Ring L^{2B} represents a benzene ring, a pyridine ring or a diazabenzene ring.].
[10] The light emitting device according to any one of [1] to [9], wherein the above-described second layer is a layer containing at least one selected from the group consisting of the above-described compound containing an alkali metal element and the above-described compound containing a group 2 element.
[11] The light emitting device according to any one of [1] to [10], wherein the above-described third layer is a layer containing at least one selected from the group consisting of the above-described compound containing an alkali metal element and the above-described simple substance composed only of a group 2 element.
[12] The light emitting device according to any one of [1] to [11], wherein the above-described second layer and the above-described third layer are adjacent.
[13] The light emitting device according to any one of [1] to [12], wherein the above-described first layer and the above-described second layer are adjacent.

### Effect of the Invention

According to the present invention, a light emitting device excellent in luminance life can be provided.

### Modes for Carrying Out the Invention

Suitable embodiments of the present invention will be illustrated in detail below.

### <Explanation of common terms>

Terms commonly used in the present specification have the following meanings unless otherwise stated.

Me represents a methyl group, Et represents an ethyl group, Bu represents a butyl group, i-Pr represents an isopropyl group, and t-Bu represents a tert-butyl group.

A hydrogen atom may be a heavy hydrogen atom or a light hydrogen atom.

In the formulae representing a metal complex and a phosphorescent compound, the solid line representing the bond to a metal means an ion bond, a covalent bond or a coordinate bond.

"The polymer compound" means a polymer having molecular weight distribution and having a polystyrene-equivalent number-average molecular weight of 1×10³ to 1×10⁸.

"The low molecular compound" means a compound having no molecular weight distribution and having a molecular weight of 1×10⁴ or less.

"The constitutional unit" means a unit which is present one or more times in the polymer compound.

"The alkyl group" may be any of linear or branched. The number of carbon atoms of the linear alkyl group is, not including the number of carbon atoms of the substituent, usually 1 to 50, preferably 3 to 30, more preferably 4 to 20. The number of carbon atoms of the branched alkyl group is, not including the number of carbon atoms of the substituent, usually 3 to 50, preferably 3 to 30, more preferably 4 to 20.

The alkyl group optionally has a substituent, and examples thereof include a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group, and these groups in which its hydrogen atom is substituted with a cycloalkyl group, an alkoxy group, a cycloalkoxy group, an aryl group, a fluorine atom or the like (for example, a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl)propyl group and a 6-ethyloxyhexyl group).

The number of carbon atoms of the "cycloalkyl group" is, not including the number of carbon atoms of the substituent, usually 3 to 50, preferably 3 to 30, more preferably 4 to 20.

The cycloalkyl group optionally has a substituent, and examples thereof include a cyclohexyl group, a cyclohexylmethyl group and a cyclohexylethyl group.

"The aryl group" means an atomic group remaining after removing from an aromatic hydrocarbon one hydrogen atom directly bonding to a carbon atom constituting the ring. The number of carbon atoms of the aryl group is, not including the number of carbon atoms of the substituent, usually 6 to 60, preferably 6 to 20, more preferably 6 to 10.

The aryl group optionally has a substituent, and examples thereof include a phenyl group, a 1-naphthyl group, a 2-naphthyl group, a 1-anthracenyl group, a 2-anthracenyl group, a 9-anthracenyl group, a 1-pyrenyl group, a 2-pyrenyl group, a 4-pyrenyl group, a 2-fluorenyl group, a 3-fluorenyl group, a 4-fluorenyl group, a 2-phenylphenyl group, a 3-phenylphenyl group and a 4-phenylphenyl group, and these groups in which its hydrogen atom is substituted with an alkyl group, a cycloalkyl group, an alkoxy group, a cycloalkoxy group, an aryl group, a fluorine atom or the like.

"The alkoxy group" may be any of linear or branched. The number of carbon atoms of the linear alkoxy group is, not including the number of carbon atoms of the substituent, usually 1 to 40, preferably 4 to 10. The number of carbon atoms of the branched alkoxy group is, not including the number of carbon atoms of the substituent, usually 3 to 40, preferably 4 to 10.

The alkoxy group optionally has a substituent, and examples thereof include a methoxy group, an ethoxy group, a propyloxy group, an isopropyloxy group, a butyloxy group, an isobutyloxy group, a tert-butyloxy group, a pentyloxy group, a hexyloxy group, a heptyloxy group, an octyloxy group, a 2-ethylhexyloxy group, a nonyloxy group, a decyloxy group, a 3,7-dimethyloctyloxy group and a lauryloxy group, and these groups in which its hydrogen atom is substituted with a cycloalkyl group, an alkoxy group, a cycloalkoxy group, an aryl group, a fluorine atom or the like.

The number of carbon atoms of the "cycloalkoxy group" is, not including the number of carbon atoms of the substituent, usually 3 to 40, preferably 4 to 10.

The cycloalkoxy group optionally has a substituent, and examples thereof include a cyclohexyloxy group.

The number of carbon atoms of the "aryloxy group" is, not including the number of carbon atoms of the substituent, usually 6 to 60, preferably 6 to 48. The aryloxy group optionally has a substituent, and examples thereof include a phenoxy group, a 1-naphthyloxy group, a 2-naphthyloxy group, a 1-anthracenyloxy group, a 9-anthracenyloxy group and a 1-pyrenyloxy group, and these groups in which its hydrogen atom is substituted with an alkyl group, a cycloalkyl group, an alkoxy group, a cycloalkoxy group, a fluorine atom or the like.

"The p-valent heterocyclic group" (p represents an integer of 1 or more.) means an atomic group remaining after removing from a heterocyclic compound p hydrogen atoms among hydrogen atoms directly bonding to carbon atoms or hetero atoms constituting the ring. Of the p-valent heterocyclic groups, preferable are "p-valent aromatic heterocyclic groups" which are atomic groups remaining after removing from an aromatic heterocyclic compound p hydrogen atoms among hydrogen atoms directly bonding to carbon atoms or hetero atoms constituting the ring.

"The aromatic heterocyclic compound" means a compound in which the hetero ring itself shows aromaticity such as oxadiazole, thiadiazole, thiazole, oxazole, thiophene, pyrrole, phosphole, furan, pyridine, pyrazine, pyrimidine, triazine, pyridazine, quinoline, isoquinoline, carbazole, dibenzophosphole and the like and a compound in which an aromatic ring is condensed to the hetero ring even if the hetero ring itself shows no aromaticity such as phenoxazine, phenothiazine, dibenzoborole, dibenzosilole, benzopyran and the like.

The number of carbon atoms of the monovalent heterocyclic group is, not including the number of carbon atoms of the substituent, usually 2 to 60, preferably 4 to 20.

The monovalent heterocyclic group optionally has a substituent, and examples thereof include a thienyl group, a pyrrolyl group, a furyl group, a pyridyl group, a piperidinyl group, a quinolinyl group, an isoquinolinyl group, a pyrimidinyl group and a triazinyl group, and these groups in which its hydrogen atom is substituted with an alkyl group, a cycloalkyl group, an alkoxy group, a cycloalkoxy group or the like.

"The halogen atom" denotes a fluorine atom, a chlorine atom, a bromine atom or an iodine atom.

"The amino group" optionally has a substituent, and a substituted amino group is preferable. The substituent which the amino group has is preferably an alkyl group, a cycloalkyl group, an aryl group or a monovalent heterocyclic group.

The substituted amino group includes, for example, a dialkylamino group, a dicycloalkylamino group and a diarylamino group.

The amino group includes, for example, a dimethylamino group, a diethylamino group, a diphenylamino group, a bis(4-methylphenyl)amino group, a bis(4-tert-butylphenyl)amino group and a bis(3,5-di-tert-butylphenyl)amino group.

"The alkenyl group" may be any of linear or branched. The number of carbon atoms of the linear alkenyl group is, not including the number of carbon atoms of the substituent, usually 2 to 30, preferably 3 to 20. The number of carbon atoms of the branched alkenyl group is, not including the number of carbon atoms of the substituent, usually 3 to 30, preferably 4 to 20.

The number of carbon atoms of "the cycloalkenyl group" is, not including the number of carbon atoms of the substituent, usually 3 to 30, preferably 4 to 20.

The alkenyl group and the cycloalkenyl group optionally have a substituent, and examples thereof include a vinyl group, a 1-propenyl group, a 2-propenyl group, a 2-butenyl group, a 3-butenyl group, a 3-pentenyl group, a 4-pentenyl group, a 1-hexenyl group, a 5-hexenyl group and a 7-octenyl group, and these groups having a substituent.

"The alkynyl group" may be any of linear or branched. The number of carbon atoms of the alkynyl group is, not including the number of carbon atoms of the substituent, usually 2 to 20, preferably 3 to 20. The number of carbon atoms of the branched alkynyl group is, not including the number of carbon atoms of the substituent, usually 4 to 30, preferably 4 to 20.

The number of carbon atoms of "the cycloalkynyl group" is, not including the number of carbon atoms of the substituent, usually 4 to 30, preferably 4 to 20.

The alkynyl group and the cycloalkynyl group optionally have a substituent, and examples thereof include an ethynyl group, a 1-propynyl group, a 2-propynyl group, a 2-butynyl group, a 3-butynyl group, a 3-pentynyl group, a 4-pentynyl group, a 1-hexynyl group and a 5-hexynyl group, and these groups having a substituent.

"The arylene group" means an atomic group remaining after removing from an aromatic hydrocarbon two hydrogen atoms directly bonding to carbon atoms constituting the ring. The number of carbon atoms of the arylene group is, not including the number of carbon atoms of the substituent, usually 6 to 60, preferably 6 to 30, more preferably 6 to 18.

The arylene group optionally has a substituent, and examples thereof include a phenylene group, a naphthalenediyl group, an anthracenediyl group, a phenanthrenediyl group, a dihydrophenanthrenediyl group, a naphthacenediyl group, a fluorenediyl group, a pyrenediyl group, a perylenediyl group and a chrysenediyl group, and these groups having a substituent, and preferable are groups represented by the formula (A-1) to the formula (A-20). The arylene group includes groups in which a plurality of these groups are connected. 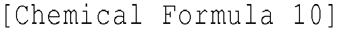 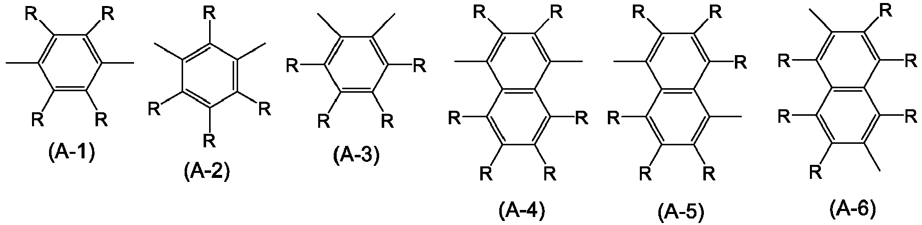 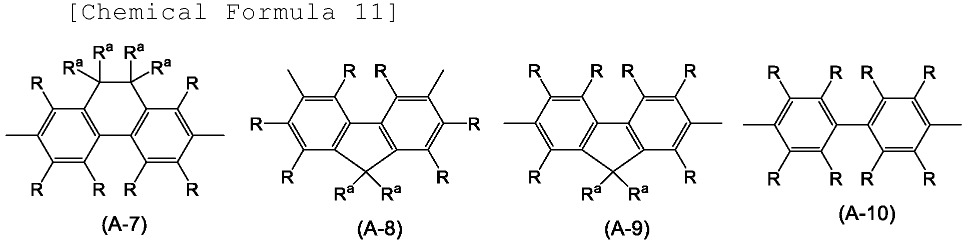 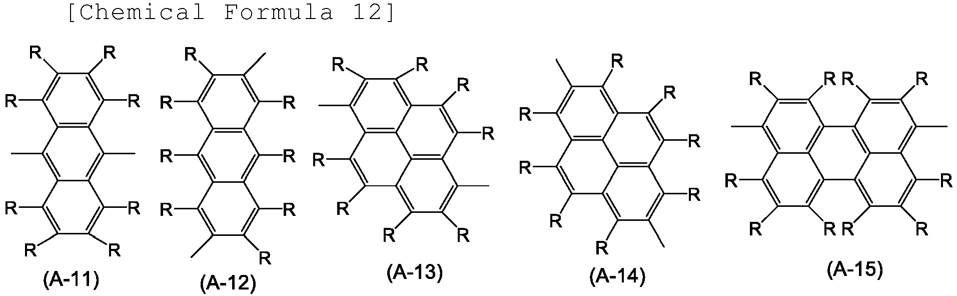 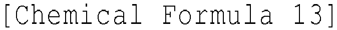 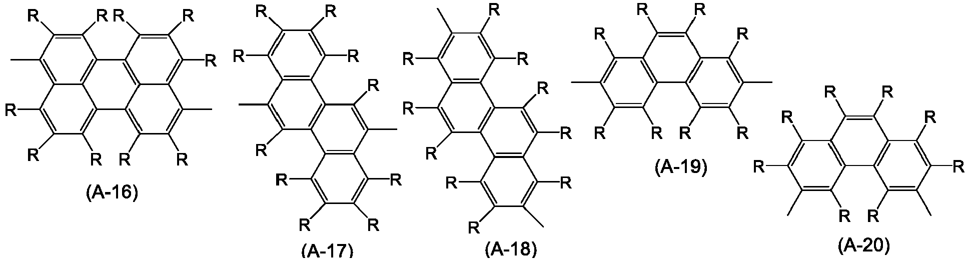 [wherein, R and R^{a} each independently represent a hydrogen atom, an alkyl group, a cycloalkyl group, an aryl group or a monovalent heterocyclic group. A plurality of R and R^{a} each may be the same or different, and the groups R^{a} may be combined together to form a ring together with the atoms to which they are attached.]

The number of carbon atoms of the divalent heterocyclic group is, not including the number of carbon atoms of the substituent, usually 2 to 60, preferably 3 to 20, more preferably 4 to 15.

The divalent heterocyclic group optionally has a substituent, and examples thereof include divalent groups obtained by removing from pyridine, diazabenzene, triazine, azanaphthalene, diazanaphthalene, carbazole, dibenzofuran, dibenzothiophene, dibenzosilole, phenoxazine, phenothiazine, acridine, dihydroacridine, furan, thiophene, azole, diazole or triazole two hydrogen atoms among hydrogen atoms directly bonding to carbon atoms or hetero atoms constituting the ring, and preferable are groups represented by the formula (AA-1) to the formula (AA-34). The divalent heterocyclic group includes groups in which a plurality of these groups are connected. 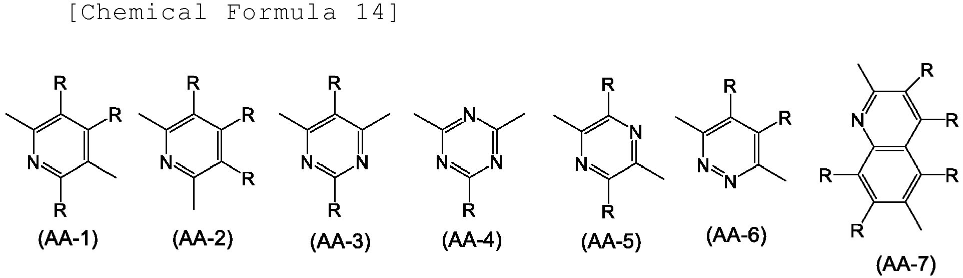 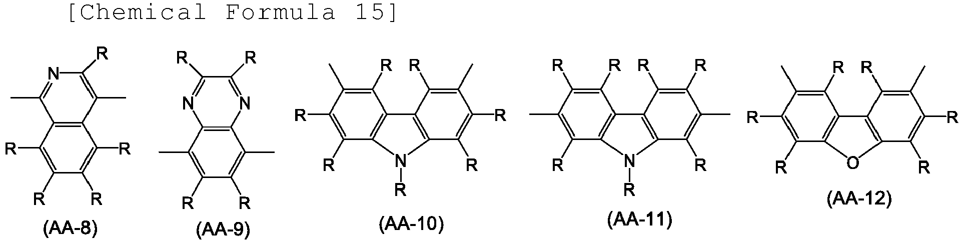 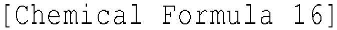 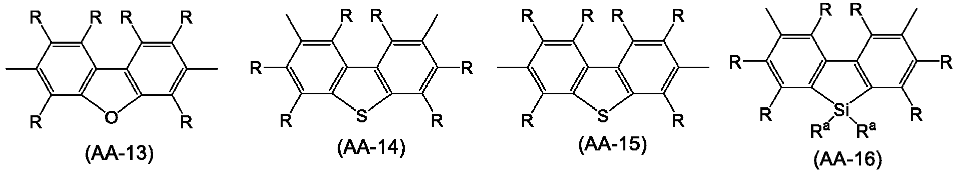 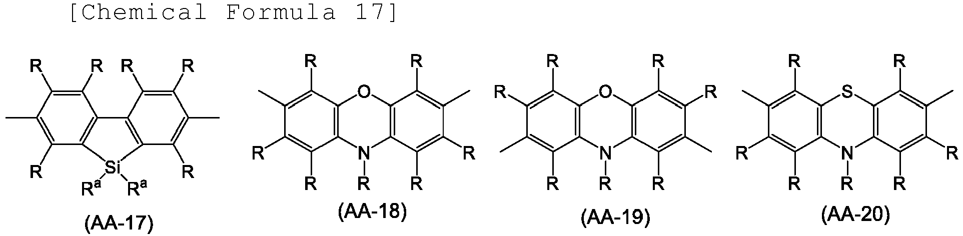 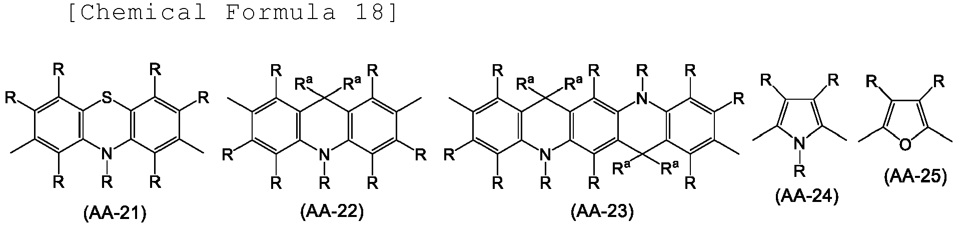 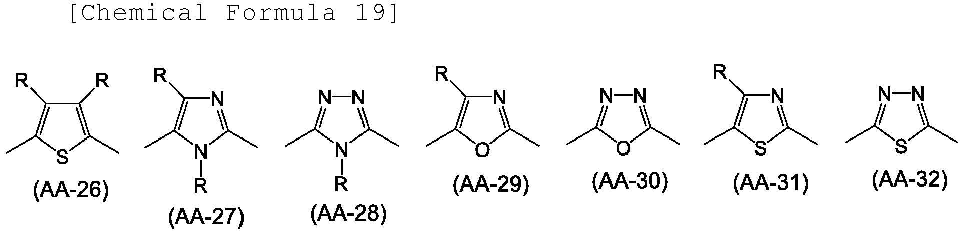 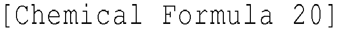 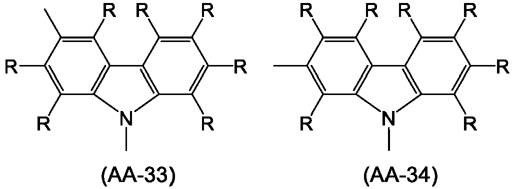 [wherein, R and R^{a} represent the same meaning as described above.]

"The crosslinking group" is a group capable of generating a new bond by being subjected to heating, ultraviolet irradiation, near ultraviolet irradiation, visible light irradiation, infrared irradiation, radical reaction and the like, and preferable are crosslinking groups represented by the formula (XL-1) to the formula (XL-17) in Group A of crosslinking group.

### (Group A of crosslinking group)

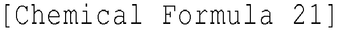

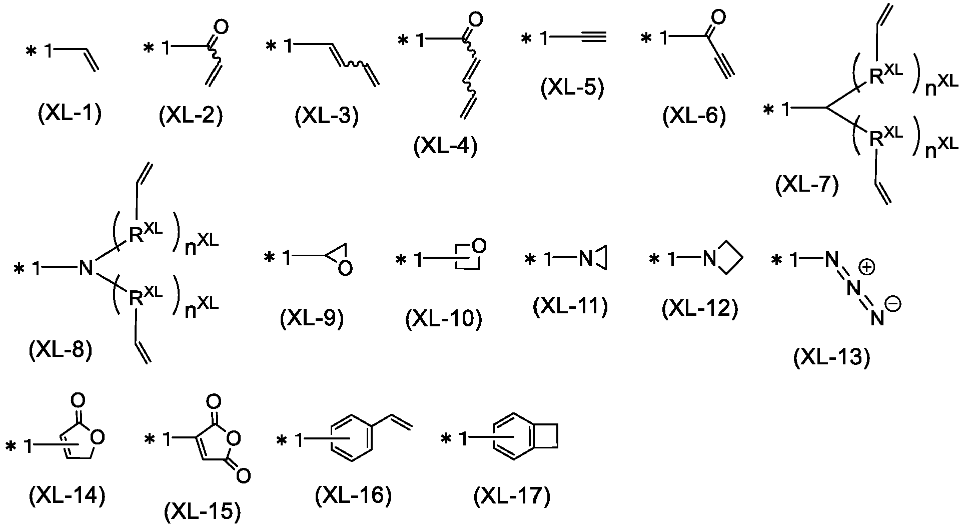

[wherein, R^{XL} represents a methylene group, an oxygen atom or a sulfur atom, and n^{XL} represents an integer of 0 to 5. When a plurality of R^{XL} are present, they may be the same or different, and when a plurality of n^{XL} are present, they may be the same or different. *1 represents a binding position. These crosslinking groups optionally have a substituent.]

"The substituent" denotes a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group or a cycloalkynyl group. The substituent may be a crosslinking group.

### <Light emitting device>

### [First layer]

The first layer which the light emitting device of the present invention has is a layer containing a compound represented by the formula (C-1).

### [Compound represented by the formula (C-1)]

The compound represented by the formula (C-1) has a molecular weight of preferably 2×10² to 5×10⁴, more preferably 2×10² to 5×10³, further preferably 3×10² to 3×10³, particularly preferably 4×10² to 1×10³.

The number of carbon atoms of the aromatic hydrocarbon ring represented by Ring R^{1C}, Ring R^{2C}, Ring R^{3C} and Ring R^{4C} is, not including the number of carbon atoms of the substituent, usually 6 to 60, preferably 6 to 30, more preferably 6 to 18.

The aromatic hydrocarbon ring represented by Ring R^{1C}, Ring R^{2C}, Ring R^{3C} and Ring R^{4C} includes, for example, a benzene ring, a naphthalene ring, an anthracene ring, an indene ring, a fluorene ring, a spirobifluorene ring, a phenanthrene ring, a dihydrophenanthrene ring, a pyrene ring, a chrysene ring and a triphenylene ring, and is preferably a benzene ring, a naphthalene ring, an anthracene ring, a fluorene ring, a spirobifluorene ring, a phenanthrene ring or a dihydrophenanthrene ring, more preferably a benzene ring, a naphthalene ring, a fluorene ring or a spirobifluorene ring, further preferably a benzene ring, and the foregoing rings optionally have a substituent.

The number of carbon atoms of the aromatic hetero ring represented by Ring R^{1C}, Ring R^{2C}, Ring R^{3C} and Ring R^{4C} is, not including the number of carbon atoms of the substituent, usually 2 to 60, preferably 3 to 30, more preferably 4 to 15.

The aromatic hetero ring represented by Ring R^{1C}, Ring R^{2C}, Ring R^{3C} and Ring R^{4C} includes, for example, a pyrrole ring, a diazole ring, a triazole ring, a furan ring, a thiophene ring, an oxadiazole ring, a thiadiazole ring, a pyridine ring, a diazabenzene ring, a triazine ring, an azanaphthalene ring, a diazanaphthalene ring, a triazanaphthalene ring, an azaanthracene ring, a diazaanthracene ring, a triazaanthracene ring, an azaphenanthrene ring, a diazaphenanthrene ring, a triazaphenanthrene ring, a dibenzofuran ring, a dibenzothiophene ring, a dibenzosilole ring, a dibenzophosphole ring, a carbazole ring, an azacarbazole ring, a diazacarbazole ring, a phenoxazine ring, a phenothiazine ring, a dihydroacridine ring and a dihydrophenazine ring, and is preferably a pyridine ring, a diazabenzene ring, an azanaphthalene ring, a diazanaphthalene ring, an azaanthracene ring, a diazaphenanthrene ring, a dibenzofuran ring, a dibenzothiophene ring, a carbazole ring, a phenoxazine ring, a phenothiazine ring, a dihydroacridine ring or a dihydrophenazine ring, more preferably a pyridine ring, a diazabenzene ring, an azanaphthalene ring, a diazanaphthalene ring, a dibenzofuran ring, a dibenzothiophene ring or a carbazole ring, further preferably a pyridine ring or a diazabenzene ring, and the foregoing rings optionally have a substituent.

It is preferable that at least one of Ring R^{1C}, Ring R^{2C}, Ring R^{3C} and Ring R^{4C} is an aromatic hydrocarbon ring, it is more preferable that at least two of Ring R^{1C}, Ring R^{2C}, Ring R^{3C} and Ring R^{4C} are aromatic hydrocarbon rings, it is further preferable that all Ring R^{1C}, Ring R^{2C}, Ring R^{3C} and Ring R^{4C} represent an aromatic hydrocarbon ring, it is particularly preferable that all Ring R^{1C}, Ring R^{2C}, Ring R^{3C} and Ring R^{4C} represent a benzene ring, and the foregoing rings optionally have a substituent, since the light emitting device of the present invention is more excellent in luminance life.

The substituent which Ring R^{1C}, Ring R^{2C}, Ring R^{3C} and Ring R^{4C} optionally have is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an aryl group, a monovalent heterocyclic group or a substituted amino group, further preferably an aryl group or a monovalent heterocyclic group, particularly preferably a monovalent heterocyclic group, and the foregoing groups optionally further have a substituent.

The number of carbon atoms of the aryl group as the substituent which Ring R^{1C}, Ring R^{2C}, Ring R^{3C} and Ring R^{4C} optionally have is, not including the number of carbon atoms of the substituent, usually 6 to 60, preferably 6 to 40, more preferably 6 to 25.

The aryl group as the substituent which Ring R^{1C}, Ring R^{2C}, Ring R^{3C} and Ring R^{4C} optionally have includes, for example, groups obtained by removing from a benzene ring, a naphthalene ring, an anthracene ring, an indene ring, a fluorene ring, a spirobifluorene ring, a phenanthrene ring, a dihydrophenanthrene ring, a pyrene ring, a chrysene ring, a triphenylene ring or a ring in which these rings are condensed one hydrogen atom bonding directly to a carbon atom constituting the ring, and is preferably a group obtained by removing from a benzene ring, a naphthalene ring, a fluorene ring, a spirobifluorene ring, a phenanthrene ring, a dihydrophenanthrene ring or a triphenylene ring one hydrogen atom bonding directly to a carbon atom constituting the ring, more preferably a group obtained by removing from a benzene ring, a fluorene ring or a spirobifluorene ring one hydrogen atom bonding directly to a carbon atom constituting the ring, further preferably a group obtained by removing from a fluorene ring or a spirobifluorene ring one hydrogen atom bonding directly to a carbon atom constituting the ring, and the foregoing groups optionally further have a substituent.

The number of carbon atoms of the monovalent heterocyclic group as the substituent which Ring R^{1C}, Ring R^{2C}, Ring R^{3C} and Ring R^{4C} optionally have is, not including the number of carbon atoms of the substituent, usually 2 to 60, preferably 3 to 30, more preferably 3 to 15.

The monovalent heterocyclic group as the substituent which Ring R^{1C}, Ring R^{2C}, Ring R^{3C} and Ring R^{4C} optionally have includes, for example, groups obtained by removing from a pyrrole ring, a diazole ring, a triazole ring, a furan ring, a thiophene ring, an oxadiazole ring, a thiadiazole ring, a pyridine ring, a diazabenzene ring, a triazine ring, an azanaphthalene ring, a diazanaphthalene ring, a triazanaphthalene ring, an azaanthracene ring, a diazaanthracene ring, a triazaanthracene ring, an azaphenanthrene ring, a diazaphenanthrene ring, a triazaphenanthrene ring, a dibenzofuran ring, a dibenzothiophene ring, a dibenzosilole ring, a dibenzophosphole ring, a carbazole ring, an azacarbazole ring, a diazacarbazole ring, a phenoxazine ring, a phenothiazine ring, a dihydroacridine ring, a dihydrophenazine ring or a ring obtained by condensing an aromatic ring to these rings one hydrogen atom bonding directly to a carbon atom or a hetero atom constituting the ring, and is preferably a group obtained by removing from a pyridine ring, a diazabenzene ring, a triazine ring, an azanaphthalene ring, a diazanaphthalene ring, a dibenzofuran ring, a dibenzothiophene ring, a carbazole ring, an azacarbazole ring, a diazacarbazole ring, a phenoxazine ring, a phenothiazine ring, a dihydroacridine ring or a dihydrophenazine ring one hydrogen atom bonding directly to a carbon atom or a hetero atom constituting the ring, more preferably a group obtained by removing from a pyridine ring, a diazabenzene ring, a triazine ring, a dibenzofuran ring, a dibenzothiophene ring, a carbazole ring, an azacarbazole ring, a diazacarbazole ring, a phenoxazine ring, a phenothiazine ring, a dihydroacridine ring or a dihydrophenazine ring one hydrogen atom bonding directly to a carbon atom or a hetero atom constituting the ring, further preferably a group obtained by removing from a dibenzofuran ring, a dibenzothiophene ring, a carbazole ring, a phenoxazine ring, a phenothiazine ring, a dihydroacridine ring or a dihydrophenazine ring one hydrogen atom bonding directly to a carbon atom or a hetero atom constituting the ring, particularly preferably a group obtained by removing from a dibenzofuran ring, a dibenzothiophene ring, a carbazole ring or a dihydroacridine ring one hydrogen atom bonding directly to a carbon atom or a hetero atom constituting the ring, especially preferably a group obtained by removing from a dibenzofuran ring or a dibenzothiophene ring one hydrogen atom bonding directly to a carbon atom constituting the ring, and the foregoing rings optionally have a substituent.

In the substituted amino group as the substituent which Ring R^{1C}, Ring R^{2C}, Ring R^{3C} and Ring R^{4C} optionally have, the substituent which the amino group has is preferably an aryl group or a monovalent heterocyclic group, more preferably an aryl group, and the foregoing groups optionally further have a substituent. The examples and preferable ranges of the aryl group as the substituent which the amino group has are the same as the examples and preferable ranges of the aryl group as the substituent which Ring R^{1C}, Ring R^{2C}, Ring R^{3C} and Ring R^{4C} optionally have. The examples and preferable ranges of the monovalent heterocyclic group as the substituent which the amino group has are the same as the examples and preferable ranges of the monovalent heterocyclic group as the substituent which Ring R^{1C}, Ring R^{2C}, Ring R^{3C} and Ring R^{4C} optionally have.

The substituent which the substituent which Ring R^{1C}, Ring R^{2C}, Ring R^{3C} and Ring R^{4C} optionally have optionally further has is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an aryl group or a monovalent heterocyclic group, further preferably an alkyl group or an aryl group, particularly preferably an alkyl group, and the foregoing groups optionally further have a substituent, but it is preferable that these groups have no substituent.

The examples and preferable ranges of the aryl group, the monovalent heterocyclic group and the substituted amino group as the substituent which the substituent which Ring R^{1C}, Ring R^{2C}, Ring R^{3C} and Ring R^{4C} optionally have optionally further has are the same as the examples and preferable ranges of the aryl group, the monovalent heterocyclic group and the substituted amino group as the substituent which Ring R^{1C}, Ring R^{2C}, Ring R^{3C} and Ring R^{4C} optionally have, respectively.

R^{C} is preferably a carbon atom, a silicon atom or a germanium atom, more preferably a carbon atom or a silicon atom, further preferably a carbon atom, since the light emitting device of the present invention is more excellent in luminance life.

It is preferable that at least one of Ring R^{1C}, Ring R^{2C}, Ring R^{3C} and Ring R^{4C} has an aryl group or a monovalent heterocyclic group as a substituent, it is more preferable that at least one of Ring R^{1C}, Ring R^{2C}, Ring R^{3C} and Ring R^{4C} has a group represented by the formula (D-1) as a substituent, and the foregoing groups optionally have a substituent, since the light emitting device of the present invention is more excellent in luminance life.

When at least one of Ring R^{1C}, Ring R^{2C}, Ring R^{3C} and Ring R^{4C} has an aryl group or a monovalent heterocyclic group as a substituent, the total number of the aryl group and the monovalent heterocyclic group which Ring R^{1C}, Ring R^{2C}, Ring R^{3C} and Ring R4c have is preferably 1 to 5, more preferably 1 to 3, further preferably 1 or 2, particularly preferably 1.

When at least one of Ring R^{1C}, Ring R^{2C}, Ring R^{3C} and Ring R^{4C} has a group represented by the formula (D-1) as a substituent, the total number of the group represented by the formula (D-1) which Ring R^{1C}, Ring R^{2C}, Ring R^{3C} and Ring R^{4C} have is preferably 1 to 5, more preferably 1 to 3, further preferably 1 or 2, particularly preferably 1.

### Group represented by the formula (D-1)

The examples and preferable range of the aromatic hydrocarbon ring and the aromatic hetero ring represented by Ring R^{D} are the same as the examples and preferable range of the aromatic hydrocarbon ring and the aromatic hetero ring represented by Ring R^{1C}, Ring R^{2C}, Ring R^{3C} and Ring R^{4C}, respectively.

The examples and preferable range of the substituent which Ring R^{D} optionally has are the same as the examples and preferable range of the substituent which the substituent which Ring R^{1C}, Ring R^{2C}, Ring R^{3C} and Ring R^{4C} optionally have optionally further has.

Ring R^{D} is preferably an aromatic hydrocarbon ring, more preferably a benzene ring, since the light emitting device of the present invention is more excellent in luminance life.

X^{D1} and X^{D2} are each preferably a single bond, an oxygen atom, a sulfur atom or a group represented by-C(R^{XD2})₂-, more preferably a single bond, an oxygen atom or a sulfur atom, further preferably a single bond or a sulfur atom, and it is particularly preferable that one of X^{D1} and X^{D2} is a single bond and the other is a sulfur atom, since the light emitting device of the present invention is more excellent in luminance life.

It is preferable that at least one of X^{D1} and X^{D2} is a single bond, it is more preferable that X^{D2} is a single bond.

R^{XD1} is preferably an alkyl group, a cycloalkyl group, an aryl group or a monovalent heterocyclic group, more preferably an aryl group or a monovalent heterocyclic group, further preferably an aryl group, and the foregoing groups optionally have a substituent.

R^{XD2} is preferably an alkyl group, a cycloalkyl group, an aryl group or a monovalent heterocyclic group, more preferably an alkyl group or an aryl group, and the foregoing groups optionally have a substituent.

The examples and preferable range of the aryl group, the monovalent heterocyclic group and the substituted amino group represented by R^{XD1} and R^{XD2} are the same as the examples and preferable range of the aryl group, the monovalent heterocyclic group and the substituted amino group as the substituent which Ring R^{1C}, Ring R^{2C}, Ring R^{3C} and Ring R^{4C} optionally have, respectively.

Regarding the combination of two groups R^{XD2} in the group represented by -C(R^{XD2})₂- represented by X^{D1} and X^{D2}, it is preferable that both are alkyl groups or cycloalkyl groups, both are aryl groups, both are monovalent heterocyclic groups, or one is an alkyl group or a cycloalkyl group and the other is an aryl group or a monovalent heterocyclic group, it is more preferable that both are aryl groups, or one is an alkyl group or a cycloalkyl group and the other is an aryl group, it is further preferable that both are aryl groups, and the foregoing groups optionally have a substituent. It is preferable that two groups R^{XD2} are combined together to form a ring together with the carbon atoms to which they are attached. When groups R^{XD2} form a ring, the group represented by -C(R^{XD2})₂- is preferably a group represented by the formula (Y-A1)-the formula (Y-A5), more preferably a group represented by the formula (Y-A4), and the foregoing groups optionally have a substituent.

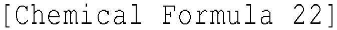

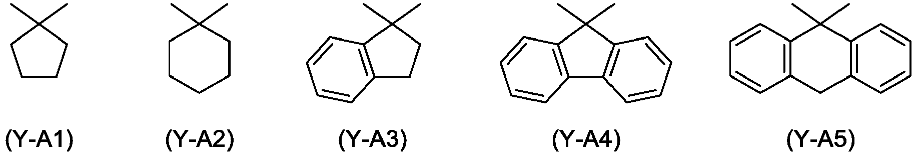

The examples and preferable range of the substituent which R^{XD1} and R^{XD2} optionally have are the same as the examples and preferable range of the substituent which the substituent which Ring R^{1C}, Ring R^{2C}, Ring R^{3C} and Ring R^{4C} optionally have optionally further has.

E^{1D}, E^{2D} and E^{3D} are each preferably a carbon atom.

R^{1D}, R^{2D} and R^{3D} are each preferably a hydrogen atom, an alkyl group, a cycloalkyl group, an aryl group, a monovalent heterocyclic group or a substituted amino group, more preferably a hydrogen atom, an alkyl group or an aryl group, further preferably a hydrogen atom, and the foregoing groups optionally further have a substituent.

The examples and preferable range of the aryl group, the monovalent heterocyclic group and the substituted amino group represented by R^{1D}, R^{2D} and R^{3D} are the same as the examples and preferable range of the aryl group, the monovalent heterocyclic group and the substituted amino group as the substituent which Ring R^{1C}, Ring R^{2C}, Ring R^{3C} and Ring R^{4C} optionally have, respectively.

The examples and preferable range of the substituent which R^{1D}, R^{2D} and R^{3D} optionally have are the same as the examples and preferable range of the substituent which R^{XD1} and R^{XD2} optionally have.

R^{1D} and R^{2D}, R^{2D} and R^{3D}, R^{1D} and R^{XD1}, R^{1D} and R^{XD2}, R^{XD1} and a substituent which Ring R^{D} optionally has, and R^{XD2} and a substituent which Ring R^{D} optionally has each may be combined together to form a ring together with the carbon atoms to which they are attached, but it is preferable that they do not form a ring.

The group represented by the formula (D-1) is preferably a group represented by the formula (D-2), since the light emitting device of the present invention is more excellent in luminance life.

E^{4D}, E^{5D}, E^{6D} and E^{7D} are each preferably a carbon atom.

The examples and preferable range of R^{4D}, R^{5D}, R^{6D} and R^{7D} are the same as the examples and preferable range of R^{1D}, R^{2D} and R^{3D}.

The examples and preferable range of the substituent which R^{4D}, R^{5D}, R^{6D} and R^{7D} optionally have are the same as the examples and preferable range of the substituent which R^{1D}, R^{2D} and R^{3D} optionally have.

R^{4D} and R^{5D}, R^{5D} and R^{6D}, and R^{6D} and R^{7D} each may be combined together to form a ring together with the carbon atoms to which they are attached, but it is preferable that they do not form a ring.

### [Compound represented by the formula (C-2)]

The compound represented by the formula (C-1) is preferably a compound represented by the formula (C-2), since the light emitting device of the present invention is more excellent in luminance life.

E^{11C}, E^{12C}, E^{13C}, E^{14C}, E^{21C}, E^{22C}, E^{23C}, E^{24C}, E^{31C}, E^{32C}, E^{33C}, E^{34C}, E^{41C}, E^{42C}, E^{43C} and E^{44C} are each preferably a carbon atom.

Ring R^{1C'}, Ring R^{2C'}, Ring R^{3C'} and Ring R^{4C'} are each preferably a benzene ring.

R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} are each preferably a hydrogen atom, an alkyl group, a cycloalkyl group, an aryl group, a monovalent heterocyclic group or a substituted amino group, more preferably a hydrogen atom, an aryl group or a monovalent heterocyclic group, further preferably a hydrogen atom or a group represented by the formula (D-1), and the foregoing groups optionally further have a substituent.

It is preferable that at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is an aryl group or a monovalent heterocyclic group, more preferably a group represented by the formula (D-1), and the foregoing groups optionally further have a substituent.

The examples and preferable range of the aryl group, the monovalent heterocyclic group and the substituted amino group represented by R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} are the same as the examples and preferable range of the aryl group, the monovalent heterocyclic group and the substituted amino group as the substituent which Ring R^{1C}, Ring R^{2C}, Ring R^{3C} and Ring R^{4C} optionally have, respectively.

The examples and preferable range of the substituent which R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} optionally have are the same as the examples and preferable range of the substituent which the substituent which Ring R^{1C}, Ring R^{2C}, Ring R^{3C} and Ring R^{4C} optionally have optionally further has.

When at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is an aryl group or a monovalent heterocyclic group, the total number of the aryl group or the monovalent heterocyclic group represented by R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is preferably 1 to 5, more preferably 1 to 3, further preferably 1 or 2, particularly preferably 1.

When at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is a group represented by the formula (D-1), the total number of the group represented by the formula (D-1) represented by R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is preferably 1 to 5, more preferably 1 to 3, further preferably 1 or 2, particularly preferably 1.

When at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is an aryl group or a monovalent heterocyclic group, it is preferable that at least one of R^{11C}, R^{12C}, R^{14C}, R^{21C}, R^{22C}, R^{24C}, R^{31C}, R^{32C}, R^{34C}, R^{41C}, R^{42C} and R^{44C} is an aryl group or a monovalent heterocyclic group, it is more preferable that at least one of R^{11C}, R^{12C}, R^{21C}, R^{22C}, R^{31C}, R^{32C}, R^{41C} and R^{42C} is an aryl group or a monovalent heterocyclic group, it is further preferable that at least one of R^{11C}, R^{12C}, R^{21C} and R^{22C} is an aryl group or a monovalent heterocyclic group, it is particularly preferable that at least one of R^{11C} and R^{12C} is an aryl group or a monovalent heterocyclic group, and the foregoing groups optionally have a substituent.

When at least one of R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} is a group represented by the formula (D-1), it is preferable that at least one of R^{11C}, R^{12C}, R^{14C}, R^{21C}, R^{22C}, R^{24C}, R^{31C}, R^{32C}, R^{34C}, R^{41C}, R^{42C} and R^{44C} is a group represented by the formula (D-1), it is more preferable that at least one of R^{11C}, R^{12C}, R^{21C}, R^{22C}, R^{31C}, R^{32C}, R^{41C} and R^{42C} is a group represented by the formula (D-1), it is further preferable that at least one of R^{11C}, R^{12C}, R^{21C} and R^{22C} is a group represented by the formula (D-1), it is particularly preferable that at least one of R^{11C} and R^{12C} is a group represented by the formula (D-1), it is especially preferable that R^{12C} is a group represented by the formula (D-1).

R^{11C} and R^{12C}, R^{12C} and R^{13C}, R^{13C} and R^{14C}, R^{14C} and R^{34C}, R^{34C} and R^{33C}, R^{33C} and R^{32C}, R^{32C} and R^{31C}, R^{31C} and R^{41C}, R^{41C} and R^{42C}, R^{42C} and R^{43C}, R^{43C} and R^{44C}, R^{44C} and R^{24C}, R^{24C} and R^{23C}, R^{23C} and R^{22C}, R^{22C} and R^{21C}, and R^{21C} and R^{11C} each may be combined together to form a ring together with the carbon atoms to which they are attached, but it is preferable that they do not form a ring.

### [Compound represented by the formula (C-3)]

The compound represented by the formula (C-2) is preferably a compound represented by the formula (C-3), since the light emitting device of the present invention is more excellent in luminance life.

The compound represented by the formula (C-1) includes, for example, compounds represented by the formula (C-101) to the formula (C-137). 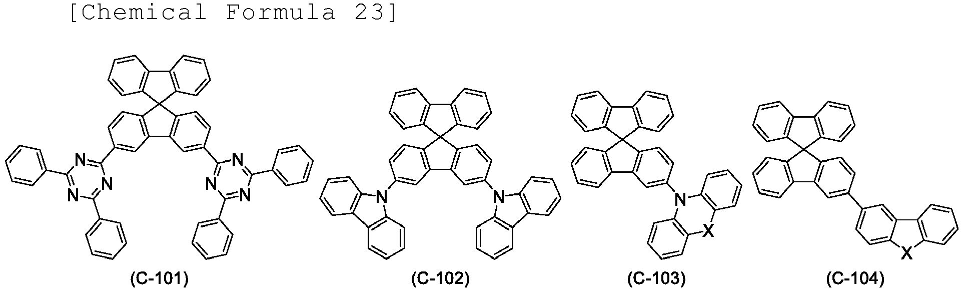 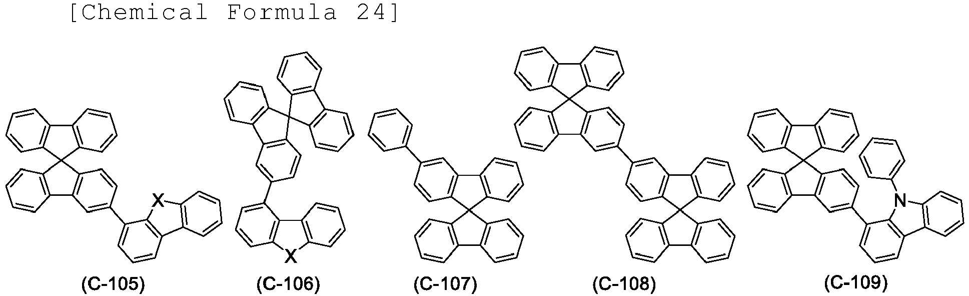 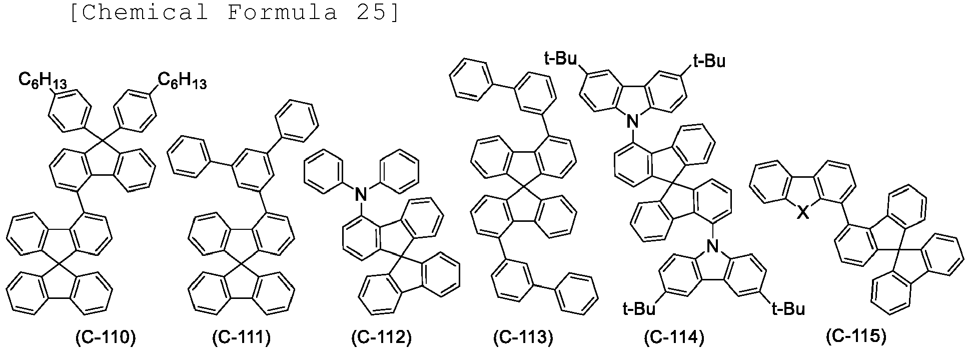 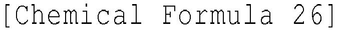 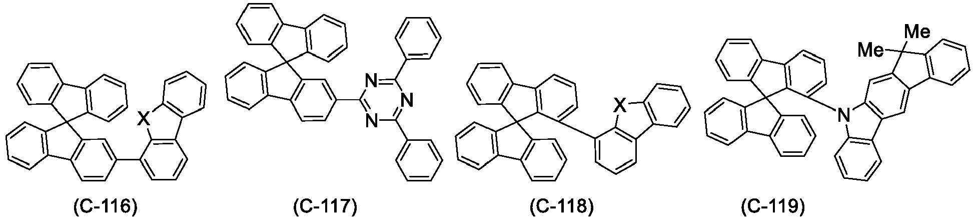 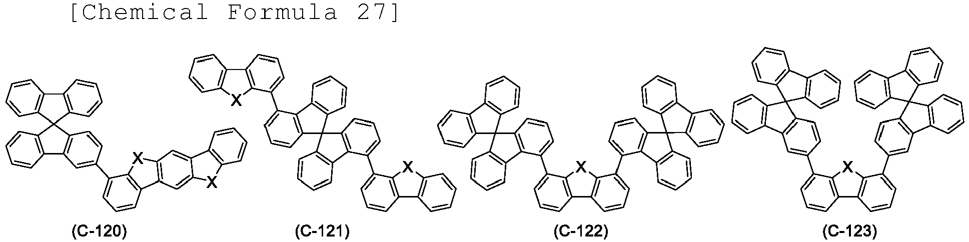 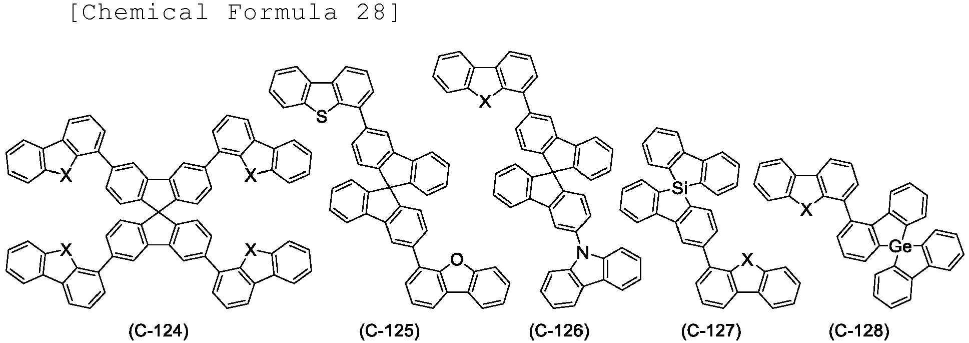 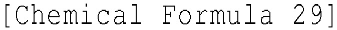 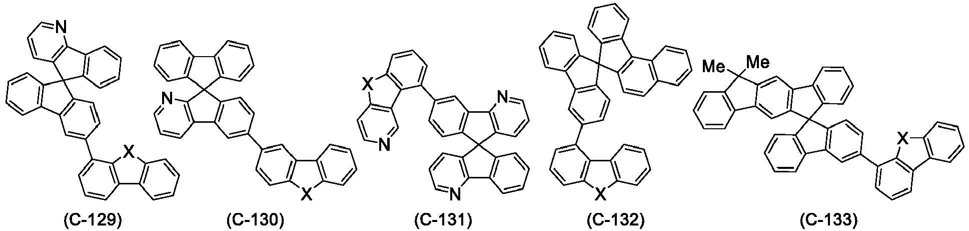 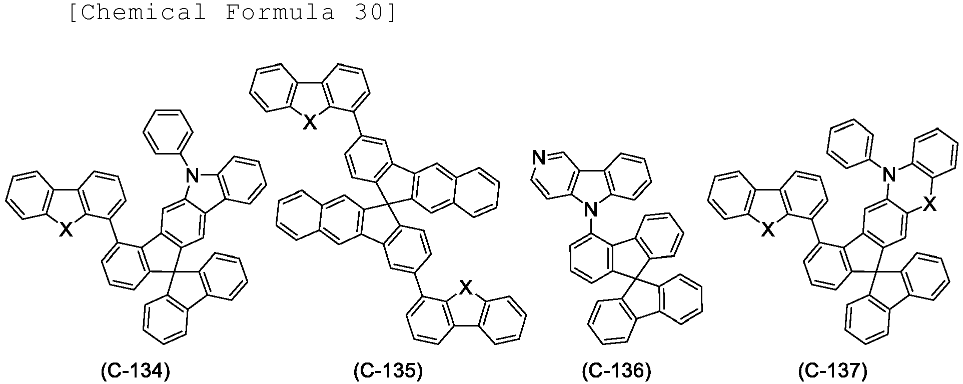 [wherein, X represents an oxygen atom or a sulfur atom. When a plurality of X are present, they may be the same or different.]

X is preferably a sulfur atom.

The compound represented by the formula (C-1) is available, for example, from Aldrich and Luminescence Technology Corp. The compound represented by the formula (C-1) can be synthesized according to methods described, for example, in International Publication WO 2014/023388, International Publication WO 2013/045408, International Publication WO 2013/045410, International Publication WO 2013/045411, International Publication WO 2012/048820, International Publication WO 2012/048819, International Publication WO 2011/006574 and "Organic Electronics vol. 14, pp. 902-908 (2013)", as other means.

### Others

The first layer is preferably a layer containing a compound represented by the formula (C-1) and a phosphorescent compound, since the light emitting device of the present invention is more excellent in luminance life.

In the first layer, the compounds represented by the formula (C-1) may be contained singly or in combination of two or more. Further, when the first layer is a layer containing a compound represented by the formula (C-1) and a phosphorescent compound, the phosphorescent compounds may be contained singly or in combination of two or more.

When the first layer is a layer containing a compound represented by the formula (C-1) and a phosphorescent compound, the content of the phosphorescent compound is usually 0.01 to 95 parts by mass, preferably 0.1 to 70 parts by mass, more preferably 1 to 50 parts by mass, further preferably 10 to 40 parts by mass, when the sum of the phosphorescent compound and the compound represented by the formula (C-1) is taken as 100 parts by mass.

When the first layer is a layer containing a compound represented by the formula (C-1) and a phosphorescent compound, the compound represented by the formula (C-1) is preferably a host material having at least one function selected from hole injectability, hole transportability, electron injectability and electron transportability, since the light emitting device of the present invention is more excellent in luminance life.

When the first layer is a layer containing a compound represented by the formula (C-1) and a phosphorescent compound, the lowest excited triplet state (T₁) of the compound represented by the formula (C-1) is preferably at energy level corresponding to T₁ of the phosphorescent compound contained in the first layer or higher energy level than it, more preferably at higher energy level than it, since the light emitting device of the present invention is more excellent in luminance life.

The phosphorescent compound is preferably one that shows solubility in a solvent which is capable of dissolving a compound represented by the formula (C-1) contained in the first layer, since the light emitting device of the present invention can be fabricated by a solution application process.

"The phosphorescent compound" means usually a compound showing phosphorescence at room temperature (25°C), preferably a metal complex showing light emission from triplet excited state at room temperature. This metal complex showing light emission from triplet excited state has a central metal atom and a ligand.

The central metal atom includes, for example, metal atoms having an atomic number of 40 or more, having the spin-orbital interaction to the complex and capable of causing intersystem crossing between singlet state and triplet state. The metal atom includes, for example, a ruthenium atom, a rhodium atom, a palladium atom, an iridium atom and a platinum atom, and is preferably an iridium atom or a platinum atom, since the light emitting device of the present invention is more excellent in luminance life.

The ligand includes, for example, neutral or anionic monodentate ligands or neutral or anionic polydentate ligands forming at least one bond selected from the group consisting of a coordination bond and a covalent bond with a central metal atom. The bond between a central metal atom and a ligand includes, for example, a metal-nitrogen bond, a metal-carbon bond, a metal-oxygen bond, a metal-phosphorus bond, a metal-sulfur bond and a metal-halogen bond. The polydentate ligand means usually a bidentate or more and hexadentate or less ligand.

### Phosphorescent compound represented by the formula (1)

The phosphorescent compound is preferably a phosphorescent compound represented by the above-described formula (1).

M is preferably an iridium atom or a platinum atom, more preferably an iridium atom, since the light emitting device of the present invention is more excellent in luminance life.

When M is a ruthenium atom, a rhodium atom or an iridium atom, n¹ is preferably 2 or 3, more preferably 3.

When M is a palladium atom or a platinum atom, n¹ is preferably 2.

E¹ and E² are each preferably a carbon atom.

Ring L¹ is preferably a 5-membered aromatic hetero ring or a 6-membered aromatic hetero ring, more preferably a 5-membered aromatic hetero ring having two or more and four or less nitrogen atoms as a constituent atom or a 6-membered aromatic hetero ring having one or more and four or less nitrogen atoms as a constituent atom, further preferably a 5-membered aromatic hetero ring having two or more and three or less nitrogen atoms as a constituent atom or a 6-membered aromatic hetero ring having one or more and two or less nitrogen atoms as a constituent atom, and the foregoing rings optionally have a substituent. When Ring L¹ is a 6-membered aromatic hetero ring, E¹ is preferably a carbon atom.

Ring L¹ includes, for example, a diazole ring, a triazole ring, a pyridine ring, a diazabenzene ring, a triazine ring, an azanaphthalene ring and a diazanaphthalene ring, and is preferably a diazole ring, a triazole ring, a pyridine ring, a diazabenzene ring, a quinoline ring or an isoquinoline ring, more preferably a diazole ring, a triazole ring, a pyridine ring, a quinoline ring or an isoquinoline ring, further preferably a diazole ring, a triazole ring or a pyridine ring, particularly preferably a diazole ring or a pyridine ring, and the foregoing rings optionally have a substituent.

Ring L² is preferably a 5-membered or 6-membered aromatic hydrocarbon ring or a 5-membered or 6-membered aromatic hetero ring, more preferably a 6-membered aromatic hydrocarbon ring or a 6-membered aromatic hetero ring, further preferably a 6-membered aromatic hydrocarbon ring, and the foregoing rings optionally have a substituent. When Ring R² is a 6-membered aromatic hetero ring, E² is preferably a carbon atom.

Ring L² includes, for example, a benzene ring, a naphthalene ring, a fluorene ring, a phenanthrene ring, an indene ring, a pyridine ring, a diazabenzene ring and a triazine ring, and is preferably a benzene ring, a naphthalene ring, a fluorene ring, a pyridine ring or a diazabenzene ring, more preferably a benzene ring, a pyridine ring or a diazabenzene ring, further preferably a benzene ring, and the foregoing rings optionally have a substituent.

The substituent which Ring L¹ and Ring L² optionally have is preferably an alkyl group, a cycloalkyl group, an alkoxy group, a cycloalkoxy group, an aryl group, an aryloxy group, a monovalent heterocyclic group, a substituted amino group or a halogen atom, more preferably an alkyl group, a cycloalkyl group, an alkoxy group, a cycloalkoxy group, an aryl group, a monovalent heterocyclic group, a substituted amino group or a fluorine atom, further preferably an alkyl group, a cycloalkyl group, an aryl group, a monovalent heterocyclic group or a substituted amino group, particularly preferably an aryl group or a monovalent heterocyclic group, especially preferably an aryl group, and the foregoing groups optionally further have a substituent.

The aryl group as the substituent which Ring L¹ and Ring L² optionally have is preferably a phenyl group, a naphthyl group, an anthracenyl group, a phenanthrenyl group, a dihydrophenanthrenyl group, a fluorenyl group or a pyrenyl group, more preferably a phenyl group, a naphthyl group or a fluorenyl group, further preferably a phenyl group, and the foregoing groups optionally further have a substituent.

The monovalent heterocyclic group as the substituent which Ring L¹ and Ring L² optionally have is preferably a pyridyl group, a pyrimidinyl group, a triazinyl group, a quinolinyl group, an isoquinolinyl group, a dibenzofuranyl group, a dibenzothienyl group, a carbazolyl group, an azacarbazolyl group, a diazacarbazolyl group, a phenoxazinyl group or a phenothiazinyl group, more preferably a pyridyl group, a pyrimidinyl group, a triazinyl group, a carbazolyl group, an azacarbazolyl group or a diazacarbazolyl group, further preferably a pyridyl group, a pyrimidinyl group or a triazinyl group, particularly preferably a triazinyl group, and the foregoing groups optionally further have a substituent.

In the substituted amino group as the substituent which Ring L¹ and Ring L² optionally have, the substituent which the amino group has is preferably an aryl group or a monovalent heterocyclic group, more preferably an aryl group, and the foregoing groups optionally further have a substituent. The examples and preferable range of the aryl group as the substituent which the amino group has are the same as the examples and preferable range of the aryl group as the substituent which Ring L¹ and Ring L² optionally have. The examples and preferable range of the monovalent heterocyclic group as the substituent which the amino group has are the same as the examples and preferable range of the monovalent heterocyclic group as the substituent which Ring L¹ and Ring L² optionally have.

The substituent which the substituent which Ring L¹ and Ring L² optionally have optionally further has is preferably an alkyl group, a cycloalkyl group, an aryl group, a monovalent heterocyclic group, an alkoxy group, a cycloalkoxy group or a substituted amino group, more preferably an alkyl group, a cycloalkyl group, an aryl group, a monovalent heterocyclic group or a substituted amino group, further preferably an alkyl group, a cycloalkyl group or an aryl group, particularly preferably an alkyl group or a cycloalkyl group, and the foregoing groups optionally further have a substituent, but it is preferable that these groups do not further have a substituent.

The examples and preferable range of the aryl group, the monovalent heterocyclic group and the substituted amino group as the substituent which the substituent which Ring L¹ and Ring L² optionally have optionally further has are the same as the examples and preferable range of the aryl group, the monovalent heterocyclic group and the substituted amino group as the substituent which Ring L¹ and Ring L² optionally have, respectively.

The aryl group, the monovalent heterocyclic group or the substituted amino group as the substituent which Ring L¹ and Ring L² optionally have is preferably a group represented by the formula (D-A), the formula (D-B) or the formula (D-C), more preferably a group represented by the formula (D-A) or the formula (D-C), since the light emitting device of the present invention is more excellent in luminance life. 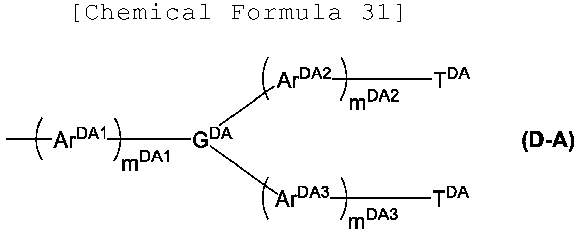 [wherein,
m^{DA1}, m^{DA2} and m^{DA3} each independently represent an integer of 0 or more.
G^{DA} represents a nitrogen atom, an aromatic hydrocarbon group or a heterocyclic group, and the foregoing groups optionally have a substituent.
Ar^{DA1}, Ar^{DA2} and Ar^{DA3} each independently represent an arylene group or a divalent heterocyclic group, and the foregoing groups optionally have a substituent. When a plurality of Ar^{DA1}, Ar^{DA2} and Ar^{DA3} are present, they may be the same or different at each occurrence.
T^{DA} represents an aryl group or a monovalent heterocyclic group, and the foregoing groups optionally have a substituent. A plurality of T^{DA} may be the same or different.] 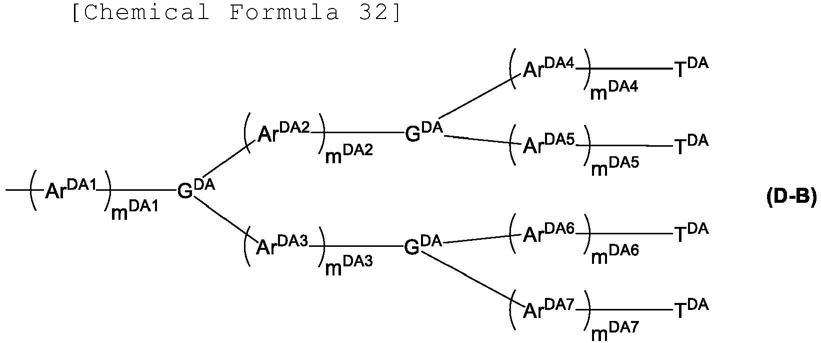
[wherein,
m^{DA1}, m^{DA2}, m^{DA3}, m^{DA4}, m^{DA5}, m^{DA6} and m^{DA7} each independently represent an integer of 0 or more.
G^{DA} represents a nitrogen atom, an aromatic hydrocarbon group or a heterocyclic group, and the foregoing groups optionally have a substituent. A plurality of G^{DA} may be the same or different.
Ar^{DA1}, Ar^{DA2}, Ar^{DA3}, Ar^{DA4}, Ar^{DA5}, Ar^{DA6} and Ar^{DA7} each independently represent an arylene group or a divalent heterocyclic group, and the foregoing groups optionally have a substituent. When a plurality of Ar^{DA1}, Ar^{DA2}, Ar^{DA3}, Ar^{DA4}, Ar^{DA5}, Ar^{DA6} and Ar^{DA7} are present, they may be the same or different at each occurrence.
T^{DA} represents an aryl group or a monovalent heterocyclic group, and the foregoing groups optionally have a substituent. A plurality of T^{DA} may be the same or different.] 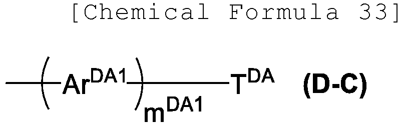
[wherein,
m^{DA1} represents an integer of 0 or more.
Ar^{DA1} represents an arylene group or a divalent heterocyclic group, and the foregoing groups optionally have a substituent. When a plurality of Ar^{DA1} are present, they may be the same or different.
T^{DA} represents an aryl group or a monovalent heterocyclic group, and the foregoing groups optionally have a substituent.]

m^{DA1}, m^{DA2}, m^{DA3}, m^{DA4}, m^{DA5}, m^{DA6} and m^{DA7} are each usually an integer of 10 or less, preferably an integer of 5 or less, more preferably an integer of 2 or less, further preferably 0 or 1. It is preferable that m^{DA2}, m^{DA3}, m^{DA4}, m^{DA5}, m^{DA6} and m^{DA7} are the same integer, it is more preferable that m^{DA1}, m^{DA2}, m^{DA3}, m^{DA4}, m^{DA5}, m^{DA6} and m^{DA7} are the same integer.

G^{DA} is preferably an aromatic hydrocarbon group or a heterocyclic group, more preferably a group obtained by removing from a benzene ring, a pyridine ring, a pyrimidine ring, a triazine ring or a carbazole ring three hydrogen atoms directly bonding to carbon atoms or nitrogen atoms constituting the ring, and the foregoing groups optionally have a substituent.

The substituent which G^{DA} optionally has is preferably an alkyl group, a cycloalkyl group, an alkoxy group, a cycloalkoxy group, an aryl group or a monovalent heterocyclic group, more preferably an alkyl group, a cycloalkyl group, an alkoxy group or a cycloalkoxy group, further preferably an alkyl group or a cycloalkyl group, and the foregoing groups optionally further have a substituent, but it is preferable that these groups do not further have a substituent.

G^{DA} is preferably a group represented by the formula (GDA-11) to the formula (GDA-15), more preferably a group represented by the formula (GDA-11) to the formula (GDA-14), further preferably a group represented by the formula (GDA-11) or the formula (GDA-14), particularly preferably a group represented by the formula (GDA-11). 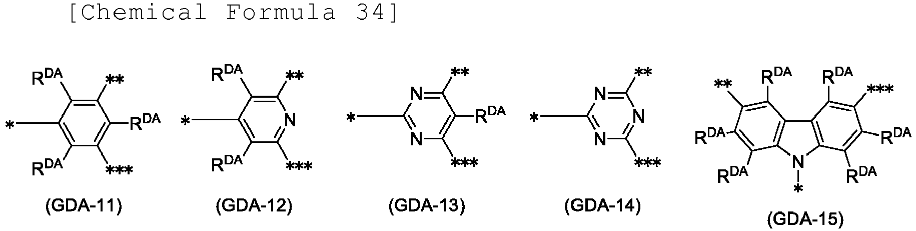 [wherein,
* represents a bond to Ar^{DA1} in the formula (D-A), to Ar^{DA1} in the formula (D-B), to Ar^{DA2} in the formula (D-B) or to Ar^{DA3} in the formula (D-B).
** represents a bond to Ar^{DA2} in the formula (D-A), to Ar^{DA2} in the formula (D-B), to Ar^{DA4} in the formula (D-B) or to Ar^{DA6} in the formula (D-B).
*** represents a bond to Ar^{DA3} in the formula (D-A), to Ar^{DA3} in the formula (D-B), to Ar^{DA5} in the formula (D-B) or to Ar^{DA7} in the formula (D-B).

R^{DA} represents a hydrogen atom, an alkyl group, a cycloalkyl group, an alkoxy group, a cycloalkoxy group, an aryl group or a monovalent heterocyclic group, and the foregoing groups optionally further have a substituent. When a plurality of R^{DA} are present, they may be the same or different.]

R^{DA} is preferably a hydrogen atom, an alkyl group, a cycloalkyl group, an alkoxy group or a cycloalkoxy group, more preferably a hydrogen atom, an alkyl group or a cycloalkyl group, and the foregoing groups optionally have a substituent.

Ar^{DA1}, Ar^{DA2}, Ar^{DA3}, Ar^{DA4}, Ar^{DA5}, Ar^{DA6} and Ar^{DA7} are each preferably a phenylene group, a fluorenediyl group or a carbazolediyl group, more preferably a group represented by the formula (ArDA-1) to the formula (ArDA-5), further preferably a group represented by the formula (ArDA-1) to the formula (ArDA-3), particularly preferably a group represented by the formula (ArDA-1) or the formula (ArDA-2), especially preferably a group represented by the formula (ArDA-1), and the foregoing groups optionally have a substituent. 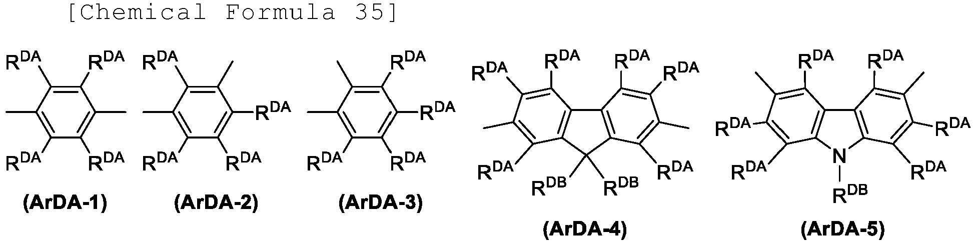 [wherein,
R^{DA} represents the same meaning as described above.
R^{DB} represents a hydrogen atom, an alkyl group, a cycloalkyl group, an aryl group or a monovalent heterocyclic group, and the foregoing groups optionally have a substituent. When a plurality of R^{DB} are present, they may be the same or different.]

R^{DB} is preferably an alkyl group, a cycloalkyl group, an aryl group or a monovalent heterocyclic group, more preferably an aryl group or a monovalent heterocyclic group, further preferably an aryl group, and the foregoing groups optionally have a substituent.

The examples and preferable range of the substituent which Ar^{DA1}, Ar^{DA2}, Ar^{DA3}, Ar^{DA4}, Ar^{DA5}, Ar^{DA6}, Ar^{DA7} and R^{DB} optionally have are the same as the examples and preferable range of the substituent which G^{DA} optionally has.

T^{DA} is preferably a group represented by the formula (TDA-1) to the formula (TDA-3), more preferably a group represented by the formula (TDA-1). 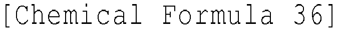 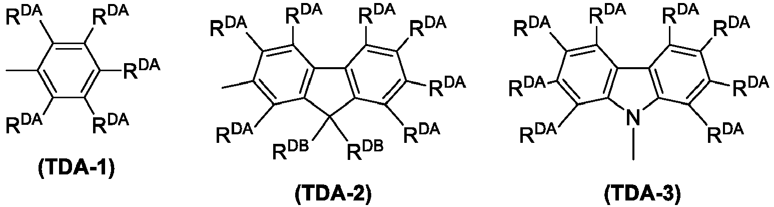 [wherein, R^{DA} and R^{DB} represent the same meaning as described above.]

The group represented by the formula (D-A) is preferably a group represented by the formula (D-A1) to the formula (D-A5), more preferably a group represented by the formula (D-A1) or the formula (D-A3) to the formula (D-A5), further preferably a group represented by the formula (D-A1) or the formula (D-A3), particularly preferably a group represented by the formula (D-A1). 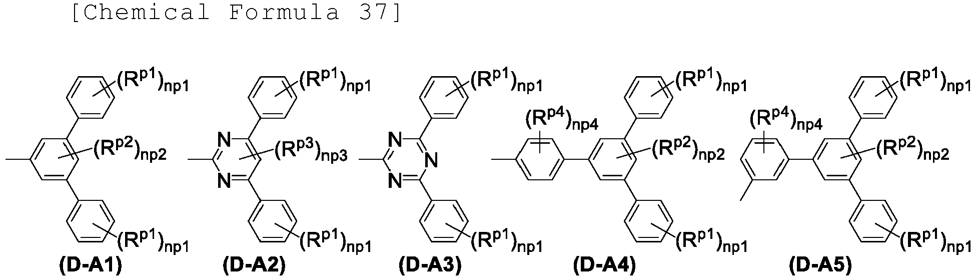 [wherein,
R^{p1}, R^{p2}, R^{p3} and R^{p4} each independently represent an alkyl group, a cycloalkyl group, an alkoxy group, a cycloalkoxy group or a halogen atom. When a plurality of R^{p1}, R^{p2} and R^{p4} are present, they may be the same or different at each occurrence.
np1 represents an integer of 0 to 5, np2 represents an integer of 0 to 3, np3 represents 0 or 1, and np4 represents an integer of 0 to 4. A plurality of np1 may be the same or different.]

The group represented by the formula (D-B) is preferably a group represented by the formula (D-B1) to the formula (D-B6), more preferably a group represented by the formula (D-B1) to the formula (D-B3) or the formula (D-B5), further preferably a group represented by the formula (D-B1). 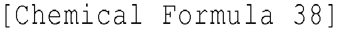 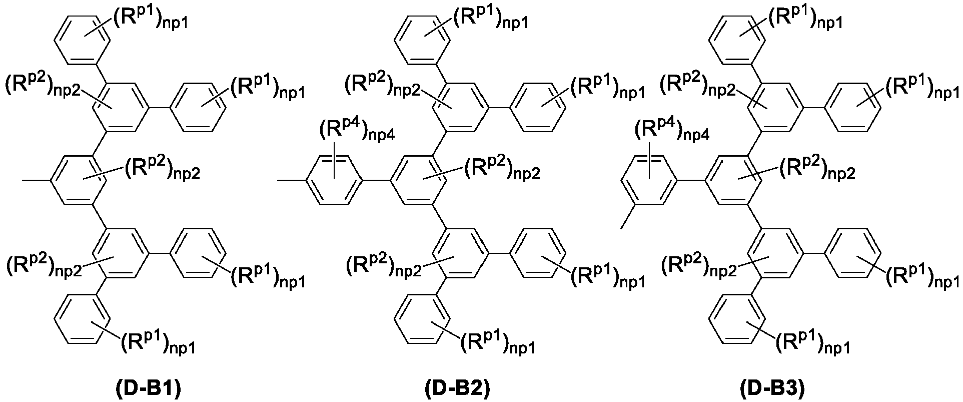 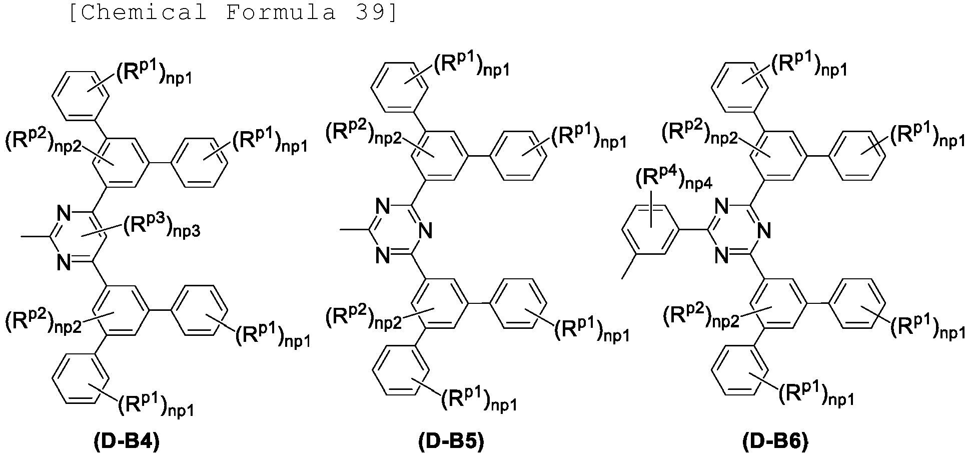 [wherein,
R^{p1}, R^{p2}, R^{p3} and R^{p4} each independently represent an alkyl group, a cycloalkyl group, an alkoxy group, a cycloalkoxy group or a halogen atom. When a plurality of R^{p1}, R^{p2} and R^{p4} are present, they may be the same or different at each occurrence.
np1 represents an integer of 0 to 5, np2 represents an integer of 0 to 3, np3 represents 0 or 1, np4 represents an integer of 0 to 4. A plurality of np1 may be the same or different. A plurality of np2 may be the same or different.]

The group represented by the formula (D-C) is preferably a group represented by the formula (D-C1) to the formula (D-C4), more preferably a group represented by the formula (D-C1) or the formula (D-C2), further preferably a group represented by the formula (D-C2). 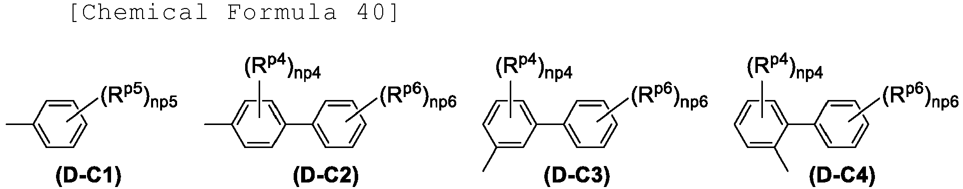 [wherein,
R^{p4}, R^{p5} and R^{p6} each independently represent an alkyl group, a cycloalkyl group, an alkoxy group, a cycloalkoxy group or a halogen atom. When a plurality of R^{p4}, R^{p5} and R^{p6} are present, they may be the same or different at each occurrence.
np4 represents an integer of 0 to 4, np5 represents an integer of 0 to 5, np6 represents an integer of 0 to 5.]

np1 is preferably an integer of 0 to 2, more preferably 0 or 1. np2 is preferably 0 or 1, more preferably 0. np3 is preferably 0. np4 is preferably an integer of 0 to 2, more preferably 0. np5 is preferably an integer of 0 to 3, more preferably 0 or 1. np6 is preferably an integer of 0 to 2, more preferably 0 or 1.

The alkyl group or the cycloalkyl group represented by R^{p1}, R^{p2}, R^{p3}, R^{p4}, R^{p5} and R^{p6} is preferably a methyl group, an ethyl group, an isopropyl group, a tert-butyl group, a hexyl group, a 2-ethylhexyl group, a cyclohexyl group or a tert-octyl group.

The alkoxy group or the cycloalkoxy group represented by R^{p1}, R^{p2}, R^{p3}, R^{p4}, R^{p5} and R^{p6} is preferably a methoxy group, a 2-ethylhexyloxy group or a cyclolhexyloxy group.

R^{p1}, R^{p2}, R^{p3}, R^{p4}, R^{p5} and R^{p6} are each preferably an alkyl group optionally having a substituent or a cycloalkyl group optionally having a substituent, more preferably an alkyl group optionally having a substituent, further preferably a methyl group, an ethyl group, an isopropyl group, a tert-butyl group, a hexyl group, a 2-ethylhexyl group or a tert-octyl group.

When a plurality of the substituents which Ring L¹ optionally has are present, it is preferable that they are not combined together to form a ring together with the atoms to which they are attached.

When a plurality of the substituents which Ring L² optionally has are present, it is preferable that they are not combined together to form a ring together with the atoms to which they are attached.

It is preferable that the substituent which Ring L¹ optionally has and the substituent which Ring L² optionally has are not combined together to form a ring together with the atoms to which they are attached.

### [Anionic bidentate ligand]

The anionic bidentate ligand represented by A¹-G¹-A² includes, for example, ligands represented by the following formulae. However, the anionic bidentate ligand represented by A¹-G¹-A² is different from a ligand of which number is defined by subscript n¹. 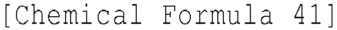 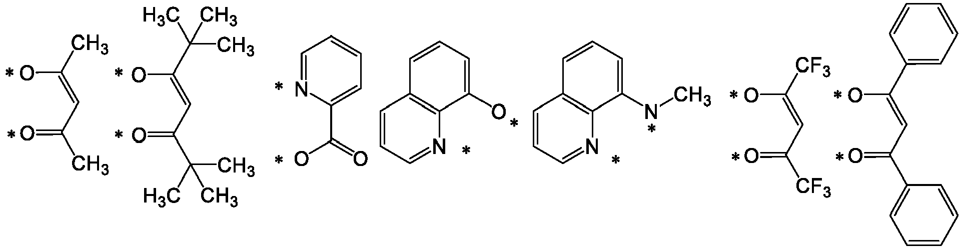 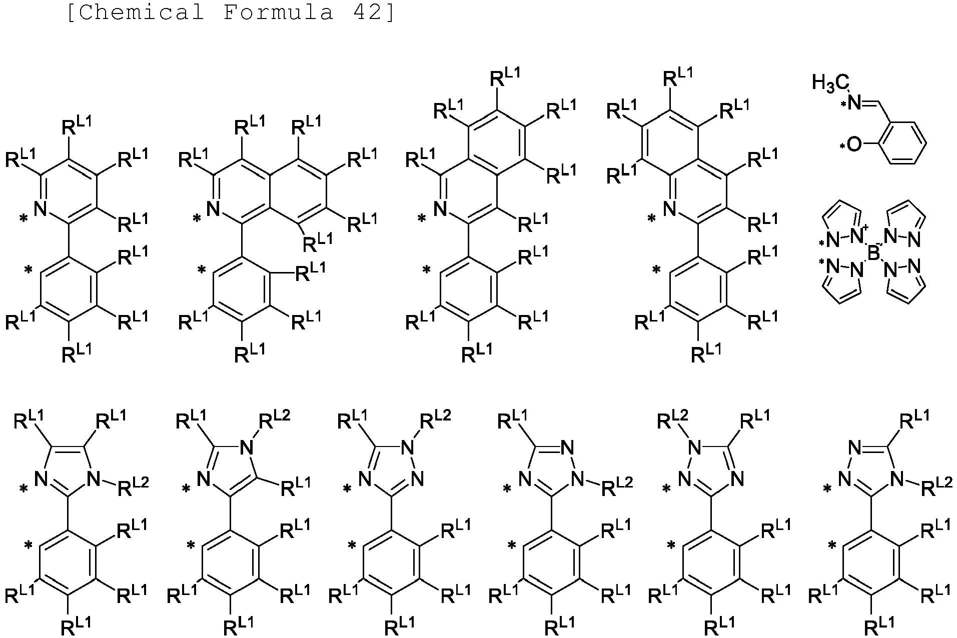 [wherein,
* represents a site binding to M.
R^{L1} represents a hydrogen atom, an alkyl group, a cycloalkyl group, an aryl group, a monovalent heterocyclic group or a halogen atom, and the foregoing groups optionally have a substituent. A plurality of R^{L1} may be the same or different.
R^{L2} represents an alkyl group, a cycloalkyl group, an aryl group, a monovalent heterocyclic group or a halogen atom, and the foregoing groups optionally have a substituent.]

R^{L1} is preferably a hydrogen atom, an alkyl group, a cycloalkyl group, an aryl group or a fluorine atom, more preferably a hydrogen atom or an alkyl group, and the foregoing groups optionally have a substituent.

R^{L2} is preferably an alkyl group or an aryl group, and the foregoing groups optionally have a substituent.

The phosphorescent compound represented by the formula (1) is preferably a phosphorescent compound represented by the formula (1-A) or a phosphorescent compound represented by the formula (1-B), since the light emitting device of the present invention is more excellent in luminance life.

### [Phosphorescent compound represented by the formula (1-A)]

When Ring L^{1A} is a diazole ring, an imidazole ring in which E^{11A} is a nitrogen atom or an imidazole ring in which E^{12A} is a nitrogen atom is preferable, an imidazole ring in which E^{11A} is a nitrogen atom is more preferable.

When Ring L^{1A} is a triazole ring, a triazole ring in which E^{11A} and E^{12A} are each a nitrogen atom or a triazole ring in which E^{11A} and E^{13A} are each a nitrogen atom is preferable, a triazole ring in which E^{11A} and E^{13A} are each a nitrogen atom is more preferable.

Ring L^{1A} is preferably a diazole ring.

The examples and preferable range of the aryl group, the monovalent heterocyclic group and the substituted amino group represented by R^{11A}, R^{12A}, R^{13A}, R^{21A}, R^{22A}, R^{23A} and R^{24A} are the same as the examples and preferable range of the aryl group, the monovalent heterocyclic group and the substituted amino group as the substituent which Ring L¹ and Ring L² optionally have, respectively.

The examples and preferable range of the substituent which R^{11A}, R^{12A}, R^{13A}, R^{21A}, R^{22A}, R^{23A} and R^{24A} optionally have are the same as the examples and preferable range of the substituent which the substituent which Ring L¹ and Ring L² optionally have optionally further has.

When E^{11A} is a nitrogen atom and R^{11A} is present, R^{11A} is preferably an alkyl group, a cycloalkyl group, an aryl group or a monovalent heterocyclic group, more preferably an aryl group or a monovalent heterocyclic group, further preferably an aryl group, and the foregoing groups optionally have a substituent.

When E^{11A} is a carbon atom, R^{11A} is preferably a hydrogen atom, an alkyl group, a cycloalkyl group, an aryl group, a monovalent heterocyclic group or a substituted amino group, more preferably a hydrogen atom, an alkyl group, a cycloalkyl group or an aryl group, further preferably a hydrogen atom, an alkyl group or a cycloalkyl group, particularly preferably a hydrogen atom, and the foregoing groups optionally have a substituent.

When E^{12A} is a nitrogen atom and R^{12A} is present, R^{12A} is preferably an alkyl group, a cycloalkyl group, an aryl group or a monovalent heterocyclic group, more preferably an aryl group or a monovalent heterocyclic group, further preferably an aryl group, and the foregoing groups optionally have a substituent.

When E^{12A} is a carbon atom, R^{12A} is preferably a hydrogen atom, an alkyl group, a cycloalkyl group, an aryl group, a monovalent heterocyclic group or a substituted amino group, more preferably a hydrogen atom, an alkyl group, a cycloalkyl group or an aryl group, further preferably a hydrogen atom, an alkyl group or a cycloalkyl group, particularly preferably a hydrogen atom, and the foregoing groups optionally have a substituent.

When E^{13A} is a nitrogen atom and R^{13A} is present, R^{13A} is preferably an alkyl group, a cycloalkyl group, an aryl group or a monovalent heterocyclic group, more preferably an aryl group or a monovalent heterocyclic group, further preferably an aryl group, and the foregoing groups optionally have a substituent.

When E^{13A} is a carbon atom, R^{13A} is preferably a hydrogen atom, an alkyl group, a cycloalkyl group, an aryl group, a monovalent heterocyclic group or a substituted amino group, more preferably a hydrogen atom, an alkyl group, a cycloalkyl group or an aryl group, further preferably a hydrogen atom, an alkyl group or a cycloalkyl group, particularly preferably a hydrogen atom, and the foregoing groups optionally have a substituent.

When Ring L^{1A} is a diazole ring, Ring L^{1A} is preferably an imidazole ring in which E^{11A} is a nitrogen atom and R^{11A} is present or an imidazole ring in which E^{12A} is a nitrogen atom and R^{12A} is present, more preferably an imidazole ring in which E^{11A} is a nitrogen atom and R^{11A} is present.

When Ring L^{1A} is a triazole ring, Ring L^{1A} is preferably a triazole ring in which E^{11A} and E^{12A} are each a nitrogen atom and R^{11A} is present and R^{12A} is absent or a triazole ring in which E^{11A} and E^{13A} are each a nitrogen atom and R^{11A} is present and R^{13A} is absent, more preferably a triazole ring in which E^{11A} and E^{13A} are each a nitrogen atom and R^{11A} is present and R^{13A} is absent.

When Ring L^{1A} is a triazole ring, two of R^{11A}, R^{12A} and R^{13A} are each preferably an alkyl group, a cycloalkyl group, an aryl group or a monovalent heterocyclic group, more preferably an alkyl group or an aryl group, and the foregoing groups optionally have a substituent.

When Ring L^{2A} is a pyridine ring, Ring L^{2A} is preferably a pyridine ring in which E^{21A} is a nitrogen atom, a pyridine ring in which E^{22A} is a nitrogen atom or a pyridine ring in which E^{23A} is a nitrogen atom, more preferably a pyridine ring in which E^{22A} is a nitrogen atom.

When Ring L^{2A} is a diazabenzene ring, Ring L^{2A} is preferably a pyrimidine ring in which E^{22A} and E^{24A} are each a nitrogen atom or a pyrimidine ring in which E^{22A} and E^{24A} are each a nitrogen atom, more preferably a pyrimidine ring in which E^{22A} and E^{24A} are each a nitrogen atom.

Ring L^{2A} is preferably a benzene ring.

R^{21A}, R^{22A}, R^{23A} and R^{24A} are each preferably a hydrogen atom, an alkyl group, a cycloalkyl group, an alkoxy group, a cycloalkoxy group, an aryl group, a monovalent heterocyclic group, a substituted amino group or a fluorine atom, more preferably a hydrogen atom, an alkyl group, a cycloalkyl group, an aryl group, a monovalent heterocyclic group or a substituted amino group, further preferably a hydrogen atom, an alkyl group or a group represented by the formula (D-A), the formula (D-B) or the formula (D-C), particularly preferably a hydrogen atom or a group represented by the formula (D-A), especially preferably a hydrogen atom, and the foregoing groups optionally have a substituent.

When Ring L^{2A} has an aryl group, a monovalent heterocyclic group or a substituted amino group, it is preferable that R^{22A} or R^{23A} is an aryl group, a monovalent heterocyclic group or a substituted amino group, it is more preferable that R^{22A} is an aryl group, a monovalent heterocyclic group or a substituted amino group.

It is preferable that R^{11A} and R^{12A}, R^{12A} and R^{13A}, R^{11A} and R^{21A}, R^{21A} and R^{22A}, R^{22A} and R^{23A}, and R^{23A} and R^{24A} are not each combined together to form a ring together with the atoms to which they are attached.

The phosphorescent compound represented by the formula (1-A) is preferably a phosphorescent compound represented by the formula (1-A1) to the formula (1-A5), more preferably a phosphorescent compound represented by the formula (1-A1), the formula (1-A3) or the formula (1-A4), further preferably a phosphorescent compound represented by the formula (1-A3) or the formula (1-A4), particularly preferably a phosphorescent compound represented by the formula (1-A4), since the light emitting device of the present invention is further excellent in luminance life. 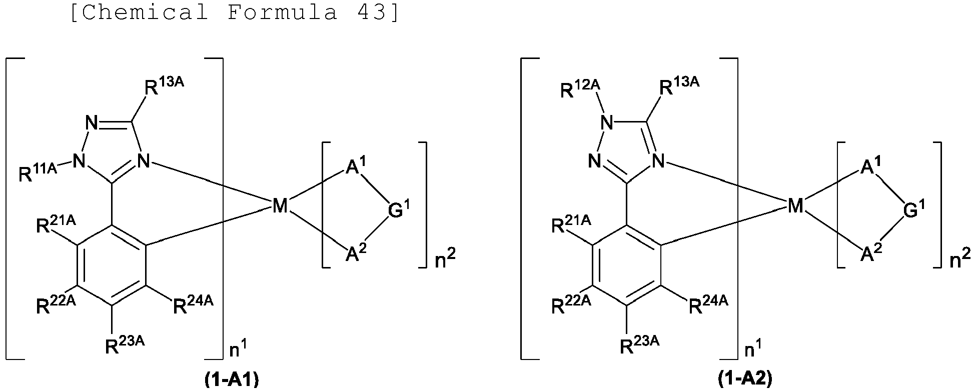 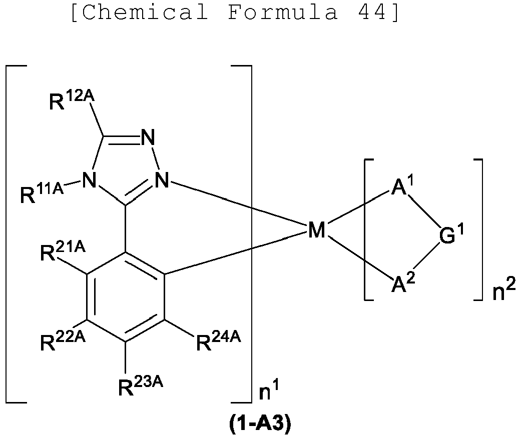 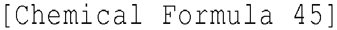 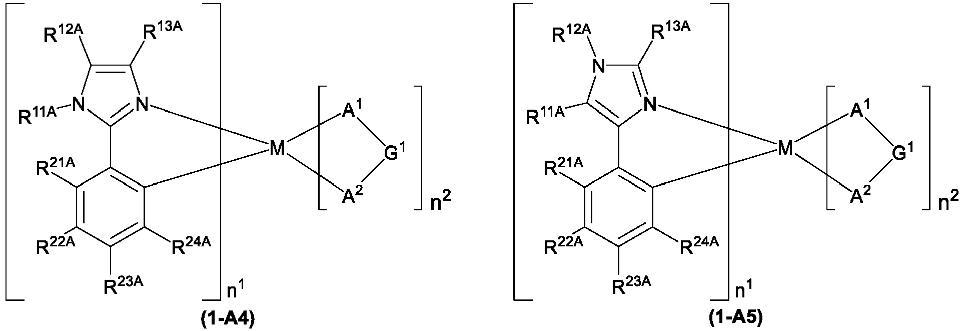 [wherein, M, n¹, n², R^{11A}, R^{12A}, R^{13A}, R^{21A}, R^{22A}, R^{23A}, R^{24A} and A¹-G¹-A² represent the same meaning as described above.]

### [Phosphorescent compound represented by the formula (1-B)]

When Ring L^{1B} is a diazabenzene ring, Ring L^{1B} is preferably a pyrimidine ring in which E^{11B} is a nitrogen atom or a pyrimidine ring in which E^{13B} is a nitrogen atom, more preferably a pyrimidine ring in which E^{11B} is a nitrogen atom.

Ring L^{1B} is preferably a pyridine ring.

When Ring L^{2B} is a pyridine ring, Ring L^{2B} is preferably a pyridine ring in which E^{21B} is a nitrogen atom, a pyridine ring in which E^{22B} is a nitrogen atom or a pyridine ring in which E^{23B} is a nitrogen atom, more preferably a pyridine ring in which E^{22B} is a nitrogen atom.

When Ring L^{2B} is a diazabenzene ring, Ring L^{2B} is preferably a pyrimidine ring in which E^{22B} and E^{24B} are each a nitrogen atom or a pyrimidine ring in which E^{21B} and E^{23B} are each a nitrogen atom, more preferably a pyrimidine ring in which E^{22B} and E^{24B} are each a nitrogen atom.

Ring L^{2B} is preferably a benzene ring.

The examples and preferable range of the aryl group, the monovalent heterocyclic group and the substituted amino group represented by R^{11B}, R^{12B}, R^{13B}, R^{14B}, R^{21B}, R^{22B}, R^{23B} and R^{24B} are the same as the examples and preferable range of the aryl group, the monovalent heterocyclic group and the substituted amino group as the substituent which Ring L¹ and Ring L² optionally have, respectively.

The examples and preferable range of the substituent which R^{11B}, R^{12B}, R^{13B}, R^{14B}, R^{21B}, R^{22B}, R^{23B} and R^{24B} optionally have are the same as the examples and preferable range of the substituent which the substituent which Ring L¹ and Ring L² optionally have optionally further has.

R^{11B}, R^{12B}, R^{13B}, R^{14B}, R^{21B}, R^{22B}, R^{23B} and R^{24B} are each preferably a hydrogen atom, an alkyl group, a cycloalkyl group, an alkoxy group, a cycloalkoxy group, an aryl group, an alkenyl group, a monovalent heterocyclic group, a substituted amino group or a fluorine atom, more preferably a hydrogen atom, an alkyl group, a cycloalkyl group, an aryl group, an alkenyl group, a monovalent heterocyclic group or a substituted amino group, further preferably a hydrogen atom, an alkyl group or a group represented by the formula (D-A), the formula (D-B) or the formula (D-C), particularly preferably a hydrogen atom or a group represented by the formula (D-A), and the foregoing groups optionally have a substituent.

When Ring L^{1B} has an aryl group, a monovalent heterocyclic group or a substituted amino group, it is preferable that R^{11B}, R^{12B} or R^{13B} is an aryl group, a monovalent heterocyclic group or a substituted amino group, it is more preferable that R^{12B} or R^{13B} is an aryl group, a monovalent heterocyclic group or a substituted amino group, it is further preferable that R^{13B} is an aryl group, a monovalent heterocyclic group or a substituted amino group.

When Ring L^{2B} has an aryl group, a monovalent heterocyclic group or a substituted amino group, it is preferable that R^{22B} or R^{23B} is an aryl group, a monovalent heterocyclic group or a substituted amino group, it is more preferable that R^{22B} is an aryl group, a monovalent heterocyclic group or a substituted amino group.

The phosphorescent compound represented by the formula (1-B) is preferably a phosphorescent compound represented by the formula (1-B1) to the formula (1-B5), more preferably a phosphorescent compound represented by the formula (1-B1) to the formula (1-B3), further preferably a phosphorescent compound represented by the formula (1-B1) or a phosphorescent compound represented by the formula (1-B2), particularly preferably a phosphorescent compound represented by the formula (1-B1), since the light emitting device of the present invention is further excellent in luminance life. 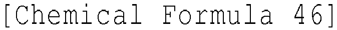 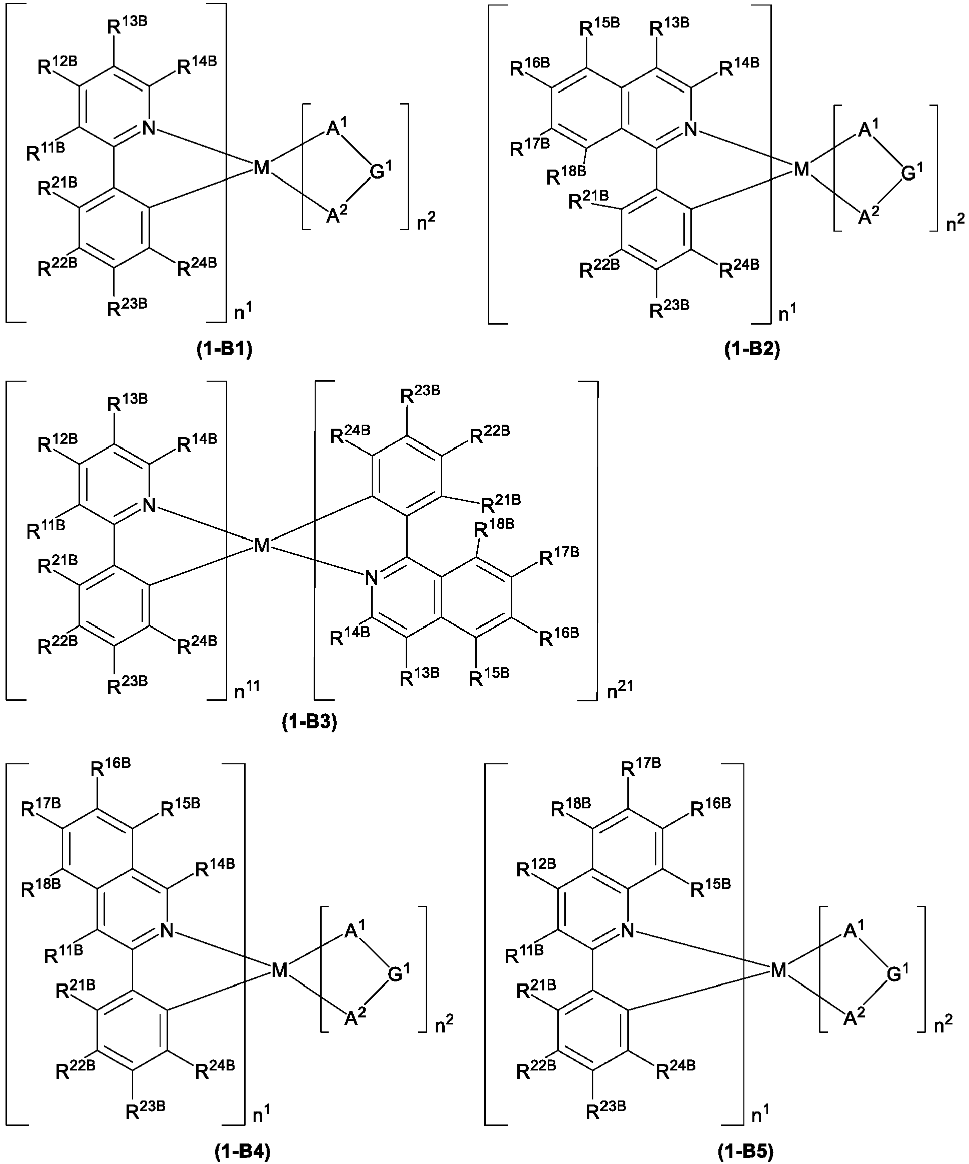 [wherein,
M, n¹, n², A¹-G¹-A², R^{11B}, R^{12B}, R^{13B}, R^{14B}, R^{21B}, R^{22B}, R^{23B} and R^{24B} represent the same meaning as described above.
n¹¹ and n¹² each independently represent an integer of 1 or more. n¹¹+n¹² is 3 when M is a ruthenium atom, a rhodium atom or an iridium atom, while n¹¹+n¹² is 2 when M is a palladium atom or a platinum atom.
R^{15B}, R^{16B}, R^{17B} and R^{18B} each independently represent a hydrogen atom, an alkyl group, a cycloalkyl group, an alkoxy group, a cycloalkoxy group, an aryl group, an aryloxy group, an alkenyl group, a monovalent heterocyclic group, a substituted amino group or a halogen atom, and the foregoing groups optionally have a substituent. When a plurality of R^{15B}, R^{16B}, R^{17B} and R^{18B} are present, they may be the same or different at each occurrence. R^{13B} and R^{15B}, R^{15B} and R^{16B}, R^{16B} and R^{17B}, R^{17B} and R^{18B}, and R^{18B} and R^{21B} each may be combined together to form a ring together with the atoms to which they are attached.]

The examples and preferable range of the aryl group, the monovalent heterocyclic group and the substituted amino group represented by R^{15B}, R^{16B}, R^{17B} and R^{18B} are the same as the examples and preferable range of the aryl group, the monovalent heterocyclic group and the substituted amino group as the substituent which Ring L¹ and Ring L² optionally have, respectively.

The examples and preferable range of the substituent which R^{15B}, R^{16B}, R^{17B} and R^{18B} optionally have are the same as the examples and preferable range of the substituent which the substituent which Ring L¹ and Ring L² optionally have optionally further has.

R^{15B}, R^{16B}, R^{17B} and R^{18B} are each preferably a hydrogen atom, an alkyl group, a cycloalkyl group, an alkoxy group, a cycloalkoxy group, a fluorine atom, an aryl group, an alkenyl group, a monovalent heterocyclic group or a substituted amino group, more preferably a hydrogen atom, an alkyl group, a cycloalkyl group, an aryl group, a monovalent heterocyclic group or a substituted amino group, further preferably a hydrogen atom, an alkyl group or a cycloalkyl group, particularly preferably a hydrogen atom, and the foregoing groups optionally have a substituent.

The phosphorescent compound includes, for example, phosphorescent compounds represented by the following formulae.

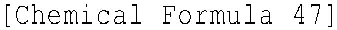

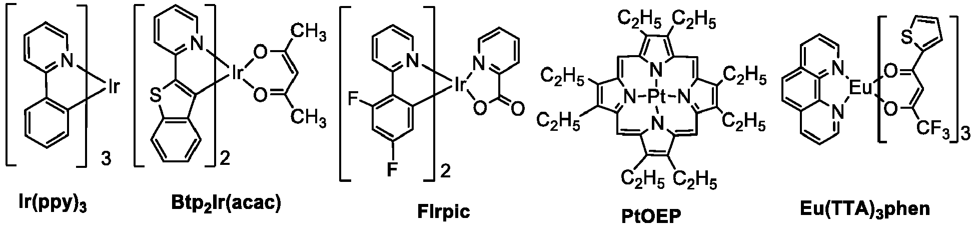

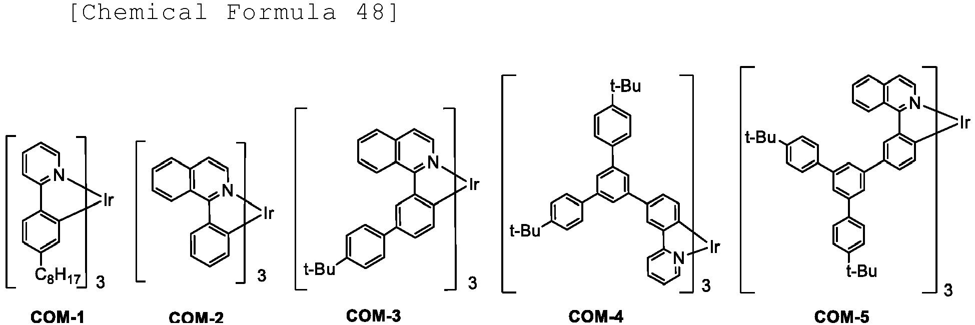

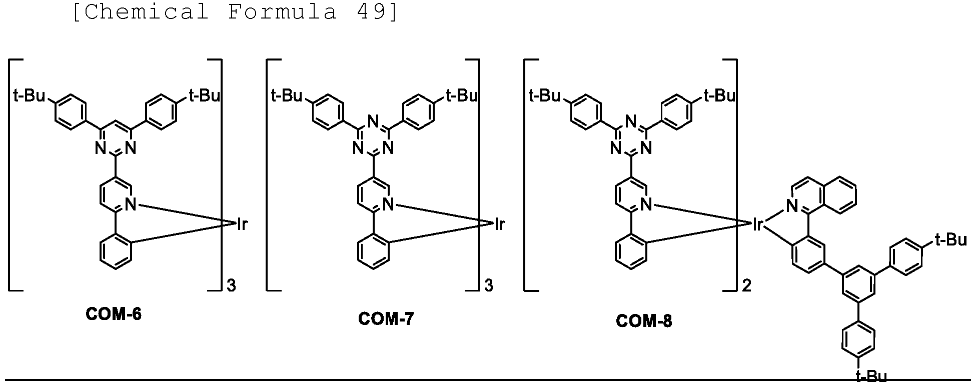

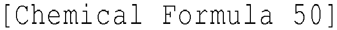

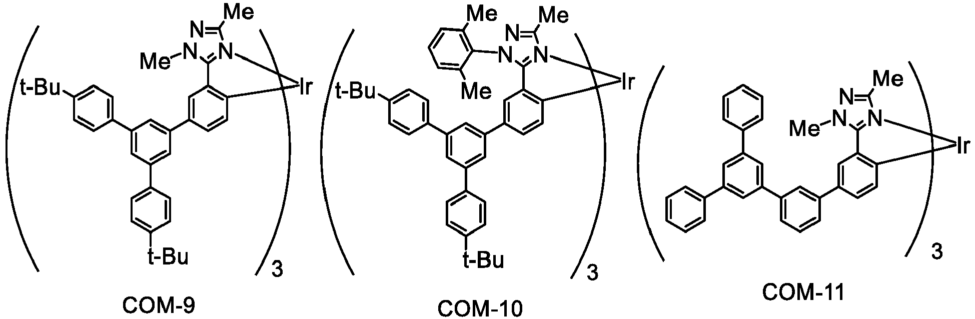

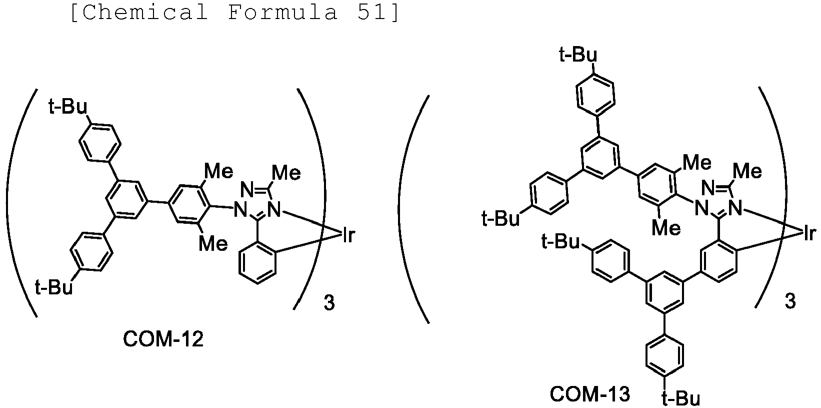

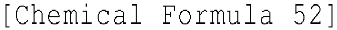

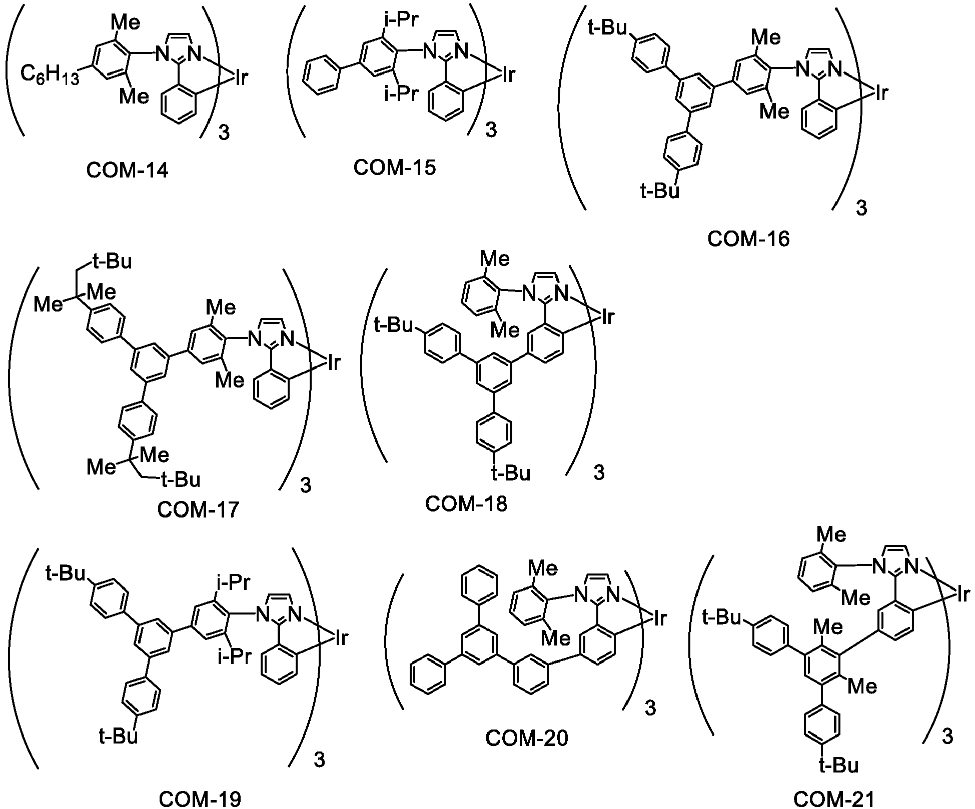

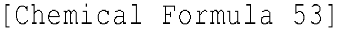

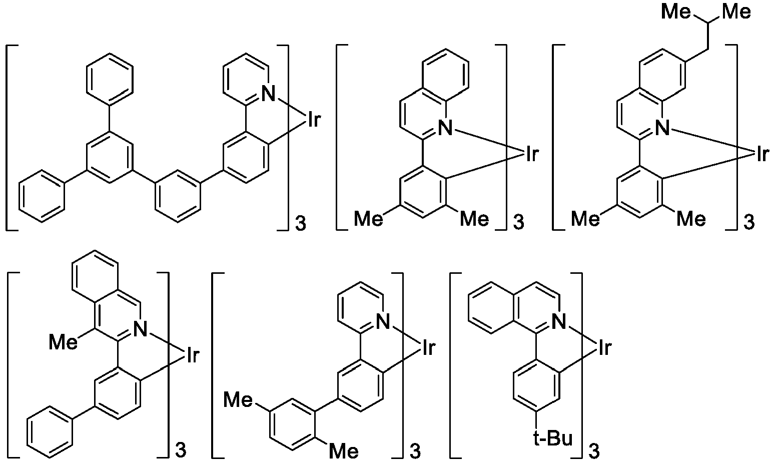

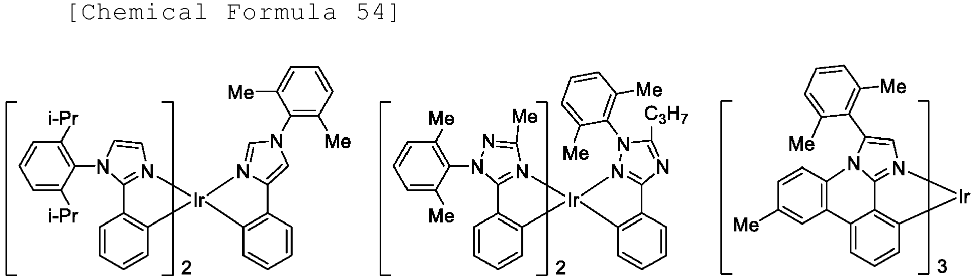

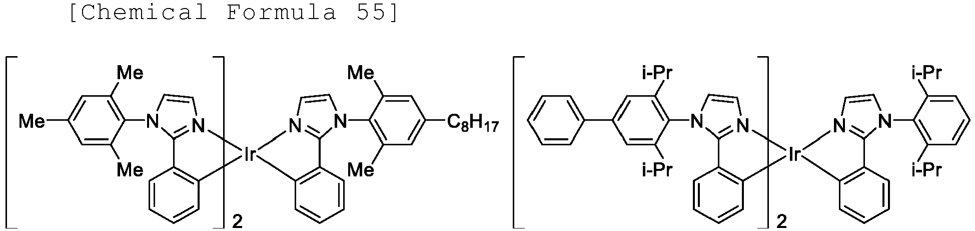

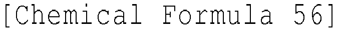

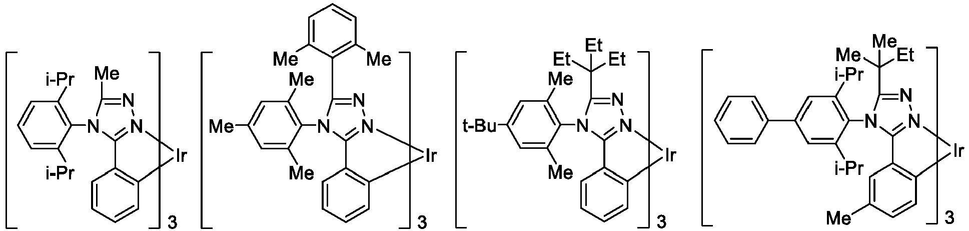

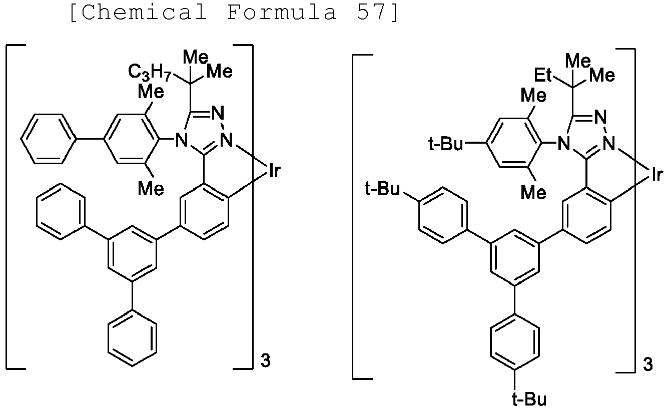

### [First composition]

The first layer may be a layer containing a composition (hereinafter, referred to also as "first composition") containing a compound represented by the formula (C-1) and at least one material selected from the group consisting of the phosphorescent compound described above, the hole transporting material, the hole injection material, the electron transporting material, the electron injection material, the light emitting material and the antioxidant. The hole transporting material, the hole injection material, the electron transporting material and the electron injection material contained in the first composition are different from the compound represented by the formula (C-1), and the light emitting material contained in the first composition is different from the compound represented by the formula (C-1) and different from the phosphorescent compound.

### [Hole transporting material]

The hole transporting material is classified into low molecular compounds and polymer compounds, and preferable are polymer compounds. The hole transporting material may have a crosslinking group.

The polymer compound includes, for example, polyvinylcarbazole, and derivatives thereof; polyarylenes having an aromatic amine structure in the side chain or main chain, and derivatives thereof. The polymer compound may also be a compound to which an electron accepting site is bound. The electron accepting site includes, for example, fullerene, tetrafluorotetracyanoquinodimethane, tetracyanoethylene, trinitrofluorenone and the like, preferably fullerene.

In the first composition, the compounding amount of the hole transporting material is usually 1 to 400 parts by mass, preferably 5 to 150 parts by mass, when the amount of the compound represented by the formula (C-1) is taken as 100 parts by mass.

The hole transporting material may be used each singly or in combination of two or more.

### [Electron transporting material]

The electron transporting material is classified into low molecular compounds and polymer compounds. The electron transporting material may have a crosslinking group.

The low molecular compound includes, for example, metal complexes having 8-hydroxyquinoline as a ligand; oxadiazole, anthraquinodimethane, benzoquinone, naphthoquinone, anthraquinone, tetracyanoanthraquinodimethane, fluorenone, diphenyldicyanoethylene and diphenoquinone, and derivatives thereof.

The polymer compound includes, for example, polyphenylene, polyfluorene, and derivatives thereof. The polymer compound may be doped with a metal.

In the first composition, the compounding amount of the electron transporting material is usually 1 to 400 parts by mass, preferably 5 to 150 parts by mass, when the amount of the compound represented by the formula (C-1) is taken as 100 parts by mass.

The electron transporting material may be used each singly or in combination of two or more.

### [Hole injection material and electron injection material]

The hole injection material and the electron injection material are each classified into low molecular compounds and polymer compounds. The hole injection material and the electron injection material optionally have a crosslinking group.

The low molecular compound includes, for example, metal phthalocyanines such as copper phthalocyanine and the like; carbon; oxides of metals such as molybdenum, tungsten and the like; and metal fluorides such as lithium fluoride, sodium fluoride, cesium fluoride, potassium fluoride and the like.

The polymer compound includes electrically conductive polymers such as, for example, polyaniline, polythiophene, polypyrrole, polyphenylenevinylene, polythienylenevinylene, polyquinoline and polyquinoxaline, and derivatives thereof; polymers containing an aromatic amine structure in the main chain or side chain, and the like.

In the first composition, the compounding amounts of the hole injection material and the electron injection material are each usually 1 to 400 parts by mass, preferably 5 to 150 parts by mass, when the amount of the compound represented by the formula (C-1) is taken as 100 parts by mass,.

The electron injection material and the hole injection material may each be used singly or in combination of two or more.

### [Ion doping]

When the hole injection material or the electron injection material contains an electrically conductive polymer, the electric conductivity of the electrically conductive polymer is preferably 1×10⁻⁵ S/cm to 1×10³ S/cm. For adjusting the electric conductivity of the electrically conductive polymer within such a range, the electrically conductive polymer can be doped with an appropriate amount of ions.

The kind of the ion for doping is an anion for the hole injection material and a cation for the electron injection material. The anion includes, for example, a polystyrenesulfonate ion, an alkylbenzenesulfonate ion and a camphor sulfonate ion. The cation includes, for example, a lithium ion, a sodium ion, a potassium ion and a tetrabutylammonium ion.

The ion for doping may be used each singly or in combination of two or more.

### [Light emitting material]

The light emitting material is classified into low molecular compounds and polymer compounds. The light emitting material may have a crosslinking group.

The low molecular compound includes, for example, naphthalene and derivatives thereof, anthracene and derivatives thereof, and perylene and derivatives thereof.

The polymer compound includes polymer compounds containing, for example, a phenylene group, a naphthalenediyl group, an anthracenediyl group, a fluorenediyl group, a phenanthrenediyl group, a dihydrophenanthrenediyl group, a group represented by the formula (X) described later, a carbazolediyl group, a phenoxazinediyl group, a phenothiazinediyl group, a pyrenediyl group and the like.

In the first composition, the compounding amount of the light emitting material is usually 1 to 400 parts by mass, preferably 5 to 150 parts by mass, when the amount of the compound represented by the formula (C-1) is taken as 100 parts by mass.

The light emitting material may be used singly or in combination of two or more.

### [Antioxidant]

The antioxidant may be a compound which is soluble in the same solvent as for a compound represented by the formula (C-1) and which does not disturb light emission and charge transportation, and includes, for example, phenol type antioxidants and phosphorus-based antioxidants.

In the first composition, the compounding amount of the antioxidant is usually 0.001 to 10 parts by mass, when the amount of the compound represented by the formula (C-1) is taken as 100 parts by mass.

The antioxidant may be used each singly or in combination of two or more.

### First ink

The compound represented by the formula (C-1) can be, for example, dissolved in a solvent and used. The composition containing a compound represented by the formula (C-1) and a solvent (hereinafter, referred to also as "first ink") can be suitably used for application methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet printing method, a capillary coat method, a nozzle coat method and the like.

The viscosity of the first ink may be adjusted depending on the kind of the application method, and when applied to a printing method in which a solution passes through a discharge device such as an inkjet printing method and the like, it is preferably 1 to 20 mPa•s at 25°C since clogging and flight bending hardly occur during discharge.

The solvent contained in the first ink is preferably a solvent which can dissolve or uniformly disperse solid components in the ink. The solvent includes, for example, chlorine-based solvents such as 1,2-dichloroethane, 1,1,2-trichloroethane, chlorobenzene, o-dichlorobenzene and the like; ether solvents such as tetrahydrofuran, dioxane, anisole, 4-methylanisole and the like; aromatic hydrocarbon solvents such as toluene, xylene, mesitylene, ethylbenzene, n-hexylbenzene, cyclohexylbenzene and the like; aliphatic hydrocarbon solvents such as cyclohexane, methylcyclohexane, n-pentane, n-hexane, n-heptane, n-octane, n-nonane, n-decane, n-dodecane, bicyclohexyl and the like; ketone solvents such as acetone, methyl ethyl ketone, cyclohexanone, acetophenone and the like; ester solvents such as ethyl acetate, butyl acetate, ethylcellosolve acetate, methyl benzoate, phenyl acetate and the like; polyhydric alcohol solvents such as ethylene glycol, glycerin, 1,2-hexanediol and the like; alcohol solvents such as isopropyl alcohol, cyclohexanol and the like; sulfoxide solvents such as dimethyl sulfoxide and the like; and amide solvents such as N-methyl-2-pyrrolidone, N,N-dimethylformamide and the like. The solvent may be used each singly or in combination of two or more.

In the first ink, the compounding amount of the solvent is usually 1000 to 100000 parts by mass, preferably 2000 to 20000 parts by mass, when the amount of the compound represented by the formula (C-1) is taken as 100 parts by mass.

### [Simple substance or compound contained in second layer and third layer]

The second layer and the third layer which the light emitting device of the present invention has are each a layer containing at least one selected from the group consisting of a simple substance composed only of an alkali metal element, a simple substance composed only of a group 2 (meaning periodic table group 2, the same shall apply in the present specification) element, a compound containing an alkali metal element and a compound containing a group 2 element.

In each of the second layer and the third layer, the simple substance composed only of an alkali metal element, the simple substance composed only of a group 2 element, the compound containing an alkali metal element and the compound containing a group 2 element each may be contained singly or in combination of two or more.

In the light emitting device of the present invention, at least one selected from the group consisting of a simple substance composed only of an alkali metal element, a simple substance composed only of a group 2 element, a compound containing an alkali metal element and a compound containing a group 2 element contained in the second layer (hereinafter, referred to also as "simple substance or compound of second layer") and at least one selected from the group consisting of a simple substance composed only of an alkali metal element, a simple substance composed only of a group 2 element, a compound containing an alkali metal element and a compound containing a group 2 element contained in the third layer (hereinafter, referred to also as "simple substance or compound of third layer") are mutually different (hereinafter, referred to as "mutually different relation").

"Mutually different relation" means that when the second layer contains only one kind of "simple substance or compound of second layer" and when the third layer contains only one kind of "simple substance or compound of third layer", the simple substance or compound of the second layer and the simple substance or compound of the third layer are different mutually.

Further, "mutually different relation" means that when the second layer contains two or more kinds of "simple substance or compound of second layer", the third layer contains at least one of "simple substance or compound of third layer".

Furthermore, "mutually different relation" means that when the third layer contains two or more kinds of "simple substance or compound of third layer", the second layer contains at least one of "simple substance or compound of second layer".

For "mutually different relation", it is preferable that all components of "simple substance or compound of second layer" contained in the second layer and all components of "simple substance or compound of third layer" contained in the third layer are not identical completely, it is more preferable that one of "simple substance or compound of second layer" contained in the second layer is not contained in the third layer, it is further preferable that all components of "simple substance or compound of second layer" contained in the second layer are not contained in the third layer, since the light emitting device of the present invention is more excellent in luminance life.

In the simple substance composed only of an alkali metal element and the compound containing an alkali metal element, the alkali metal element is preferably lithium, sodium, potassium or cesium, more preferably lithium, sodium or cesium, since the light emitting device of the present invention is more excellent in luminance life.

In the simple substance composed only of a group 2 element and the compound containing a group 2 element, the group 2 element is preferably beryllium, magnesium, calcium or barium, more preferably calcium or barium, since the light emitting device of the present invention is more excellent in luminance life.

The compound containing an alkali metal element may be a polymer compound containing an alkali metal element or a low molecular compound containing an alkali metal element, and it is preferably a polymer compound containing an alkali metal element, since the light emitting device of the present invention can be fabricated by an application method.

The compound containing an alkali metal element may contain only one kind of the alkali metal element or two or more kinds of the alkali metal element.

The compound containing a group 2 element may be a polymer compound containing a group 2 element or a low molecular compound containing a group 2 element, and it is preferably a low molecular compound containing a group 2 element, since the light emitting device of the present invention is more excellent in luminance life.

The compound containing a group 2 element may contain only one kind of the group 2 element or may contain two or more kinds of the group 2 element.

The compound containing an alkali metal element and the compound containing a group 2 element each may be any of a simple salt, a double salt and a complex salt.

The low molecular compound containing an alkali metal element includes, for example, metal complexes containing an alkali metal element and inorganic compounds containing an alkali metal element.

The inorganic compound containing an alkali metal element includes, for example, alkali metal halides, alkali metal oxides, alkali metal hydroxides, alkali metal cyanides, alkali metal salts of phosphoric acid, alkali metal salts of carbonic acid, alkali metal salts of sulfuric acid and alkali metal salts of nitric acid, and it is preferably an alkali metal halide, an alkali metal oxide, an alkali metal hydroxide or an alkali metal salt or carbonic acid, more preferably an alkali metal halide, an alkali metal oxide or an alkali metal salt of carbonic acid, further preferably an alkali metal halide, and these compounds may be hydrates or non-hydrates, since the light emitting device of the present invention is more excellent in luminance life.

The alkali metal halide is preferably an alkali metal fluoride or an alkali metal chloride, more preferably an alkali metal fluoride, and these compounds may be hydrates or non-hydrates. Further, the alkali metal halide is preferably lithium halide, sodium halide, potassium halide or cesium halide, more preferably lithium halide or sodium halide. That is, the alkali metal halide is preferably lithium fluoride, sodium fluoride, potassium fluoride or cesium fluoride, more preferably lithium fluoride or sodium fluoride.

The alkali metal oxide is preferably lithium oxide, sodium oxide, potassium oxide or cesium oxide, more preferably lithium oxide or cesium oxide, further preferably lithium oxide, and these compounds may be hydrates or non-hydrates.

The alkali metal hydroxide is preferably lithium hydroxide, sodium hydroxide, potassium hydroxide or cesium hydroxide, more preferably lithium hydroxide or cesium hydroxide, and these compounds may be hydrates or non-hydrates.

The alkali metal cyanide is preferably lithium cyanide, sodium cyanide, potassium cyanide or cesium cyanide, and these compounds may be hydrates or non-hydrates.

The alkali metal salt of phosphoric acid includes, for example, tri-alkali phosphate metal salts, di-alkali hydrogen phosphate metal salts and alkali dihydrogen phosphate metal salts, and it is preferably a tri-alkali phosphate metal salt, and these compounds may be hydrates or non-hydrates. The tri-alkali phosphate metal salt includes, for example, trilithium phosphate, trisodium phosphate, tripotassium phosphate and tricesium phosphate, and it is preferably trilithium phosphate or tricesium phosphate. The di-alkali hydrogen phosphate metal salt includes, for example, dilithium hydrogen phosphate, disodium hydrogen phosphate, dipotassium hydrogen phosphate and dicesium hydrogen phosphate. The alkali dihydrogen phosphate metal salt includes, for example, lithium dihydrogen phosphate, sodium dihydrogen phosphate, potassium dihydrogen phosphate and cesium dihydrogen phosphate.

The alkali metal salt of carbonic acid includes, for example, alkali metal carbonates and alkali metal hydrogen carbonates, and it is preferably an alkali metal carbonate, and these compounds may be hydrates or non-hydrates. The alkali metal carbonate is preferably lithium carbonate, sodium carbonate, potassium carbonate or cesium carbonate, more preferably cesium carbonate. The alkali metal hydrogen carbonate is preferably lithium hydrogen carbonate, sodium hydrogen carbonate, potassium hydrogen carbonate or cesium hydrogen carbonate.

The alkali metal salt of sulfuric acid includes, alkali metal sulfate salts and alkali metal hydrogen sulfate salts, and it is preferably an alkali metal sulfate salt, and these compounds may be hydrates or non-hydrates. The alkali metal sulfate salt includes, for example, lithium sulfate, sodium sulfate, potassium sulfate and cesium sulfate, and it is preferably sodium sulfate or potassium sulfate. The alkali metal hydrogen sulfate salt includes, for example, lithium hydrogen sulfate, sodium hydrogen sulfate, potassium hydrogen sulfate or cesium hydrogen sulfate.

The alkali metal salt of nitric acid includes, for example, lithium nitrate, sodium nitrate, potassium nitrate and cesium nitrate, and it is preferably sodium nitrate or potassium nitrate, and these compounds may be hydrates or non-hydrates.

The low molecular compound containing a group 2 element includes, for example, metal complexes containing a group 2 element and inorganic compounds containing a group 2 element.

The inorganic compound containing a group 2 element includes, for example, halides of a group 2 element, oxides of a group 2 element, hydroxides of a group 2 element, cyanides of a group 2 element, phosphoric acid group 2 element salts, carbonic acid group 2 element salts, sulfuric acid group 2 element salts and nitric acid group 2 element salts, and it is preferably a halide of a group 2 element, an oxide of a group 2 element, a hydroxide of a group 2 element or a carbonic acid group 2 element salt, more preferably a halide of a group 2 element, an oxide of a group 2 element or a carbonic acid group 2 element salt, and these compounds may be hydrates or non-hydrates, since the light emitting device of the present invention is more excellent in luminance life.

The halide of a group 2 element is preferably a fluoride of a group 2 element or a chloride of a group 2 element, more preferably a fluoride of a group 2 element, and these compounds may be hydrates or non-hydrates. Further, the alkali metal halide is preferably beryllium halide, magnesium halide, calcium halide or barium halide, more preferably calcium halide or barium halide. That is, the halide of a group 2 element is preferably beryllium fluoride, magnesium fluoride, calcium fluoride or barium fluoride, more preferably calcium fluoride or barium fluoride.

The oxide of a group 2 element is preferably beryllium oxide, magnesium oxide, calcium oxide or barium oxide, more preferably calcium oxide or barium oxide, and these compounds may be hydrates or non-hydrates.

The hydroxides of a group 2 element is preferably beryllium hydroxide, magnesium hydroxide, calcium hydroxide or barium hydroxide, more preferably calcium hydroxide or barium hydroxide, and these compounds may be hydrates or non-hydrates.

The cyanide of a group 2 element includes, for example, magnesium cyanide, calcium cyanide or barium cyanide, and these compounds may be hydrates or non-hydrates.

The phosphoric acid group 2 element salt includes, for example, phosphate salts of a group 2 element, hydrogen phosphate salts of a group 2 element and dihydrogen phosphate salts of a group 2 element, and it is preferably a phosphate salt of a group 2 element, and these compounds may be hydrates or non-hydrates. The phosphate salt of a group 2 element is preferably calcium phosphate or barium phosphate. The hydrogen phosphate salt of a group 2 element includes, for example, calcium hydrogen phosphate and barium hydrogen phosphate. The dihydrogen phosphate salt of a group 2 element includes, for example, calcium dihydrogen phosphate and barium hydrogen phosphate.

The carbonic acid group 2 element salt includes, for example, carbonates of a group 2 element and hydrogen carbonates of a group 2 element, and it is preferably a carbonate of a group 2 element, and these compounds may be hydrates or non-hydrates. The carbonate of a group 2 element includes, for example, beryllium carbonate, magnesium carbonate, calcium carbonate or barium carbonate, and it is preferably calcium carbonate or barium carbonate. The hydrogen carbonate of a group 2 element includes, for example, beryllium hydrogen carbonate, magnesium hydrogen carbonate, calcium hydrogen carbonate or barium hydrogen carbonate, and it is preferably calcium hydrogen carbonate or barium hydrogen carbonate.

The sulfuric acid group 2 element salt includes sulfate salts of a group 2 element and hydrogen sulfate salts of a group 2 element, and it is preferably a sulfate salt of a group 2 element, and these compounds may be hydrates or non-hydrates. The sulfate salt of a group 2 element includes, for example, beryllium sulfate, magnesium sulfate, calcium sulfate or barium sulfate, and it is preferably calcium sulfate or barium sulfate. The hydrogen sulfate salt of a group 2 element includes, for example, beryllium sulfate, magnesium sulfate, calcium sulfate or barium sulfate.

The nitric acid group 2 element salt includes, for example, beryllium nitrate, magnesium nitrate, calcium nitrate or barium nitrate, and it is preferably calcium nitrate or barium nitrate, and these compounds may be hydrates or non-hydrates.

The metal complex containing an alkali metal element is a metal complex having at least one organic ligand and forming at least one bond selected from the group consisting of an ion bond, a coordination bond and a covalent bond between this organic ligand and the alkali metal atom.

The metal complex containing a group 2 element is a metal complex having at least one organic ligand and forming at least one bond selected from the group consisting of an ion bond, a coordination bond and a covalent bond between this organic ligand and the periodic table group 2 metal atom.

The metal complex containing an alkali metal element and the metal complex containing a group 2 element each may further have an inorganic ligand such as a halide ion, a hydroxide ion, a cyanide ion, a phosphate ion, a carbonate ion, a hydrogen carbonate ion, a sulfate ion, a nitrate ion and the like.

When the metal complex containing an alkali metal element and the metal complex containing a group 2 element have a plurality of the organic ligands, they may be the same or different.

When the metal complex containing an alkali metal element and the metal complex containing a group 2 element have a plurality of the inorganic ligands, they may be the same or different.

The metal complex containing an alkali metal element and the metal complex containing a group 2 element each may be a hydrate or a non-hydrate.

The organic ligand which the metal complex containing an alkali metal element and the metal complex containing a group 2 element have includes neutral or anionic monodentate ligands or neutral or anionic polydentate ligands forming at least one bond selected from the group consisting of an ion bond, a coordination bond and a covalent bond between the ligand and the alkali metal atom or the periodic table group 2 metal atom. The bond between the alkali metal atom or the periodic table group 2 metal atom and the ligand includes, for example, a metal-nitrogen bond, a metal-oxygen bond, a metal-phosphorus bond and a metal-sulfur bond, and it is preferably a metal-nitrogen bond, a metal-oxygen bond or a metal-sulfur bond, more preferably a metal-nitrogen bond or a metal-oxygen bond. The polydentate ligand is usually a bidentate or more and hexadentate or less ligand, preferably a bidentate or more and tetradentate or less ligand, more preferably a bidentate ligand.

The organic ligand which the metal complex containing an alkali metal element and the metal complex containing a group 2 element have is preferably a monodentate or more and tetradentate or less ligand, more preferably a monodentate ligand or a bidentate ligand, further preferably a bidentate ligand.

The metal complex containing an alkali metal element and the metal complex containing a group 2 element are each preferably a metal complex composed only of an organic ligand. That is, it is preferable that the metal complex containing an alkali metal element and the metal complex containing a group 2 element have no inorganic ligand.

The organic ligand which the metal complex containing an alkali metal element and the metal complex containing a group 2 element have includes, for example, nitrogen-containing heterocyclic compounds such as pyridine, bipyridine, quinoline, isoquinoline, phenanthroline, azole and the like; conjugated bases of organic Broenstead acids such as acetic acid, benzoic acid, picolinic acid, hydroxyquinoline, acetylacetone, phenol, thiophenol, methanol and the like; and heterocyclic macrocyclic compounds such as crown ether, cryptand, azacrown ether, phthalocyanine and the like.

The metal complex containing an alkali metal element and the metal complex containing a group 2 element is preferably a compound represented by the formula (S-1), since the light emitting device of the present invention is more excellent in luminance life. That is, the metal complex containing an alkali metal element is preferably a compound represented by the formula (S-1) in which M^{ES1} is an alkali metal atom. Further, the metal complex containing a group 2 element is preferably a compound represented by the formula (S-1) in which M^{ES1} is a periodic table group 2 metal atom.

The compound represented by the formula (S-1) may be a hydrate or a non-hydrate. 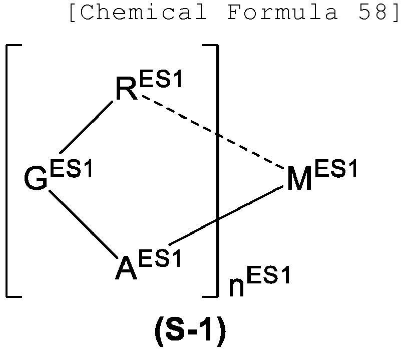 [wherein,
M^{ES1} represents an alkali metal atom or a periodic table group 2 metal atom.
n^{ES1} is 1 when M^{ES1} is an alkali metal atom, while n^{ES1} is 2 when M^{ES2} is a periodic table group 2 metal atom.
A^{ES1} represents a group represented by -X^{ES1}-, - (X^{ES2} =) C-X^{ES1}-, -(X^{ES2} =)₂S-X^{ES1}-, -(X^{ES2} =) S-X^{ES1} - or (X^{ES2}=)P-X^{ES1}-. When a plurality of A^{ES1} are present, they may be the same or different. X^{ES1} represents an oxygen atom, a sulfur atom or a group represented by - N(R^{ES2})-. X^{ES2} represents an oxygen atom, a sulfur atom or a group represented by =N(R^{ES3}). When a plurality of X^{ES2} are present, they may be the same or different. R^{ES2} and R^{ES3} each independently represent a hydrogen atom, an alkyl group, a cycloalkyl group, an aryl group or a monovalent heterocyclic group, and the foregoing groups optionally have a substituent.
G^{ES1} represents a single bond or a divalent group. When a plurality of G^{ES1} are present, they may be the same or different.
R^{ES1} represents a group represented by R^{ES1}'-(X^{ES3}=)C-, an alkyl group, a cycloalkyl group, an alkoxy group, a cycloalkoxy group, an aryl group, an aryloxy group, a monovalent heterocyclic group or a substituted amino group, and the foregoing groups optionally have a substituent. X^{ES3} represents an oxygen atom, a sulfur atom or a group represented by =N(R^{ES3}). When a plurality of X^{ES3} are present, they may be the same or different. R^{ES1}' represents a hydrogen atom, an alkyl group, a cycloalkyl group, an alkoxy group, a cycloalkoxy group, an aryl group, an aryloxy group, a monovalent heterocyclic group or a substituted amino group, and the foregoing groups optionally have a substituent. R^{ES3} represent the same meaning as described above.
When a chemical bond is present between M^{ES1} and R^{ES1}, the dotted line means a solid line. When a chemical bond is not present between M^{ES1} and R^{ES1}, the dotted line is absent. When a plurality of dotted lines are present, they may be the same or different.

The alkali metal atom represented by M^{ES1} is preferably a lithium atom, a sodium atom, potassium atom or a cesium atom, more preferably a lithium atom, a sodium atom or a cesium atom, further preferably a lithium atom or a sodium atom, particularly preferably a lithium atom, since the light emitting device of the present invention is more excellent in luminance life.

The periodic table group 2 metal atom represented by M^{ES1} is preferably a beryllium atom, a magnesium atom, a calcium atom or a barium atom, more preferably a beryllium atom, a calcium atom or a barium atom, further preferably a beryllium atom, since synthesis of a compound represented by the formula (S-1) is easy.

The periodic table group 2 metal atom represented by M^{ES1} is preferably a beryllium atom, a magnesium atom, a calcium atom or a barium atom, more preferably a beryllium atom, a calcium atom or a barium atom, further preferably a beryllium atom, since the light emitting device of the present invention is more excellent in luminance life.

M^{ES1} is preferably an alkali metal atom, since the light emitting device of the present invention is more excellent in luminance life.

M^{ES1} is preferably a lithium atom, a sodium atom, a cesium atom, a beryllium atom, a calcium atom or a barium atom, more preferably a lithium atom, a sodium atom, a cesium atom or a beryllium atom, since the light emitting device of the present invention is more excellent in luminance life and since synthesis of a compound represented by the formula (S-1) is easy.

A^{ES1} is preferably a group represented by -X^{ES1}-, - (X^{ES2}=) C-X^{ES1}-, -(X^{ES2}=)₂S-X^{ES1} - or -(X^{ES2}=) P-X^{ES1}-, more preferably a group represented by -X^{ES1}- or - (X^{E32}=)C-X^{ES1}-, further preferably a group represented by -X^{ES1}-.

X^{ES1} is preferably an oxygen atom or a sulfur atom, more preferably an oxygen atom. X^{ES2} is preferably an oxygen atom or a sulfur atom, more preferably an oxygen atom.

R^{ES2} and R^{ES3} are each preferably an alkyl group or an aryl group, more preferably an aryl group, and the foregoing groups optionally have a substituent.

The substituent which R^{ES2} and R^{ES3} optionally have is preferably an alkyl group, a cycloalkyl group, an alkoxy group, a cycloalkoxy group, an aryl group, a monovalent heterocyclic group, a substituted amino group or a halogen atom, more preferably an alkyl group, a cycloalkyl group, an aryl group, a monovalent heterocyclic group or a fluorine atom, further preferably an alkyl group or a cycloalkyl group, and the foregoing groups optionally further have a substituent, since synthesis of a compound represented by the formula (S-1) is easy.

The examples and preferable range of the aryl group, the monovalent heterocyclic group and the substituted amino group as the substituent which R^{ES2} and R^{ES3} optionally have are the same as the examples and preferable range of the aryl group, the monovalent heterocyclic group and the substituted amino group represented by R^{ES1} described later, respectively.

The divalent group represented by G^{ES1} includes, for example, an alkylene group, a cycloalkylene group, an alkenediyl group, a cycloalkenediyl group, an alkynediyl group, a cycloalkynediyl group, an arylene group, a divalent heterocyclic group, and a group in which 2 to 10 (preferably 2 to 5) of these groups are bonded directly, and it is preferably an alkylene group, an alkenediyl group, an arylene group or a divalent heterocyclic group, more preferably an alkenediyl group or an arylene group, and the foregoing groups optionally have a substituent.

The alkylene group represented by G^{ES1} is a group obtained by removing one hydrogen atom bonding directly to a carbon atom of the alkyl group described above, and is preferably a methylene group optionally having a substituent.

The cycloalkylene group represented by G^{ES1} is a group obtained by removing one hydrogen atom bonding directly to a carbon atom of the cycloalkyl group described above.

The alkenediyl group represented by G^{ES1} is a group obtained by removing one hydrogen atom bonding directly to a carbon atom of the alkenyl group described above, and is preferably a vinylidene group optionally having a substituent or a vinylene group optionally having a substituent.

The cycloalkenediyl group represented by G^{ES1} is a group obtained by removing one hydrogen atom bonding directly to a carbon atom of the cycloalkenyl group described above.

The alkynediyl group represented by G^{ES1} is a group obtained by removing one hydrogen atom bonding directly to a carbon atom of the alkynyl group described above.

The cycloalkynediyl group represented by G^{ES1} is a group obtained by removing one hydrogen atom bonding directly to a carbon atom of the cycloalkynyl group described above.

G^{ES1} is preferably a single bond, an alkenediyl group or an arylene group, more preferably a single bond, a vinylene group or a phenylene group, further preferably a single bond or a phenylene group, particularly preferably a single bond.

The examples and preferable range of the substituent which G^{ES1} optionally has are the same as the examples and preferable range of the substituent which R^{ES2} and R^{ES3} optionally have.

R^{ES1} is preferably a group represented by R^{ES1}'-(X^{ES3}=)C-, an alkyl group, a cycloalkyl group, an aryl group or a monovalent heterocyclic group, more preferably a group represented by R^{ES1'}-(X^{ES3}=)C-, an aryl group or a monovalent heterocyclic group, further preferably a monovalent heterocyclic group, and the foregoing groups optionally have a substituent.

The examples and preferable range of the aryl group and the substituted amino group represented by R^{ES1} are the same as the examples and preferable range of the aryl group and the substituted amino group as the substituent which Ring L¹ and Ring L² optionally have described above, respectively.

The examples of the monovalent heterocyclic group represented by R^{ES1} are the same as the examples of the monovalent heterocyclic group as the substituent which Ring R^{1C}, Ring R^{2C}, Ring R^{3C} and Ring R^{4C} optionally have described above. The monovalent heterocyclic group represented by R^{ES1} is preferably a group obtained by removing from a benzodiazole ring, a benzotriazole ring, a benzooxadiazole ring, a benzothiadiazole ring, a pyridine ring, a diazabenzene ring, a triazine ring, an azanaphthalene ring, a diazanaphthalene ring, a triazanaphthalene ring, an azaanthracene ring, a diazaanthracene ring, a triazaanthracene ring, an azaphenanthrene ring, a diazaphenanthrene ring or a triazaphenanthrene ring one hydrogen atom bonding directly to a carbon atom or a hetero atom constituting the ring, more preferably a group obtained by removing from a pyridine ring, a diazabenzene ring, a triazine ring, an azanaphthalene ring, a diazanaphthalene ring, a triazanaphthalene ring, an azaphenanthrene ring, a diazaphenanthrene ring or a triazaphenanthrene ring one hydrogen atom bonding directly to a carbon atom or a hetero atom constituting the ring, further preferably a group obtained by removing from a pyridine ring, an azanaphthalene ring or an azaphenanthrene ring one hydrogen atom bonding directly to a carbon atom or a hetero atom constituting the ring, and the foregoing groups optionally have a substituent, since the light emitting device of the present invention is more excellent in luminance life.

X^{ES3} is preferably an oxygen atom or a group represented by =N(R^{ES3}), more preferably a group represented by =N(R^{ES3}).

R^{ES1}' is a hydrogen atom, an alkyl group, a cycloalkyl group, an aryl group or a monovalent heterocyclic group, and the foregoing groups optionally have a substituent.

The examples and preferable range of the substituent which R^{ES1} and R^{ES1}' optionally have are the same as the examples and preferable range of the substituent which R^{ES2} and R^{ES3} optionally have.

The metal complex containing an alkali metal element and the metal complex containing a group 2 element include, for example, compounds represented by the following formulae, and these compounds may be hydrates or non-hydrates. 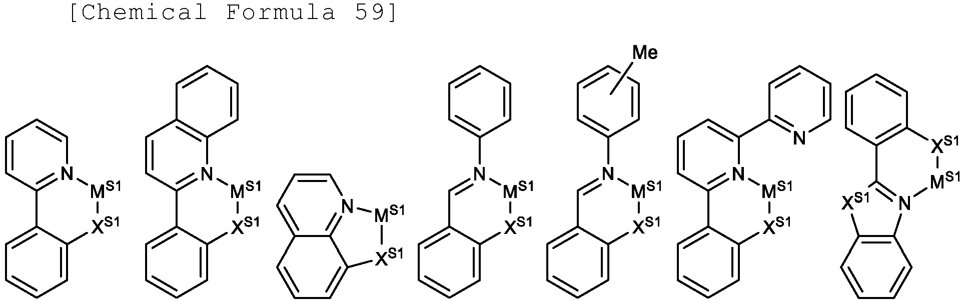 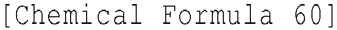 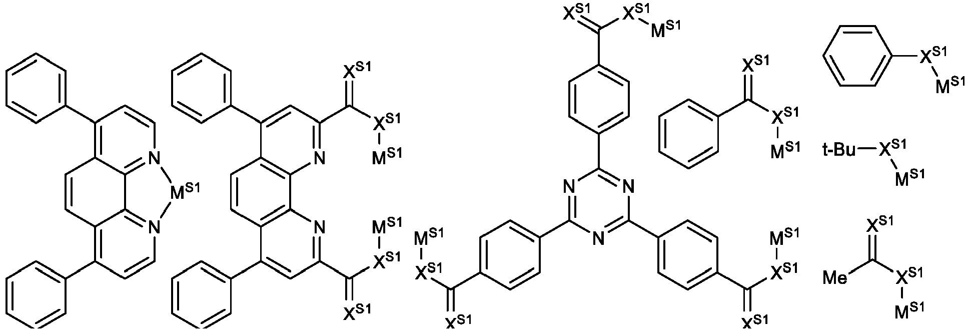 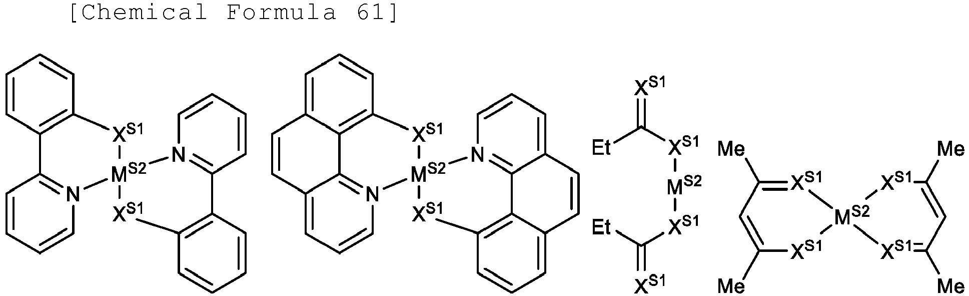 [wherein,
M^{S1} represents an alkali metal atom. M^{S2} represents a periodic table group 2 metal atom.
X^{S1} represents an oxygen atom or a sulfur atom. When a plurality of X^{S1} are present, they may be the same or different.]

M^{S1} is preferably a lithium atom, a sodium atom, a potassium atom or a cesium atom, more preferably a lithium atom.

M^{S2} is a beryllium atom, a magnesium atom, a calcium atom or a barium atom, preferably a beryllium atom.

X^{S1} is preferably an oxygen atom.

The polymer compound containing an alkali metal element and the polymer compound containing a group 2 element are each preferably a polymer compound containing a constitutional unit represented by the formula (ET-1), since the light emitting device of the present invention is more excellent in luminance life. That is, the polymer compound containing an alkali metal element is preferably a polymer compound containing a constitutional unit represented by the formula (ET-1) in which M^{E1} in the formula (ET-1) is an alkali metal cation. Further, the polymer compound containing a group 2 element is preferably a polymer compound containing a constitutional unit represented by the formula (ET-1) in which M^{E1} in the formula (ET-1) is a periodic table group 2 metal cation. 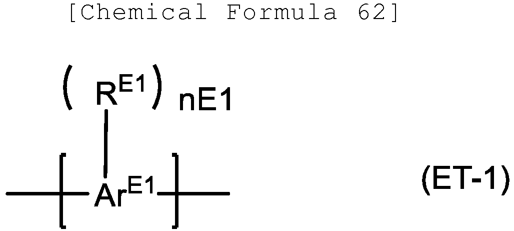 [wherein,
nE1 represents an integer of 1 or more.
Ar^{E1} represents an aromatic hydrocarbon group or a heterocyclic group, and the foregoing groups optionally have a substituent other than R^{E1}.
R^{E1} represents a group represented by the formula (ES-1). When a plurality of R^{E1} are present, they may be the same or different.]

-R^{E3}-{(Q^{E1})_{nE3}-Y^{E1}(M^{E1})_{aE1}(Z^{E1})_{bE1}}_{mE1} (ES-1)

[wherein,

nE3 represents an integer of 0 or more, aE1 represents an integer of 1 or more, bE1 represents an integer of 0 or more, mE1 represents an integer of 1 or more. When a plurality of nE3, aE1 and bE1 are present, they may be the same or different at each occurrence. When R^{E3} is a single bond, mE1 is 1. Further, aE1 and bE1 are selected so that the charge of a group represented by the formula (ES-1) is 0.

R^{E3} represents a single bond, a hydrocarbon group, a heterocyclic group or O-R^{E3}' (R^{E3}' represents a hydrocarbon group or a heterocyclic group), and the foregoing groups optionally have a substituent.

Q^{E1} represents an alkylene group, a cycloalkylene group, an arylene group, an oxygen atom or a sulfur atom, and the foregoing groups optionally have a substituent. When a plurality of Q^{E1} are present, they may be the same or different.

Y^{E1} represents CO₂⁻, SO₃⁻, SO₂⁻ or PO₃²⁻. When a plurality of Y^{E1} are present, they may be the same or different.

M^{E1} represents an alkali metal cation or a periodic table group 2 metal cation. When a plurality of M^{E1} are present, they may be the same or different.

Z^{E1} represents F⁻, Cl⁻, Br⁻, I⁻, OH⁻, B (R^{E4})₄⁻, R^{E4}SO₃⁻, R^{E4}COO⁻, NO₃⁻, SO₄²⁻, HSO₄⁻, PO₄³⁻, HPO₄²⁻, H₂PO₄⁻, BF₄⁻ or PF₆⁻. R^{E4} represents an alkyl group, a cycloalkyl group or an aryl group, and the foregoing groups optionally have a substituent. When a plurality of Z^{E1} are present, they may be the same or different.]

nE1 is usually an integer of 1 to 10, preferably an integer of 1 to 4, more preferably 1 or 2.

The aromatic hydrocarbon group represented by Ar^{E1} includes, for example, groups obtained by removing nE1 hydrogen atoms from the arylene group described above, and it is preferably a group obtained by removing from a phenylene group, a naphthalenediyl group, a fluorenediyl group or a phenanthrenediyl group nE1 hydrogen atoms bonding directly to atoms constituting the ring, more preferably a group obtained by removing from a fluorenediyl group nE1 hydrogen atoms bonding directly to atoms constituting the ring, and the foregoing groups optionally have a substituent other than R^{E1}.

The heterocyclic group represented by Ar^{E1} includes, for example, groups obtained by removing nE1 hydrogen atoms from the divalent heterocyclic group described above, and it is preferably a group obtained by removing from a carbazolediyl group nE1 hydrogen atoms bonding directly to atoms constituting the ring, and the group optionally has a substituent other than R^{E1}.

Ar^{E1} is preferably an aromatic hydrocarbon group optionally having a substituent other than R^{E1}.

The substituent other than R^{E1} which Ar^{E1} optionally has includes a halogen atom, a cyano group, an alkyl group, a cycloalkyl group, an aryl group, a monovalent heterocyclic group, an alkoxy group, a cycloalkoxy group, an aryloxy group, an amino group, a substituted amino group, an alkenyl group, a cycloalkenyl group, an alkynyl group, a cycloalkynyl group, a carboxyl group and a group represented by the formula (ES-3), and it is preferably an alkyl group, a cycloalkyl group, an aryl group, a monovalent heterocyclic group, an alkoxy group, a cycloalkoxy group or a group represented by the formula (ES-3), more preferably an alkyl group or an aryl group, and the foregoing groups optionally further have a substituent.

-O-(C_{n'}H_{2n'}O)ₙₓ-C_{m'}H_{2m'+1} (ES-3)

[wherein, n', m' and nx each independently represent an integer of 1 or more.]

nE3 is usually an integer of 0 to 10, and it is preferably an integer of 0 to 8, more preferably an integer of 0 to 2, since synthesis of a polymer compound containing a constitutional unit represented by the formula (ET-1) is easy.

aE1 is usually an integer of 1 to 10, preferably an integer of 1 to 5, more preferably 1 or 2.

bE1 is usually an integer of 0 to 10, preferably an integer of 0 to 4, more preferably 0 or 1.

mE1 is usually an integer of 1 to 5, and it is preferably 1 or 2, more preferably 1, since synthesis of a polymer compound containing a constitutional unit represented by the formula (ET-1) is easy.

When R^{E3} is -O-R^{E3'}, the group represented by the formula (ES-1) is a group represented by the following formula.

-O-R^{E3}'-{(Q^{E1})_{nE3}-Y^{E1}(M^{E1})_{aE1}(Z^{E1})_{bE1}}_{mE1}

In R^{E3} and R^{E3}', the number of carbon atoms of a hydrocarbon group is, not including the number of carbon atoms of the substituent, preferably 1 to 20, more preferably 1 to 10, further preferably 1 to 6.

In R^{E3} and R^{E3}', the hydrocarbon group may be an aliphatic hydrocarbon group optionally having a substituent or an aromatic hydrocarbon group optionally having a substituent.

The hydrocarbon group represented by R^{E3} is preferably an aromatic hydrocarbon group optionally having a substituent, since a polymer compound containing a constitutional unit represented by the formula (ET-1) is excellent in electron transportability.

The hydrocarbon group represented by R^{E3}' is preferably an aliphatic hydrocarbon group optionally having a substituent, since synthesis of a polymer compound containing a constitutional unit represented by the formula (ET-1) is easy.

The aliphatic hydrocarbon group as the hydrocarbon group represented by R^{E3} and R^{E3}' includes, for example, groups obtained by removing mE1 hydrogen atoms bonding directly to carbon atoms of the alkyl group described above, and this group optionally has a substituent.

The aromatic hydrocarbon group as the hydrocarbon group represented by R^{E3} and R^{E3}' includes, for example, groups obtained by removing mE1 hydrogen atoms bonding directly to carbon atoms of the aryl group described above, and it is preferably a group obtained by removing mE1 hydrogen atoms bonding directly to carbon atoms of a phenyl group, and the foregoing groups optionally have a substituent.

In R^{E3} and R^{E3}', the number of carbon atoms of a heterocyclic group is, not including the number of carbon atoms of the substituent, usually 2 to 60, preferably 3 to 20, more preferably 3 to 15.

In R^{E3} and R^{E3}', the heterocyclic group is preferably an aromatic heterocyclic group, since synthesis of a polymer compound containing a constitutional unit represented by the formula (ET-1) is easy.

In R^{E3} and R^{E3}', the heterocyclic group includes, for example, groups obtained by removing mE1 hydrogen atoms bonding directly to atoms of the monovalent heterocyclic group described above.

R^{E3} is preferably a hydrocarbon group or a heterocyclic group, more preferably an aromatic hydrocarbon group or an aromatic heterocyclic group, further preferably an aromatic hydrocarbon group, and the foregoing groups optionally have a substituent, since the light emitting device of the present invention is more excellent in luminance life.

R^{E3}' is preferably a hydrocarbon group, more preferably an aliphatic hydrocarbon group, and the foregoing groups optionally have a substituent.

The substituent which R^{E3} and R^{E3}' optionally have is preferably an alkyl group, a cycloalkyl group, an aryl group, a monovalent heterocyclic group and a group represented by the formula (ES-3), more preferably a group represented by the formula (ES-3), and the foregoing groups optionally further have a substituent.

Q^{E1} is preferably an alkylene group, an arylene group or an oxygen atom, more preferably an alkylene group or an oxygen atom.

The examples and preferable range of the alkylene group and the cycloalkylene group represented by Q^{E1} are the same as the examples and preferable range of the alkylene group and the cycloalkylene group represented by G^{ES1} described above, respectively.

Y^{E1} is preferably CO₂⁻, SO₂⁻ or PO₃²⁻, more preferably CO₂⁻.

The alkali metal cation represented by M^{E1} includes, for example, Li⁺, Na⁺, K⁺, Rb⁺ and Cs⁺, and it is preferably Li⁺, Na⁺, K⁺ or Cs⁺, more preferably Li⁺, Na⁺ or Cs⁺, further preferably Cs⁺.

The periodic table group 2 metal cation represented by M^{E1} includes, for example, Be²⁺, Mg²⁺, Ca²⁺, Sr²⁺ and Ba²⁺, and it is preferably Be²⁺, Mg²⁺, Ca²⁺ or Ba²⁺, more preferably Ca²⁺ or Ba²⁺.

M^{E1} is an alkali metal cation, since the light emitting device of the present invention is more excellent in luminance life.

Z^{E1} is preferably F⁻, Cl⁻, Br⁻, I⁻, OH⁻, B(R^{E4})₄⁻, R^{E4}SO₃⁻, R^{E4}COO⁻ or NO₃⁻, more preferably F⁻, Cl⁻, Br⁻, I⁻, OH⁻, R^{E4}SO₃⁻ or R^{E4}COO⁻. R^{E4} is preferably an alkyl group.

The group represented by the formula (ES-1) includes, for example, groups represented by the following formulae. 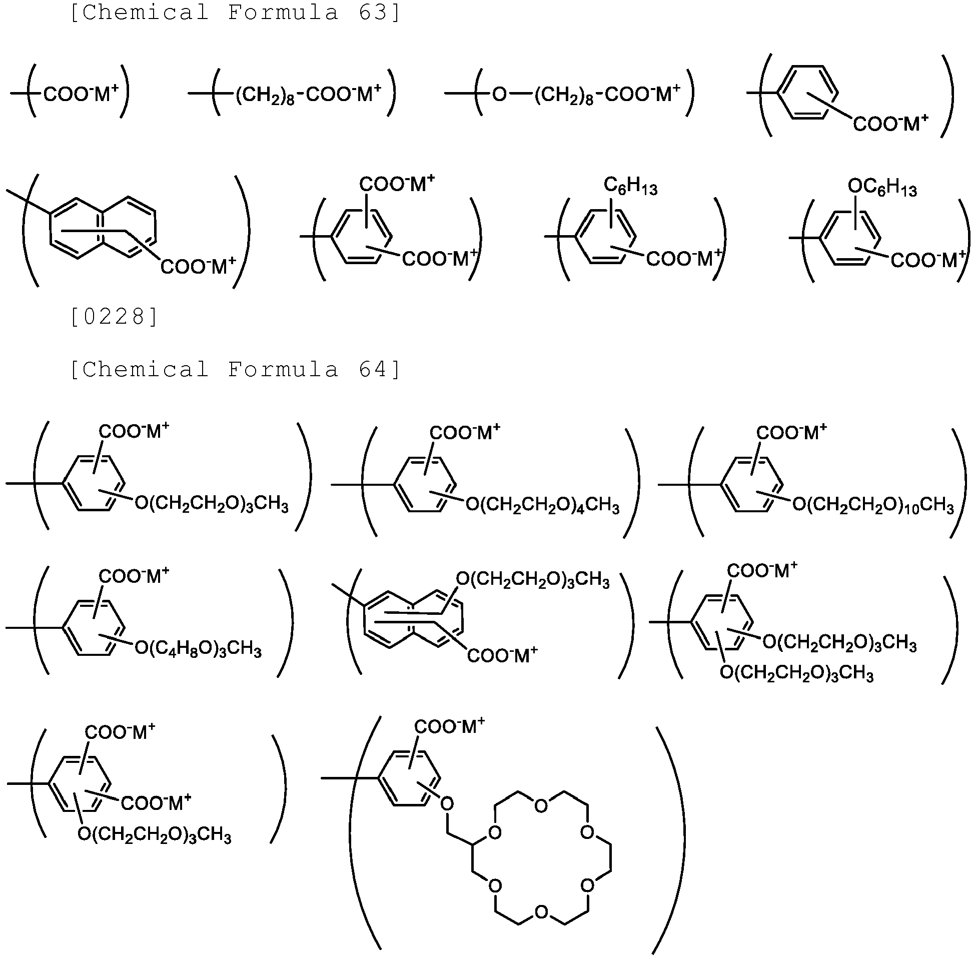 [wherein, M⁺ represents Li⁺, Na⁺, K⁺ or Cs⁺. When a plurality of M⁺ are present, they may be the same or different.]

The constitutional unit represented by the formula (ET-1) includes, for example, constitutional units represented by the following formulae.

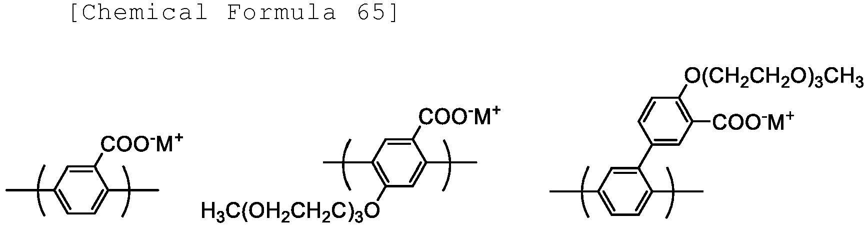

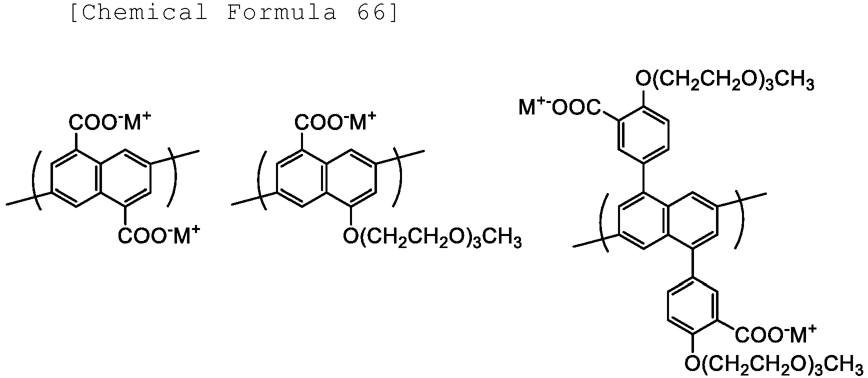

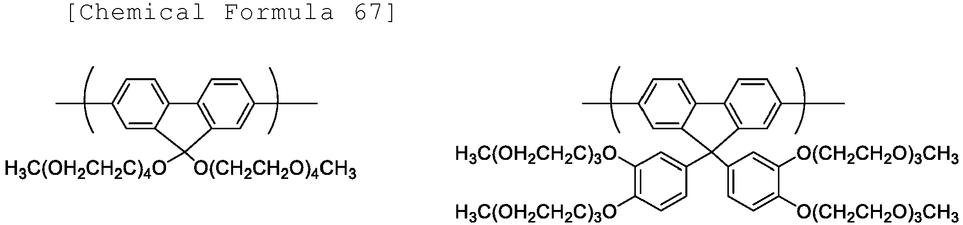

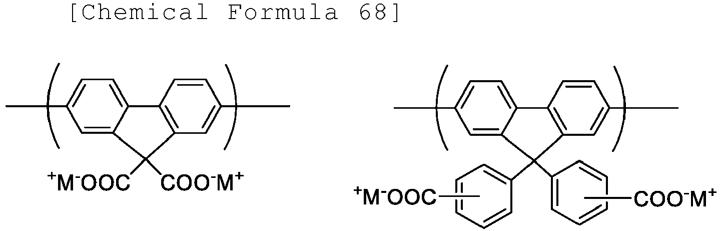

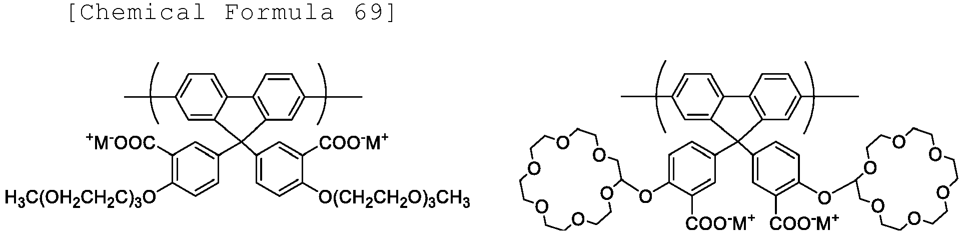

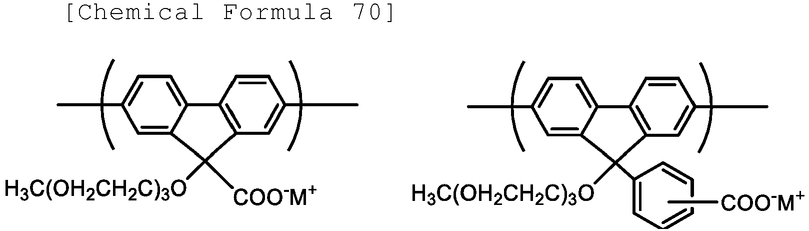

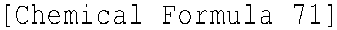

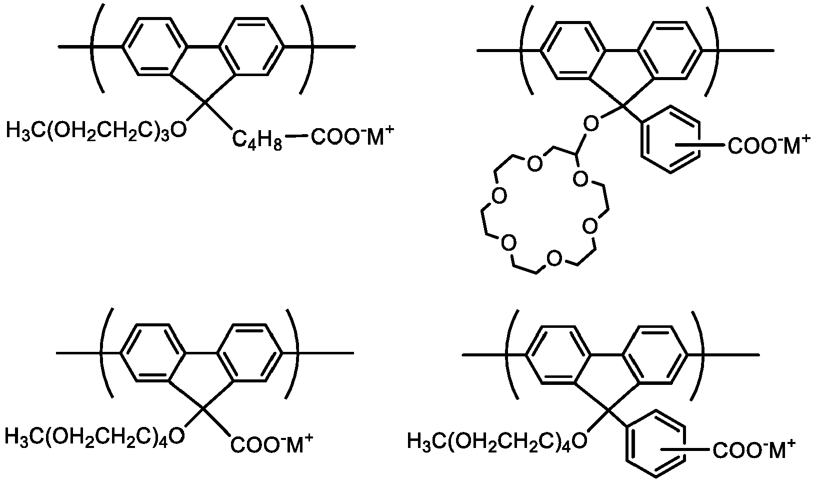

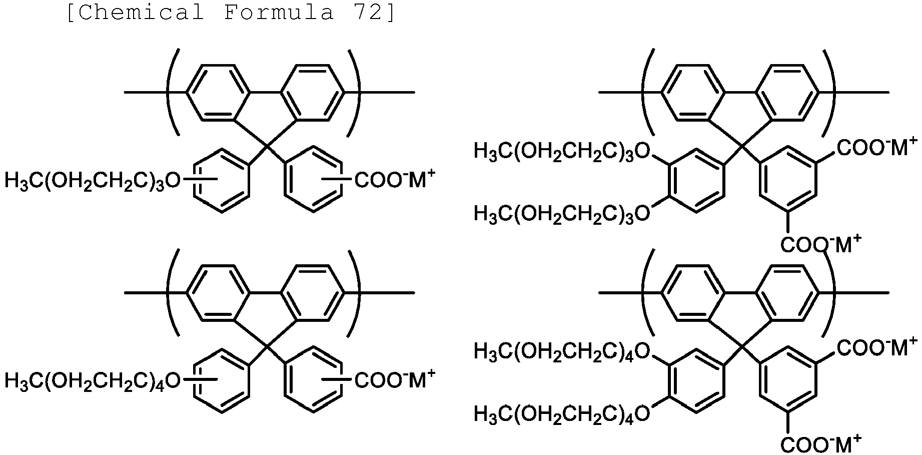

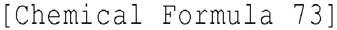

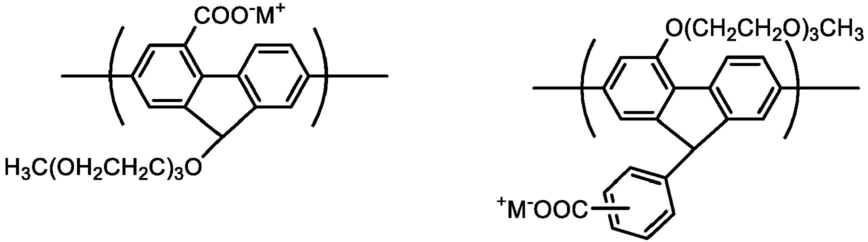

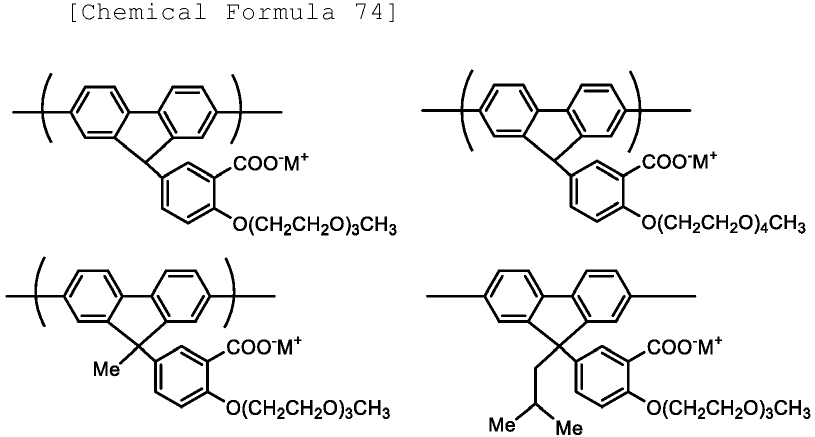

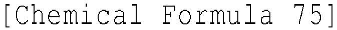

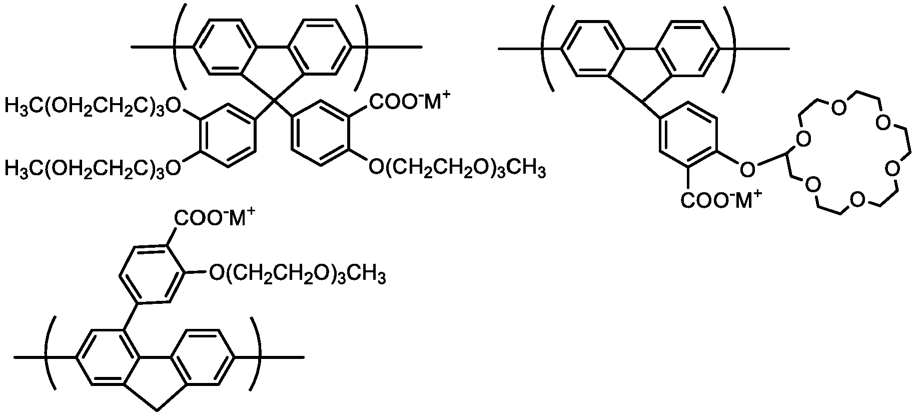

The polymer compound containing a constitutional unit represented by the formula (ET-1) preferably further contains at least one constitutional unit selected from the group consisting of a constitutional unit represented by the formula (Y) and a constitutional unit represented by the formula (X), more preferably contains a constitutional unit represented by the formula (Y), since the light emitting device of the present invention is more excellent in luminance life. 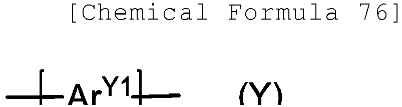 [wherein, Ar^{Y1} represents an arylene group, a divalent heterocyclic group, or a divalent group in which at least one arylene group and at least one divalent heterocyclic group are bonded directly, and the foregoing groups optionally have a substituent.]

The arylene group represented by Ar^{Y1} is more preferably a group represented by the formula (A-1), the formula (A-2), the formulae (A-6)-(A-10), the formula (A-19) or the formula (A-20), further preferably a group represented by the formula (A-1), the formula (A-2), the formula (A-7), the formula (A-9) or the formula (A-19), and the foregoing groups optionally have a substituent.

The divalent heterocyclic group represented by Ar^{Y1} is more preferably a group represented by the formulae (AA-1)-(AA-4), the formula (AA-10)-(AA-15), the formulae (AA-18)-(AA-21), the formula (AA-33) or the formula (AA-34), further preferably a group represented by the formula (AA-4), the formula (AA-10), the formula (AA-12), the formula (AA-14) or the formula (AA-33), and the foregoing groups optionally have a substituent.

In the divalent group in which at least one arylene group and at least one divalent heterocyclic group are bonded directly represented by Ar^{Y1}, the more preferable range and the further preferable range of the arylene group and the divalent heterocyclic group are the same as the more preferable range and the further preferable range of the arylene group and the divalent heterocyclic group represented by Ar^{Y1} described above, respectively.

"The divalent group in which at least one arylene group and at least one divalent heterocyclic group are bonded directly" includes, for example, groups represented by the following formulae, and these groups optionally have a substituent. 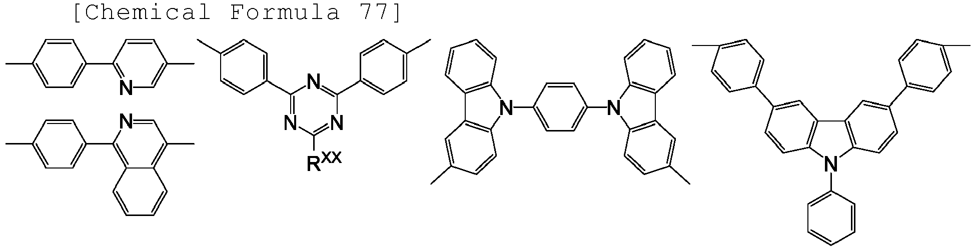 [wherein, R^{XX} represents a hydrogen atom, an alkyl group, a cycloalkyl group, an aryl group or a monovalent heterocyclic group, and the foregoing groups optionally have a substituent.]

R^{XX} is preferably an alkyl group, a cycloalkyl group or an aryl group, and the foregoing groups optionally have a substituent.

The substituent which the group represented by Ar^{Y1} optionally has is preferably an alkyl group, a cycloalkyl group or an aryl group, and the foregoing groups optionally further have a substituent.

The constitutional unit represented by the formula (Y) includes, for example, constitutional units represented by the formulae (Y-1)-(Y-10), and it is preferably a constitutional unit represented by the formulae (Y-1)-(Y-3) from the standpoint of the luminance life of the light emitting device, preferably a constitutional unit represented by the formulae (Y-4)-(Y-7) from the standpoint of electron transportability, and preferably a constitutional unit represented by the formulae (Y-8)-(Y-10) from the standpoint of hole transportability. 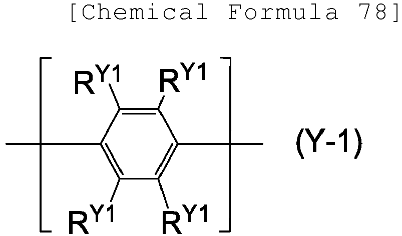 [wherein, R^{Y1} represents a hydrogen atom, an alkyl group, a cycloalkyl group, an alkoxy group, a cycloalkoxy group, an aryl group or a monovalent heterocyclic group, and the foregoing groups optionally have a substituent. A plurality of R^{Y1} may be the same or different, and adjacent groups R^{Y1} may be combined together to form a ring together with the carbon atoms to which they are attached.]

R^{Y1} is preferably a hydrogen atom, an alkyl group, a cycloalkyl group or an aryl group, and the foregoing groups optionally have a substituent.

The constitutional unit represented by the formula (Y-1) is preferably a constitutional unit represented by the formula (Y-1'). 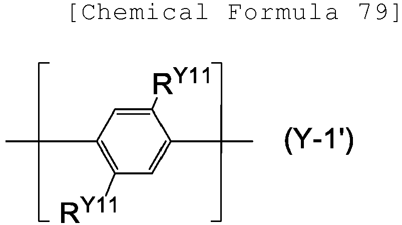 [wherein, R^{Y11} represents an alkyl group, a cycloalkyl group, an alkoxy group, a cycloalkoxy group, an aryl group or a monovalent heterocyclic group, and the foregoing groups optionally have a substituent. A plurality of R^{Y11} may be the same or different.]

R^{Y11} is preferably an alkyl group, a cycloalkyl group or an aryl group, more preferably an alkyl group or a cycloalkyl group, and the foregoing groups optionally have a substituent. 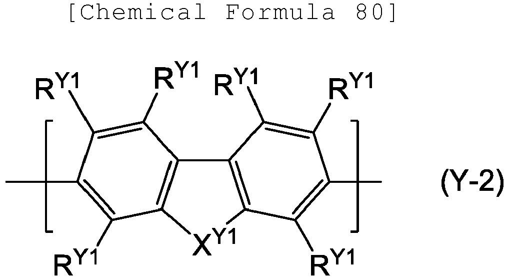 [wherein,
R^{Y1} represent the same meaning as described above.
X^{Y1} represents a group represented by -C(R^{Y2})₂-, - C(R^{Y2})=C(R^{Y2})- or -C(R^{Y2})₂-C(R^{Y2})₂-. R^{Y2} represents a hydrogen atom, an alkyl group, a cycloalkyl group, an alkoxy group, a cycloalkoxy group, an aryl group or a monovalent heterocyclic group, and the foregoing groups optionally have a substituent. A plurality of R^{Y2} may be the same or different, and groups R^{Y2} may be combined together to form a ring together with the carbon atoms to which they are attached.]

R^{Y2} is preferably an alkyl group, a cycloalkyl group, an aryl group or a monovalent heterocyclic group, more preferably an alkyl group, a cycloalkyl group or an aryl group, and the foregoing groups optionally have a substituent.

Regarding the combination of two groups R^{Y2} in the group represented by -C(R^{Y2})₂- represented by X^{Y1}, it is preferable that both are alkyl groups, both are aryl groups, both are monovalent heterocyclic groups, or one is an alkyl group and the other is an aryl group or a monovalent heterocyclic group, and the foregoing groups optionally have a substituent. Two groups R^{Y2} may be combined together to form a ring together with the atoms to which they are attached, and when groups R^{Y2} form a ring, the group represented by -C(R^{Y2})₂- is preferably a group represented by the formulae (Y-A1)-(Y-A5), more preferably a group represented by the formula (Y-A4), and the foregoing groups optionally have a substituent.

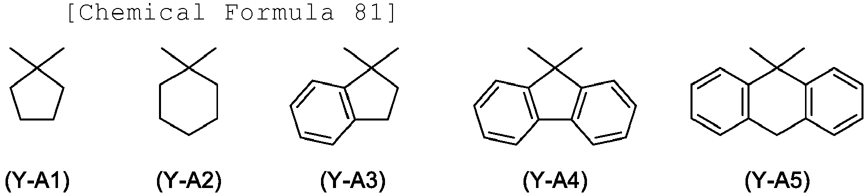

Regarding the combination of two groups R^{Y2} in the group represented by -C(R^{Y2})=C(R^{Y2})- represented by X^{Y1}, it is preferable that both are alkyl groups or cycloalkyl groups, or one is an alkyl group or a cycloalkyl group and the other is an aryl group, and the foregoing groups optionally have a substituent.

Four groups R^{Y2} in the group represented by - C(R^{Y2})₂-C(R^{Y2})₂- represented by X^{Y1} are preferably alkyl groups or cycloalkyl groups optionally having a substituent. A plurality of R^{Y2} may be combined together to form a ring together with the atoms to which they are attached, and when groups R^{Y2} form a ring, the group represented by -C(R^{Y2})₂-C(R^{Y2})₂- is preferably a group represented by the formulae (Y-B1)-(Y-B5), more preferably a group represented by the formula (Y-B3), and the foregoing groups optionally have a substituent. 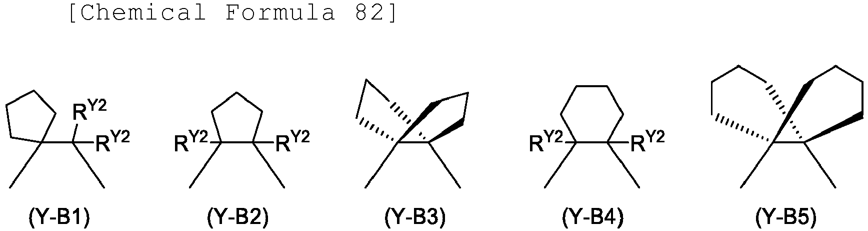 [wherein, R^{Y2} represent the same meaning as described above.]

The constitutional unit represented by the formula (Y-2) is preferably a constitutional unit represented by the formula (Y-2'). 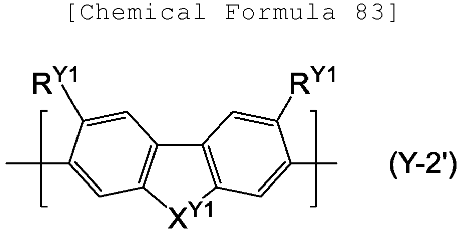 [wherein, R^{Y1} and X^{Y1} represent the same meaning as described above.] 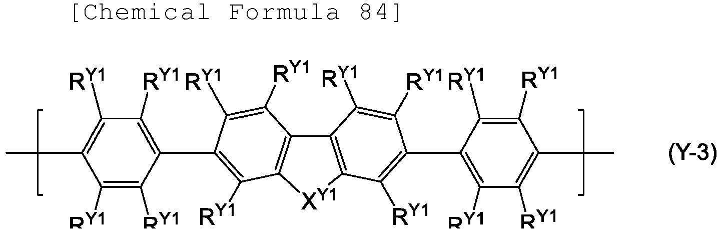 [wherein, R^{Y1} and X^{Y1} represent the same meaning as described above.]

The constitutional unit represented by the formula (Y-3) is preferably a constitutional unit represented by the formula (Y-3'). 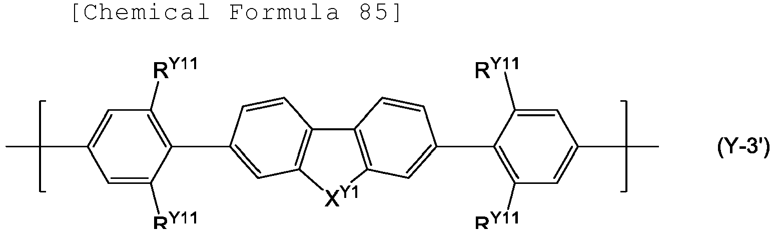 [wherein, R^{Y11} and X^{Y1} represent the same meaning as described above.] 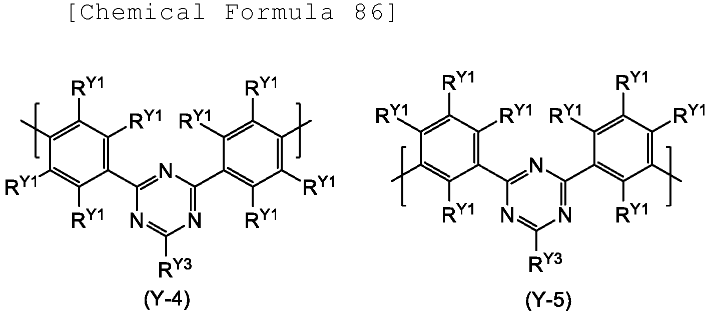 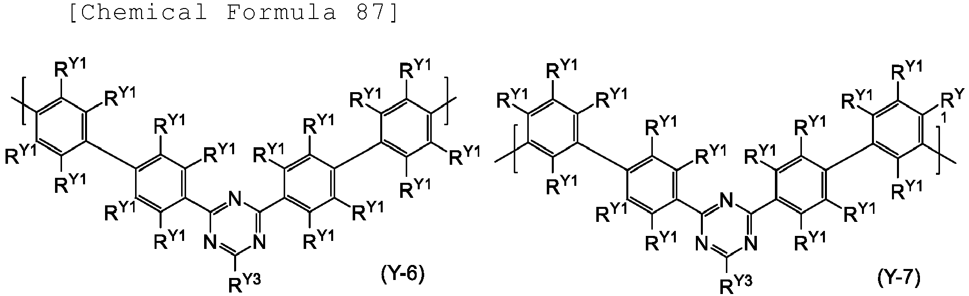 [wherein, R^{Y1} represents the same meaning as described above. R^{Y3} represents a hydrogen atom, an alkyl group, a cycloalkyl group, an alkoxy group, a cycloalkoxy group, an aryl group or a monovalent heterocyclic group, and the foregoing groups optionally have a substituent.]

R^{Y3} is preferably an alkyl group, a cycloalkyl group, an alkoxy group, a cycloalkoxy group, an aryl group or a monovalent heterocyclic group, more preferably an aryl group, and the foregoing groups optionally have a substituent.

The constitutional unit represented by the formula (Y-4) is preferably a constitutional unit represented by the formula (Y-4'), and the constitutional unit represented by the formula (Y-6) is preferably a constitutional unit represented by the formula (Y-6'). 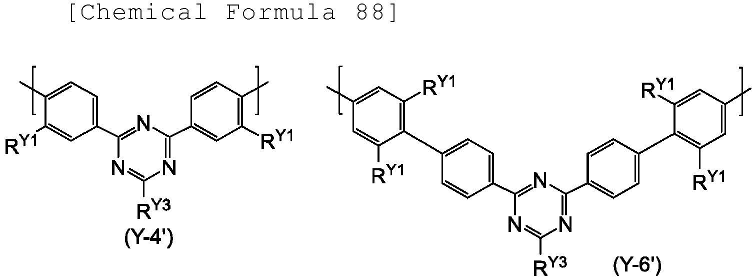 [wherein, R^{Y1} and R^{Y3} represent the same meaning as described above.] 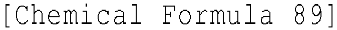 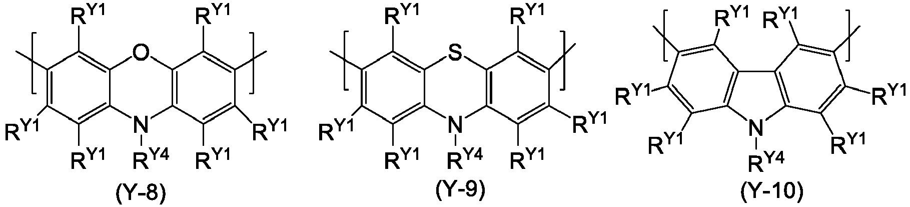 [wherein, R^{Y1} represents the same meaning as described above. R^{Y4} represents a hydrogen atom, an alkyl group, a cycloalkyl group, an alkoxy group, a cycloalkoxy group, an aryl group or a monovalent heterocyclic group, and the foregoing groups optionally have a substituent.]

R^{Y4} is preferably an alkyl group, a cycloalkyl group, an alkoxy group, a cycloalkoxy group, an aryl group or a monovalent heterocyclic group, more preferably an aryl group, and the foregoing groups optionally have a substituent.

The constitutional unit represented by the formula (Y) includes, for example, constitutional units composed of an arylene group represented by the formula (Y-101) to the formula (Y-121), constitutional units composed of a divalent heterocyclic group represented by the formula (Y-201) to the formula (Y-206), and constitutional units composed of a divalent group in which at least one arylene group and at least one divalent heterocyclic group are bonded directly represented by the formula (Y-300) to the formula (Y-304) .

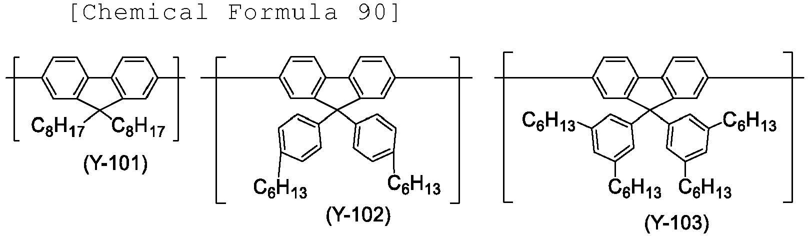

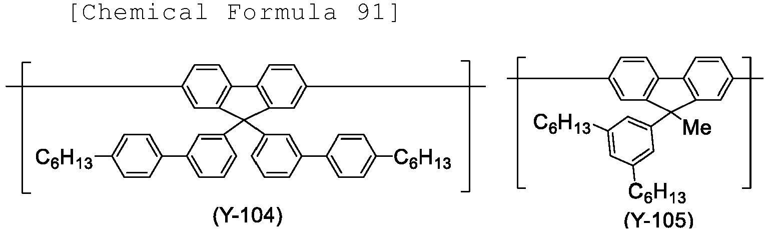

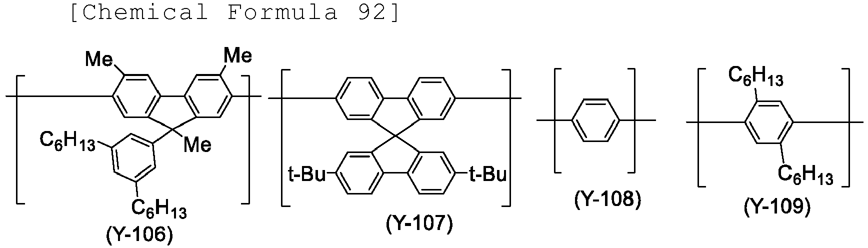

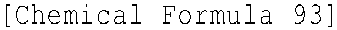

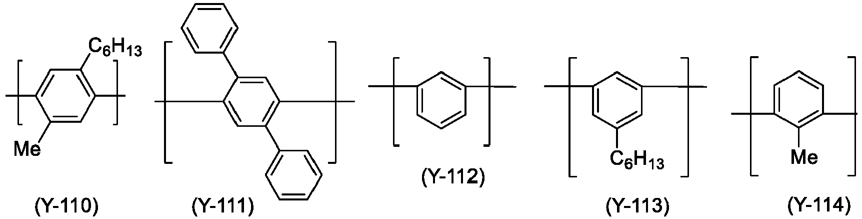

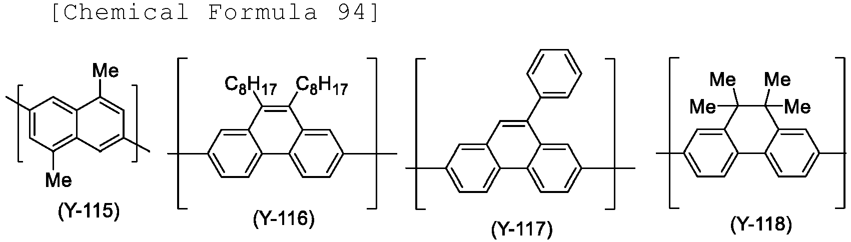

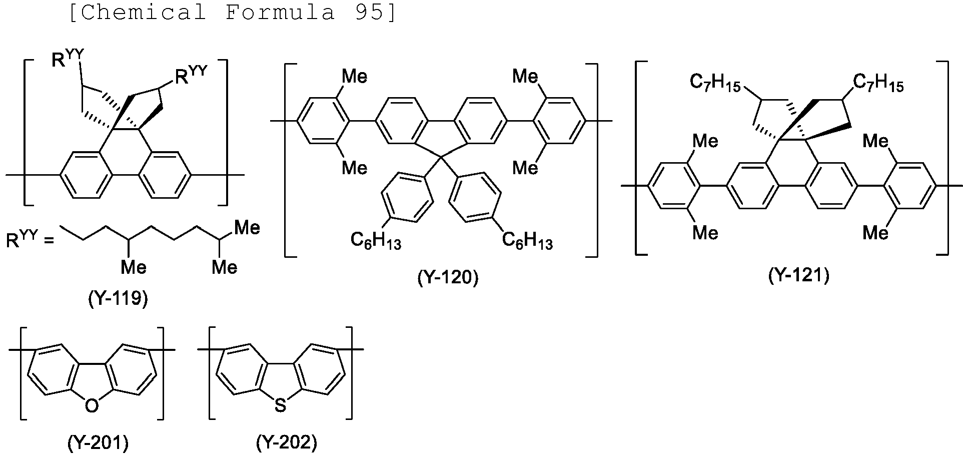

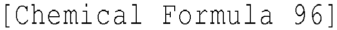

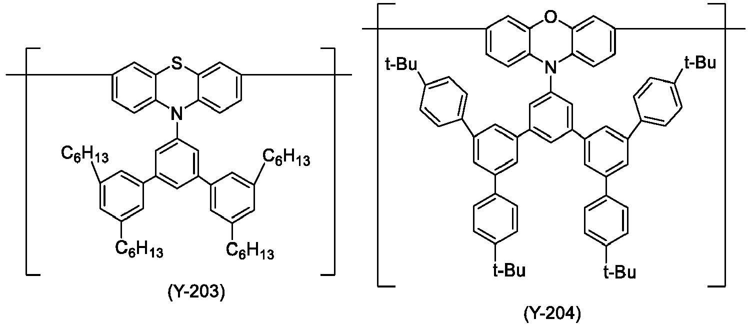

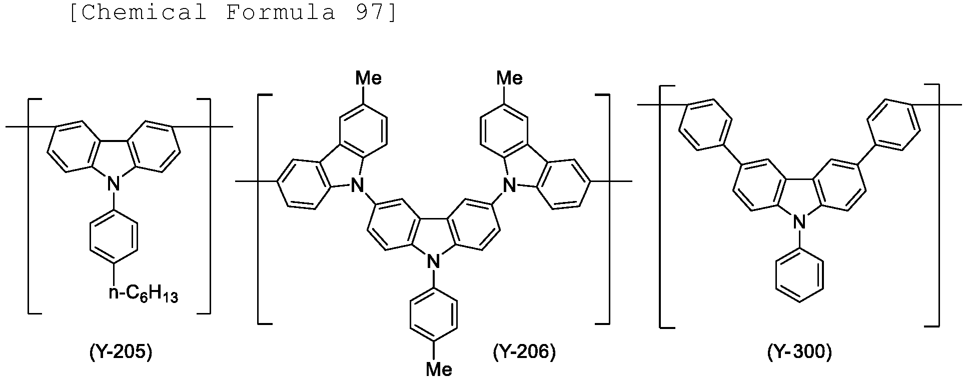

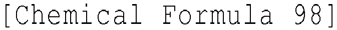

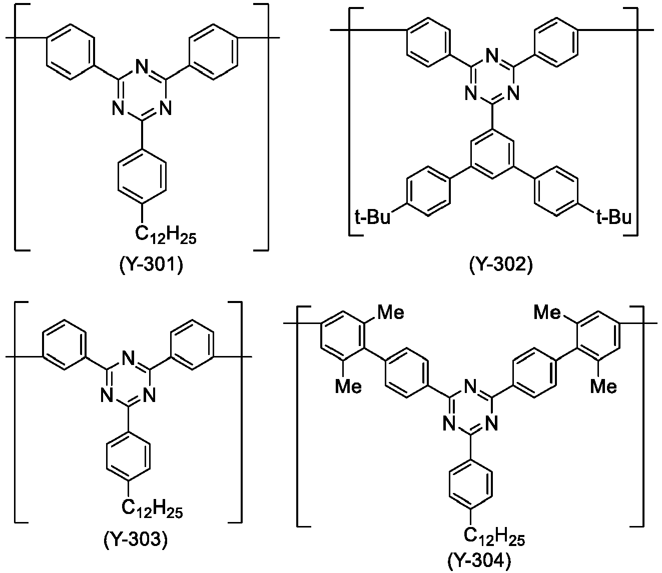

The amount of the constitutional unit represented by the formula (Y) in which Ar^{Y1} is an arylene group is preferably 0.5 to 80% by mol, more preferably 30 to 60% by mol, with respect to the total amount of constitutional units contained in a polymer compound containing a constitutional unit represented by the formula (ET-1), since the light emitting device is more excellent in luminance life.

The amount of the constitutional unit represented by the formula (Y) in which Ar^{Y1} is a divalent heterocyclic group or a divalent group in which at least one arylene group and at least one divalent heterocyclic group are bonded directly is preferably 0.5 to 30% by mol, more preferably 3 to 20% by mol, with respect to the total amount of constitutional units contained in a polymer compound containing a constitutional unit represented by the formula (ET-1), since the light emitting device is excellent in charge transportability.

The constitutional unit represented by the formula (Y) may be contained only singly or in combination of two or more in the polymer compound containing a constitutional unit represented by the formula (ET-1). 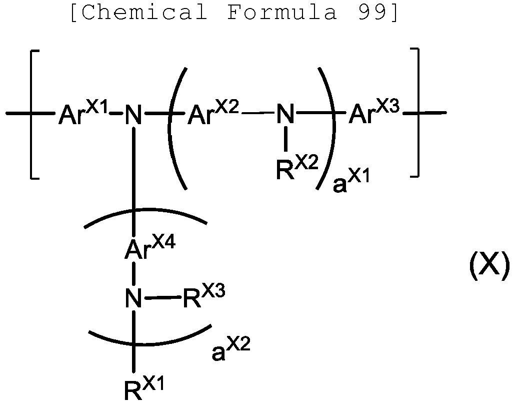 [wherein,
a^{X1} and a^{X2} each independently represent an integer of 0 or more. Ar^{X1} and Ar^{X3} each independently represent an arylene group or a divalent heterocyclic group, and the foregoing groups optionally have a substituent.
Ar^{X2} and Ar^{X4} each independently represent an arylene group, a divalent heterocyclic group, or a divalent group in which at least one arylene group and at least one divalent heterocyclic group are bonded directly, and the foregoing groups optionally have a substituent. When a plurality of Ar^{X2} and Ar^{X4} are present, they may be the same or different at each occurrence.
R^{X1}, R^{X2} and R^{X3} each independently represent a hydrogen atom, an alkyl group, a cycloalkyl group, an aryl group or a monovalent heterocyclic group, and the foregoing groups optionally have a substituent. When a plurality of R^{X2} and R^{X3} are present, they may be the same or different at each occurrence.]

a^{X1} is preferably 2 or less, more preferably 1, since the light emitting device is more excellent in luminance life.

a^{X2} is preferably 2 or less, more preferably 0, since the light emitting device is more excellent in luminance life.

R^{X1}, R^{X2} and R^{X3} are each preferably an alkyl group, a cycloalkyl group, an aryl group or a monovalent heterocyclic group, more preferably an aryl group, and the foregoing groups optionally have a substituent.

The arylene group represented by Ar^{X1} and Ar^{X3} is more preferably a group represented by the formula (A-1) or the formula (A-9), further preferably a group represented by the formula (A-1), and the foregoing groups optionally have a substituent.

The divalent heterocyclic group represented by Ar^{X1} and Ar^{X3} is more preferably a group represented by the formula (AA-1), the formula (AA-2) or the formulae (AA-7)-(AA-26), and the foregoing groups optionally have a substituent.

Ar^{X1} and Ar^{X3} are each preferably an arylene group optionally having a substituent.

The arylene group represented by Ar^{X2} and Ar^{X4} is more preferably a group represented by the formula (A-1), the formula (A-6), the formula (A-7), the formulae (A-9)-(A-11) or the formula (A-19), and the foregoing groups optionally have a substituent.

The more preferable range of the divalent heterocyclic group represented by Ar^{X2} and Ar^{X4} is the same as the more preferable range of the divalent heterocyclic group represented by Ar^{X1} and Ar^{X3}.

In the divalent group in which at least one arylene group and at least one divalent heterocyclic group are bonded directly represented by Ar^{X2} and Ar^{X4}, the more preferable range and the further preferable range of the arylene group and the divalent heterocyclic group are the same as the more preferable range and the further preferable range of the arylene group and the divalent heterocyclic group represented by Ar^{X1} and Ar^{X3}, respectively.

The divalent group in which at least one arylene group and at least one divalent heterocyclic group are bonded directly represented by Ar^{X2} and Ar^{X4} includes those that are the same as the divalent group in which at least one arylene group and at least one divalent heterocyclic group are bonded directly represented by Ar⁷¹ in the formula (Y).

Ar^{X2} and Ar^{X4} are each preferably an arylene group optionally having a substituent.

The substituent which the groups represented by Ar^{X1} to Ar^{X4} and R^{X1} to R^{X3} optionally has is preferably an alkyl group, a cycloalkyl group or an aryl group, and the foregoing groups optionally further have a substituent.

The constitutional unit represented by the formula (X) is preferably a constitutional unit represented by the formulae (X-1)-(X-7), more preferably a constitutional unit represented by the formulae (X-1)-(X-6), further preferably a constitutional unit represented by the formulae (X-3)-(X-6). 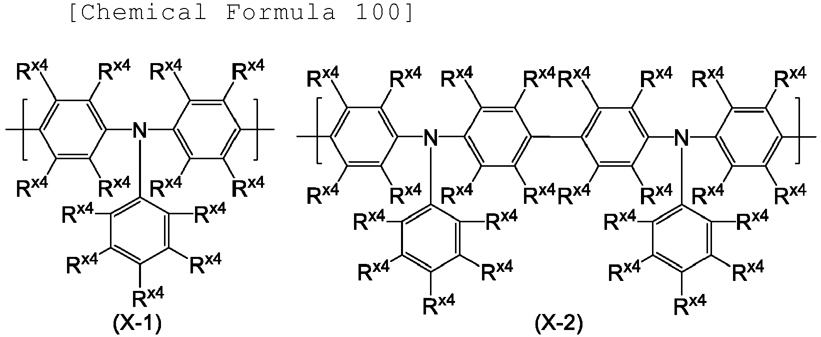 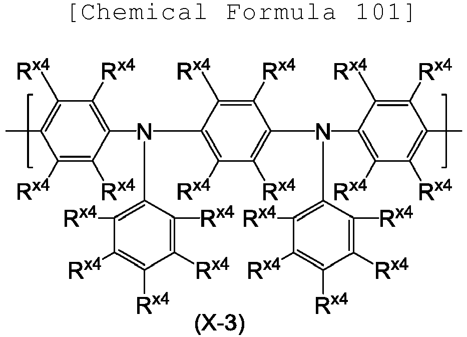 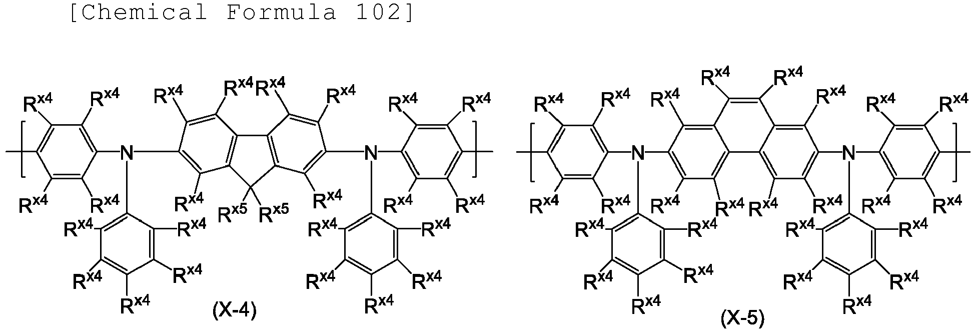 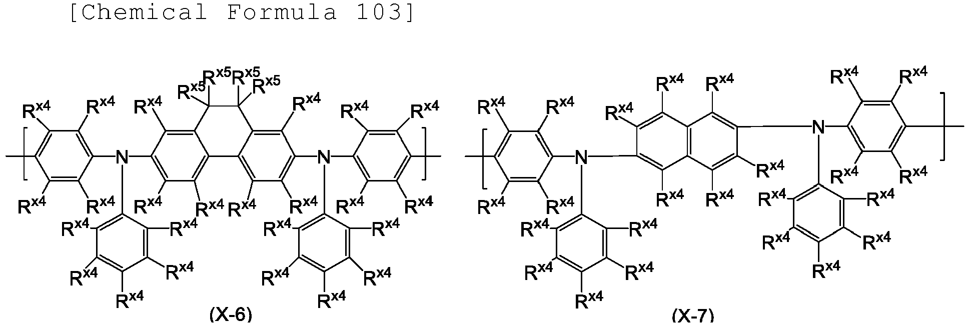 [wherein, R^{X4} and R^{X5} each independently represent a hydrogen atom, an alkyl group, a cycloalkyl group, an alkoxy group, a cycloalkoxy group, an aryl group, an aryloxy group, a halogen atom, a monovalent heterocyclic group or a cyano group, and the foregoing groups optionally have a substituent. A plurality of R^{X4} may be the same or different. A plurality of R^{X5} may be the same or different, and adjacent groups R^{X5} may be combined together to form a ring together with the carbon atoms to which they are attached.]

The amount of the constitutional unit represented by the formula (X) is preferably 0.1 to 50% by mol, more preferably 1 to 40% by mol, further preferably 5 to 30% by mol, with respect to the total amount of constitutional units contained in a polymer compound containing a constitutional unit represented by the formula (ET-1), since the light emitting device is more excellent in luminance life.

The constitutional unit represented by the formula (X) includes, for example, constitutional units represented by the formula (X1-1) to the formula (X1-11), and it is preferably a constitutional unit represented by the formula (X1-3) to the formula (X1-10) .

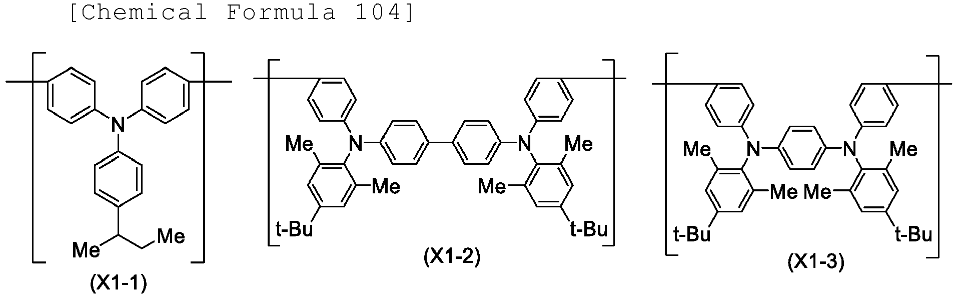

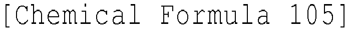

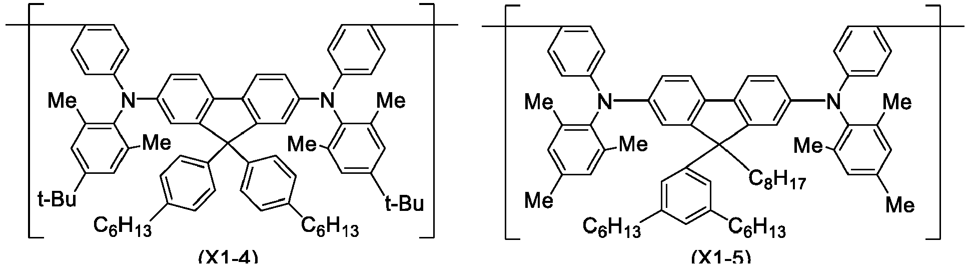

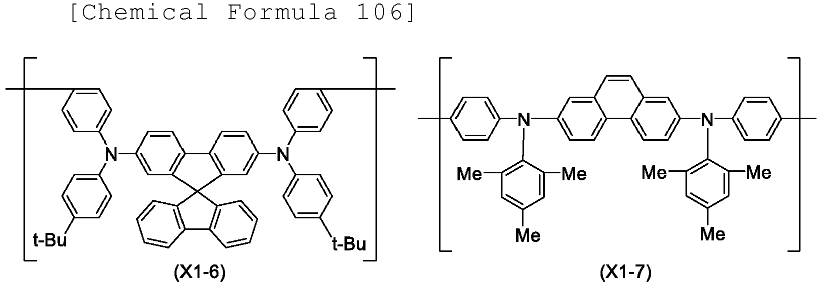

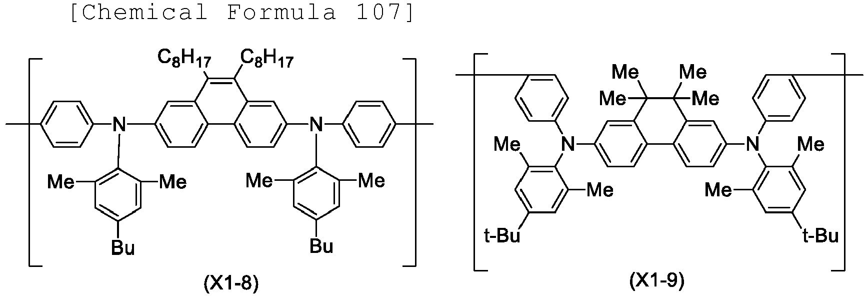

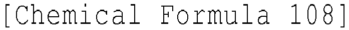

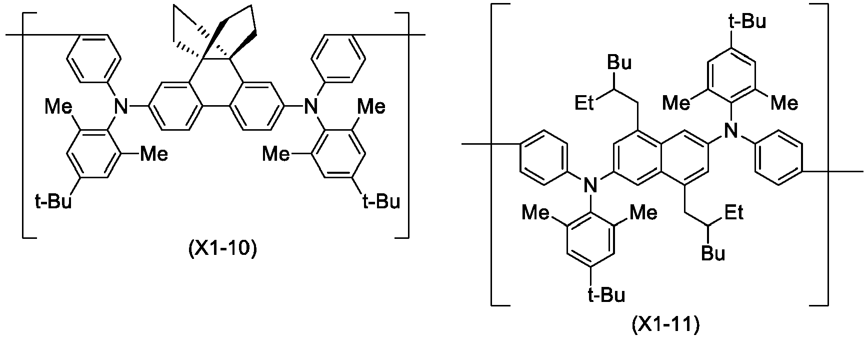

The constitutional unit represented by the formula (X) may be contained only singly or in combination of two or more in the polymer compound containing a constitutional unit represented by the formula (ET-1).

The polymer compound containing a constitutional unit represented by the formula (ET-1) includes, for example, polymer compounds P-1 to P-14. "Other" constitutional unit denotes a constitutional unit other than the constitutional units represented by the formula (ET-1), the formula (X) and the formula (Y).

**[Table 1]**

| polymer compound | constitutional unit and mole fraction thereof | | | | | |
|---|---|---|---|---|---|---|
| | the formula (ET-1) | the formula (Y) | | | the formula (X) | other |
| | | (Y-1) to (Y-3) | (Y-4) to (Y-7) | (Y-8) to (Y-10) | (X-1) to (X-7) | |
| | p' | q' | r' | s' | t' | u' |
| P-1 | 70 to 100 | 0 | 0 | 0 | 0 | 0 to 30 |
| P-2 | 0.1 to 99.9 | 0.1 99.9 | 0 | 0 | 0 | 0 to 30 |
| P-3 | 0.1 to 99.9 | 0 | 0.1 to 99.9 | 0 | 0 | 0 to 30 |
| P-4 | 0.1 to 99.9 | 0 | 0 | 0.1 99.9 | 0 | 0 to 30 |
| P-5 | 0.1 to 99.8 | 0.1 to 99.8 | 0.1 to 99.8 | 0 | 0 | 0 to 30 |
| P-6 | 0.1 to 99.8 | 0.1 99.8 | 0 | 0.1 99.8 | 0 | 0 to 30 |
| P-7 | 0.1 to 99.8 | 0 | 0.1 to 99.8 | 0.1 to 99.8 | 0 | 0 to 30 |
| P-8 | 0.1 to 99.8 | 0.1 to 99.8 | 0 | 0 | 0.1 to 99.8 | 0 to 30 |
| P-9 | 0.1 to 99.8 | 0 | 0.1 to 99.8 | 0 | 0.1 to 99.8 | 0 to 30 |
| P-10 | 0.1 to 99.8 | 0 | 0 | 0.1 to 99.8 | 0.1 to 99.8 | 0 to 30 |
| P-11 | 0.1 to 99.7 | 0.1 to 99.7 | 0.1 to 99.7 | 0 | 0.1 to 99.7 | 0 to 30 |
| P-12 | 0.1 to 99.7 | 0.1 to 99.7 | 0 | 0.1 to 99.7 | 0.1 to 99.7 | 0 to 30 |
| P-13 | 0.1 to 99.7 | 0 | 0.1 to 99.7 | 0.1 to 99.7 | 0.1 to 99.7 | 0 to 30 |
| P-14 | 0.1 to 99.6 | 0.1 to 99.6 | 0.1 to 99.6 | 0.1 to 99.6 | 0.1 to 99.6 | 0 to 30 |

| | | | | | | |
|---|---|---|---|---|---|---|
| [in the table, p', q', r', s', t' and u' represent the mole fraction of each constitutional unit. p'+q'+r'+s'+t'+u' = 100, and 100 ≥ p'+q'+r'+s'+t' ≥ 70.] | | | | | | |

The polymer compound containing a constitutional unit represented by the formula (ET-1) can be synthesized according to methods described, for example, in Japanese Unexamined Patent Application Publication (JP-A) No. 2009-239279, JP-A No. 2012-033845, JP-A No. 2012-216821, JP-A No. 2012-216822 and JP-A No. 2012-216815.

### [Second layer]

The second layer which the light emitting device of the present invention has is preferably a layer containing at least one selected from the group consisting of a compound containing an alkali metal element and a compound containing a group 2 element, more preferably a layer containing a compound containing an alkali metal element, since luminance life is more excellent.

In the second layer, the compound containing an alkali metal element is preferably a metal complex containing an alkali metal element, an inorganic compound containing an alkali metal element or a polymer compound containing an alkali metal element, since the light emitting device of the present invention is more excellent in luminance life. In the second layer, the compound containing an alkali metal element is preferably a polymer compound containing an alkali metal element, since the light emitting device of the present invention can be fabricated by an application method.

In the second layer, the compound containing a group 2 element is preferably a metal complex containing a group 2 element, an inorganic compound containing a group 2 element or a polymer compound containing a group 2 element, since the light emitting device of the present invention is more excellent in luminance life. In the second layer, the compound containing a group 2 element is preferably a polymer compound containing a group 2 element, since the light emitting device of the present invention can be fabricated by an application method.

The second layer is preferably a layer containing at least one selected from the group consisting of a simple substance composed only of an alkali metal element, a simple substance composed only of a group 2 element, a compound containing an alkali metal element and a compound containing a group 2 element, and a compound represented by the formula (H-1) (hereinafter, referred to as "the second' layer"), since the light emitting device of the present invention is more excellent in luminance life.

The second' layer is preferably a layer containing at least one selected from the group consisting of a compound containing an alkali metal element and a compound containing a group 2 element, and a compound represented by the formula (H-1), more preferably a layer containing a compound containing an alkali metal element, and a compound represented by the formula (H-1), since the light emitting device of the present invention is further excellent in luminance life.

In the second' layer, the compound containing an alkali metal element is preferably a metal complex containing an alkali metal element, an inorganic compound containing an alkali metal element or a polymer compound containing an alkali metal element, more preferably a metal complex containing an alkali metal element, since the light emitting device of the present invention is more excellent in luminance life. In the second' layer, the compound containing a group 2 element is preferably a metal complex containing a group 2 element, an inorganic compound containing a group 2 element or a polymer compound containing a group 2 element, more preferably a metal complex containing an alkali metal element, since the light emitting device of the present invention is more excellent in luminance life.

In the second' layer, the compound represented by the formula (H-1) may be contained singly or in combination of two or more.

In the second' layer, the content of the compound represented by the formula (H-1) is usually 1 to 99 parts by mass, preferably 10 to 95 parts by mass, more preferably 50 to 90 parts by mass when the sum of a simple substance composed only of an alkali metal element, a simple substance composed only of a group 2 element, a compound containing an alkali metal element and a compound containing a group 2 element and a compound represented by the formula (H-1) is taken as 100 parts by mass. 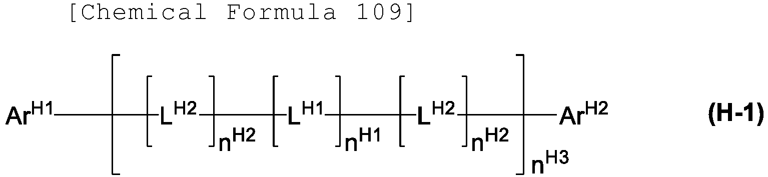 [wherein,
Ar^{H1} and Ar^{H2} each independently represent an aryl group or a monovalent heterocyclic group, and the foregoing groups optionally have a substituent.
n^{H1} and n^{H2} each independently represent 0 or 1. When a plurality of n^{H1} are present, they may be the same or different. A plurality of n^{H2} may be the same or different.
n^{H3} represents an integer of 0 or more.
L^{H1} represents an arylene group, a divalent heterocyclic group or a group represented by - [C(R^{H11})₂]n^{H11}-, and the foregoing groups optionally have a substituent. When a plurality of L^{H1} are present, they may be the same or different. n^{H11} represents an integer of 1 or more and 10 or less. R^{H11} represents a hydrogen atom, an alkyl group, a cycloalkyl group, an alkoxy group, a cycloalkoxy group, an aryl group or a monovalent heterocyclic group, and the foregoing groups optionally have a substituent. A plurality of R^{H11} may be the same or different, and may be combined together to form a ring together with the carbon atoms to which they are attached.
L^{H2} represents a group represented by -N(-L^{H21}-R^{H21})-. When a plurality of L^{H2} are present, they may be the same or different. L^{H21} represents a single bond, an arylene group or a divalent heterocyclic group, and the foregoing groups optionally have a substituent. R^{H21} represents a hydrogen atom, an alkyl group, a cycloalkyl group, an aryl group or a monovalent heterocyclic group, and the foregoing groups optionally have a substituent.]

Ar^{H1} and Ar^{H2} are each preferably a phenyl group, a fluorenyl group, a spirobifluorenyl group, a pyridyl group, a pyrimidinyl group, a triazinyl group, a quinolinyl group, an isoquinolinyl group, a thienyl group, a benzothienyl group, a dibenzothienyl group, a furyl group, a benzofuryl group, a dibenzofuryl group, a pyrrolyl group, an indolyl group, an azaindolyl group, a carbazolyl group, an azacarbazolyl group, a diazacarbazolyl group, a phenoxazinyl group or a phenothiazinyl group, more preferably a phenyl group, a pyridyl group, a pyrimidinyl group, a triazinyl group, a dibenzothienyl group, a dibenzofuryl group, a carbazolyl group, an azacarbazolyl group or a diazacarbazolyl group, further preferably an azacarbazolyl group or a diazacarbazolyl group, and the foregoing groups optionally have a substituent.

The substituent which Ar^{H1} and Ar^{H2} optionally have is preferably a halogen atom, an alkyl group, a cycloalkyl group, an alkoxy group, a cycloalkoxy group, an aryl group or a monovalent heterocyclic group, more preferably an alkyl group, a cycloalkyl group, an alkoxy group or a cycloalkoxy group, further preferably an alkyl group or a cycloalkyl group, and the foregoing groups optionally further have a substituent.

n^{H1} is preferably 1. n^{H2} is preferably 0.

n^{H3} is usually an integer of 0 or more and 10 or less, preferably an integer of 0 or more and 5 or less, further preferably an integer of 1 or more and 3 or less, particularly preferably 1.

n^{H11} is preferably an integer of 1 or more and 5 or less, more preferably an integer of 1 or more and 3 or less, further preferably 1.

R^{H11} is preferably a hydrogen atom, an alkyl group, a cycloalkyl group, an aryl group or a monovalent heterocyclic group, more preferably a hydrogen atom, an alkyl group or a cycloalkyl group, further preferably a hydrogen atom or an alkyl group, and the foregoing groups optionally have a substituent.

L^{H1} is preferably an arylene group or a divalent heterocyclic group, more preferably a divalent heterocyclic group, and the foregoing groups optionally have a substituent.

The arylene group represented by L^{H1} is preferably a group represented by the formula (A-1) to the formula (A-3) or the formula (A-8) to the formula (A-10), more preferably a group represented by the formula (A-1), the formula (A-2), the formula (A-8) or the formula (A-9), further preferably a group represented by the formula (A-1) or the formula (A-2).

The divalent heterocyclic group represented by L^{H1} is preferably a group represented by the formula (AA-1) to the formula (AA-6), the formula (AA-10) to the formula (AA-21) or the formula (AA-24) to the formula (AA-34), more preferably a group represented by the formula (AA-1) to the formula (AA-4), the formula (AA-10) to the formula (AA-15) or the formula (AA-29) to the formula (AA-34), further preferably a group represented by the formula (AA-2), the formula (AA-4) or the formula (AA-10) to the formula (AA-15), particularly preferably a group represented by the formula (AA-12) or the formula (AA-14).

The substituent which L^{H1} optionally has is preferably a halogen atom, an alkyl group, a cycloalkyl group, an alkoxy group, a cycloalkoxy group, an aryl group or a monovalent heterocyclic group, more preferably an alkyl group, an alkoxy group, an aryl group or a monovalent heterocyclic group, further preferably an alkyl group, an aryl group or a monovalent heterocyclic group, particularly preferably an alkyl group, and the foregoing groups optionally further have a substituent.

L^{H21} is preferably a single bond or an arylene group, more preferably a single bond, and this arylene group optionally has a substituent.

The definition and examples of the arylene group or the divalent heterocyclic group represented by L^{H21} are the same as the definition and examples of the arylene group or the divalent heterocyclic group represented by L^{H1}.

R^{H21} is preferably an aryl group or a monovalent heterocyclic group, and the foregoing groups optionally have a substituent.

The definition and examples of the aryl group and the monovalent heterocyclic group represented by R^{H21} are the same as the definition and examples of the aryl group and the monovalent heterocyclic group represented by Ar^{H1} and Ar^{H2}.

The definition and examples of the substituent which R^{H21} optionally has are the same as the definition and examples of the substituent which Ar^{H1} and Ar^{H2} optionally have.

The compound represented by the formula (H-1) is preferably a compound represented by the formula (H-2). 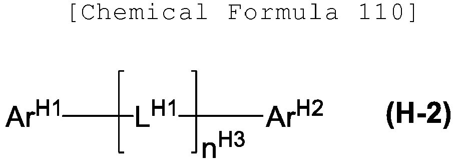 [wherein, Ar^{H1}, Ar^{H2}, n^{H3} and L^{H1} represent the same meaning as described above.]

As the compound represented by the formula (H-1), compounds represented by the formula (H-101) to the formula (H-121) are exemplified.

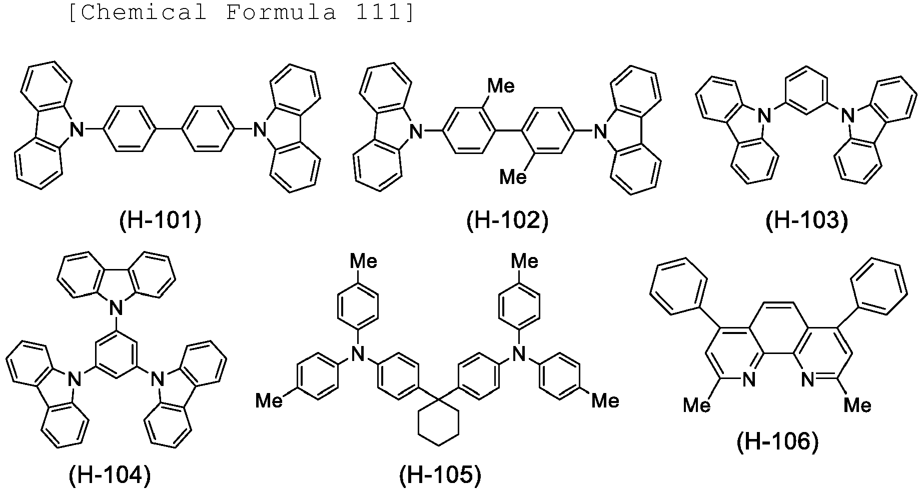

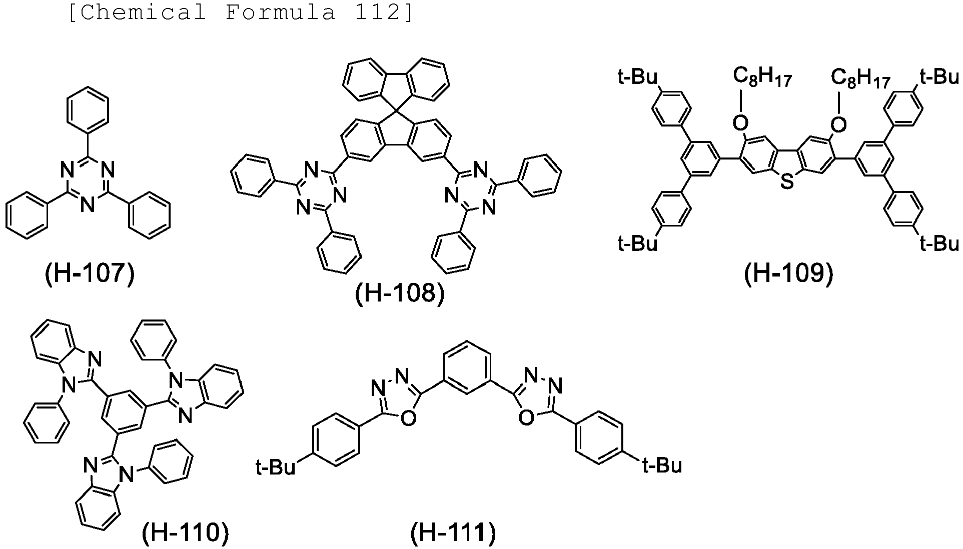

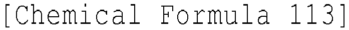

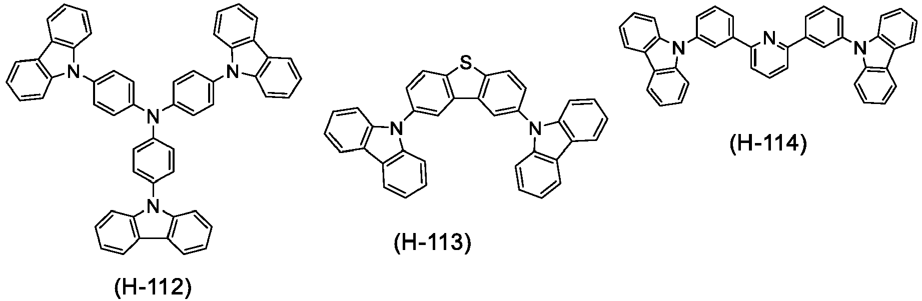

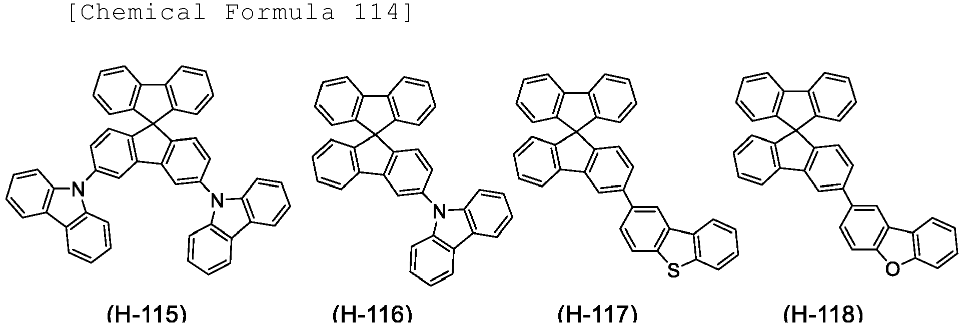

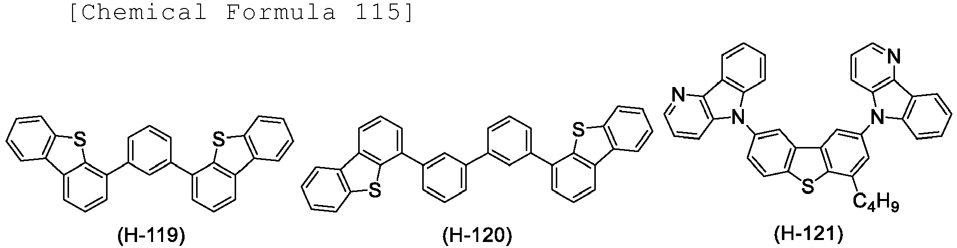

### [Second composition]

The second layer may be a layer containing a composition containing
at least one selected from the group consisting of a simple substance composed only of an alkali metal element, a simple substance composed only of a group 2 element, a compound containing an alkali metal element and a compound containing a group 2 element, and
at least one material selected from the group consisting of a compound represented by the formula (H-1), a hole transporting material, a hole injection material, an electron transporting material, an electron injection material, a light emitting material and an antioxidant (hereinafter, referred to also as "second composition").

In the second composition, the hole transporting material, the hole injection material, the electron transporting material, the electron injection material and the light emitting material are different from the simple substance composed only of an alkali metal element, the simple substance composed only of a group 2 element, the compound containing an alkali metal element, the compound containing a group 2 element and the compound represented by the formula (H-1), respectively.

The examples and preferable range of the hole transporting material, the electron transporting material, the hole injection material, the electron injection material and the light emitting material contained in the second composition are the same as the examples and preferable range of the hole transporting material, the electron transporting material, the hole injection material, the electron injection material and the light emitting material contained in the first composition.

In the second composition, the compounding amounts of the hole transporting material, the electron transporting material, the hole injection material, the electron injection material and the light emitting material are each usually 1 to 10000 parts by mass, preferably 10 to 1000 parts by mass, more preferably 100 to 500 parts by mass, when the sum of a simple substance composed only of an alkali metal element, a simple substance composed only of a group 2 element, a compound containing an alkali metal element and a compound containing a group 2 element is taken as 100 parts by mass.

The examples and preferable range of the antioxidant contained in the second composition are the same as the examples and preferable range of the antioxidant contained in the first composition. In the second composition, the compounding amount of the antioxidant is usually 0.001 to 10 parts by mass, when the sum of a simple substance composed only of an alkali metal element, a simple substance composed only of a group 2 element, a compound containing an alkali metal element and a compound containing a group 2 element is taken as 100 parts by mass.

### [Second ink]

The at least one material selected from the group consisting of a simple substance composed only of an alkali metal element, a simple substance composed only of a group 2 element, a compound containing an alkali metal element and a compound containing a group 2 element can be, for example, dissolved in a solvent and used. A composition containing this material and a solvent (hereinafter, referred to also as "second ink") can be suitably used for the application methods explained in the section of the first ink. The preferable range of the viscosity of the second ink is the same as the preferable range of the viscosity of the first ink.

The solvent contained in the second ink is preferably a solvent that can dissolve or uniformly disperse solid components in the ink. The solvent is preferably water, alcohol, ether, ester, nitrile compound, nitro compound, fluorinated alcohol, thiol, sulfide, sulfoxide, thioketone, amide or carboxylic acid, since the second layer can be laminated on an underlayer (for example, first layer) utilizing a difference in solubility. Examples of the solvent include methanol, ethanol, 2-propanol, 1-butanol, tert-butyl alcohol, acetonitrile, 1,2-ethanediol, N,N-dimethylformamide, dimethyl sulfoxide, acetic acid, nitromethane, propylene carbonate, pyridine, carbon disulfide, and mixed solvent thereof. When the mixed solvent is used, it may be a mixed solvent composed of at least one solvent among water, alcohol, ether, ester, nitrile compound, nitro compound, fluorinated alcohol, thiol, sulfide, sulfoxide, thioketone, amide, carboxylic acid and the like and at least one solvent among chlorine-based solvents, aromatic hydrocarbon solvents, aliphatic hydrocarbon solvents and ketone solvents.

In the second ink, the compounding amount of the solvent is usually 1000 to 100000 parts by mass, preferably 2000 to 20000 parts by mass, when the sum of a simple substance composed only of an alkali metal element, a simple substance composed only of a group 2 element, a compound containing an alkali metal element and a compound containing a group 2 element is taken as 100 parts by mass,.

### [Third layer]

The third layer which the light emitting device of the present invention has is preferably a layer containing at least one selected from the group consisting of a compound containing an alkali metal element and a simple substance composed only of a group 2 element, since luminance life is more excellent.

In the third layer, the compound containing an alkali metal element is preferably a metal complex containing an alkali metal element, an inorganic compound containing an alkali metal element or a polymer compound containing an alkali metal element, more preferably a metal complex containing an alkali metal element or an inorganic compound containing an alkali metal element, further preferably an inorganic compound containing an alkali metal element, since the light emitting device of the present invention is more excellent in luminance life.

In the third layer, the compound containing a group 2 element is preferably a metal complex containing a group 2 element, an inorganic compound containing a group 2 element or a polymer compound containing a group 2 element, more preferably a metal complex containing a group 2 element or an inorganic compound containing a group 2 element, further preferably an inorganic compound containing a group 2 element, since the light emitting device of the present invention is more excellent in luminance life.

The third layer may be a layer containing a composition containing

at least one selected from the group consisting of a simple substance composed only of an alkali metal element, a simple substance composed only of a group 2 element, a compound containing an alkali metal element and a compound containing a group 2 element, and

at least one material selected from the group consisting of a compound represented by the formula (H-1), a hole transporting material, a hole injection material, an electron transporting material, an electron injection material, a light emitting material and an antioxidant (hereinafter, referred to also as "third composition").

In the third composition, the hole transporting material, the hole injection material, the electron transporting material, the electron injection material and the light emitting material are different from the simple substance composed only of an alkali metal element, the simple substance composed only of a group 2 element, the compound containing an alkali metal element, the compound containing a group 2 element and the compound represented by the formula (H-1), respectively.

The examples and preferable range of the hole transporting material, the electron transporting material, the hole injection material, the electron injection material and the light emitting material contained in the third composition are the same as the examples and preferable range of the hole transporting material, the electron transporting material, the hole injection material, the electron injection material and the light emitting material contained in the first composition.

In the third composition, the compounding amounts of the hole transporting material, the electron transporting material, the hole injection material, the electron injection material and the light emitting material are each usually 1 to 10000 parts by mass, preferably 10 to 1000 parts by mass, more preferably 100 to 500 parts by mass, when the sum of a simple substance composed only of an alkali metal element, a simple substance composed only of a group 2 element, a compound containing an alkali metal element and a compound containing a group 2 element is taken as 100 parts by mass.

The examples and preferable range of the antioxidant contained in the third composition are the same as the examples and preferable range of the antioxidant contained in the first composition. In the third composition, the compounding amount of the antioxidant is usually 0.001 to 10 parts by mass, when the sum a simple substance composed only of an alkali metal element, a simple substance composed only of a group 2 element, a compound containing an alkali metal element and a compound containing a group 2 element is taken as 100 parts by mass.

The third layer is preferably a layer composed only of one or two or more selected from the group consisting of a simple substance composed only of an alkali metal element, a simple substance composed only of a group 2 element, a compound containing an alkali metal element and a compound containing a group 2 element, more preferably a layer composed only of one or two or more selected from the group consisting of a compound containing an alkali metal element and a simple substance composed only of a group 2 element, since the light emitting device of the present invention is further excellent in luminance life.

Further, the third layer is preferably a layer composed only of one or more and five or less selected from the group consisting of a simple substance composed only of an alkali metal element, a simple substance composed only of a group 2 element, a compound containing an alkali metal element and a compound containing a group 2 element, more preferably a layer composed only of one or more and three or less selected from the group consisting of a simple substance composed only of an alkali metal element, a simple substance composed only of a group 2 element, a compound containing an alkali metal element and a compound containing a group 2 element, further preferably a layer composed only of one selected from the group consisting of a simple substance composed only of an alkali metal element, a simple substance composed only of a group 2 element, a compound containing an alkali metal element and a compound containing a group 2 element, since the light emitting device of the present invention can be produced easily.

### [Third ink]

The at least one material selected from the group consisting of a simple substance composed only of an alkali metal element, a simple substance composed only of a group 2 element, a compound containing an alkali metal element and a compound containing a group 2 element can be, for example, dissolved in a solvent and used. A composition containing this material and a solvent (hereinafter, referred to also as "third ink") can be suitably used for the application methods explained in the section of the first ink. The preferable range of the viscosity of the third ink is the same as the preferable range of the viscosity of the first ink. The examples and preferable range of the solvent contained in the third ink are the same as the examples and preferable range of the solvent contained in the first ink or the second ink.

In the third ink, the compounding amount of the solvent is usually 1000 to 100000 parts by mass, preferably 2000 to 20000 parts by mass, when the sum of a simple substance composed only of an alkali metal element, a simple substance composed only of a group 2 element, a compound containing an alkali metal element and a compound containing a group 2 element is taken as 100 parts by mass.

### [Layer constitution]

The light emitting device of the present invention has an anode, a cathode, a first layer disposed between the anode and the cathode, a second layer disposed between the first layer and the cathode, and a third layer disposed between the second layer and the cathode in contact with the above-described cathode.

In the light emitting device of the present invention, one layer of the second layer and one layer of the third layer are present, respectively.

In the light emitting device of the present invention, two or more layers of the first layer may be provided, but it is preferable that one layer of the first layer is present, since production of the light emitting device of the present invention is easy.

The light emitting device of the present invention may have layers other than the anode, the cathode, the first layer, the second layer and the third layer.

In the light emitting device of the present invention, the first layer is usually a light emitting layer (hereinafter, referred to as "first light emitting layer").

In the light emitting device of the present invention, the second layer is preferably a light emitting layer (a light emitting layer different from the first light emitting layer, hereinafter, referred to as "second light emitting layer"), an electron transporting layer or an electron injection layer, more preferably an electron transporting layer or an electron injection layer, further preferably an electron transporting layer.

In the light emitting device of the present invention, the third layer is preferably an electron transporting layer or an electron injection layer, more preferably an electron injection layer.

In the light emitting device of the present invention, it is preferable that the first layer is a light emitting layer, the second layer is an electron transporting layer and the third layer is an electron injection layer.

In the light emitting device of the present invention, it is preferable that the first layer and the second layer are adjacent, since the light emitting device of the present invention is more excellent in luminance life.

In the light emitting device of the present invention, it is preferable that the second layer and the third layer are adjacent, since the light emitting device of the present invention is more excellent in luminance life.

In the light emitting device of the present invention, it is preferable that the first layer, the second layer and the third layer are adjacent, since the light emitting device of the present invention is further excellent in luminance life.

In the light emitting device of the present invention, the second layer is preferably an electron transporting layer or an electron injection layer disposed between the cathode and the first layer, more preferably an electron transporting layer disposed between the cathode and the first layer, since the light emitting device of the present invention is more excellent in luminance life.

In the light emitting device of the present invention, the third layer is preferably an electron injection layer disposed between the cathode and the second layer in adjacent to the cathode, since the light emitting device of the present invention is more excellent in luminance life.

In the light emitting device of the present invention, it is preferable that at least one layer out of a hole injection layer and a hole transporting layer is further present between the anode and the first layer, it is more preferable that both a hole injection layer and a hole transporting layer are further present, since luminance life is more excellent.

When the light emitting device of the present invention has a hole injection layer between the anode and the first layer, it is preferable that the anode and the hole injection layer are adjacent, since the light emitting device of the present invention is more excellent in luminance life. When the light emitting device of the present invention has a hole transporting layer between the anode and the first layer, it is preferable that the first layer and the hole transporting layer are adjacent, since the light emitting device of the present invention is more excellent in luminance life. When the light emitting device of the present invention has a hole injection layer and a hole transporting layer between the anode and the first layer, the hole injection layer is preferably a layer disposed between the anode and the hole transporting layer, more preferably a layer disposed between the anode and the hole transporting layer in adjacent to the anode or the hole transporting layer, further preferably a layer disposed between the anode and the hole transporting layer in adjacent to the anode and the hole transporting layer, since the light emitting device of the present invention is more excellent in luminance life.

The light emitting device of the present invention preferably has a second light emitting layer, since the emission color can be controlled.

When the light emitting device of the present invention has a second light emitting layer, it is preferable that the first light emitting layer and the second light emitting layer are adjacent, since luminance life is more excellent.

When the light emitting device of the present invention has a second light emitting layer, it is preferable that the second light emitting layer is a layer disposed between the anode and the first layer, it is more preferable that the second light emitting layer is a layer disposed between the anode and the first layer in adjacent to the first layer, since luminance life is more excellent.

When the light emitting device of the present invention has a second light emitting layer and the second light emitting layer is a layer disposed between the cathode and the first layer, it is preferable that at least one layer among the hole injection layer and the hole transporting layer is further present between the anode and the first layer, it is more preferable that both the hole injection layer and the hole transporting layer are further present, since luminance life is more excellent.

When the light emitting device of the present invention has a second light emitting layer and the second light emitting layer is a layer disposed between the anode and the first layer, it is preferable that at least one layer among the hole injection layer and the hole transporting layer is further present between the anode and the second light emitting layer, it is more preferable that the hole injection layer is further present, since luminance life is more excellent.

When the light emitting device of the present invention has a second light emitting layer and the second light emitting layer is a layer disposed between the anode and the first layer and the hole injection layer is present between the anode and the second light emitting layer, the hole injection layer is preferably a layer disposed in adjacent to the anode or the second light emitting layer, more preferably a layer disposed in adjacent to the anode, since luminance life is more excellent. When the light emitting device of the present invention has a second light emitting layer and the second light emitting layer is a layer disposed between the anode and the first layer and the hole transporting layer is present between the anode and the second light emitting layer, the hole transporting layer is preferably a layer disposed in adjacent to the anode or the second light emitting layer, more preferably a layer disposed in adjacent to the second light emitting layer, since luminance life is more excellent. When the light emitting device of the present invention has a second light emitting layer and the second light emitting layer is a layer disposed between the anode and the first layer and the hole injection layer and the hole transporting layer are present between the anode and the second light emitting layer, the hole injection layer is preferably a layer disposed between the anode and the hole transporting layer, more preferably a layer disposed between the anode and the hole transporting layer in adjacent to the anode or the hole transporting layer, further preferably a layer disposed between the anode and the hole transporting layer in adjacent to the anode and the hole transporting layer, since luminance life is more excellent.

The layer constitution of the light emitting device of the present invention includes, for example, layer constitutions represented by (D1) to (D9), and layer constitutions represented by (D3) to (D6) are preferable. The light emitting device of the present invention usually has a substrate, and an anode may be first laminated on the substrate, or a cathode may be first laminated on the substrate.

(D1) anode/first light emitting layer (first layer)/second light emitting layer (second layer)/electron transporting layer (third layer)/cathode
(D2) anode/first light emitting layer (first layer)/electron transporting layer (second layer)/electron injection layer (third layer)/cathode
(D3) anode/hole injection layer/second light emitting layer/first light emitting layer (first layer)/electron transporting layer (second layer)/electron injection layer (third layer)/cathode
(D4) anode/hole transporting layer/second light emitting layer/first light emitting layer (first layer)/electron transporting layer (second layer)/electron injection layer (third layer)/cathode
(D5) anode/hole injection layer/hole transporting layer/first light emitting layer (first layer)/electron transporting layer (second layer)/electron injection layer (third layer)/cathode
(D6) anode/hole injection layer/hole transporting layer/second light emitting layer/first light emitting layer (first layer)/electron transporting layer (second layer)/electron injection layer (third layer)/cathode
(D7) anode/hole injection layer/hole transporting layer/first light emitting layer (first layer)/second light emitting layer/electron transporting layer (second layer)/electron injection layer (third layer)/cathode
(D8) anode/hole injection layer/hole transporting layer/first light emitting layer (first layer)/electron transporting layer (second layer)/electron transporting layer/electron injection layer (third layer)/cathode
(D9) anode/hole injection layer/hole transporting layer/first light emitting layer (first layer)/electron transporting layer (second layer)/electron injection layer/electron injection layer (third layer)/cathode

In (D1) to (D9), "/" means that layers before and behind that are laminated adjacently. Specifically, "first light emitting layer (first layer)/electron transporting layer (second layer)/electron injection layer (third layer)" means that a first light emitting layer (first layer), and electron transporting layer (second layer) and an electron injection layer (third layer) are laminated adjacently.

In the light emitting device of the present invention, two or more layers of the anode, two or more layers of the hole injection layer, two or more layers of the hole transporting layer, two or more layers of the light emitting layer, two or more layers of the electron transporting layer, two or more layers of the electron injection layer and two or more layers of the cathode may be provided, respectively, if necessary, but it is preferable that one layer of the anode, one layer of the hole injection layer, one layer of the hole transporting layer, one layer of the electron transporting layer, one layer of the electron injection layer and one layer of the cathode are provided, since production of the light emitting device of the present invention is easy.

When a plurality of anodes, hole injection layers, hole transporting layers, light emitting layers, electron transporting layers, electron injection layers and cathodes are present, they may be the same or different at each occurrence.

The thicknesses of the anode, the hole injection layer, the hole transporting layer, the light emitting layer, the electron transporting layer, the electron injection layer and the cathode are each usually 1 nm to 1 µm, preferably 2 nm to 500 nm, further preferably 5 nm to 150 nm.

In the light emitting device of the present invention, the order, number and thickness of layers to be laminated may be controlled in view of the luminance life of the light emitting device, the driving voltage and the device life.

### [Second light emitting layer]

The second light emitting layer is usually a second layer or a layer containing a light emitting material, preferably a layer containing a light emitting material. When the second light emitting layer is a layer containing a light emitting material, the light emitting material contained in the second light emitting layer includes, for example, light emitting materials and phosphorescent compounds which may be contained in the first composition described above. The light emitting material contained in the second light emitting layer may be contained singly or in combination of two or more.

When the light emitting device of the present invention has a second light emitting layer and the second layer is not an electron transporting layer and an electron injection layer, it is preferable that the second light emitting layer is the second layer.

### [Hole transporting layer]

The hole transporting layer is usually a layer containing a hole transporting material. When the hole transporting layer is a layer containing a hole transporting material, the hole transporting material includes, for example, hole transporting materials which may be contained in the first composition described above. The hole transporting material contained in the hole transporting layer may be contained singly or in combination of two or more.

### [Electron transporting layer]

The electron transporting layer is usually a second layer, a third layer or a layer containing an electron transporting material, preferably a second layer or a third layer, more preferably a second layer. When the electron transporting layer is a layer containing an electron transporting material, the electron transporting material contained in the electron transporting layer includes, for example, electron transporting materials which may be contained in the first composition described above. The electron transporting material contained in the electron transporting layer may be contained singly or in combination of two or more.

When the light emitting device of the present invention has an electron transporting layer and the second layer is not a second light emitting layer and an electron injection layer, it is preferable that the electron transporting layer is a second layer.

When the light emitting device of the present invention has an electron transporting layer and the third layer is an electron injection layer, it is preferable that the electron transporting layer is a second layer.

When the light emitting device of the present invention has an electron transporting layer and the third layer is not an electron injection layer, it is preferable that the electron transporting layer is a third layer.

When the light emitting device of the present invention has an electron transporting layer and the second layer is a second light emitting layer, it is preferable that the electron transporting layer is a third layer.

### [Hole injection layer and electron injection layer]

The hole injection layer is usually a layer containing a hole injection material. The hole injection material contained in the hole injection layer includes, for example, hole injection materials which may be contained in the first composition described above. The hole injection material contained in the hole injection layer may be contained singly or in combination of two or more.

The electron injection layer is usually a second layer, a third layer or a layer containing an electron injection material, preferably a second layer or a third layer, more preferably a third layer. The electron injection material contained in the electron injection layer includes, for example, electron injection materials which may be contained in the first composition described above. The electron injection material contained in the electron injection layer may be contained singly or in combination of two or more.

When the light emitting device of the present invention has an electron injection layer and the second layer is not a second light emitting layer and an electron transporting layer, it is preferable that the electron injection layer is a second layer.

When the light emitting device of the present invention has an electron injection layer and the third layer is not an electron transporting layer, it is preferable that the electron injection layer is a third layer.

When the light emitting device of the present invention has an electron injection layer and the second layer is a second light emitting layer or an electron transporting layer, it is preferable that the electron injection layer is a third layer.

### [Substrate/electrode]

The substrate in the light emitting device may advantageously be a substrate on which an electrode can be formed and which does not change chemically in forming an organic layer, and is, for example, a substrate made of a material such as glass, plastic, silicon and the like. When an opaque substrate is used, it is preferable that the electrode farthest from the substrate is transparent or semi-transparent.

The material of the anode includes, for example, electrically conductive metal oxides and semi-transparent metals, preferably includes indium oxide, zinc oxide, tin oxide; electrically conductive compounds such as indium-tin-oxide (ITO), indium-zinc-oxide and the like; silver-palladium-copper (APC) complex; NESA, gold, platinum, silver and copper.

The material of the cathode includes, for example, metals such as aluminum, zinc, indium and the like; alloys composed of two or more of them; alloys composed of at least one of them and at least one of silver, copper, manganese, titanium, cobalt, nickel, tungsten and tin; and graphite and graphite intercalation compounds. The alloy includes, for example, an indium-silver alloy.

In the light emitting device of the present invention, at least one of the anode and the cathode is usually transparent or semi-transparent, and it is preferable that the anode is transparent or semi-transparent.

The method for forming the anode and the cathode includes, for example, a vacuum vapor deposition method, a sputtering method, an ion plating method, a plating method and a lamination method.

### [Production method]

The method for forming a first layer, a second layer, a third layer and other layers in the light emitting device of the present invention includes, for example, a vacuum vapor deposition method from a powder and a method by film formation from a solution or melted state when a low molecular compound is used, and, for example, a method by film formation from a solution or melted state when a polymer compound is used. The first layer, the second layer, the third layer and other layers may be formed by application methods explained in the section of the first ink described above using the above-described various inks and inks containing various materials, or may be formed by a dry method such as a vacuum vapor deposition method and the like.

When the first layer is formed by an application method, it is preferable that the first ink is used. It is preferable that the first layer is formed by an application method, since production of the light emitting device of the present invention is easy.

When the second layer is formed by an application method, it is preferable that the second ink is used. It is preferable that the second layer is formed by an application method, since production of the light emitting device of the present invention is easy.

When the third layer is formed by an application method, it is preferable that the third ink is used.

It is preferable that the third layer is formed by a dry method, since the light emitting device of the present invention is more excellent in luminance life.

The light emitting device of the present embodiment can be produced, for example, by laminating layers in series on a substrate. Specifically, an anode is provided on a substrate, layers such as a hole injection layer, a hole transporting layer and the like are provided thereon, a light emitting layer is provided thereon, and layers such as an electron transporting layer, and electron injection layer and the like are provided thereon, and further, a cathode is laminated thereon, thus, a light emitting device can be produced. As another production method, a cathode is provided on a substrate, layers such as an electron injection layer, an electron transporting layer, a light emitting layer, a hole transporting layer, a hole injection layer and the like are provided thereon, and further, an anode is laminated thereon, thus, a light emitting device can be produced. As still another production method, an anode or an anode side substrate prepared by laminating various layers on an anode, and a cathode or a cathode side substrate prepared by laminating various layers on a cathode can be connected facing each other, to produce a light emitting device.

### [Application]

In order to obtain planar light emission using a light emitting device, the planar anode and the planar cathode may be arranged so as to overlap each other. In order to obtain patterned light emission, there are a method of installing a mask having a patterned window on the surface of a planar light emitting device, a method in which a layer to be formed as a non-light emitting part is formed extremely thick so as to cause substantially non light emission and a method of forming an anode or a cathode, or both electrodes in a pattern. A segment type display capable of displaying numerals, letters and the like can be obtained by forming a pattern by any one of these methods and disposing several electrodes so that several electrodes can be turned on and off independently. In order to obtain a dot matrix display, both the anode and the cathode may be formed in a stripe shape and arranged so as to be orthogonal to each other. Partial color display and multicolor display become possible by a method of separately coating plural kinds of polymer compounds having different emission colors or a method using a color filter or a fluorescence conversion filter. The dot matrix display can be driven passively or can be driven actively in combination with a TFT and the like. These displays can be used for displays of computers, televisions, portable terminals, and the like. The planar light emitting device can be suitably used as a planar light source for backlight of a liquid crystal display, or as a planar light source for illumination. If a flexible substrate is used, it can be used as a curved light source and a curved display.

### EXAMPLES

The present invention will be illustrated further in detail by examples below, but the present invention is not limited to these examples.

In examples, the polystyrene-equivalent number-average molecular weight (Mn) and the polystyrene-equivalent weight-average molecular weight (Mw) of polymer compounds were determined by the following size exclusion chromatography (SEC) using tetrahydrofuran as a mobile phase.

A polymer compound to be measured was dissolved in tetrahydrofuran at a concentration of about 0.05% by mass, and 10 µL of the solution was injected into SEC. The mobile phase was flowed at a flow rate of 1.0 mL/min. As a column, PLgel MIXED-B (manufactured by Polymer Laboratories Ltd.) was used. As a detector, a UV-VIS detector (trade name: UV-8320GPC manufactured by Tosoh Corp.) was used.

NMR was measured by the following method.

Five to ten mg of a measurement sample was dissolved in about 0.5 mL of deutero-chloroform (CDCl₃), deutero-tetrahydrofuran, deutero-dimethyl sulfoxide, deutero-acetone, deutero-N,N-dimethylformamide, deutero-toluene , deutero-methanol, deutero-ethanol, deutero-2-propanol or deutero-methylene chloride, and NMR thereof was measured using an NMR apparatus (trade name: JNM-ECZ400S/L1 manufactured by JEOL RESONANCE).

As an indicator of the purity of the compound, high performance liquid chromatography (HPLC) area percentage value was used. This value is a value by HPLC (trade name: LC-20A manufactured by Shimadzu Corp.) at UV = 254 nm unless otherwise stated. In this operation, the compound to be measured was dissolved in tetrahydrofuran or chloroform so as to give a concentration of 0.01 to 0.2% by mass, and 1 to 10 µL of the solution was injected into HPLC depending on the concentration. As a mobile phase of HPLC, acetonitrile/tetrahydrofuran were used while changing the ratio thereof from 100/0 to 0/100 (volume ratio), and flowed at a flow rate of 1.0 mL/min. As a column, SUMIPAX ODS Z-CLUE (manufactured by Sumika Chemical Analysis Service, Ltd., internal diameter: 4.6 mm, length: 250 mm, particle size: 3 µm) or an ODS column having the equivalent performance was used. As a detector, a photodiode array detector (trade name: SPD-M20A manufactured by Shimadzu Corp.) was used.

In the present example, the maximum peak wavelength of the emission spectrum of the compound was measured by a spectrophotometer (manufactured by JASCO Corp., FP-6500) at room temperature. The compound was dissolved in xylene at a concentration of about 0.8×10⁻⁴ % by mass to prepare a xylene solution which was then used as a sample. As the excitation light, UV light having a wavelength of 325 nm was used.

### <Synthesis Example M1> Synthesis of compounds M1 to M9 and metal complex RM1

Compounds M1, M2 and M3 were synthesized according to a method described in International Publication WO 2013/146806.

A compound M4 was synthesized according to a method described in JP-A No. 2012-33845.

A compound M5 was synthesized according to a method described in JP-A No. 2010-189630.

A compound M6 was synthesized according to a method described in JP-A No. 2011-174062.

A compound M7 was synthesized according to a method described in International Publication WO 2002/045184.

A compound M8 was synthesized according to a method described in International Publication WO 2005/049546.

A compound M9 was synthesized according to a method described in JP-A No. 2008-106241.

A metal complex RM1 was synthesized according to a method described in International Publication WO 2009/157424.

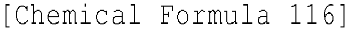

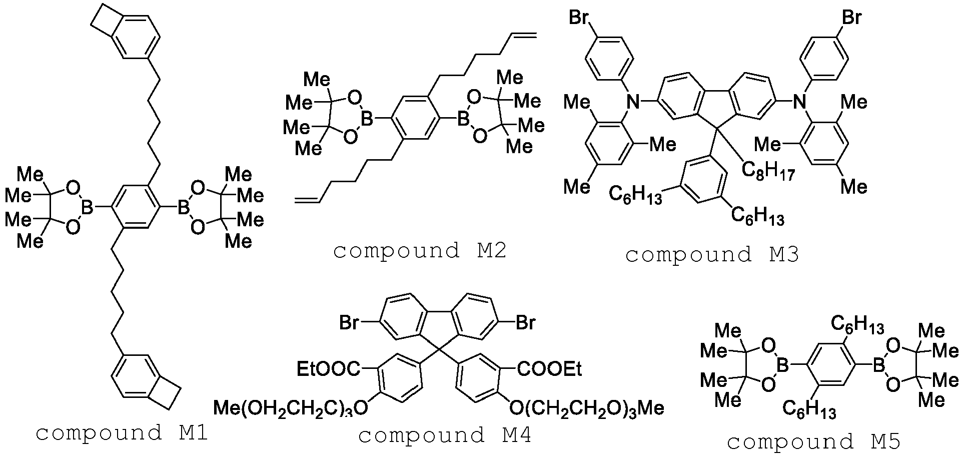

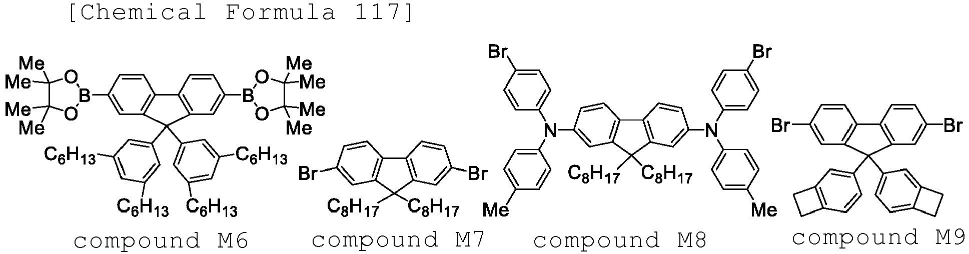

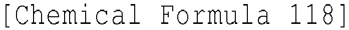

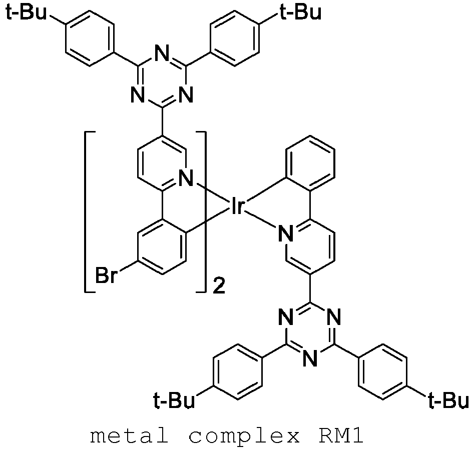

### <Synthesis Example HTL1> Synthesis of polymer compound HTL-1

An inert gas atmosphere was prepared in a reaction vessel, then, the compound M1 (2.52 g), the compound M2 (0.470 g), the compound M3 (4.90 g), the metal complex RM1 (0.530 g), dichlorobis(tris-o-methoxyphenylphosphine)palladium (4.2 mg) and toluene (158 mL) were added, and the mixture was heated at 100°C. Thereafter, into this was dropped a 20% by mass tetraethylammonium hydroxide aqueous solution (16 mL), and the solution was refluxed for 8 hours. Thereafter, to this were added phenylboronic acid (116 mg) and dichlorobis(tris-o-methoxyphenylphosphine)palladium (4.2 mg), and the solution was refluxed for 15 hours. Thereafter, to this was added a sodium diethyldithiacarbamate aqueous solution, and the mixture was stirred at 85°C for 2 hours. The resultant reaction liquid was cooled, then, washed with 3.6% by mass hydrochloric acid, 2.5% by mass ammonia water, and water, respectively. The resultant solution was dropped into methanol, to generate a precipitate. The resultant precipitate was dissolved in toluene, and purified by passing through an alumina column and a silica gel column in this order. The resultant solution was dropped into methanol, stirred, then, the resultant precipitate was collected by filtration, and dried, to obtain 6.02 g of a polymer compound HTL-1. The polymer compound HTL-1 had an Mn of 3.8×10⁴ and an Mw of 4.5×10⁵.

The polymer compound HTL-1 is a copolymer constituted of a constitutional unit derived from the compound M1, a constitutional unit derived from the compound M2, a constitutional unit derived from the compound M3 and a constitutional unit derived from the metal complex RM1 at a molar ratio of 40:10:47:3 according to the theoretical values calculated from the amounts of the charged raw materials.

The emission spectrum of the polymer compound HTL-1 had a maximal wavelength at 404 nm and 600 nm and the maximum peak wavelength of the emission spectrum of the polymer compound HTL-1 was 404 nm.

### <Synthesis Example HTL2> Synthesis of polymer compound HTL-2

A polymer compound HTL-2 was synthesized according to a method described in JP-A No. 2011-174062 using the compound M6, the compound M7, the compound M8 and the compound M9. The polymer compound HTL-2 had an Mn of 7.8×10⁴ and an Mw of 2.6×10⁵.

The polymer compound HTL-2 is a copolymer constituted of a constitutional unit derived from the compound M6, a constitutional unit derived from the compound M7, a constitutional unit derived from the compound M8 and a constitutional unit derived from the compound M9 at a molar ratio of 50:12.5:30:7.5 according to the theoretical values calculated from the amounts of the charged raw materials.

### <Synthesis Example HTL3> Synthesis of polymer compound HTL-3

An inert gas atmosphere was prepared in a reaction vessel, then, the compound M1 (0.800 g), the compound M2 (0.149 g), the compound M3 (1.66 g), dichlorobis(tris-o-methoxyphenylphosphine)palladium (1.4 mg) and toluene (45 mL) were added, and the mixture was heated at 100°C. Thereafter, into this was dropped a 20% by mass tetraethylammonium hydroxide aqueous solution (16 mL), and the solution was refluxed for 7 hours. Thereafter, to this were added 2-ethylphenylboronic acid (90 mg) and dichlorobis(tris-o-methoxyphenylphosphine)palladium (1.3 mg), and the solution was refluxed for 17.5 hours. Thereafter, to this was added a sodium diethyldithiacarbamate aqueous solution, and the mixture was stirred at 85°C for 2 hours. The resultant reaction liquid was cooled, then, washed with 3.6% by mass hydrochloric acid, 2.5% by mass ammonia water, and water, respectively. The resultant solution was dropped into methanol, to generate a precipitate. The resultant precipitate was dissolved in toluene, and purified by passing through an alumina column and a silica gel column in this order. The resultant solution was dropped into methanol, stirred, then, the resultant precipitate was collected by filtration, and dried, to obtain 1.64 g of a polymer compound HTL-1. The polymer compound HTL-3 had an Mn of 3.5×10⁴ and an Mw of 2.2×10⁵.

The polymer compound HTL-3 is a copolymer constituted of a constitutional unit derived from the compound M1, a constitutional unit derived from the compound M2 and a constitutional unit derived from the compound M3 at a molar ratio of 40:10:50 according to the theoretical values calculated from the amounts of the charged raw materials.

### <Synthesis Examples B1, B3, B4, G1 to G4 and R1 to R4>

### Synthesis of phosphorescent compounds B1, B3, B4, G1 to G4 and R1 to R4

A phosphorescent compound B1 was synthesized with reference to methods described in International Publication WO 2006/121811 and JP-A No. 2013-048190.

A phosphorescent compound B3 was synthesized with reference to a method described in International Publication WO 2016/185183.

A phosphorescent compound B4 was synthesized with reference to a method described in International Publication WO 2006/121811.

A phosphorescent compound G1 was synthesized with reference to a method described in International Publication WO 2009/131255.

A phosphorescent compound G2 was synthesized according to a method described in JP-A No. 2013-237789.

Phosphorescent compounds G3 and G4 were synthesized according to a method described in JP-A No. 2014-224101.

A phosphorescent compound R1 was synthesized with reference to a method described in JP-A No. 2006-188673.

A phosphorescent compound R2 was synthesized according to a method described in JP-A No. 2008-179617.

A phosphorescent compound R3 was synthesized with reference to a method described in International Publication WO 2002/044189.

A phosphorescent compound R4 was synthesized according to a method described in JP-A No. 2011-105701.

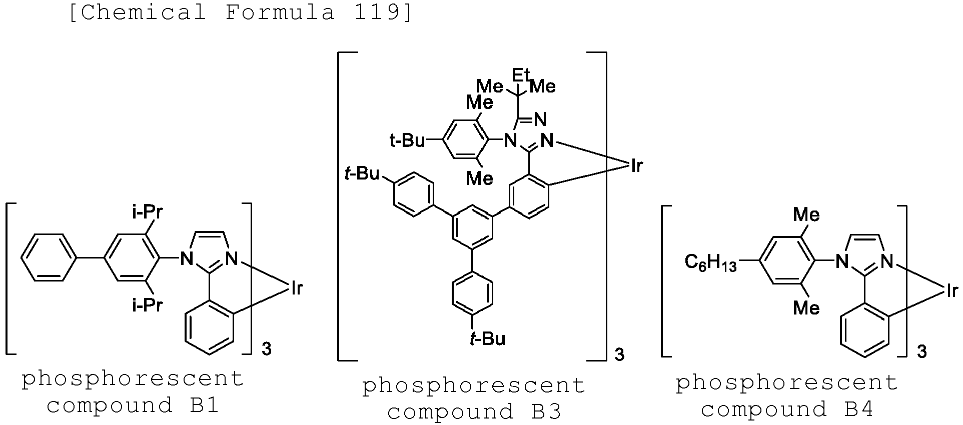

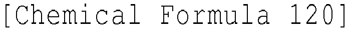

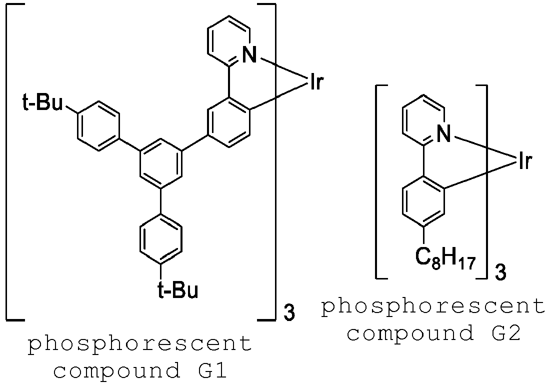

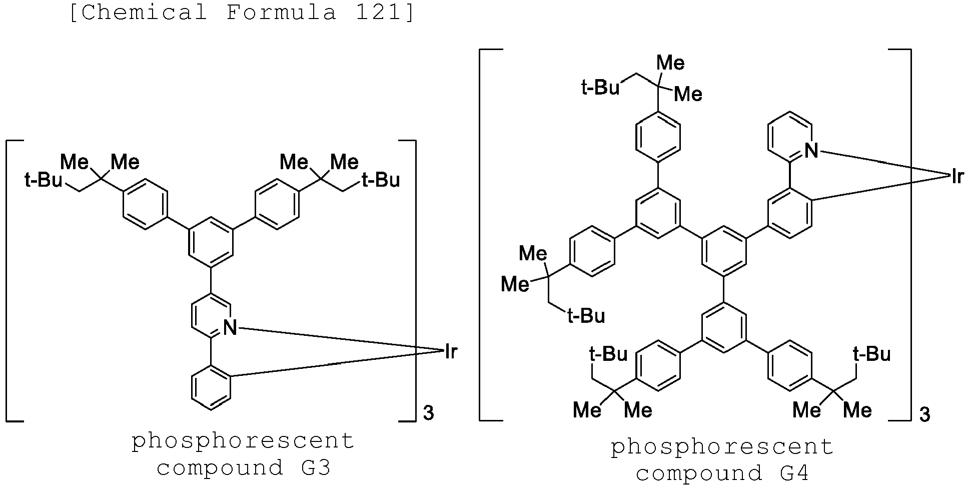

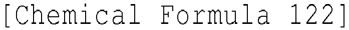

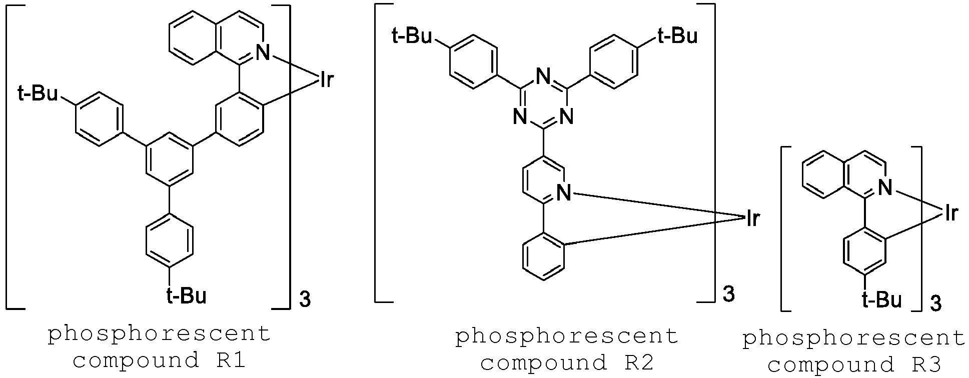

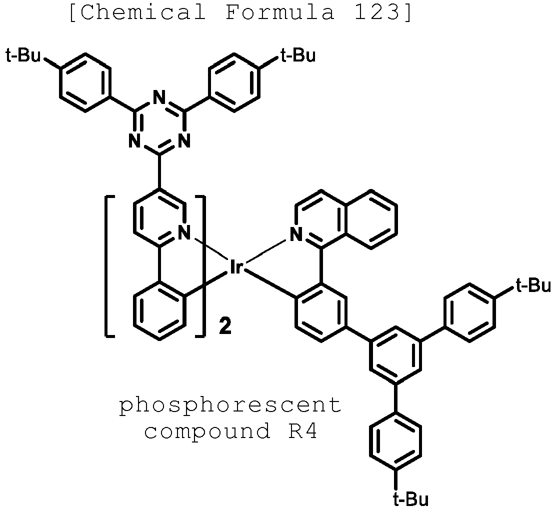

The maximum peak wavelength of the emission spectrum of the phosphorescent compound B1 was 471 nm.

The maximum peak wavelength of the emission spectrum of the phosphorescent compound B3 was 476 nm. The maximum peak wavelength of the emission spectrum of the phosphorescent compound B4 was 469 nm.

The maximum peak wavelength of the emission spectrum of the phosphorescent compound G1 was 514 nm.

The maximum peak wavelength of the emission spectrum of the phosphorescent compound G2 was 508 nm.

The maximum peak wavelength of the emission spectrum of the phosphorescent compound G3 was 545 nm.

The maximum peak wavelength of the emission spectrum of the phosphorescent compound G4 was 514 nm.

The maximum peak wavelength of the emission spectrum of the phosphorescent compound R1 was 619 nm.

The maximum peak wavelength of the emission spectrum of the phosphorescent compound R2 was 594 nm.

The maximum peak wavelength of the emission spectrum of the phosphorescent compound R3 was 617 nm.

The maximum peak wavelength of the emission spectrum of the phosphorescent compound R4 was 611 nm.

### <Synthesis Example B2> Synthesis of phosphorescent compound B2

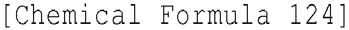

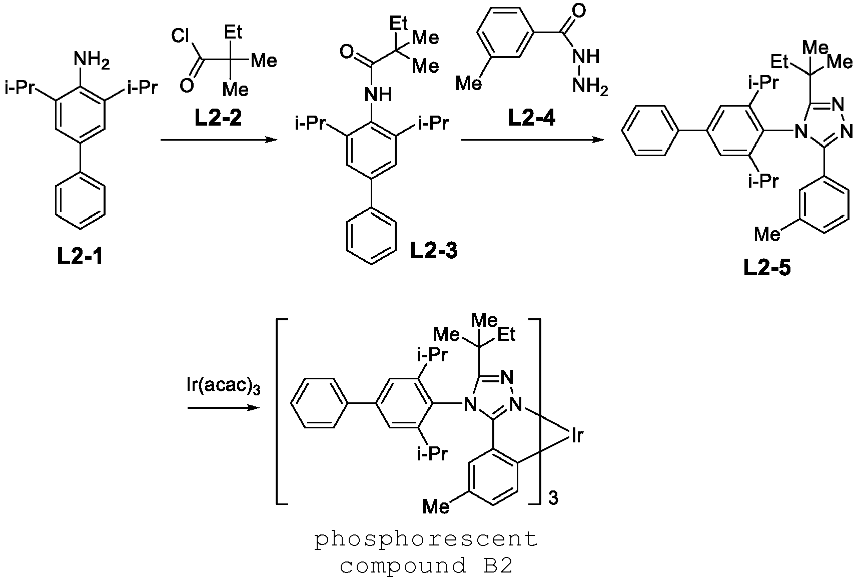

### (Synthesis of compound L2-3)

A nitrogen gas atmosphere was prepared in a reaction vessel, then, a compound L2-1 (21.4 g), triethylamine (13.0 mL) and tetrahydrofuran (300 mL) were added, and the mixture was cooled to 0°C. Thereafter, into this was dropped a compound L2-2 (12.8 mL), and the mixture was stirred at room temperature for 16 hours. Thereafter, to this was added ion exchanged water (100 mL), to generate a precipitate. The reaction liquid containing the resultant precipitate was filtrated, to obtain a residue L2-3-1 and a filtrate L2-3-2.

The resultant residue L2-3-1 was washed with toluene, then, dried under reduced pressure, to obtain a solid L2-3' (24.5 g).

From the resultant filtrate L2-3-2, an aqueous layer was removed, and the resultant organic layer was dried over magnesium sulfate, then, filtrated. The resultant filtrate was condensed under reduced pressure, then, crystallized with a mixed solvent of toluene and heptane. The resultant solid was dried under reduced pressure, to obtain a solid L2-3" (3.9 g).

The resultant solid L2-3' and the solid L2-3" were combined, then, crystallized with a mixed solvent of toluene and heptane. The resultant solid was dried under reduced pressure, to obtain a compound L2-3 (27.8 g, white solid). The HPLC area percentage value of the compound L2-3 was 98.9%.

The analysis result of the compound L2-3 was as described below.
¹H-NMR (600 MHz, THF-d₈): δ (ppm) = 7.57 (d, 2H), 7.43 (t, 2H), 7.35 (s, 2H), 7.34 (t, 1H), 6.82 (brs, 1H), 3.08 (septet, 2H), 1.73 (q, 2H), 1.34 (s, 6H), 1.25 (d, 12H), 1.00 (t, 3H).

### (Synthesis of compound L2-5)

A compound L2-5 was synthesized with reference to a method described in Organic Letters, vol. 17, pp. 1184-1187, 2015 using the compound L2-3 (19.1 g), a compound L2-4 (9.0 g), chlorobenzene (150 mL), 2-fluoropyridine (5.15 mL) and trifluoromethanesulfonic anhydride (10.0 mL).

The analysis result of the compound L2-5 was as described below.
¹H-NMR (600 MHz, CDCl₃): δ (ppm) = 7.68 (d, 2H), 7.52 (t, 2H), 7.50 (s, 2H), 7.43 (t, 1H), 7.26 (s, 1H), 7.05-6.98 (m, 3H), 2.53 (septet, 2H), 2.15 (s, 3H), 1.88 (q, 2H), 1.28 (d, 6H), 1.23 (s, 6H), 0.89 (t, 3H), 0.77 (d, 6H).

### (Synthesis of phosphorescent compound B2)

A nitrogen gas atmosphere was prepared in a reaction vessel, then, tris(acetylacetonato)iridium(III) (0.72 g), the compound L2-5 (2.8 g) and pentadecane (2 mL) were added, and the mixture was stirred at 300°C for 24 hours. The resultant reaction liquid was cooled down to room temperature, then, purified by silica gel column chromatography (a mixed solvent of methylene chloride and ethyl acetate), then, crystallized using a mixed solvent of acetonitrile and toluene, a mixed solvent of toluene and methanol and a mixed solvent of methylene chloride and acetonitrile in series. The resultant solid was washed with methylene chloride, then, dried under reduced pressure, to obtain a phosphorescent compound B2 (0.82 g). The HPLC area percentage value of the phosphorescent compound B2 was 98.8%.

The analysis result of the phosphorescent compound B2 was as described below.
¹H-NMR (600 MHz, THF-d₈): δ (ppm) = 7.74 (d, 6H), 7.64 (dd, 6H), 7.48 (t, 6H), 7.38 (t, 3H), 6.68 (d, 3H), 6.30 (d, 3H), 5.62 (s, 3H), 2.94 (septet, 3H), 2.37 (septet, 3H), 1.75 (s, 9H), 1.71-1.64 (m, 6H), 1.34 (d, 9H), 1.22-1.17 (m, 27H), 0.98 (d, 9H), 0.90 (d, 9H), 0.81 (d, 9H).

The maximum peak wavelength of the emission spectrum of the phosphorescent compound B2 was 474 nm.

### <Synthesis Example B5> Synthesis of phosphorescent compound B5

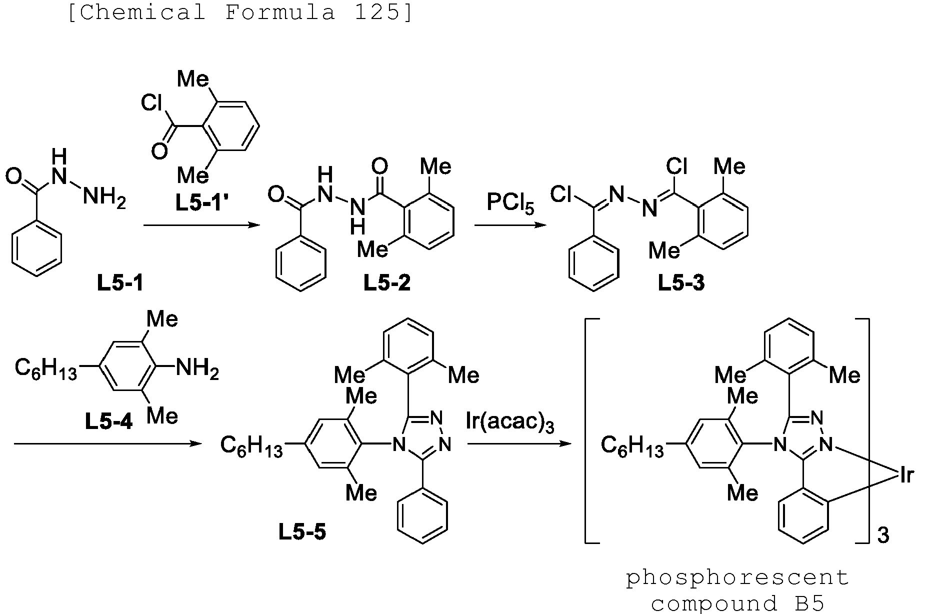

### (Synthesis of compound L5-2)

A nitrogen gas atmosphere was prepared in a reaction vessel, then, a compound L5-1 (50 g) and N-methyl-2-pyrrolidone (200 mL) were added, and the mixture was stirred at 0°C. Thereafter, into this was dropped a compound L5-1' (40 g) dissolved in N-methyl-2-pyrrolidone (40 mL), and the mixture was stirred at room temperature for 18 hours. The resultant reaction liquid was poured into ion exchanged water (1.2L), to generate a precipitate. The resultant precipitate was collected by filtration, then, washed with a 1M hydrochloric acid aqueous solution, ion exchanged water and heptane, in series. The resultant solid was dried under reduced pressure, to obtain a compound L5-2 (43g, white solid).

The analysis result of the compound L5-2 was as described below.
¹H-NMR (600 MHz, CDCl₃): δ (ppm) = 9.64 (br, 1H), 8.90 (br, 1H), 7.86 (d, 2H), 7.56 (t, 1H), 7.45 (t, 2H), 7.02-7.08 (m, 3H), 2.41 (s, 6H).

### (Synthesis of compound L5-3)

A nitrogen gas atmosphere was prepared in a reaction vessel, then, the compound L5-2 (43 g) and toluene (740 mL) were added, and the mixture was stirred at room temperature. Thereafter, to this was added phosphorus pentachloride (67 g), then, the mixture was stirred at 110°C for 21 hours. The resultant reaction liquid was cooled down to room temperature, then, poured into ice water (500 mL), and the mixture was stirred for 2 hours, then, an aqueous layer was removed. The resultant organic layer was washed with ion exchanged water and a 10% by mass sodium hydrogen carbonate aqueous solution, respectively. The resultant organic layer was dried over magnesium sulfate, then, filtrated. The resultant filtrate was condensed under reduced pressure, to obtain a compound L5-3 (40 g).

### (Synthesis of compound L5-5)

A nitrogen gas atmosphere was prepared in a reaction vessel, then, the compound L5-3 (40 g), a compound L5-4 (32 g) and xylene (800 mL) were added, and the mixture was stirred at room temperature. Thereafter, to this was added p-toluenesulfonic acid (3 g), and the mixture was stirred at 120°C for 116 hours. The resultant reaction liquid was cooled down to room temperature, then, ion exchanged water (800 mL) was added, and the mixture was stirred at room temperature for 1 hour. From the resultant reaction liquid, an aqueous layer was removed, then, the resultant organic layer was washed with a 5% by mass sodium hydrogen carbonate aqueous solution. The resultant organic layer was dried over magnesium sulfate, then, filtrated. The resultant filtrate was condensed under reduced pressure, to obtain a coarse product. The resultant coarse product was purified by silica gel column chromatography (a mixed solvent of heptane and ethyl acetate) and purified by silica gel column chromatography (acetonitrile and tetrahydrofuran) in series, to obtain a compound L4-5 (1.3 g, white solid). The HPLC area percentage value of the compound L5-5 was 99.5% or more. The above-described operation was conducted repeatedly, to obtain a necessary amount of the compound L4-5.

The analysis result of the compound L5-5 was as described below.
¹H-NMR (600 MHz, THF-d₈): δ (ppm) = 7.42 (d, 2H), 7.30 (t, 1H), 7.24 (t, 2H), 7.15 (t, 1H), 6.98 (d, 2H), 6.85 (s, 2H), 2.51 (t, 2H), 2.07 (s, 6H), 1.81 (s, 6H), 1.56 (m, 2H), 1.26-1.32 (m, 6H), 0.88 (t, 3H).

### (Synthesis of phosphorescent compound B5)

A nitrogen gas atmosphere was prepared in a reaction vessel, then, tris(acetylacetonato)iridium(III) (0.6 g), the compound L5-5 (2.0 g) and tridecane (2 mL) were added, and the mixture was stirred at 250°C for 120 hours. The resultant reaction liquid was cooled down to room temperature, then, purified by silica gel column chromatography (a mixed solvent of heptane and ethyl acetate), then, crystallized using a mixed solvent of methylene chloride and acetonitrile. The resultant solid was dried under reduced pressure, to obtain a phosphorescent compound B5 (0.6 g, yellow solid). The HPLC area percentage value of the phosphorescent compound B5 was 99.2%.

The analysis result of the phosphorescent compound B5 was as described below.
¹H-NMR (600 MHz, THF-d₈): δ (ppm) = 7.04-7.08 (m, 6H),6.93 (s, 3H), 6.92 (s, 3H), 6.88 (d, 3H), 6.84 (d, 3H), 6.61 (t, 3H), 6.43 (t, 3H), 6.29 (d, 3H), 2.57 (t, 6H), 2.12 (s, 9H), 1.95 (s, 9H), 1.82 (s, 9H), 1.70 (s, 9H), 1.62 (m, 6H), 1.28-1.36 (m, 18H), 0.89 (t, 9H).

The maximum peak wavelength of the emission spectrum of the phosphorescent compound B5 was 468 nm.

### <Synthesis Example B6> Synthesis of phosphorescent compound B6

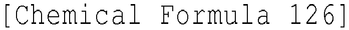

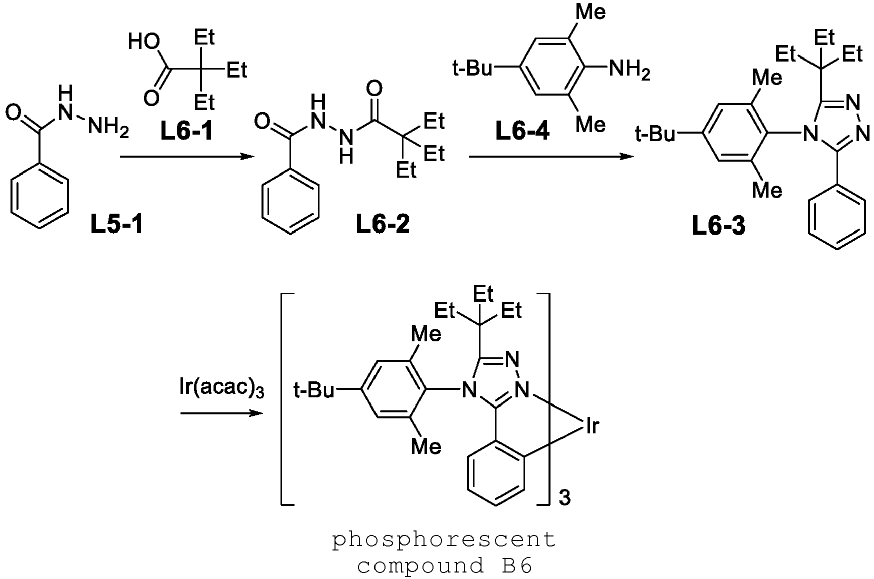

### (Synthesis of reaction mixture L6-1')

A nitrogen gas atmosphere was prepared in a reaction vessel, then, a compound L6-1 (50 g) and thionyl chloride (100 mL) were added, and the mixture was stirred under reflux for 3 hours. The resultant reaction mixture was cooled down to room temperature, then, thionyl chloride was distilled off under reduced pressure, to obtain a reaction mixture L6-1'.

### (Synthesis of compound L6-2)

A nitrogen gas atmosphere was prepared in a reaction vessel, then, the compound L5-1 (47 g) and tetrahydrofuran (1L) were added, and the mixture was cooled to 0°C. Thereafter, to this was added triethylamine (54 mL), and the mixture was stirred at 0°C for 45 minutes. Thereafter, to this was added the reaction mixture L6-1' (whole amount) obtained in (Synthesis of reaction mixture L6-1'), and the mixture was stirred at room temperature for 16 hours. The resultant reaction liquid was filtrated, then, the resultant filtrate was condensed under reduced pressure, to obtain a coarse product. The resultant coarse product was washed with a mixed solvent of ethyl acetate and hexane, then, dried under reduced pressure, to obtain a compound L6-2 (50 g). The HPLC area percentage value of the compound L6-2 was 95.2%. The above-described operation was conducted repeatedly, to obtain a necessary amount of the compound L6-2.

The analysis result of the compound L6-2 was as described below.
LC-MS (APCI, positive): m/z = 263 [M+H]⁺
¹H-NMR (300 MHz, CDCl₃): δ (ppm) = 0.84 (t, 9H), 1.64 (q, 6H), 7.39-7.54 (m, 3H), 7.81-7.84 (m, 2H), 8.72-8.74 (m, 1H), 9.66-9.68 (m, 1H).

### (Synthesis of compound L6-3)

A nitrogen gas atmosphere was prepared in a reaction vessel, then, the compound L6-2 (58 g) and toluene (600 mL) were added, and the mixture was stirred at room temperature. Thereafter, to this was added phosphorus pentachloride (92 g), then, the mixture was stirred at 110°C for 3 hours. The resultant reaction liquid was cooled down to room temperature, then, a compound L6-4 (78.2 g) and p-toluenesulfonic acid (3 g) were added, and the mixture was stirred at 130°C for 4 days. The resultant reaction liquid was cooled down to room temperature, condensed under reduced pressure, then, ethyl acetate (2L) was added, and the liquid was washed with a 10% by mass sodium hydrogen carbonate aqueous solution. The resultant organic layer was dried over magnesium sulfate, then, filtrated, and the resultant filtrate was condensed under reduced pressure, to obtain a coarse product. The resultant coarse product was purified by silica gel column chromatography (a mixed solvent of methanol and chloroform), then, crystallized using acetonitrile, then, dried under reduced pressure, to obtain a compound L6-3 (6 g). The HPLC area percentage value of the compound L6-3 was 99.1%.

The analysis result of the compound L6-3 was as described below.
LC-MS (APCI, positive): m/z = 404 [M+H]⁺
¹H-NMR (400 MHz, CDCl₃): δ (ppm) = 0.83 (t, 9H), 1.34 (s, 9H), 1.64 (q, 6H), 1.96 (s, 6H), 7.12 (s, 2H), 7.20-7.23 (m, 2H), 7.28-7.34 (m, 3H).

### (Synthesis of phosphorescent compound B6)

A nitrogen gas atmosphere was prepared in a reaction vessel, then, tris(acetylacetonato)iridium(III) (1.4 g), the compound L6-3 (4.6 g) and pentadecane (2 mL) were added, and the mixture was stirred at 300°C for 18 hours. The resultant reaction liquid was cooled down to room temperature, and dissolved in toluene, then, the solution was condensed under reduced pressure, to obtain a coarse product. The resultant coarse product was purified by silica gel column chromatography (a mixed solvent of heptane and ethyl acetate), then, crystallized using a mixed solvent of acetonitrile and toluene. The resultant solid was dried under reduced pressure, to obtain a phosphorescent compound B5 (2.8 g). The HPLC area percentage value of the phosphorescent compound B6 was 99.5% or more.

The analysis result of the phosphorescent compound B6 was as described below.
¹H-NMR (600 MHz, THF-d₈): δ (ppm) = 7.30 (s, 6H), 6.90 (d, 3H), 6.44-6.48 (m, 3H), 6.22-6.26 (m, 3H), 5.77 (d, 3H), 2.10 (s, 9H), 1.89 (s, 9H), 1.56 (s, 18H), 1.38 (s, 27H), 0.73 (t, 27H).

The maximum peak wavelength of the emission spectrum of the phosphorescent compound B6 was 464 nm.

### <Acquisition of compound HM-1, compound EM-1, compound EM-3, compound EM-4 and compound EM-5>

A compound HM-1, a compound EM-1, a compound EM-3, a compound EM-4 and a compound EM-5 were purchased from Luminescence Technology Corp.

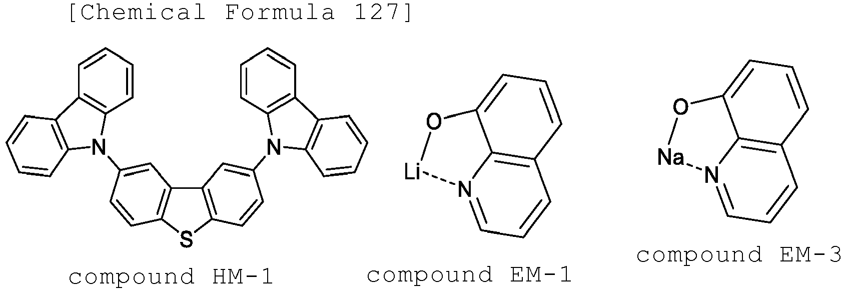

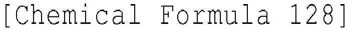

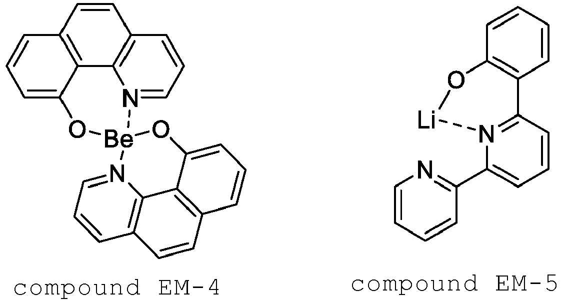

### <Synthesis Example HM-2> Synthesis of compound HM-2

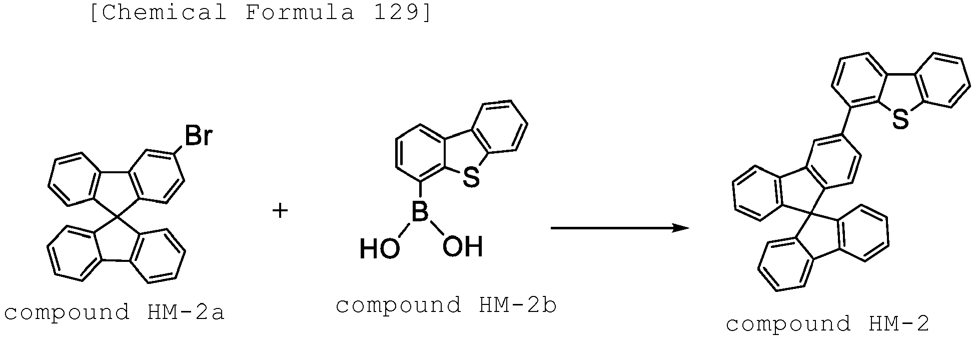

A nitrogen gas atmosphere was prepared in a reaction vessel, then, a compound HM-2a (15.6 g), a compound HM-2b (10.3 g), toluene (390 mL), tetrakis(triphenylphosphine)palladium(0) (2.2 g) and a 20% by mass tetrabutylammonium hydroxide aqueous solution (194 g) were added, and the mixture was stirred at 90°C for 4 hours. The resultant reaction liquid was cooled down to room temperature, then, filtrated through a filter paved with Celite. The resultant filtrate was washed with ion exchanged water, then, the resultant organic layer was dried over anhydrous sodium sulfate, and filtrated. The resultant filtrate was condensed under reduced pressure, to obtain a solid. The resultant solid was crystallized with a mixed solvent of toluene and 2-propanol, then, dried at 50°C under reduced pressure, to obtain a compound HM-2 (15.2 g). The HPLC area percentage value of the compound HM-2 was 99.5% or more.

The analysis result of the compound HM-2 was as described below.
¹H-NMR (CD₂Cl₂, 400 MHz): δ (ppm) = 6.70-6.83 (4H, m), 7.15 (3H, t), 7.39 (3H, t), 7.48 (3H, t), 7.59 (2H, t), 7.83-7.93 (4H, m), 8.18-8.23 (3H, m) .

### <Synthesis Example HM-3> Synthesis of compound HM-3

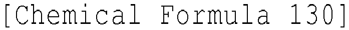

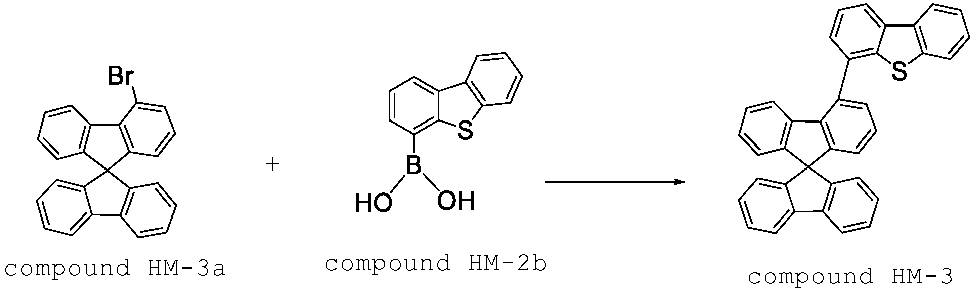

A nitrogen gas atmosphere was prepared in a reaction vessel, then, a compound HM-3a (13.5 g), a compound HM-2b (8.9 g), toluene (404 mL), tetrakis(triphenylphosphine)palladium(0) (2.0 g) and a 20% by mass tetrabutylammonium hydroxide aqueous solution (166 g) were added, and the mixture was stirred at 90°C for 3 hours. The resultant reaction liquid was cooled down to room temperature, then, filtrated through a filter paved with Celite. The resultant filtrate was washed with ion exchanged water, then, the resultant organic layer was dried over anhydrous sodium sulfate, and filtrated. The resultant filtrate was condensed under reduced pressure, to obtain a solid. The resultant solid was purified by silica gel column chromatography (a mixed solvent of hexane and chloroform), and further, crystallized with a mixed solvent of toluene and methanol, then, dried at 50°C under reduced pressure, to obtain a compound HM-3 (10.5 g). The HPLC area percentage value of the compound HM-3 was 99.5% or more.

The analysis result of the compound HM-3 was as described below.
¹H-NMR (CD₂Cl₂, 400 MHz): δ (ppm) = 6.51 (1H, d), 6.60 (1H, d), 6.80 (4H, m), 6.92 (1H, t), 7.21 (3H, m), 7.34 (1H, d), 7.39-7.50 (4H, m), 7.65 (1H, d), 7.71 (1H, t), 7.81 (1H, d), 7.88 (2H, d), 8.28-8.35 (2H, m).

### <Synthesis Example HM-4> Synthesis of compound HM-4

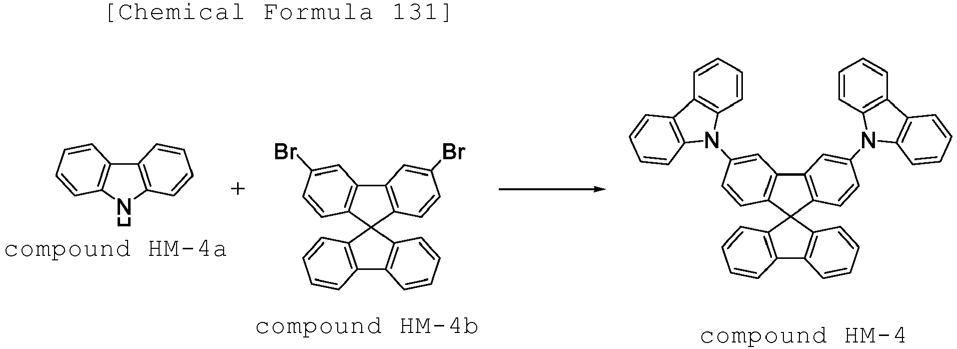

A nitrogen gas atmosphere was prepared in a reaction vessel, then, a compound HM-4a (1.6 g), a compound HM-4b (1.3 g), xylene (63 mL), palladium(II) acetate (22 mg), tri-tert-butylphosphonium tetrafluoroborate (63 mg) and sodium tert-butoxide (1.9 g) were added, and the mixture was stirred for 54 hours under reflux with heat. The resultant reaction liquid was cooled down to room temperature, then, filtrated through a filter paved with silica gel and Celite. The resultant filtrate was washed with ion exchanged water, then, the resultant organic layer was dried over anhydrous sodium sulfate, and filtrated. The resultant filtrate was condensed under reduced pressure, to obtain a solid. The resultant solid was purified by silica gel column chromatography (a mixed solvent of hexane and chloroform), and further, crystallized with a mixed solvent of chloroform and 2-propanol, then, dried at 50°C under reduced pressure, to obtain a compound HM-4 (1.0 g). The HPLC area percentage value of the compound HM-4 was 99.5% or more.

The analysis result of the compound HM-4 was as described below.
¹H-NMR (CD₂Cl₂, 400 MHz): δ (ppm) = 7.08 (4H, t), 7.34 (6H, m), 7.47-7.57 (12H, m), 8.02 (2H, d), 8.12 (2H, s), 8.22 (4H, d) .

### <Synthesis Example HM-5, compound HM-6, compound HM-8 and compound HM-9> Synthesis of compound HM-5, compound HM-6, compound HM-8 and compound HM-9

A compound HM-5 was synthesized with reference to a method described in International Publication WO 2014/023388.

A compound HM-6 and a compound HM-8 were synthesized with reference to a method described in International Publication WO 2012/048820.

A compound HM-9 was synthesized with reference to a method described in International Publication WO 2013/045411.

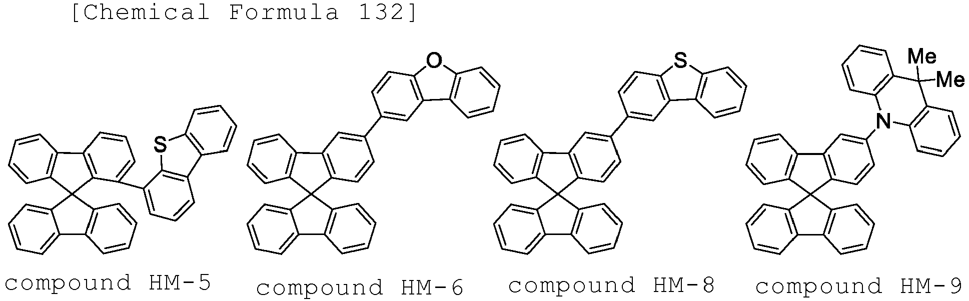

### <Synthesis Example HM-7> Synthesis of compound HM-7

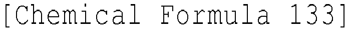

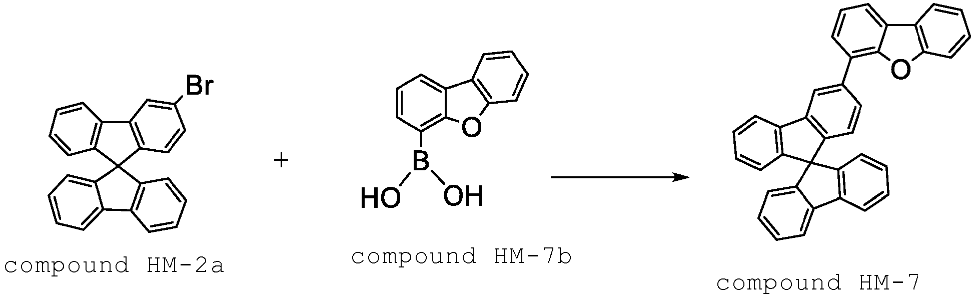

A nitrogen gas atmosphere was prepared in a reaction vessel, then, the compound HM-2a (1.64 g), a compound HM-7b (1.00 g), toluene (40 mL), tetrakis(triphenylphosphine)palladium(0) (0.24 g) and a 20% by mass tetrabutylammonium hydroxide aqueous solution (20 g) were added, and the mixture was stirred at 90°C for 3 hours. The resultant reaction liquid was cooled down to room temperature, then, toluene was added, and the mixture was washed with ion exchanged water. The resultant organic layer was dried over anhydrous magnesium sulfate, then, filtrated through a filter paved with silica gel and Celite. The resultant filtrate was condensed under reduced pressure, to obtain a solid. The resultant solid was crystallized with a mixed solvent of toluene and 2-propanol, then, dried at 50°C under reduced pressure, to obtain a compound HM-7 (1.7 g). The HPLC area percentage value of the compound HM-7 was 99.5% or more.

The analysis result of the compound HM-7 was as described below.
¹H-NMR (CDCl₃, 400 MHz): δ (ppm) = 8.36 (d, 1H), 8.03-7.99 (m, 1H), 7.98-7.93 (m, 2H), 7.89-7.86 (m, 2H), 7.70-7.60 (m, 3H), 7.51-7.35 (m, 6H), 7.17-7.12 (m, 3H), 6.89 (d, 1H), 6.86-6.82 (m, 2H), 6.78 (d, 1H).

### <Synthesis Example HM-10> Synthesis of compound HM-10

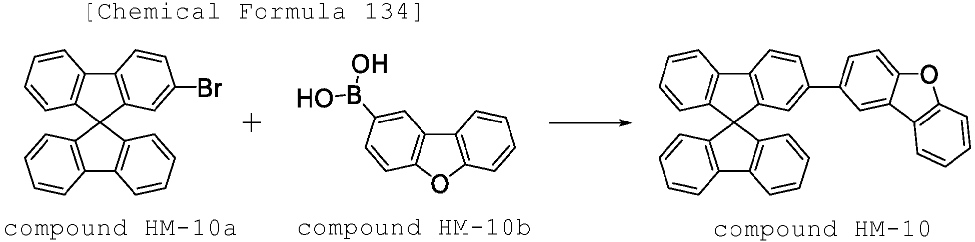

A nitrogen gas atmosphere was prepared in a reaction vessel, then, a compound HM-10a (2.0 g), a compound HM-10b (1.2 g), toluene (50 mL), tetrakis(triphenylphosphine)palladium(0) (0.29 g) and a 20% by mass tetrabutylammonium hydroxide aqueous solution (20 g) were added, and the mixture was stirred at 90°C for 3 hours. The resultant reaction liquid was cooled down to room temperature, then, toluene and ion exchanged water were added, and the mixture was filtrated through a filter paved with Celite. From the resultant filtrate, an aqueous layer was removed, then, the resultant organic layer was washed with ion exchanged water. The resultant organic layer was dried over anhydrous sodium sulfate, and filtrated. To the resultant filtrate was added activated carbon, and the mixture was stirred, then, filtrated through a filter paved with Celite and silica gel. The resultant filtrate was condensed under reduced pressure, to obtain a solid. The resultant solid was crystallized with a mixed solvent of toluene and 2-propanol, then, dried at 50°C under reduced pressure, to obtain a compound HM-10 (1.9 g). The HPLC area percentage value of the compound HM-10 was 99.5% or more.

The analysis result of the compound HM-10 was as described below.
¹H-NMR (CD₂Cl₂, 400 MHz): δ (ppm) = 6.65 (1H, d), 6.74 (2H, d), 7.01 (1H, s), 7.12 (3H, m), 7.28-7.53 (8H, m), 7.73 (1H, d), 7.87-7.99 (6H, m).

### <Synthesis Example HM-11> Synthesis of compound HM-11

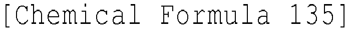

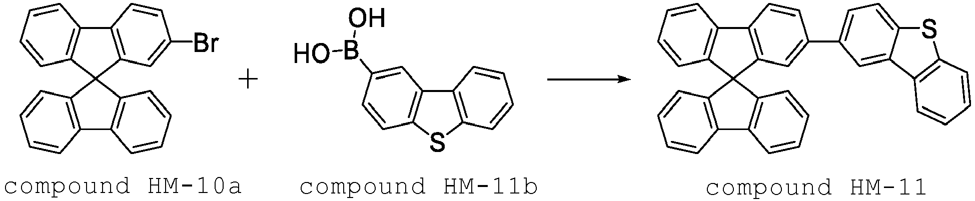

A nitrogen gas atmosphere was prepared in a reaction vessel, then, the compound HM-10a (5.0 g), a compound HM-11b (3.3 g), toluene (125 mL), tetrakis(triphenylphosphine)palladium(0) (0.73 g) and a 20% by mass tetrabutylammonium hydroxide aqueous solution (49 g) were added, and the mixture was stirred at 90°C for 3 hours. The resultant reaction liquid was cooled down to room temperature, then, toluene and ion exchanged water were added, and the mixture was filtrated through a filter paved with Celite. From the resultant filtrate, an aqueous layer was removed, then, the resultant organic layer was washed with ion exchanged water. The resultant organic layer was dried over anhydrous sodium sulfate, and filtrated. To the resultant filtrate was added activated carbon, and the mixture was stirred, then, filtrated through a filter paved with Celite and silica gel. The resultant filtrate was condensed under reduced pressure, to obtain a solid. The resultant solid was crystallized with a mixed solvent of toluene and 2-propanol, then, dried at 50°C under reduced pressure, to obtain a compound HM-11 (5.0 g). The HPLC area percentage value of the compound HM-11 was 99.5% or more.

The analysis result of the compound HM-11 was as described below.
¹H-NMR (CDCl₃, 400 MHz): δ (ppm) = 6.66 (1H, d), 6.74 (2H, d), 7.13 (4H, m), 7.37-7.52 (6H, m), 7.76-7.99 (7H, m), 8.12 (1H, d), 8.19 (1H, s).

### <Compound EM-2> Synthesis of compound EM-2

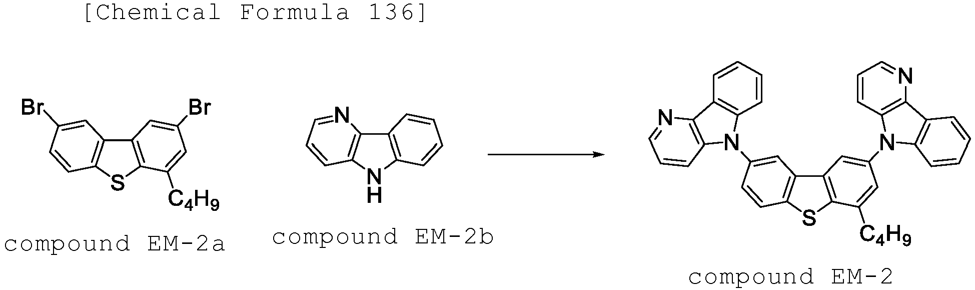

An inert gas atmosphere was prepared in a reaction vessel, then, a compound EM-2a (8.7 g), a compound EM-2b (8.1 g), dimethyl sulfoxide (218 mL), copper(I) oxide (Cu₂ O) (1.3 g), tripotassium phosphate (K₃PO₄) (16.7 g) and dipivaloylmethane (3.2 g) were added, and the mixture was stirred at 150°C for 10 hours. The resultant reaction liquid was cooled down to room temperature, then, toluene and ion exchanged water were added, and the mixture was filtrated through a glass filter paved with Celite. The resultant filtrate was washed with ion exchanged water, then, the resultant organic layer was condensed, to obtain a coarse product. The resultant coarse product was purified by silica gel column chromatography (a mixed solvent of hexane and ethyl acetate), then, crystallized with a mixed solvent of acetonitrile and toluene. The resultant solid was dried at 50°C under reduced pressure, to obtain a compound EM-2 (8.0 g). The HPLC area percentage value of the compound EM-2 was 99.5% or more.

The analysis result of the compound EM-2 was as described below.
LC-MS (ESI, positive): m/z = 573 [M+H]⁺
¹H-NMR (400 MHz, THF-d₈): δ (ppm) = 1.01 (t, 3H), 1.58-1.68 (m, 2H), 1.95-2.05 (m, 2H), 3.14-3.19 (m, 2H), 7.32-7.39 (m, 4H), 7.49-7.57 (m, 4H), 7.72 (s, 1H), 7.79-7.88 (m, 3H), 8.34-8.42 (m, 3H), 8.55-8.68 (m, 4H).

### <Synthesis Example ETL1> Synthesis of polymer compound ETL-1

An inert gas atmosphere was prepared in a reaction vessel, then, the compound M4 (9.23 g), the compound M5 (4.58 g), dichlorobis(tris-o-methoxyphenylphosphine)palladium (8.6 mg), methyltrioctylammonium chloride (manufactured by Sigma Aldrich, trade name: Aliquat336 (registered trademark)) (0.098 g) and toluene (175 mL) were added, and the mixture was heated at 105°C. Thereafter, into this was dropped a 12% by mass sodium carbonate aqueous solution (40.3 mL), and the solution was refluxed for 29 hours. Thereafter, to this were added phenylboronic acid (0.47 g) and dichlorobis(tris-o-methoxyphenylphosphine)palladium (8.7 mg), and the solution was refluxed for 14 hours. Thereafter, to this was added a sodium diethyldithiacarbamate aqueous solution, and the mixture was stirred at 80°C for 2 hours. The resultant reaction liquid was cooled, then, dropped into methanol, to generate a precipitate. The resultant precipitate was collected by filtration, and washed with methanol and water, respectively, then, dried. The resultant solid was dissolved in chloroform, and purified by passing through an alumina column and a silica gel column in series through which chloroform had been passed previously. The resultant purified liquid was dropped into methanol, and the mixture was stirred, to generate a precipitate. The resultant precipitate was collected by filtration, and dried, to obtain a polymer compound ETL-1a (7.15 g). The polymer compound ETL-1a had an Mn of 3.2×10⁴ and an Mw of 6.0×10⁴.

The polymer compound ETL-1a is a copolymer constituted of a constitutional unit derived from the compound M4 and a constitutional unit derived from the compound M5 at a molar ratio of 50:50 according to the theoretical values calculated from the amounts of the charged raw materials.

An argon gas atmosphere was prepared in a reaction vessel, then, the polymer compound ETL-1a (3.1 g), tetrahydrofuran (130 mL), methanol (66 mL), cesium hydroxide monohydrate (2.1 g) and water (12.5 mL) were added, and the mixture was stirred at 60°C for 3 hours. Thereafter, to this was added methanol (220 mL), and the mixture was stirred for 2 hours. The resultant reaction mixture was condensed, then, dropped into isopropyl alcohol, and stirred, to generate a precipitate. The resultant precipitate was collected by filtration, and dried, to obtain a polymer compound ETL-1 (3.5 g). The polymer compound ETL-1 was analyzed by ¹H-NMR, to confirm that the signal of the ethyl ester site in the polymer compound ETL-1 disappeared and the reaction was completed.

The polymer compound ETL-1 is a copolymer constituted of a constitutional unit represented by the following formula and a constitutional unit derived from the compound M5 at a molar ratio of 50:50 according to the theoretical values calculated from the amounts of the charged raw materials of the polymer compound ETL-1a.

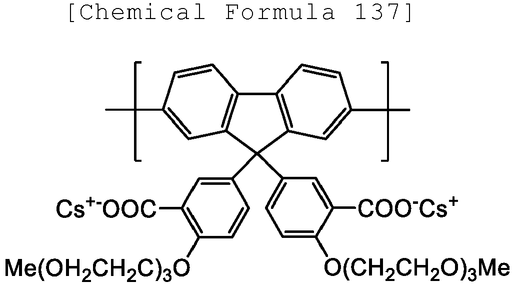

### <Example D1> Fabrication and evaluation of light emitting device D1

### (Formation of anode and hole injection layer)

An ITO film with a thickness of 45 nm was deposited on a glass substrate by a sputtering method, to form an anode. On the anode, a hole injection material ND-3202 (manufactured by Nissan Chemical Corporation) was spin-coated, to form a film with a thickness of 35 nm. In an air atmosphere, the film was heated on a hot plate at 50°C for 3 minutes, and further, heated at 230°C for 15 minutes, to form a hole injection layer.

### (Formation of second light emitting layer)

The polymer compound HTL-1 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a second light emitting layer. By this heating, the polymer compound HTL-1 became a cross-linked body.

### (Formation of first layer)

The compound HM-2, the phosphorescent compound B1 and the phosphorescent compound G1 (compound HM-2/phosphorescent compound B1/phosphorescent compound G1 = 74% by mass/25% by mass/1% by mass) were dissolved at a concentration of 2.0% by mass in toluene. The resultant toluene solution was spin-coated on the second light emitting layer, to form a film with a thickness of 75 nm, and the film was heated at 130°C for 10 minutes under a nitrogen gas atmosphere, to form a first layer (first light emitting layer).

### (Formation of second layer)

The polymer compound ETL-1 was dissolved in 2,2,3,3,4,4,5,5-octafluoro-1-pentanol at a concentration of 0.25% by mass. The resultant 2,2,3,3,4,4,5,5-octafluoro-1-pentanol solution was spin-coated on the first layer, to form a film with a thickness of 10 nm, and the film was heated at 130°C for 10 minutes under a nitrogen gas atmosphere, to form a second layer (electron transporting layer).

### (Formation of third layer)

The substrate carrying the second layer formed thereon was placed in a vapor deposition machine and the inner pressure thereof was reduced to 1.0×10⁻⁴ Pa or less, then, on the second layer, sodium fluoride (NaF) was vapor-deposited with a thickness of 4 nm as the third layer (electron injection layer).

### (Formation of cathode)

The substrate carrying the third layer formed thereon was placed in a vapor deposition machine and the inner pressure thereof was reduced to 1.0×10⁻⁴ Pa or less, then, as cathode, aluminum was vapor-deposited with a thickness of about 80 nm. After vapor deposition, sealing was performed with a glass substrate, to fabricate a light emitting device D1.

### (Evaluation of light emitting device)

Voltage was applied to the light emitting device D1, to observe EL light emission. The CIE chromaticity coordinate (x, y) at 100 cd/m² was (0.44, 0.47). The light emitting device was driven under constant current at a current value of 1 mA, and the time until the luminance reached 80% of the initial luminance was measured.

### <Example D2> Fabrication and evaluation of light emitting device D2

A light emitting device D2 was fabricated in the same manner as in Example D1, except that "the compound HM-3" was used instead of "the compound HM-2" in (Formation of first layer) in Example D1.

Voltage was applied to the light emitting device D2, to observe EL light emission. The CIE chromaticity coordinate (x, y) at 100 cd/m² was (0.43, 0.45). The light emitting device was driven under constant current at a current value of 1 mA, and the time until the luminance reached 80% of the initial luminance was measured.

### <Example D3> Fabrication and evaluation of light emitting device D3

A light emitting device D3 was fabricated in the same manner as in Example D1, except that "barium (Ba)" was used instead of "sodium fluoride" in (Formation of third layer) in Example D1.

Voltage was applied to the light emitting device D3, to observe EL light emission. The CIE chromaticity coordinate (x, y) at 100 cd/m² was (0.44, 0.47). The light emitting device was driven under constant current at a current value of 1 mA, and the time until the luminance reached 80% of the initial luminance was measured.

### <Example D4> Fabrication and evaluation of light emitting device D4

A light emitting device D4 was fabricated in the same manner as in Example D2, except that "barium (Ba)" was used instead of "sodium fluoride" in (Formation of third layer) in Example D2.

Voltage was applied to the light emitting device D4, to observe EL light emission. The CIE chromaticity coordinate (x, y) at 100 cd/m² was (0.42, 0.46). The light emitting device was driven under constant current at a current value of 1 mA, and the time until the luminance reached 80% of the initial luminance was measured.

### <Example D5> Fabrication and evaluation of light emitting device D5

A light emitting device D5 was fabricated in the same manner as in Example D1, except that "calcium (Ca) was vapor-deposited with a thickness of 5 nm." instead of "sodium fluoride (NaF) was vapor-deposited with a thickness of 4 nm." in (Formation of third layer) in Example D1.

Voltage was applied to the light emitting device D5, to observe EL light emission. The CIE chromaticity coordinate (x, y) at 100 cd/m² was (0.44, 0.47). The light emitting device was driven under constant current at a current value of 1 mA, and the time until the luminance reached 80% of the initial luminance was measured.

### <Example D6> Fabrication and evaluation of light emitting device D6

A light emitting device D6 was fabricated in the same manner as in Example D2, except that "calcium was vapor-deposited with a thickness of 5 nm." instead of "sodium fluoride (NaF) was vapor-deposited with a thickness of 4 nm." in (Formation of third layer) in Example D2.

Voltage was applied to the light emitting device D6, to observe EL light emission. The CIE chromaticity coordinate (x, y) at 100 cd/m² was (0.42, 0.46). The light emitting device was driven under constant current at a current value of 1 mA, and the time until the luminance reached 80% of the initial luminance was measured.

### <Comparative Example CD1> Fabrication and evaluation of light emitting device CD1

A light emitting device CD1 was fabricated in the same manner as in Example D1, except that "the compound HM-1" was used instead of "the compound HM-2" in (Formation of first layer) in Example D1.

Voltage was applied to the light emitting device CD1, to observe EL light emission. The CIE chromaticity coordinate (x, y) at 100 cd/m² was (0.43, 0.47). The light emitting device was driven under constant current at a current value of 1 mA, and the time until the luminance reached 80% of the initial luminance was measured.

### <Comparative Example CD2> Fabrication and evaluation of light emitting device CD2

A light emitting device CD2 was fabricated in the same manner as in Comparative Example CD1, except that "barium (Ba)" was used instead of "sodium fluoride" in (Formation of third layer) in Comparative Example CD1. Voltage was applied to the light emitting device CD2, to observe EL light emission. The CIE chromaticity coordinate (x, y) at 100 cd/m² was (0.44, 0.46). The light emitting device was driven under constant current at a current value of 1 mA, and the time until the luminance reached 80% of the initial luminance was measured.

### <Comparative Example CD3> Fabrication and evaluation of light emitting device CD3

A light emitting device CD3 was fabricated in the same manner as in Example D1, except that "calcium (Ca) was vapor-deposited with a thickness of 5 nm." instead of "sodium fluoride (NaF) was vapor-deposited with a thickness of 4 nm." in (Formation of third layer) in Comparative Example CD1.

Voltage was applied to the light emitting device CD3, to observe EL light emission. The CIE chromaticity coordinate (x, y) at 100 cd/m² was (0.43, 0.46). The light emitting device was driven under constant current at a current value of 1 mA, and the time until the luminance reached 80% of the initial luminance was measured.

### <Comparative Example CD4> Fabrication and evaluation of light emitting device CD4

A light emitting device CD4 was fabricated in the same manner as in Example D2, except that (Formation of second layer) was not conducted in Example D2.

Voltage was applied to the light emitting device CD4, to observe EL light emission. The CIE chromaticity coordinate (x, y) at 100 cd/m² was (0.37, 0.47). The light emitting device was driven under constant current at a current value of 1 mA, and the time until the luminance reached 80% of the initial luminance was measured.

### <Comparative Example CD5> Fabrication and evaluation of light emitting device CD5

A light emitting device CD5 was fabricated in the same manner as in Example D4, except that (Formation of second layer) was not conducted in Example D4.

Voltage was applied to the light emitting device CD5, to observe EL light emission. The CIE chromaticity coordinate (x, y) at 100 cd/m² was (0.37, 0.47). The light emitting device was driven under constant current at a current value of 1 mA, and the time until the luminance reached 80% of the initial luminance was measured.

### <Comparative Example CD6> Fabrication and evaluation of light emitting device CD6

A light emitting device CD6 was fabricated in the same manner as in Example D6, except that (Formation of second layer) was not conducted in Example D6.

Voltage was applied to the light emitting device CD6, to observe EL light emission. The CIE chromaticity coordinate (x, y) at 100 cd/m² was (0.36, 0.47). The light emitting device was driven under constant current at a current value of 1 mA, and the time until the luminance reached 80% of the initial luminance was measured.

The results of Examples D1 to D6 and Comparative Examples CD1 to CD6 are shown in Table 2. The relative value of the time (luminance life) until the luminance of the light emitting devices D1 to D6 and light emitting devices CD2 to CD6 reached 80% of the initial luminance when the time (luminance life) until the luminance of the light emitting device CD1 reached 80% of the initial luminance was taken as 1.0 is shown.

**[Table 2]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D1 | D1 | HM-2/B1/G1 | 74/25/1 | ETL-1 | 100 | NaF | 100 | 2.3 |
| Example D2 | D2 | HM-3/B1/G1 | 74/25/1 | ETL-1 | 100 | NaF | 100 | 1.8 |
| Example D3 | D3 | HM-2/B1/G1 | 74/25/1 | ETL-1 | 100 | Ba | 100 | 2.0 |
| Example D4 | D4 | HM-3/B1/G1 | 74/25/1 | ETL-1 | 100 | Ba | 100 | 1.8 |
| Example D5 | D5 | HM-2/B1/G1 | 74/25/1 | ETL-1 | 100 | Ca | 100 | 1.8 |
| Example D6 | D6 | HM-3/B1/G1 | 74/25/1 | ETL-1 | 100 | Ca | 100 | 1.5 |
| Comparative Example CD1 | CD1 | HM-1/B1/G1 | 74/25/1 | ETL-1 | 100 | NaF | 100 | 1.0 |
| Comparative Example CD2 | CD2 | HM-1/B1/G1 | 74/25/1 | ETL-1 | 100 | Ba | 100 | 0.9 |
| Comparative Example CD3 | CD3 | HM-1/B1/G1 | 74/25/1 | ETL-1 | 100 | Ca | 100 | 0.8 |
| Comparative Example CD4 | CD4 | HM-3/B1/G1 | 74/25/1 | - | - | NaF | 100 | 0.2 |
| Comparative Example CD5 | CD5 | HM-3/B1/G1 | 74/25/1 | - | - | Ba | 100 | 0.05 |
| Comparative Example CD6 | CD6 | HM-3/B1/G1 | 74/25/1 | - | - | Ca | 100 | 0.02 |

### <Example D7> Fabrication and evaluation of light emitting device D7

A light emitting device D7 was fabricated in the same manner as in Example D1, except that "lithium fluoride (LiF) was vapor-deposited with a thickness of 1.8 nm." instead of "sodium fluoride (NaF) was vapor-deposited with a thickness of 4 nm." in (Formation of third layer) in Example D1.

Voltage was applied to the light emitting device D7, to observe EL light emission. The CIE chromaticity coordinate (x, y) at 100 cd/m² was (0.43, 0.47). The light emitting device was driven under constant current at a current value of 1 mA, and the time until the luminance reached 80% of the initial luminance was measured.

### <Example D8> Fabrication and evaluation of light emitting device D8

A light emitting device D8 was fabricated in the same manner as in Example D2, except that "lithium fluoride (LiF) was vapor-deposited with a thickness of 1.8 nm." instead of "sodium fluoride (NaF) was vapor-deposited with a thickness of 4 nm." in (Formation of third layer) in Example D2.

Voltage was applied to the light emitting device D8, to observe EL light emission. The CIE chromaticity coordinate (x, y) at 100 cd/m² was (0.41, 0.47). The light emitting device was driven under constant current at a current value of 1 mA, and the time until the luminance reached 80% of the initial luminance was measured.

### <Example D9> Fabrication and evaluation of light emitting device D9

A light emitting device D9 was fabricated in the same manner as in Example D7, except that "the compound HM-4" was used instead of "the compound HM-2" in (Formation of first layer) in Example D7.

Voltage was applied to the light emitting device D9, to observe EL light emission. The CIE chromaticity coordinate (x, y) at 100 cd/m² was (0.33, 0.50). The light emitting device was driven under constant current at a current value of 1 mA, and the time until the luminance reached 80% of the initial luminance was measured.

### <Example D10> Fabrication and evaluation of light emitting device D10

A light emitting device D10 was fabricated in the same manner as in Example D1, except that (Formation of second layer) in Example D1 was changed to (Formation of second layer: -D10) described below, and further, (Formation of third layer) in Example D1 was changed to (Formation of third layer: -D10) described below.

### (Formation of second layer: -D10)

The substrate carrying the first layer formed thereon was placed in a vapor deposition machine and the inner pressure thereof was reduced to 1.0×10⁻⁴ Pa or less, then, on the first layer, sodium fluoride was vapor-deposited with a thickness of 4 nm as the second layer (electron transporting layer).

### (Formation of third layer: -D10)

The substrate carrying the second layer formed thereon was placed in a vapor deposition machine and the inner pressure thereof was reduced to 1.0×10⁻⁴ Pa or less, then, on the second layer, barium was vapor-deposited with a thickness of 1 nm as the third layer (electron injection layer).

Voltage was applied to the light emitting device D10, to observe EL light emission. The CIE chromaticity coordinate (x, y) at 100 cd/m² was (0.38, 0.49). The light emitting device was driven under constant current at a current value of 1 mA, and the time until the luminance reached 80% of the initial luminance was measured.

### <Example D11> Fabrication and evaluation of light emitting device D11

A light emitting device D11 was fabricated in the same manner as in Example D10, except that "the compound HM-3" was used instead of "the compound HM-2" in (Formation of first layer) in Example D10.

Voltage was applied to the light emitting device D11, to observe EL light emission. The CIE chromaticity coordinate (x, y) at 100 cd/m² was (0.37, 0.48). The light emitting device was driven under constant current at a current value of 1 mA, and the time until the luminance reached 80% of the initial luminance was measured.

### <Example D12> Fabrication and evaluation of light emitting device D12

A light emitting device D12 was fabricated in the same manner as in Example D10, except that "lithium fluoride was vapor-deposited with a thickness of 1.8 nm." instead of "sodium fluoride was vapor-deposited with a thickness of 4 nm." in (Formation of second layer: -D10), and further, "calcium was vapor-deposited with a thickness of 5 nm." instead of "barium was vapor-deposited with a thickness of 1 nm." in (Formation of third layer: -D10) in Example D10.

Voltage was applied to the light emitting device D12, to observe EL light emission. The CIE chromaticity coordinate (x, y) at 100 cd/m² was (0.39, 0.47). The light emitting device was driven under constant current at a current value of 1 mA, and the time until the luminance reached 80% of the initial luminance was measured.

### <Example D13> Fabrication and evaluation of light emitting device D13

A light emitting device D13 was fabricated in the same manner as in Example D12, except that "the compound HM-3" was used instead of "the compound HM-2" in (Formation of first layer) in Example D12.

Voltage was applied to the light emitting device D13, to observe EL light emission. The CIE chromaticity coordinate (x, y) at 100 cd/m² was (0.38, 0.47). The light emitting device was driven under constant current at a current value of 1 mA, and the time until the luminance reached 80% of the initial luminance was measured.

### <Example D14> Fabrication and evaluation of light emitting device D14

A light emitting device D14 was fabricated in the same manner as in Example D12, except that "the compound HM-4" was used instead of "the compound HM-2" in (Formation of first layer) in Example D12.

Voltage was applied to the light emitting device D14, to observe EL light emission. The CIE chromaticity coordinate (x, y) at 100 cd/m² was (0.33, 0.48). The light emitting device was driven under constant current at a current value of 1 mA, and the time until the luminance reached 80% of the initial luminance was measured.

### <Comparative Example CD7> Fabrication and evaluation of light emitting device CD7

A light emitting device CD7 was fabricated in the same manner as in Example D7, except that (Formation of second layer) was not conducted in Example D8.

Voltage was applied to the light emitting device CD7, to observe EL light emission. The CIE chromaticity coordinate (x, y) at 100 cd/m² was (0.37, 0.47). The light emitting device was driven under constant current at a current value of 1 mA, and the time until the luminance reached 80% of the initial luminance was measured.

The results of Examples D7 to D14 and Comparative Example CD7 are shown in Table 3. The relative value of the time (luminance life) until the luminance of the light emitting devices D7 to D14 reached 80% of the initial luminance when the time (luminance life) until the luminance of the light emitting device CD7 reached 80% of the initial luminance was taken as 1 is shown.

**[Table 3]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D7 | D7 | HM-2/B1/G1 | 74/25/1 | ETL-1 | 100 | LiF | 100 | 2164 |
| Example D8 | D8 | HM-3/B1/G1 | 74/25/1 | ETL-1 | 100 | LiF | 100 | 1825 |
| Example D9 | D9 | HM-4/B1/G1 | 74/25/1 | ETL-1 | 100 | LiF | 100 | 230 |
| Example D10 | D10 | HM-2/B1/G1 | 74/25/1 | NaF | 100 | Ba | 100 | 2024 |
| Example D11 | D11 | HM-3/B1/G1 | 74/25/1 | NaF | 100 | Ba | 100 | 1885 |
| Example D12 | D12 | HM-2/B1/G1 | 74/25/1 | LiF | 100 | Ca | 100 | 523 |
| Example D13 | D13 | HM-3/B1/G1 | 74/25/1 | LiF | 100 | Ca | 100 | 397 |
| Example D14 | D14 | HM-4/B1/G1 | 74/25/1 | LiF | 100 | Ca | 100 | 282 |
| Comparative Example CD7 | CD7 | HM-3/B1/G1 | 74/25/1 | - | - | LiF | 100 | 1 |

### <Example D15> Fabrication and evaluation of light emitting device D15

A light emitting device D1 was fabricated in the same manner as in Example D1 (in the present example, referred to as "light emitting device D15").

Voltage was applied to the light emitting device D15, to observe EL light emission. The CIE chromaticity coordinate (x, y) at 100 cd/m² was (0.44, 0.47). The light emitting device was driven under constant current at a current value of 1 mA, and the time until the luminance reached 95% of the initial luminance was measured.

### <Example D16> Fabrication and evaluation of light emitting device D16

A light emitting device D16 was fabricated in the same manner as in Example D1, except that "the phosphorescent compound B4" was used instead of "the phosphorescent compound B1" in (Formation of first layer) in Example D1.

Voltage was applied to the light emitting device D16, to observe EL light emission. The CIE chromaticity coordinate (x, y) at 100 cd/m² was (0.47, 0.46). The light emitting device was driven under constant current at a current value of 1 mA, and the time until the luminance reached 95% of the initial luminance was measured.

### <Example D17> Fabrication and evaluation of light emitting device D17

A light emitting device D17 was fabricated in the same manner as in Example D1, except that "the compound HM-7" was used instead of "the compound HM-2" in (Formation of first layer) in Example D1.

Voltage was applied to the light emitting device D17, to observe EL light emission. The CIE chromaticity coordinate (x, y) at 100 cd/m² was (0.46, 0.46). The light emitting device was driven under constant current at a current value of 1 mA, and the time until the luminance reached 95% of the initial luminance was measured.

### <Example D18> Fabrication and evaluation of light emitting device D18

A light emitting device D18 was fabricated in the same manner as in Example D1, except that "the compound HM-5" was used instead of "the compound HM-2" in (Formation of first layer) in Example D1.

Voltage was applied to the light emitting device D18, to observe EL light emission. The CIE chromaticity coordinate (x, y) at 100 cd/m² was (0.42, 0.46). The light emitting device was driven under constant current at a current value of 1 mA, and the time until the luminance reached 95% of the initial luminance was measured.

### <Example D19> Fabrication and evaluation of light emitting device D19

A light emitting device D19 was fabricated in the same manner as in Example D1, except that "the compound HM-3" was used instead of "the compound HM-2" in (Formation of first layer) in Example D1.

Voltage was applied to the light emitting device D19, to observe EL light emission. The CIE chromaticity coordinate (x, y) at 100 cd/m² was (0.43, 0.45). The light emitting device was driven under constant current at a current value of 1 mA, and the time until the luminance reached 95% of the initial luminance was measured.

### <Example D20> Fabrication and evaluation of light emitting device D20

A light emitting device D20 was fabricated in the same manner as in Example D1, except that "the compound HM-8" was used instead of "the compound HM-2" in (Formation of first layer) in Example D1.

Voltage was applied to the light emitting device D20, to observe EL light emission. The CIE chromaticity coordinate (x, y) at 100 cd/m² was (0.46, 0.46). The light emitting device was driven under constant current at a current value of 1 mA, and the time until the luminance reached 95% of the initial luminance was measured.

### <Example D21> Fabrication and evaluation of light emitting device D21

A light emitting device D21 was fabricated in the same manner as in Example D1, except that "the compound HM-6" was used instead of "the compound HM-2" in (Formation of first layer) in Example D1.

Voltage was applied to the light emitting device D21, to observe EL light emission. The CIE chromaticity coordinate (x, y) at 100 cd/m² was (0.44, 0.47). The light emitting device was driven under constant current at a current value of 1 mA, and the time until the luminance reached 95% of the initial luminance was measured.

### <Comparative Example CD8> Fabrication and evaluation of light emitting device CD8

A light emitting device CD8 was fabricated in the same manner as in Example D1, except that "the compound HM-1" was used instead of "the compound HM-2" in (Formation of first layer) in Example D1.

Voltage was applied to the light emitting device CD8, to observe EL light emission. The CIE chromaticity coordinate (x, y) at 100 cd/m² was (0.43, 0.47). The light emitting device was driven under constant current at a current value of 1 mA, and the time until the luminance reached 95% of the initial luminance was measured.

The results of Examples D15 to D21 and Comparative Example CD8 are shown in Table 4. The relative value of the time (luminance life) until the luminance of the light emitting devices D15 to D21 reached 95% of the initial luminance when the time (luminance life) until the luminance of the light emitting device CD8 reached 95% of the initial luminance was taken as 1.0 is shown.

**[Table 4]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D15 | D15 | HM-2/B1/G1 | 74/25/1 | ETL-1 | 100 | NaF | 100 | 2.1 |
| Example D16 | D16 | HM-2/B4/G1 | 74/25/1 | ETL-1 | 100 | NaF | 100 | 4.0 |
| Example D17 | D17 | HM-7/B1/G1 | 74/25/1 | ETL-1 | 100 | NaF | 100 | 2.1 |
| Example D18 | D18 | HM-5/B1/G1 | 74/25/1 | ETL-1 | 100 | NaF | 100 | 3.3 |
| Example D19 | D19 | HM-3/B1/G1 | 74/25/1 | ETL-1 | 100 | NaF | 100 | 2.0 |
| Example D20 | D20 | HM-8/B1/G1 | 74/25/1 | ETL-1 | 100 | NaF | 100 | 1.2 |
| Example D21 | D21 | HM-6/B1/G1 | 74/25/1 | ETL-1 | 100 | NaF | 100 | 1.5 |
| Comparative Example CD8 | CD8 | HM-1/B1/G1 | 74/25/1 | ETL-1 | 100 | NaF | 100 | 1.0 |

### <Example D22> Fabrication and evaluation of light emitting device D22

A light emitting device D22 was fabricated in the same manner as in Example D1, except that (Formation of first layer) was changed to (Formation of first layer: -D22) described below, in Example D1.

### (Formation of first layer: -D22)

The compound HM-2, the phosphorescent compound B3 and the phosphorescent compound G1 (compound HM-2/phosphorescent compound B3/phosphorescent compound G1 = 74% by mass/25% by mass/1% by mass) were dissolved in xylene at a concentration of 2.0% by mass. The resultant xylene solution was spin-coated on the second light emitting layer, to form a film with a thickness of 75 nm, and the film was heated at 130°C for 10 minutes under a nitrogen gas atmosphere, to form a first layer (first light emitting layer).

Voltage was applied to the light emitting device D22, to observe EL light emission. The CIE chromaticity coordinate (x, y) at 100 cd/m² was (0.41, 0.46). The light emitting device was driven under constant current at an initial luminance of 6000 cd/m², and the time until the luminance reached 70% of the initial luminance was measured.

### <Comparative Example CD9> Fabrication and evaluation of light emitting device CD9

A light emitting device CD9 was fabricated in the same manner as in Example D22, except that "the compound HM-1" was used instead of "the compound HM-2" in (Formation of first layer: -D22) in Example D22.

Voltage was applied to the light emitting device CD9, to observe EL light emission. The CIE chromaticity coordinate (x, y) at 100 cd/m² was (0.41, 0.44). The light emitting device was driven under constant current at an initial luminance of 6000 cd/m², and the time until the luminance reached 70% of the initial luminance was measured.

The results of Example D22 and Comparative Example CD9 are shown in Table 5. The relative value of the time (luminance life) until the luminance of the light emitting device D22 reached 70% of the initial luminance when the time (luminance life) until the luminance of the light emitting device CD9 reached 70% of the initial luminance was taken as 1.0 is shown.

**[Table 5]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D22 | D22 | HM-2/B3/G1 | 74/25/1 | ETL-1 | 100 | NaF | 100 | 2.6 |
| Comparative Example CD9 | CD9 | HM-1/B3/G1 | 74/25/1 | ETL-1 | 100 | NaF | 100 | 1.0 |

### <Example D23> Fabrication and evaluation of light emitting device D23

A light emitting device D23 was fabricated in the same manner as in Example D1, except that "the phosphorescent compound B2" was used instead of "the phosphorescent compound B1" in (Formation of first layer) in Example D1.

Voltage was applied to the light emitting device D23, to observe EL light emission. The CIE chromaticity coordinate (x, y) at 100 cd/m² was (0.46, 0.45). The light emitting device was driven under constant current at a current value of 1 mA, and the time until the luminance reached 75% of the initial luminance was measured.

### <Example D24> Fabrication and evaluation of light emitting device D24

A light emitting device D24 was fabricated in the same manner as in Example D23, except that "the compound HM-3" was used instead of "the compound HM-2" in (Formation of first layer) in Example D23.

Voltage was applied to the light emitting device D24, to observe EL light emission. The CIE chromaticity coordinate (x, y) at 100 cd/m² was (0.45, 0.46). The light emitting device was driven under constant current at a current value of 1 mA, and the time until the luminance reached 75% of the initial luminance was measured.

### <Example D25> Fabrication and evaluation of light emitting device D25

A light emitting device D25 was fabricated in the same manner as in Example D23, except that "the compound HM-6" was used instead of "the compound HM-2" in (Formation of first layer) in Example D23.

Voltage was applied to the light emitting device D25, to observe EL light emission. The CIE chromaticity coordinate (x, y) at 100 cd/m² was (0.45, 0.46). The light emitting device was driven under constant current at a current value of 1 mA, and the time until the luminance reached 75% of the initial luminance was measured.

### <Example D26> Fabrication and evaluation of light emitting device D26

A light emitting device D26 was fabricated in the same manner as in Example D23, except that "the compound HM-7" was used instead of "the compound HM-2" in (Formation of first layer) in Example D23.

Voltage was applied to the light emitting device D26, to observe EL light emission. The CIE chromaticity coordinate (x, y) at 100 cd/m² was (0.47, 0.46). The light emitting device was driven under constant current at a current value of 1 mA, and the time until the luminance reached 75% of the initial luminance was measured.

### <Example D27> Fabrication and evaluation of light emitting device D27

A light emitting device D27 was fabricated in the same manner as in Example D23, except that "the compound HM-8" was used instead of "the compound HM-2" in (Formation of first layer) in Example D23.

Voltage was applied to the light emitting device D27, to observe EL light emission. The CIE chromaticity coordinate (x, y) at 100 cd/m² was (0.46, 0.46). The light emitting device was driven under constant current at a current value of 1 mA, and the time until the luminance reached 75% of the initial luminance was measured.

### <Comparative Example CD10> Fabrication and evaluation of light emitting device CD10

A light emitting device CD10 was fabricated in the same manner as in Example D23, except that "the compound HM-1" was used instead of "the compound HM-2" in (Formation of first layer) in Example D23.

Voltage was applied to the light emitting device CD10, to observe EL light emission. The CIE chromaticity coordinate (x, y) at 100 cd/m² was (0.48, 0.45). The light emitting device was driven under constant current at a current value of 1 mA, and the time until the luminance reached 75% of the initial luminance was measured.

The results of Examples D23 to D27 and Comparative Example CD10 are shown in Table 6. The relative value of the time (luminance life) until the luminance of the light emitting devices D23 to D27 reached 75% of the initial luminance when the time (luminance life) until the luminance of the light emitting device CD10 reached 75% of the initial luminance was taken as 1.00 is shown.

**[Table 6]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D23 | D23 | HM-2/B2/G1 | 74/25/1 | ETL-1 | 100 | NaF | 100 | 1.92 |
| Example D24 | D24 | HM-3/B2/G1 | 74/25/1 | ETL-1 | 100 | NaF | 100 | 1.42 |
| Example D25 | D25 | HM-6/B2/G1 | 74/25/1 | ETL-1 | 100 | NaF | 100 | 1.17 |
| Example D26 | D26 | HM-7/B2/G1 | 74/25/1 | ETL-1 | 100 | NaF | 100 | 1.43 |
| Example D27 | D27 | HM-8/B2/G1 | 74/25/1 | ETL-1 | 100 | NaF | 100 | 1.20 |
| Comparative Example CD10 | CD10 | HM-1/B2/G1 | 74/25/1 | ETL-1 | 100 | NaF | 100 | 1.00 |

### <Example D28> Fabrication and evaluation of light emitting device D28

### (Formation of anode and hole injection layer)

An ITO film with a thickness of 45 nm was deposited on a glass substrate by a sputtering method, to form an anode. On the anode, a hole injection material ND-3202 (manufactured by Nissan Chemical Corporation) was spin-coated, to form a film with a thickness of 35 nm. In an air atmosphere, the film was heated on a hot plate at 50°C for 3 minutes, and further, heated at 230°C for 15 minutes, to form a hole injection layer.

### (Formation of second light emitting layer)

The polymer compound HTL-1 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a second light emitting layer. By this heating, the polymer compound HTL-1 became a cross-linked body.

### (Formation of first layer)

The compound HM-2 and the phosphorescent compound B1 (compound HM-2/phosphorescent compound B1 = 75% by mass/25% by mass) were dissolved at a concentration of 2.0% by mass in toluene. The resultant toluene solution was spin-coated on the second light emitting layer, to form a film with a thickness of 75 nm, and the film was heated at 130°C for 10 minutes under a nitrogen gas atmosphere, to form a first layer (first light emitting layer).

### (Formation of second layer)

The substrate carrying the first organic layer formed thereon was placed in a vapor deposition machine and the inner pressure thereof was reduced to 3.0×10⁻⁴ Pa or less, then on the first layer, the compound EM-1 and the compound EM-2 (compound EM-1/compound EM-2 = 25% by mass/75% by mass) were co-vapor-deposited with a thickness of 10 nm as the second layer (electron transporting layer).

### (Formation of third layer)

The substrate carrying the second layer formed thereon was placed in a vapor deposition machine and the inner pressure thereof was reduced to 1.0×10⁻⁴ Pa or less, then, on the second layer, barium was vapor-deposited with a thickness of 4 nm as the third layer (electron injection layer).

### (Formation of cathode)

The substrate carrying the third layer formed thereon was placed in a vapor deposition machine and the inner pressure thereof was reduced to 1.0×10⁻⁴ Pa or less, then, as cathode, aluminum was vapor-deposited with a thickness of about 80 nm. After vapor deposition, sealing was performed using a glass substrate, to fabricate a light emitting device D28.

### (Evaluation of light emitting device)

Voltage was applied to the light emitting device D28, to observe EL light emission. The CIE chromaticity coordinate (x, y) at 100 cd/m² was (0.32, 0.42). The light emitting device was driven under constant current at a current value of 1 mA, and the time until the luminance reached 80% of the initial luminance was measured.

### <Comparative Example CD11> Fabrication and evaluation of light emitting device CD11

A light emitting device CD11 was fabricated in the same manner as in Example D28, except that "the compound HM-1" was used instead of "the compound HM-2" in (Formation of first layer) in Example D28.

Voltage was applied to the light emitting device CD11, to observe EL light emission. The CIE chromaticity coordinate (x, y) at 100 cd/m² was (0.34, 0.41). The light emitting device was driven under constant current at a current value of 1 mA, and the time until the luminance reached 80% of the initial luminance was measured.

### <Comparative Example CD12> Fabrication and evaluation of light emitting device CD12

A light emitting device CD12 was fabricated in the same manner as in Example D28, except that (Formation of third layer) of Example D28 was changed to (Formation of third layer: -CD12) described below.

### (Formation of third layer: -CD12)

The substrate carrying the second layer formed thereon was placed in a vapor deposition machine and the inner pressure thereof was reduced to 3.0×10⁻⁴ Pa or less, then, on the second layer, the compound EM-1 was vapor-deposited with a thickness of 4 nm as the third layer (electron injection layer).

### (Evaluation of light emitting device)

Voltage was applied to the light emitting device CD12, to observe EL light emission. The CIE chromaticity coordinate (x, y) at 100 cd/m² was (0.31, 0.42). The light emitting device was driven under constant current at a current value of 1 mA, and the time until the luminance reached 80% of the initial luminance was measured.

### <Comparative Example CD13> Fabrication and evaluation of light emitting device CD13

A light emitting device CD13 was fabricated in the same manner as in Example D28, except that (Formation of second layer) was not conducted in Example D28.

Voltage was applied to the light emitting device CD13, to observe EL light emission. The CIE chromaticity coordinate (x, y) at 100 cd/m² was (0.34, 0.42). The light emitting device was driven under constant current at a current value of 1 mA, and the time until the luminance reached 80% of the initial luminance was measured.

The results of Example D28 and Comparative Examples CD11 to CD13 are shown in Table 7. The relative value of the time (luminance life) until the luminance of the light emitting device D28 and light emitting devices CD11 and CD13 reached 80% of the initial luminance when the time (luminance life) until the luminance of the light emitting device CD12 reached 80% of the initial luminance was taken as 1.0 is shown.

**[Table 7]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D28 | D28 | HM-2/B1 | 75/25 | EM-1/EM-2 | 25/75 | Ba | 100 | 6.0 |
| Comparative Example CD11 | CD11 | HM-1/B1 | 75/25 | EM-1/EM-2 | 25/75 | Ba | 100 | 2.8 |
| Comparative Example CD12 | CD12 | HM-2/B1 | 75/25 | EM-1/EM-2 | 25/75 | EM-1 | 100 | 1.0 |
| Comparative Example CD13 | CD13 | HM-2/B1 | 75/25 | - | - | Ba | 100 | 0.6 |

### <Example D29> Fabrication and evaluation of light emitting device D29

A light emitting device D29 was fabricated in the same manner as in Example D10, except that "the compound HM-2 and the phosphorescent compound B1 (compound HM-2/phosphorescent compound B1 = 75% by mass/25% by mass)" were used instead of "the compound HM-2, the phosphorescent compound B1 and the phosphorescent compound G1 (compound HM-2/phosphorescent compound B1/phosphorescent compound G1 = 74% by mass/25% by mass/1% by mass)" in (Formation of first layer) in Example D10.

Voltage was applied to the light emitting device D29, to observe EL light emission. The CIE chromaticity coordinate (x, y) at 100 cd/m² was (0.39, 0.41). The light emitting device was driven under constant current at a current value of 1 mA, and the time until the luminance reached 50% of the initial luminance was measured.

### <Comparative Example CD14> Fabrication and evaluation of light emitting device CD14

A light emitting device CD14 was fabricated in the same manner as in Example D29, except that "the compound HM-1" was used instead of "the compound HM-2" in (Formation of first layer) in Example D29.

Voltage was applied to the light emitting device CD14, to observe EL light emission. The CIE chromaticity coordinate (x, y) at 100 cd/m² was (0.40, 0.42). The light emitting device was driven under constant current at a current value of 1 mA, and the time until the luminance reached 50% of the initial luminance was measured.

The results of Example D29 and Comparative Example CD14 are shown in Table 8. The relative value of the time (luminance life) until the luminance of the light emitting device D29 reached 50% of the initial luminance when the time (luminance life) until the luminance of the light emitting device CD14 reached 50% of the initial luminance was taken as 1.0 is shown.

**[Table 8]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D29 | D29 | HM-2/B1 | 75/25 | NaF | 100 | Ba | 100 | 5.0 |
| Comparative Example CD14 | CD14 | HM-1/B1 | 75/25 | NaF | 100 | Ba | 100 | 1.0 |

### <Example D30> Fabrication and evaluation of light emitting device D30

A light emitting device D30 was fabricated in the same manner as in Example D3, except that "the compound HM-2 and the phosphorescent compound B1 (compound HM-2/phosphorescent compound B1 = 75% by mass/25% by mass)" were used instead of "the compound HM-2, the phosphorescent compound B1 and the phosphorescent compound G1 (compound HM-2/phosphorescent compound B1/phosphorescent compound G1 = 74% by mass/25% by mass/1% by mass)" in (Formation of first layer) in Example D3.

Voltage was applied to the light emitting device D30, to observe EL light emission. The CIE chromaticity coordinate (x, y) at 100 cd/m² was (0.47, 0.41). The light emitting device was driven under constant current at a current value of 1 mA, and the time until the luminance reached 60% of the initial luminance was measured.

### <Example D31> Fabrication and evaluation of light emitting device D31

A light emitting device D31 was fabricated in the same manner as in Example D30, except that (Formation of second layer) of Example D30 was changed to (Formation of second layer: -D31) described below.

### (Formation of second layer: -D31)

The substrate carrying the first organic layer formed thereon was placed in a vapor deposition machine and the inner pressure thereof was reduced to 3.0×10⁻⁴ Pa or less, then on the first layer, the compound EM-1 was vapor-deposited with a thickness of 4 nm as the second layer (electron transporting layer).

Voltage was applied to the light emitting device D31, to observe EL light emission. The CIE chromaticity coordinate (x, y) at 100 cd/m² was (0.33, 0.41). The light emitting device was driven under constant current at a current value of 1 mA, and the time until the luminance reached 60% of the initial luminance was measured.

### <Example D32> Fabrication and evaluation of light emitting device D32

A light emitting device D32 was fabricated in the same manner as in Example D31, except that "the compound EM-3" was used instead of "the compound EM-1" in (Formation of second layer: -D31) in Example D31.

Voltage was applied to the light emitting device D32, to observe EL light emission. The CIE chromaticity coordinate (x, y) at 100 cd/m² was (0.33, 0.41). The light emitting device was driven under constant current at a current value of 1 mA, and the time until the luminance reached 60% of the initial luminance was measured.

### <Example D33> Fabrication and evaluation of light emitting device D33

A light emitting device D33 was fabricated in the same manner as in Example D31, except that "the compound EM-4" was used instead of "the compound EM-1" in (Formation of second layer: -D31) in Example D31.

Voltage was applied to the light emitting device D33, to observe EL light emission. The CIE chromaticity coordinate (x, y) at 100 cd/m² was (0.35, 0.41). The light emitting device was driven under constant current at a current value of 1 mA, and the time until the luminance reached 60% of the initial luminance was measured.

### <Example D34> Fabrication and evaluation of light emitting device D34

A light emitting device D34 was fabricated in the same manner as in Example D31, except that "the compound EM-5" was used instead of "the compound EM-1" in (Formation of second layer: -D31) in Example D31.

Voltage was applied to the light emitting device D34, to observe EL light emission. The CIE chromaticity coordinate (x, y) at 100 cd/m² was (0.34, 0.40). The light emitting device was driven under constant current at a current value of 1 mA, and the time until the luminance reached 60% of the initial luminance was measured.

### <Comparative Example CD15> Fabrication and evaluation of light emitting device CD15

A light emitting device CD15 was fabricated in the same manner as in Example D31, except that (Formation of third layer) was not conducted in Example D31.

Voltage was applied to the light emitting device CD15, to observe EL light emission. The CIE chromaticity coordinate (x, y) at 100 cd/m² was (0.33, 0.40). The light emitting device was driven under constant current at a current value of 1 mA, and the time until the luminance reached 60% of the initial luminance was measured.

### <Comparative Example CD16> Fabrication and evaluation of light emitting device CD16

A light emitting device CD16 was fabricated in the same manner as in Example D33, except that (Formation of third layer) was not conducted in Example D33.

Voltage was applied to the light emitting device CD16, to observe EL light emission. The CIE chromaticity coordinate (x, y) at 100 cd/m² was (0.32, 0.40). The light emitting device was driven under constant current at a current value of 1 mA, and the time until the luminance reached 60% of the initial luminance was measured.

The results of Examples D30 to D34 and Comparative Examples CD15 to CD16 are shown in Table 9. The relative value of the time (luminance life) until the luminance of the light emitting devices D30 to D34 and the light emitting device CD15 reached 60% of the initial luminance when the time (luminance life) until the luminance of the light emitting device CD16 reached 60% of the initial luminance was taken as 1.0 is shown.

**[Table 9]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D30 | D30 | HM-2/B1 | 75/25 | ETL-1 | 100 | Ba | 100 | 44.9 |
| Example D31 | D31 | HM-2/B1 | 75/25 | EM-1 | 100 | Ba | 100 | 21.3 |
| Example D32 | D32 | HM-2/B1 | 75/25 | EM-3 | 100 | Ba | 100 | 26.6 |
| Example D33 | D33 | HM-2/B1 | 75/25 | EM-4 | 100 | Ba | 100 | 21.8 |
| Example D34 | D34 | HM-2/B1 | 75/25 | EM-5 | 100 | Ba | 100 | 13.9 |
| Comparative Example CD15 | CD15 | HM-2/B1 | 75/25 | EM-1 | 100 | - | - | 0.1 |
| Comparative Example CD16 | CD16 | HM-2/B1 | 75/25 | EM-4 | 100 | - | - | 1.0 |

### <Example D35> Fabrication and evaluation of light emitting device D35

A light emitting device D35 was fabricated in the same manner as in Example D33, except that (Formation of third layer) of Example D33 was changed to (Formation of third layer: -D35) described below.

### (Formation of third layer: -D35)

The substrate carrying the second layer formed thereon was placed in a vapor deposition machine and the inner pressure thereof was reduced to 1.0×10⁻⁴ Pa or less, then, on the second layer, sodium fluoride (NaF) was vapor-deposited with a thickness of 4 nm as the third layer.

Voltage was applied to the light emitting device D35, to observe EL light emission. The CIE chromaticity coordinate (x, y) at 100 cd/m² was (0.38, 0.40). The light emitting device was driven under constant current at a current value of 1 mA, and the time until the luminance reached 65% of the initial luminance was measured.

### <Example D36> Fabrication and evaluation of light emitting device D36

A light emitting device D35 was fabricated in the same manner as in Example D35, except that (Formation of second layer) of Example D35 was changed to (Formation of second layer: -D36) described below.

### (Formation of second layer: -D36)

The compound EM-5 was dissolved in 2,2,3,3,4,4,5,5-octafluoro-1-pentanol at a concentration of 0.1% by mass. The resultant 2,2,3,3,4,4,5,5-octafluoro-1-pentanol solution was spin-coated on the first layer, to form a film with a thickness of 4 nm, and the film was heated at 130°C for 10 minutes under a nitrogen gas atmosphere, to form a second layer.

Voltage was applied to the light emitting device D36, to observe EL light emission. The CIE chromaticity coordinate (x, y) at 100 cd/m² was (0.31, 0.40). The light emitting device was driven under constant current at a current value of 1 mA, and the time until the luminance reached 65% of the initial luminance was measured.

### <Comparative Example CD17> Fabrication and evaluation of light emitting device CD17

A light emitting device CD17 was fabricated in the same manner as in Example D36, except that (Formation of third layer) of Example D36 was changed to (Formation of third layer: -CD17) described below.

### (Formation of third layer: -CD17)

The substrate carrying the second layer formed thereon was placed in a vapor deposition machine and the inner pressure thereof was reduced to 3.0×10⁻⁴ Pa or less, then, on the second layer, the compound EM-5 was vapor-deposited with a thickness of 4 nm as the third layer.

Voltage was applied to the light emitting device CD17, to observe EL light emission. The light emitting device was driven under constant current at a current value of 1 mA, and the time until the luminance reached 65% of the initial luminance was measured.

The results of Examples D35 to D36 and Comparative Example CD17 are shown in Table 10. The relative value of the time (luminance life) until the luminance of the light emitting devices D35 to D36 reached 65% of the initial luminance when the time (luminance life) until the luminance of the light emitting device CD17 reached 65% of the initial luminance was taken as 1.0 is shown.

**[Table 10]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D35 | D35 | HM-2/B1 | 75/25 | EM-4 | 100 | NaF | 100 | 17.6 |
| Example D36 | D36 | HM-2/B1 | 75/25 | EM-5 | 100 | NaF | 100 | 1.9 |
| Comparative Example CD17 | CD17 | HM-2/B1 | 75/25 | EM-5 | 100 | EM-5 | 100 | 1.0 |

### <Example D37> Fabrication and evaluation of light emitting device D37

A light emitting device D37 was fabricated in the same manner as in Example D30, except that (Formation of third layer) of Example D30 was changed to (Formation of third layer: -D37) described below.

### (Formation of third layer: -D37)

The substrate carrying the second layer formed thereon was placed in a vapor deposition machine and the inner pressure thereof was reduced to 3.0×10⁻⁴ Pa or less, then, on the second layer, the compound EM-1 was vapor-deposited with a thickness of 4 nm as the third layer.

Voltage was applied to the light emitting device D37, to observe EL light emission. The CIE chromaticity coordinate (x, y) at 100 cd/m² was (0.37, 0.41). The light emitting device was driven under constant current at a current value of 1 mA, and the time until the luminance reached 70% of the initial luminance was measured.

### <Example D38> Fabrication and evaluation of light emitting device D38

A light emitting device D38 was fabricated in the same manner as in Example D37, except that "the compound EM-3" was used instead of "the compound EM-1" in (Formation of third layer: -D37) in Example D37.

Voltage was applied to the light emitting device D38, to observe EL light emission. The CIE chromaticity coordinate (x, y) at 100 cd/m² was (0.40, 0.41). The light emitting device was driven under constant current at a current value of 1 mA, and the time until the luminance reached 70% of the initial luminance was measured.

### <Example D39> Fabrication and evaluation of light emitting device D39

A light emitting device D39 was fabricated in the same manner as in Example D37, except that "the compound EM-5" was used instead of "the compound EM-1" in (Formation of third layer: -D37) in Example D37.

Voltage was applied to the light emitting device D39, to observe EL light emission. The CIE chromaticity coordinate (x, y) at 100 cd/m² was (0.34, 0.41). The light emitting device was driven under constant current at a current value of 1 mA, and the time until the luminance reached 70% of the initial luminance was measured.

### <Comparative Example CD18> Fabrication and evaluation of light emitting device CD18

A light emitting device CD18 was fabricated in the same manner as in Example D30, except that (Formation of third layer) was not conducted in Example D30.

Voltage was applied to the light emitting device CD18, to observe EL light emission. The CIE chromaticity coordinate (x, y) at 100 cd/m² was (0.34, 0.41). The light emitting device was driven under constant current at a current value of 1 mA, and the time until the luminance reached 70% of the initial luminance was measured.

The results of Examples D37 to D39 and Comparative Example CD18 are shown in Table 11. The relative value of the time (luminance life) until the luminance of the light emitting devices D37 to D39 reached 70% of the initial luminance when the time (luminance life) until the luminance of the light emitting device CD18 reached 70% of the initial luminance was taken as 1.0 is shown.

**[Table 11]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D37 | D37 | HM-2/B1 | 75/25 | ETL-1 | 100 | EM-1 | 100 | 8.1 |
| Example D38 | D38 | HM-2/B1 | 75/25 | ETL-1 | 100 | EM-3 | 100 | 4.0 |
| Example D39 | D39 | HM-2/B1 | 75/25 | ETL-1 | 100 | EM-5 | 100 | 1.4 |
| Comparative Example CD18 | CD18 | HM-2/B1 | 75/25 | ETL-1 | 100 | - | - | 1.0 |

### <Example D40> Fabrication and evaluation of light emitting device D40

A light emitting device D40 was fabricated in the same manner as in Example D1, except that "the compound HM-2 and the phosphorescent compound B1 (compound HM-2/phosphorescent compound B1 = 75% by mass/25% by mass)" were used instead of "the compound HM-2, the phosphorescent compound B1 and the phosphorescent compound G1 (compound HM-2/phosphorescent compound B1/phosphorescent compound G1 = 74% by mass/25% by mass/1% by mass)" in (Formation of first layer) in Example D1.

Voltage was applied to the light emitting device D40, to observe EL light emission. The CIE chromaticity coordinate (x, y) at 100 cd/m² was (0.47, 0.41). The light emitting device was driven under constant current at a current value of 1 mA, and the time until the luminance reached 75% of the initial luminance was measured.

### <Example D41> Fabrication and evaluation of light emitting device D41

A light emitting device D41 was fabricated in the same manner as in Example D40, except that "the compound HM-3" was used instead of "the compound HM-2" in (Formation of first layer) in Example D40.

Voltage was applied to the light emitting device D41, to observe EL light emission. The CIE chromaticity coordinate (x, y) at 100 cd/m² was (0.45, 0.41). The light emitting device was driven under constant current at a current value of 1 mA, and the time until the luminance reached 75% of the initial luminance was measured.

### <Comparative Example CD19> Fabrication and evaluation of light emitting device CD19

A light emitting device CD19 was fabricated in the same manner as in Example D40, except that (Formation of second layer) was not conducted in Example D40.

Voltage was applied to the light emitting device CD19, to observe EL light emission. The CIE chromaticity coordinate (x, y) at 100 cd/m² was (0.37, 0.41). The light emitting device was driven under constant current at a current value of 1 mA, and the time until the luminance reached 75% of the initial luminance was measured.

### <Comparative Example CD20> Fabrication and evaluation of light emitting device CD20

A light emitting device CD20 was fabricated in the same manner as in Example D40, except that "the compound HM-1" was used instead of "the compound HM-2" in (Formation of first layer) in Example D40.

Voltage was applied to the light emitting device CD20, to observe EL light emission. The CIE chromaticity coordinate (x, y) at 100 cd/m² was (0.45, 0.41). The light emitting device was driven under constant current at a current value of 1 mA, and the time until the luminance reached 75% of the initial luminance was measured.

The results of Examples D40 to D41 and Comparative Examples CD19 to CD20 are shown in Table 12. The relative value of the time (luminance life) until the luminance of the light emitting devices D40 to D41 and the light emitting device CD19 reached 75% of the initial luminance when the time (luminance life) until the luminance of the light emitting device CD20 reached 75% of the initial luminance was taken as 1.0 is shown.

**[Table 12]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D40 | D40 | HM-2/B1 | 75/25 | ETL-1 | 100 | NaF | 100 | 2.2 |
| Example D41 | D41 | HM-3/B1 | 75/25 | ETL-1 | 100 | NaF | 100 | 1.6 |
| Comparative Example CD19 | CD19 | HM-2/B1 | 75/25 | - | - | NaF | 100 | 0.3 |
| Comparative Example CD20 | CD20 | HM-1/B1 | 75/25 | ETL-1 | 100 | NaF | 100 | 1.0 |

### <Example D42> Fabrication and evaluation of light emitting device D42

### (Formation of anode and hole injection layer)

An ITO film with a thickness of 45 nm was deposited on a glass substrate by a sputtering method, to form an anode. On the anode, a hole injection material ND-3202 (manufactured by Nissan Chemical Corporation) was spin-coated, to form a film with a thickness of 35 nm. In an air atmosphere, the film was heated on a hot plate at 50°C for 3 minutes, and further, heated at 230°C for 15 minutes, to form a hole injection layer.

### (Formation of hole transporting layer)

The polymer compound HTL-3 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a hole transporting layer. By this heating, the polymer compound HTL-3 became a cross-linked body.

### (Formation of first layer)

The compound HM-2, the phosphorescent compound B1 and the phosphorescent compound R2 (compound HM-2/phosphorescent compound B1/phosphorescent compound R2 = 74.9% by mass/25% by mass/0.1% by mass) were dissolved at a concentration of 2.0% by mass in toluene. The resultant toluene solution was spin-coated on the hole transporting layer, to form a film with a thickness of 75 nm, and the film was heated at 130°C for 10 minutes under a nitrogen gas atmosphere, to form a first layer (light emitting layer).

### (Formation of second layer)

The polymer compound ETL-1 was dissolved in 2,2,3,3,4,4,5,5-octafluoro-1-pentanol at a concentration of 0.25% by mass. The resultant 2,2,3,3,4,4,5,5-octafluoro-1-pentanol solution was spin-coated on the first layer, to form a film with a thickness of 10 nm, and the film was heated at 130°C for 10 minutes under a nitrogen gas atmosphere, to form a second layer (electron transporting layer).

### (Formation of third layer)

The substrate carrying the second layer formed thereon was placed in a vapor deposition machine and the inner pressure thereof was reduced to 1.0×10⁻⁴ Pa or less, then, on the second layer, sodium fluoride (NaF) was vapor-deposited with a thickness of 4 nm as the third layer (electron injection layer).

### (Formation of cathode)

The substrate carrying the third layer formed thereon was placed in a vapor deposition machine and the inner pressure thereof was reduced to 1.0×10⁻⁴ Pa or less, then, as cathode, aluminum was vapor-deposited with a thickness of about 80 nm. After vapor deposition, sealing was performed with a glass substrate, to fabricate a light emitting device D42.

### (Evaluation of light emitting device)

Voltage was applied to the light emitting device D42, to observe EL light emission. The CIE chromaticity coordinate (x, y) at 100 cd/m² was (0.35, 0.43). The light emitting device was driven under constant current at a current value of 1 mA, and the time until the luminance reached 90% of the initial luminance was measured.

### <Comparative Example CD21> Fabrication and evaluation of light emitting device CD21

A light emitting device CD21 was fabricated in the same manner as in Example D42, except that "the compound HM-1" was used instead of "the compound HM-2" in (Formation of first layer) in Example D42.

Voltage was applied to the light emitting device CD21, to observe EL light emission. The CIE chromaticity coordinate (x, y) at 100 cd/m² was (0.31, 0.42). The light emitting device was driven under constant current at a current value of 1 mA, and the time until the luminance reached 90% of the initial luminance was measured.

The results of Example D42 and Comparative Example CD21 are shown in Table 13. The relative value of the time (luminance life) until the luminance of the light emitting device D42 reached 90% of the initial luminance when the time (luminance life) until the luminance of the light emitting device CD21 reached 90% of the initial luminance was taken as 1.0 is shown.

**[Table 13]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D42 | D42 | MH-2/B1/R2 | 74.9/25/0.1 | ETL-1 | 100 | NaF | 100 | 3.2 |
| Comparative Example CD21 | CD21 | HM-1/B1/R2 | 74.9/25/0.1 | ETL-1 | 100 | NaF | 100 | 1.0 |

### <Example D43> Fabrication and evaluation of light emitting device D43

A light emitting device D43 was fabricated in the same manner as in Example D42, except that "the compound HM-3, the phosphorescent compound B1 and the phosphorescent compound R1 (compound HM-3/phosphorescent compound B1/phosphorescent compound R1 = 74.9% by mass/25% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D43, to observe EL light emission. The CIE chromaticity coordinate (x, y) at 100 cd/m² was (0.35, 0.42). The light emitting device was driven under constant current at a current value of 1 mA, and the time until the luminance reached 80% of the initial luminance was measured.

### <Example D44> Fabrication and evaluation of light emitting device D44

A light emitting device D44 was fabricated in the same manner as in Example D43, except that "the phosphorescent compound R3" was used instead of "the phosphorescent compound R1" in (Formation of first layer) in Example D43.

Voltage was applied to the light emitting device D44, to observe EL light emission. The CIE chromaticity coordinate (x, y) at 100 cd/m² was (0.31, 0.41). The light emitting device was driven under constant current at a current value of 1 mA, and the time until the luminance reached 80% of the initial luminance was measured.

### <Example D45> Fabrication and evaluation of light emitting device D45

A light emitting device D45 was fabricated in the same manner as in Example D43, except that "the compound HM-4" was used instead of "the compound HM-3" in (Formation of first layer) in Example D43.

Voltage was applied to the light emitting device D45, to observe EL light emission. The CIE chromaticity coordinate (x, y) at 100 cd/m² was (0.33, 0.43). The light emitting device was driven under constant current at a current value of 1 mA, and the time until the luminance reached 80% of the initial luminance was measured.

The results of Examples D43 to D45 are shown in Table 14. The relative value of the time (luminance life) until the luminance of the light emitting devices D43 to D44 reached 80% of the initial luminance when the time (luminance life) until the luminance of the light emitting device D45 reached 80% of the initial luminance was taken as 1.0 is shown.

**[Table 14]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D43 | D43 | HM-3/B1/R1 | 74.9/25/0.1 | ETL-1 | 100 | NaF | 100 | 14.2 |
| Example D44 | D44 | HM-3/B1/R3 | 74.9/25/0.1 | ETL-1 | 100 | NaF | 100 | 8.0 |
| Example D45 | D45 | HM-4/B1/R1 | 74.9/25/0.1 | ETL-1 | 100 | NaF | 100 | 1.0 |

### <Example D46> Fabrication and evaluation of light emitting device D46

A light emitting device D46 was fabricated in the same manner as in Example D42, except that "the compound HM-2, the phosphorescent compound B1, the phosphorescent compound G1 and the phosphorescent compound R1 (compound HM-2/phosphorescent compound B1/phosphorescent compound G1/phosphorescent compound R1 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D46, to observe EL light emission. The CIE chromaticity coordinate (x, y) at 100 cd/m² was (0.32, 0.52). The light emitting device was driven under constant current at a current value of 1 mA, and the time until the luminance reached 70% of the initial luminance was measured.

### <Example D47> Fabrication and evaluation of light emitting device D47

A light emitting device D47 was fabricated in the same manner as in Example D46, except that "the compound HM-7" was used instead of "the compound HM-2" in (Formation of first layer) in Example D46.

Voltage was applied to the light emitting device D47, to observe EL light emission. The CIE chromaticity coordinate (x, y) at 100 cd/m² was (0.32, 0.52). The light emitting device was driven under constant current at a current value of 1 mA, and the time until the luminance reached 70% of the initial luminance was measured.

### <Example D48> Fabrication and evaluation of light emitting device D48

A light emitting device D48 was fabricated in the same manner as in Example D46, except that "the compound HM-8" was used instead of "the compound HM-2" in (Formation of first layer) in Example D46.

Voltage was applied to the light emitting device D48, to observe EL light emission. The CIE chromaticity coordinate (x, y) at 100 cd/m² was (0.32, 0.52). The light emitting device was driven under constant current at a current value of 1 mA, and the time until the luminance reached 70% of the initial luminance was measured.

### <Example D49> Fabrication and evaluation of light emitting device D49

A light emitting device D49 was fabricated in the same manner as in Example D46, except that "the compound HM-6" was used instead of "the compound HM-2" in (Formation of first layer) in Example D46.

Voltage was applied to the light emitting device D49, to observe EL light emission. The CIE chromaticity coordinate (x, y) at 100 cd/m² was (0.31, 0.51). The light emitting device was driven under constant current at a current value of 1 mA, and the time until the luminance reached 70% of the initial luminance was measured.

### <Example D50> Fabrication and evaluation of light emitting device D50

A light emitting device D50 was fabricated in the same manner as in Example D46, except that "the phosphorescent compound G3" was used instead of "the phosphorescent compound G1" in (Formation of first layer) in Example D46.

Voltage was applied to the light emitting device D50, to observe EL light emission. The CIE chromaticity coordinate (x, y) at 100 cd/m² was (0.38, 0.51). The light emitting device was driven under constant current at a current value of 1 mA, and the time until the luminance reached 70% of the initial luminance was measured.

### <Example D51> Fabrication and evaluation of light emitting device D51

A light emitting device D51 was fabricated in the same manner as in Example D46, except that "the phosphorescent compound G4" was used instead of "the phosphorescent compound G1" in (Formation of first layer) in Example D46.

Voltage was applied to the light emitting device D51, to observe EL light emission. The CIE chromaticity coordinate (x, y) at 100 cd/m² was (0.31, 0.48). The light emitting device was driven under constant current at a current value of 1 mA, and the time until the luminance reached 70% of the initial luminance was measured.

### <Example D52> Fabrication and evaluation of light emitting device D52

A light emitting device D52 was fabricated in the same manner as in Example D46, except that "the phosphorescent compound B4" was used instead of "the phosphorescent compound B1" in (Formation of first layer) in Example D46.

Voltage was applied to the light emitting device D52, to observe EL light emission. The CIE chromaticity coordinate (x, y) at 100 cd/m² was (0.32, 0.52). The light emitting device was driven under constant current at a current value of 1 mA, and the time until the luminance reached 70% of the initial luminance was measured.

### <Example D53> Fabrication and evaluation of light emitting device D53

A light emitting device D53 was fabricated in the same manner as in Example D46, except that "the compound HM-9" was used instead of "the compound HM-2" in (Formation of first layer) in Example D46.

Voltage was applied to the light emitting device D53, to observe EL light emission. The CIE chromaticity coordinate (x, y) at 100 cd/m² was (0.31, 0.50). The light emitting device was driven under constant current at a current value of 1 mA, and the time until the luminance reached 70% of the initial luminance was measured.

### <Example D54> Fabrication and evaluation of light emitting device D54

A light emitting device D54 was fabricated in the same manner as in Example D46, except that "the compound HM-4" was used instead of "the compound HM-2" in (Formation of first layer) in Example D46.

Voltage was applied to the light emitting device D53, to observe EL light emission. The CIE chromaticity coordinate (x, y) at 100 cd/m² was (0.36, 0.50). The light emitting device was driven under constant current at a current value of 1 mA, and the time until the luminance reached 70% of the initial luminance was measured.

### <Example D55> Fabrication and evaluation of light emitting device D55

A light emitting device D55 was fabricated in the same manner as in Example D42, except that "the compound HM-4, the phosphorescent compound B1, the phosphorescent compound G2 and the phosphorescent compound R3 (compound HM-4/phosphorescent compound B1/phosphorescent compound G2/phosphorescent compound R3 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D55, to observe EL light emission. The CIE chromaticity coordinate (x, y) at 100 cd/m² was (0.31, 0.53). The light emitting device was driven under constant current at a current value of 1 mA, and the time until the luminance reached 70% of the initial luminance was measured.

The results of Examples D46 to D55 are shown in Table 15. The relative value of the time (luminance life) until the luminance of the light emitting devices D46 to D54 reached 70% of the initial luminance when the time (luminance life) until the luminance of the light emitting device D55 reached 70% of the initial luminance was taken as 1.0 is shown.

**[Table 15]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D46 | D46 | HM-2/B1/G1/R1 | 73.9/25/1/0 .1 | ETL-1 | 100 | NaF | 100 | 15.9 |
| Example D47 | D47 | HM-7/B1/G1/R1 | 73.9/25/1/0 .1 | ETL-1 | 100 | NaF | 100 | 12.9 |
| Example D48 | D48 | HM-8/B1/G1/R1 | 73.9/25/1/0 .1 | ETL-1 | 100 | NaF | 100 | 6.6 |
| Example D49 | D49 | HM-6/B1/G1/R1 | 73.9/25/1/0 .1 | ETL-1 | 100 | NaF | 100 | 6.8 |
| Example D50 | D50 | HM-2/B1/G3/R1 | 73.9/25/1/0 .1 | ETL-1 | 100 | NaF | 100 | 10.9 |
| Example D51 | D51 | HM-2/B1/G4/R1 | 73.9/25/1/0 .1 | ETL-1 | 100 | NaF | 100 | 12.7 |
| Example D52 | D52 | HM-2/B4/G1/R1 | 73.9/25/1/0 .1 | ETL-1 | 100 | NaF | 100 | 15.3 |
| Example D53 | D53 | HM-9/B1/G1/R1 | 73.9/25/1/0 .1 | ETL-1 | 100 | NaF | 100 | 2.8 |
| Example D54 | D54 | HM-4/B1/G1/R1 | 73.9/25/1/0 .1 | ETL-1 | 100 | NaF | 100 | 1.8 |
| Example D55 | D55 | HM-4/B1/G2/R3 | 73.9/25/1/0 .1 | ETL-1 | 100 | NaF | 100 | 1.0 |

### <Example D56> Fabrication and evaluation of light emitting device D56

A light emitting device D56 was fabricated in the same manner as in Example D42, except that "the compound HM-2, the phosphorescent compound B1, the phosphorescent compound G2 and the phosphorescent compound R3 (compound HM-2/phosphorescent compound B1/phosphorescent compound G2/phosphorescent compound R3 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D56, to observe EL light emission. The CIE chromaticity coordinate (x, y) at 100 cd/m² was (0.34, 0.51). The light emitting device was driven under constant current at a current value of 1 mA, and the time until the luminance reached 80% of the initial luminance was measured.

### <Example D57> Fabrication and evaluation of light emitting device D57

A light emitting device D57 was fabricated in the same manner as in Example D42, except that "the compound HM-2, the phosphorescent compound B1, the phosphorescent compound G1 and the phosphorescent compound R2 (compound HM-2/phosphorescent compound B1/phosphorescent compound G1/phosphorescent compound R2 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D57, to observe EL light emission. The CIE chromaticity coordinate (x, y) at 100 cd/m² was (0.38, 0.49). The light emitting device was driven under constant current at a current value of 1 mA, and the time until the luminance reached 80% of the initial luminance was measured.

### <Example D58> Fabrication and evaluation of light emitting device D58

A light emitting device D58 was fabricated in the same manner as in Example D42, except that "the compound HM-2, the phosphorescent compound B1, the phosphorescent compound G1 and the phosphorescent compound R4 (compound HM-2/phosphorescent compound B1/phosphorescent compound G1/phosphorescent compound R4 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D58, to observe EL light emission. The CIE chromaticity coordinate (x, y) at 100 cd/m² was (0.36, 0.50). The light emitting device was driven under constant current at a current value of 1 mA, and the time until the luminance reached 80% of the initial luminance was measured.

### <Example D59> Fabrication and evaluation of light emitting device D59

A light emitting device D59 was fabricated in the same manner as in Example D42, except that "the compound HM-3, the phosphorescent compound B1, the phosphorescent compound G1 and the phosphorescent compound R1 (compound HM-3/phosphorescent compound B1/phosphorescent compound G1/phosphorescent compound R1 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D59, to observe EL light emission. The CIE chromaticity coordinate (x, y) at 100 cd/m² was (0.39, 0.49). The light emitting device was driven under constant current at a current value of 1 mA, and the time until the luminance reached 80% of the initial luminance was measured.

### <Example D60> Fabrication and evaluation of light emitting device D60

A light emitting device D59 was fabricated in the same manner as in Example D42, except that "the compound HM-5, the phosphorescent compound B1, the phosphorescent compound G1 and the phosphorescent compound R1 (compound HM-5/phosphorescent compound B1/phosphorescent compound G1/phosphorescent compound R1 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D60, to observe EL light emission. The CIE chromaticity coordinate (x, y) at 100 cd/m² was (0.31, 0.51). The light emitting device was driven under constant current at a current value of 1 mA, and the time until the luminance reached 80% of the initial luminance was measured.

### <Comparative Example CD22> Fabrication and evaluation of light emitting device CD22

A light emitting device CD22 was fabricated in the same manner as in Example D42, except that "the compound HM-1, the phosphorescent compound B1, the phosphorescent compound G2 and the phosphorescent compound R3 (compound HM-1/phosphorescent compound B1/phosphorescent compound G2/phosphorescent compound R3 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device CD22, to observe EL light emission. The CIE chromaticity coordinate (x, y) at 100 cd/m² was (0.30, 0.51). The light emitting device was driven under constant current at a current value of 1 mA, and the time until the luminance reached 80% of the initial luminance was measured.

### <Comparative Example CD23> Fabrication and evaluation of light emitting device CD23

A light emitting device CD23 was fabricated in the same manner as in Example D42, except that "the compound HM-1, the phosphorescent compound B1, the phosphorescent compound G1 and the phosphorescent compound R2 (compound HM-1/phosphorescent compound B1/phosphorescent compound G1/phosphorescent compound R2 = 73.9% by mass/25% by mass/1% by mass/0.1% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device CD23, to observe EL light emission. The CIE chromaticity coordinate (x, y) at 100 cd/m² was (0.38, 0.49). The light emitting device was driven under constant current at a current value of 1 mA, and the time until the luminance reached 80% of the initial luminance was measured.

The results of Examples D56 to D60 and Comparative Examples CD22 to CD23 are shown in Table 16. The relative value of the time (luminance life) until the luminance of the light emitting devices D56 to D60 and CD23 reached 80% of the initial luminance when the time (luminance life) until the luminance of the light emitting device CD22 reached 80% of the initial luminance was taken as 1.0 is shown.

**[Table 16]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D56 | D56 | HM-2/B1/G2/ R3 | 73.9/25/1/0 .1 | ETL-1 | 100 | NaF | 100 | 2.8 |
| Example D57 | D57 | HM-2/B1/G1/ R2 | 73.9/25/1/0 .1 | ETL-1 | 100 | NaF | 100 | 3.8 |
| Example D58 | D58 | HM-2/B1/G1/ R4 | 73.9/25/1/0 .1 | ETL-1 | 100 | NaF | 100 | 4.2 |
| Example D59 | D59 | HM-3/B1/G1/ R1 | 73.9/25/1/0 .1 | ETL-1 | 100 | NaF | 100 | 7.8 |
| Example D60 | D60 | HM-5/B1/G1/ R1 | 73.9/25/1/0 .1 | ETL-1 | 100 | NaF | 100 | 3.5 |
| Comparative Example CD22 | CD22 | HM-1/B1/G2/ R3 | 73.9/25/1/0 .1 | ETL-1 | 100 | NaF | 100 | 1.0 |
| Comparative Example CD23 | CD23 | HM-1/B1/G1/ R2 | 73.9/25/1/0 .1 | ETL-1 | 100 | NaF | 100 | 2.0 |

### <Example D61> Fabrication and evaluation of light emitting device D61

A light emitting device D61 was fabricated in the same manner as in Example D42, except that "the compound HM-3 and the phosphorescent compound B5 (compound HM-3/phosphorescent compound B5 = 75% by mass/25% by mass)" were used instead of "the compound HM-2, the phosphorescent compound B1 and the phosphorescent compound R2 (compound HM-2/phosphorescent compound B1/phosphorescent compound R2 = 74.9% by mass/25% by mass/0.1% by mass)" in (Formation of first layer) in Example D42.

Voltage was applied to the light emitting device D61, to observe EL light emission. The CIE chromaticity coordinate (x, y) at 100 cd/m² was (0.19, 0.41). The light emitting device was driven under constant current at a current value of 1 mA, and the time until the luminance reached 70% of the initial luminance was measured.

### <Comparative Example CD24> Fabrication and evaluation of light emitting device CD24

A light emitting device CD24 was fabricated in the same manner as in Example D61, except that "the compound HM-1" was used instead of "the compound HM-3" in (Formation of first layer) in Example D61.

Voltage was applied to the light emitting device CD24, to observe EL light emission. The CIE chromaticity coordinate (x, y) at 100 cd/m² was (0.19, 0.41). The light emitting device was driven under constant current at a current value of 1 mA, and the time until the luminance reached 70% of the initial luminance was measured.

The results of Example D61 and Comparative Example CD24 are shown in Table 17. The relative value of the time (luminance life) until the luminance of the light emitting device D61 reached 70% of the initial luminance when the time (luminance life) until the luminance of the light emitting device CD24 reached 70% of the initial luminance was taken as 1.0 is shown.

**[Table 17]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D61 | D61 | HM-3/B5 | 75/25 | ETL-1 | 100 | NaF | 100 | 2.1 |
| Comparative Example CD24 | CD24 | HM-1/B5 | 75/25 | ETL-1 | 100 | NaF | 100 | 1.0 |

### <Example D62> Fabrication and evaluation of light emitting device D62

A light emitting device D62 was fabricated in the same manner as in Example D61, except that "the compound HM-2 and the phosphorescent compound B1 (compound HM-2/phosphorescent compound B1 = 75% by mass/25% by mass)" were used instead of "the compound HM-3 and the phosphorescent compound B5 (compound HM-3/phosphorescent compound B5 = 75% by mass/25% by mass)" in (Formation of first layer) in Example D61.

Voltage was applied to the light emitting device D62, to observe EL light emission. The CIE chromaticity coordinate (x, y) at 100 cd/m² was (0.20, 0.43). The light emitting device was driven under constant current at a current value of 1 mA, and the time until the luminance reached 95% of the initial luminance was measured.

### <Comparative Example CD25> Fabrication and evaluation of light emitting device CD25

A light emitting device CD25 was fabricated in the same manner as in Example D61, except that "the compound HM-1" was used instead of "the compound HM-2" in (Formation of first layer) in Example D61.

Voltage was applied to the light emitting device CD25, to observe EL light emission. The CIE chromaticity coordinate (x, y) at 100 cd/m² was (0.20, 0.46). The light emitting device was driven under constant current at a current value of 1 mA, and the time until the luminance reached 95% of the initial luminance was measured.

The results of Example D62 and Comparative Example CD25 are shown in Table 18. The relative value of the time (luminance life) until the luminance of the light emitting device D62 reached 95% of the initial luminance when the time (luminance life) until the luminance of the light emitting device CD25 reached 95% of the initial luminance was taken as 1.0 is shown.

**[Table 18]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D62 | D62 | HM-2/B1 | 75/25 | ETL-1 | 100 | NaF | 100 | 36.5 |
| Comparative Example CD25 | CD25 | HM-1/B1 | 75/25 | ETL-1 | 100 | NaF | 100 | 1.0 |

### <Example D63> Fabrication and evaluation of light emitting device D63

A light emitting device D63 was fabricated in the same manner as in Example D61, except that "the phosphorescent compound B6" was used instead of "the phosphorescent compound B5" in (Formation of first layer) in Example D61.

Voltage was applied to the light emitting device D63, to observe EL light emission. The CIE chromaticity coordinate (x, y) at 100 cd/m² was (0.19, 0.39). The light emitting device was driven under constant current at a current value of 1 mA, and the time until the luminance reached 95% of the initial luminance was measured.

### <Comparative Example CD26> Fabrication and evaluation of light emitting device CD26

A light emitting device CD26 was fabricated in the same manner as in Example D63, except that "the compound HM-1" was used instead of "the compound HM-3" in (Formation of first layer) in Example D63.

Voltage was applied to the light emitting device CD26, to observe EL light emission. The CIE chromaticity coordinate (x, y) at 100 cd/m² was (0.19, 0.40). The light emitting device was driven under constant current at a current value of 1 mA, and the time until the luminance reached 95% of the initial luminance was measured.

The results of Example D63 and Comparative Example CD26 are shown in Table 19. The relative value of the time (luminance life) until the luminance of the light emitting device D63 reached 95% of the initial luminance when the time (luminance life) until the luminance of the light emitting device CD26 reached 95% of the initial luminance was taken as 1.0 is shown.

**[Table 19]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D63 | D63 | HM-3/B6 | 75/25 | ETL-1 | 100 | NaF | 100 | 25.1 |
| Comparative Example CD26 | CD26 | HM-1/B6 | 75/25 | ETL-1 | 100 | NaF | 100 | 1.0 |

### <Example D64> Fabrication and evaluation of light emitting device D64

### (Formation of anode and hole injection layer)

An ITO film with a thickness of 45 nm was deposited on a glass substrate by a sputtering method, to form an anode. On the anode, a hole injection material ND-3202 (manufactured by Nissan Chemical Corporation) was spin-coated, to form a film with a thickness of 35 nm. In an air atmosphere, the film was heated on a hot plate at 50°C for 3 minutes, and further, heated at 230°C for 15 minutes, to form a hole injection layer.

### (Formation of hole transporting layer)

The polymer compound HTL-2 was dissolved in xylene at a concentration of 0.7% by mass. The resultant xylene solution was spin-coated on the hole injection layer, to form a film with a thickness of 20 nm, and the film was heated on a hot plate at 180°C for 60 minutes under a nitrogen gas atmosphere, to form a hole transporting layer. By this heating, the polymer compound HTL-2 became a cross-linked body.

### (Formation of first layer)

The compound HM-2 and the phosphorescent compound G1 (compound HM-2/phosphorescent compound G1 = 70% by mass/30% by mass) were dissolved at a concentration of 2.0% by mass in toluene. The resultant toluene solution was spin-coated on the hole transporting layer, to form a film with a thickness of 75 nm, and the film was heated at 130°C for 10 minutes under a nitrogen gas atmosphere, to form a first layer (first light emitting layer).

### (Formation of second layer)

The polymer compound ETL-1 was dissolved in 2,2,3,3,4,4,5,5-octafluoro-1-pentanol at a concentration of 0.25% by mass. The resultant 2,2,3,3,4,4,5,5-octafluoro-1-pentanol solution was spin-coated on the first layer, to form a film with a thickness of 10 nm, and the film was heated at 130°C for 10 minutes under a nitrogen gas atmosphere, to form a second layer (electron transporting layer).

### (Formation of third layer)

The substrate carrying the second layer formed thereon was placed in a vapor deposition machine and the inner pressure thereof was reduced to 1.0×10⁻⁴ Pa or less, then, on the second layer, sodium fluoride (NaF) was vapor-deposited with a thickness of 4 nm as the third layer (electron injection layer).

### (Formation of cathode)

The substrate carrying the third layer formed thereon was placed in a vapor deposition machine and the inner pressure thereof was reduced to 1.0×10⁻⁴ Pa or less, then, as cathode, aluminum was vapor-deposited with a thickness of about 80 nm. After vapor deposition, sealing was performed with a glass substrate, to fabricate a light emitting device D64.

### (Evaluation of light emitting device)

Voltage was applied to the light emitting device D64, to observe EL light emission. The CIE chromaticity coordinate (x, y) at 100 cd/m² was (0.31, 0.63). The light emitting device was driven under constant current at a current value of 1 mA, and the time until the luminance reached 95% of the initial luminance was measured.

### <Example D65> Fabrication and evaluation of light emitting device D15

A light emitting device D65 was fabricated in the same manner as in Example D64 except that "the compound HM-3" was used instead of "the compound HM-2" in (Formation of first layer) in Example D64.

Voltage was applied to the light emitting device D65, to observe EL light emission. The CIE chromaticity coordinate (x, y) at 100 cd/m² was (0.30, 0.63). The light emitting device was driven under constant current at a current value of 1 mA, and the time until the luminance reached 95% of the initial luminance was measured.

### <Example D66> Fabrication and evaluation of light emitting device D66

A light emitting device D66 was fabricated in the same manner as in Example D64, except that "the compound HM-5" was used instead of "the compound HM-2" in (Formation of first layer) in Example D64.

Voltage was applied to the light emitting device D66, to observe EL light emission. The CIE chromaticity coordinate (x, y) at 100 cd/m² was (0.30, 0.64). The light emitting device was driven under constant current at a current value of 1 mA, and the time until the luminance reached 95% of the initial luminance was measured.

### <Example D67> Fabrication and evaluation of light emitting device D67

A light emitting device D67 was fabricated in the same manner as in Example D64, except that "the compound HM-8" was used instead of "the compound HM-2" in (Formation of first layer) in Example D64.

Voltage was applied to the light emitting device D67, to observe EL light emission. The CIE chromaticity coordinate (x, y) at 100 cd/m² was (0.31, 0.63). The light emitting device was driven under constant current at a current value of 1 mA, and the time until the luminance reached 95% of the initial luminance was measured.

### <Example D68> Fabrication and evaluation of light emitting device D68

A light emitting device D68 was fabricated in the same manner as in Example D64, except that "the compound HM-7" was used instead of "the compound HM-2" in (Formation of first layer) in Example D64.

Voltage was applied to the light emitting device D68, to observe EL light emission. The CIE chromaticity coordinate (x, y) at 100 cd/m² was (0.31, 0.63). The light emitting device was driven under constant current at a current value of 1 mA, and the time until the luminance reached 95% of the initial luminance was measured.

### <Example D69> Fabrication and evaluation of light emitting device D69

A light emitting device D69 was fabricated in the same manner as in Example D64, except that "the compound HM-6" was used instead of "the compound HM-2" in (Formation of first layer) in Example D64.

Voltage was applied to the light emitting device D69, to observe EL light emission. The CIE chromaticity coordinate (x, y) at 100 cd/m² was (0.30, 0.64). The light emitting device was driven under constant current at a current value of 1 mA, and the time until the luminance reached 95% of the initial luminance was measured.

### <Example D70> Fabrication and evaluation of light emitting device D70

A light emitting device D70 was fabricated in the same manner as in Example D64, except that "the compound HM-10" was used instead of "the compound HM-2" in (Formation of first layer) in Example D64.

Voltage was applied to the light emitting device D70, to observe EL light emission. The CIE chromaticity coordinate (x, y) at 100 cd/m² was (0.31, 0.63). The light emitting device was driven under constant current at a current value of 1 mA, and the time until the luminance reached 95% of the initial luminance was measured.

The results of Examples D64 to D70 are shown in Table 20. The relative value of the time (luminance life) until the luminance of the light emitting devices D64 to D69 reached 95% of the initial luminance when the time (luminance life) until the luminance of the light emitting device D70 reached 95% of the initial luminance was taken as 1.0 is shown.

**[Table 20]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D64 | D64 | HM-2/G1 | 70/30 | ETL-1 | 100 | NaF | 100 | 8.2 |
| Example D65 | D65 | HM-3/G1 | 70/30 | ETL-1 | 100 | NaF | 100 | 12.7 |
| Example D66 | D66 | HM-5/G1 | 70/30 | ETL-1 | 100 | NaF | 100 | 6.0 |
| Example D67 | D67 | HM-8/G1 | 70/30 | ETL-1 | 100 | NaF | 100 | 3.6 |
| Example D68 | D68 | HM-7/G1 | 70/30 | ETL-1 | 100 | NaF | 100 | 7.0 |
| Example D69 | D69 | HM-6/G1 | 70/30 | ETL-1 | 100 | NaF | 100 | 2.3 |
| Example D70 | D70 | HM-10/G1 | 70/30 | ETL-1 | 100 | NaF | 100 | 1.0 |

### <Example D71> Fabrication and evaluation of light emitting device D71

A light emitting device D71 was fabricated in the same manner as in Example D64, except that "the compound HM-2 and the phosphorescent compound R1 (compound HM-2/phosphorescent compound R1 = 90% by mass/10% by mass)" were used instead of "the compound HM-2 and the phosphorescent compound G1 (compound HM-2/phosphorescent compound G1 = 70% by mass/30% by mass)" in (Formation of first layer) in Example D64.

Voltage was applied to the light emitting device D71, to observe EL light emission. The CIE chromaticity coordinate (x, y) at 100 cd/m² was (0.67, 0.32). The light emitting device was driven under constant current at a current value of 1 mA, and the time until the luminance reached 75% of the initial luminance was measured.

### <Example D72> Fabrication and evaluation of light emitting device D72

A light emitting device D72 was fabricated in the same manner as in Example D71, except that "the compound HM-3" was used instead of "the compound HM-2" in (Formation of first layer) in Example D71.

Voltage was applied to the light emitting device D72, to observe EL light emission. The CIE chromaticity coordinate (x, y) at 100 cd/m² was (0.67, 0.33). The light emitting device was driven under constant current at a current value of 1 mA, and the time until the luminance reached 75% of the initial luminance was measured.

### <Example D73> Fabrication and evaluation of light emitting device D73

A light emitting device D73 was fabricated in the same manner as in Example D71, except that "the compound HM-11" was used instead of "the compound HM-2" in (Formation of first layer) in Example D71.

Voltage was applied to the light emitting device D73, to observe EL light emission. The CIE chromaticity coordinate (x, y) at 100 cd/m² was (0.67, 0.32). The light emitting device was driven under constant current at a current value of 1 mA, and the time until the luminance reached 75% of the initial luminance was measured.

### <Example D74> Fabrication and evaluation of light emitting device D74

A light emitting device D74 was fabricated in the same manner as in Example D71, except that "the compound HM-7" was used instead of "the compound HM-2" in (Formation of first layer) in Example D71.

Voltage was applied to the light emitting device D74, to observe EL light emission. The CIE chromaticity coordinate (x, y) at 100 cd/m² was (0.67, 0.32). The light emitting device was driven under constant current at a current value of 1 mA, and the time until the luminance reached 75% of the initial luminance was measured.

### <Example D75> Fabrication and evaluation of light emitting device D75

A light emitting device D75 was fabricated in the same manner as in Example D71, except that "the compound HM-6" was used instead of "the compound HM-2" in (Formation of first layer) in Example D71.

Voltage was applied to the light emitting device D75, to observe EL light emission. The CIE chromaticity coordinate (x, y) at 100 cd/m² was (0.67, 0.32). The light emitting device was driven under constant current at a current value of 1 mA, and the time until the luminance reached 75% of the initial luminance was measured.

### <Example D76> Fabrication and evaluation of light emitting device D76

A light emitting device D76 was fabricated in the same manner as in Example D71, except that "the compound HM-10" was used instead of "the compound HM-2" in (Formation of first layer) in Example D71.

Voltage was applied to the light emitting device D76, to observe EL light emission. The CIE chromaticity coordinate (x, y) at 100 cd/m² was (0.67, 0.32). The light emitting device was driven under constant current at a current value of 1 mA, and the time until the luminance reached 75% of the initial luminance was measured.

The results of Examples D71 to D76 are shown in Table 21. The relative value of the time (luminance life) until the luminance of the light emitting devices D71 to D75 reached 75% of the initial luminance when the time (luminance life) until the luminance of the light emitting device D76 reached 75% of the initial luminance was taken as 1.0 is shown.

**[Table 21]**

| | light emitting device | first layer | | second layer | | third layer | | luminance life (relative value) |
|---|---|---|---|---|---|---|---|---|
| | | material | composition ratio (% by mass) | material | composition ratio (% by mass) | material | composition ratio (% by mass) | |
| Example D71 | D71 | HM-2/R1 | 90/10 | ETL-1 | 100 | NaF | 100 | 41.5 |
| Example D72 | D72 | HM-3/R1 | 90/10 | ETL-1 | 100 | NaF | 100 | 20.8 |
| Example D73 | D73 | HM-11/R1 | 90/10 | ETL-1 | 100 | NaF | 100 | 3.1 |
| Example D74 | D74 | HM-7/R1 | 90/10 | ETL-1 | 100 | NaF | 100 | 26.9 |
| Example D75 | D75 | HM-6/R1 | 90/10 | ETL-1 | 100 | NaF | 100 | 14.6 |
| Example D76 | D76 | HM-10/R1 | 90/10 | ETL-1 | 100 | NaF | 100 | 1.0 |

### Industrial Applicability

According to the present invention, a light emitting device excellent in luminance life can be provided.

## Claims

1. A light emitting device having
an anode,
a cathode,
a first layer containing a compound represented by the formula (C-1), disposed between the anode and the cathode,
a second layer containing at least one selected from the group consisting of a simple substance composed only of an alkali metal element, a simple substance composed only of a group 2 element, a compound containing an alkali metal element and a compound containing a group 2 element, disposed between the first layer and the cathode, and
a third layer containing at least one selected from the group consisting of a simple substance composed only of an alkali metal element, a simple substance composed only of a group 2 element, a compound containing an alkali metal element and a compound containing a group 2 element, disposed between the second layer and the cathode in contact with the cathode,
wherein the at least one selected from the group consisting of a simple substance composed only of an alkali metal element, a simple substance composed only of a group 2 element, a compound containing an alkali metal element and a compound containing a group 2 element contained in the second layer and the at least one selected from the group consisting of a simple substance composed only of an alkali metal element, a simple substance composed only of a group 2 element, a compound containing an alkali metal element and a compound containing a group 2 element contained in the third layer are mutually different: 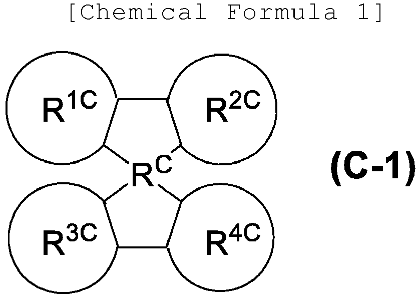 wherein,
Ring R^{1C}, Ring R^{2C}, Ring R^{3C} and Ring R^{4C} each independently represent an aromatic hydrocarbon ring or an aromatic hetero ring, and the foregoing rings optionally have a substituent, and when a plurality of the substituents are present, they may be combined together to form a ring together with the atoms to which they are attached, and
R^{C} represents a carbon atom, a silicon atom, a germanium atom, a tin atom or a lead atom.

2. The light emitting device according to Claim 1, wherein at least one of said Ring R^{1C}, said Ring R^{2C}, said Ring R^{3C} and said Ring R^{4C} has a group represented by the formula (D-1) as a substituent: 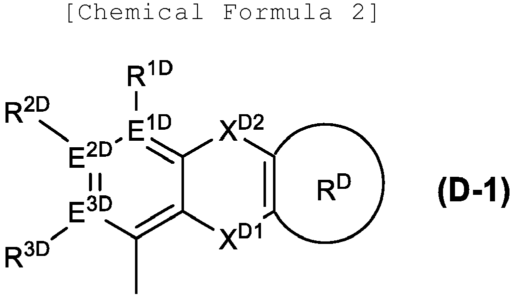 wherein,
Ring R^{D} represents an aromatic hydrocarbon ring or an aromatic hetero ring, and the foregoing rings optionally have a substituent, and when a plurality of the substituents are present, they may be combined together to form a ring together with the atoms to which they are attached,
X^{D1} and X^{D2} each independently represent a single bond, an oxygen atom, a sulfur atom, a group represented by -N(R^{XD1})- or a group represented by - C(R^{XD2})₂-, R^{XD1} and R^{XD2} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent, a plurality of R^{XD2} may be the same or different, and may be combined together to form a ring together with the carbon atoms to which they are attached,
E^{1D}, E^{2D} and E^{3D} each independently represent a nitrogen atom or a carbon atom,
R^{1D}, R^{2D} and R^{3D} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent,
when E^{1D} is a nitrogen atom, R^{1D} is absent, when E^{2D} is a nitrogen atom, R^{2D} is absent and when E^{3D} is a nitrogen atom, R^{3D} is absent,
R^{1D} and R^{2D} may be combined together to form a ring together with the carbon atoms to which they are attached, R^{2D} and R^{3D} may be combined together to form a ring together with the carbon atoms to which they are attached, R^{1D} and R^{XD1} may be combined together to form a ring together with the atoms to which they are attached, R^{1D} and R^{XD2} may be combined together to form a ring together with the carbon atoms to which they are attached, the substituent which Ring R^{D} optionally has and R^{XD1} may be combined together to form a ring together with the atoms to which they are attached, and the substituent which Ring R^{D} optionally has and R^{XD2} may be combined together to form a ring together with the carbon atoms to which they are attached.

3. The light emitting device according Claim 2, wherein said group represented by the formula (D-1) is a group represented by the formula (D-2): 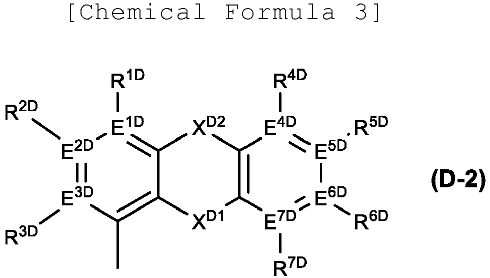 wherein,
X^{D1}, X^{D2}, E^{1D}, E^{2D}, E^{3D}, R^{1D}, R^{2D} and R^{3D} represent the same meaning as described above,
E^{4D}, E^{5D}, E^{6D} and E^{7D} each independently represent a nitrogen atom or a carbon atom,
R^{4D}, R^{5D}, R^{6D} and R^{7D} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent,
when E^{4D} is a nitrogen atom, R^{4D} is absent, when E^{5D} is a nitrogen atom, R^{5D} is absent, when E^{6D} is a nitrogen atom, R^{6D} is absent and when E^{7D} is a nitrogen atom, R^{7D} is absent, and
R^{4D} and R^{5D}, R^{5D} and R^{6D}, R^{6D} and R^{7D}, R^{4D} and R^{XD1}, R^{4D} and R^{XD2}, R^{7D} and R^{XD1}, and R^{7D} and R^{XD2} each may be combined together to form a ring together with the atoms to which they are attached.

4. The light emitting device according to any one of Claims 1 to 3, wherein said compound represented by the formula (C-1) is a compound represented by the formula (C-2): 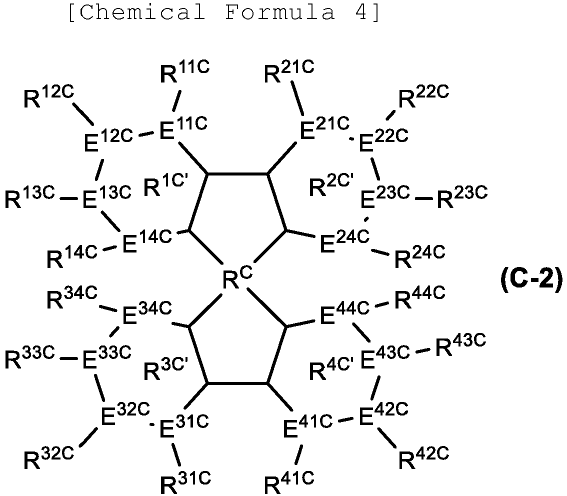 wherein,
R^{C} represents the same meaning as described above, E^{11C}, E^{12C}, E^{13C}, E^{14C}, E^{21C}, E^{22C}, E^{23C}, E^{24C}, E^{31C}, E^{32C}, E^{33C}, E^{34C}, E^{41C}, E^{42C}, E^{43C} and E^{44C} each independently represent a nitrogen atom or a carbon atom,
Ring R^{1C'}, Ring R^{2C'}, Ring R^{3C'} and Ring R^{4C'} each independently represent a benzene ring, a pyridine ring or a diazabenzene ring,
R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} each independently represent a hydrogen atom, an alkyl group, a cycloalkyl group, an alkoxy group, a cycloalkoxy group, an aryl group, an aryloxy group, a monovalent heterocyclic group, a substituted amino group or a halogen atom, and the foregoing groups optionally have a substituent,
when E^{11C} is a nitrogen atom, R^{11C} is absent, when E^{12C} is a nitrogen atom, R^{12C} is absent, when E^{13C} is a nitrogen atom, R^{13C} is absent, when E^{14C} is a nitrogen atom, R^{14C} is absent, when E^{21C} is a nitrogen atom, R^{21C} is absent, when E^{22C} is a nitrogen atom, R^{22C} is absent, when E^{23C} is a nitrogen atom, R^{23C} is absent, when E^{24C} is a nitrogen atom, R^{24C} is absent, when E^{31C} is a nitrogen atom, R^{31C} is absent, when E^{32C} is a nitrogen atom, R^{32C} is absent, when E^{33C} is a nitrogen atom, R^{33C} is absent, when E^{34C} is a nitrogen atom, R^{34C} is absent, when E^{41C} is a nitrogen atom, R^{41C} is absent, when E^{42C} is a nitrogen atom, R^{42C} is absent, when E^{43C} is a nitrogen atom, R^{43C} is absent and when E^{44C} is a nitrogen atom, R^{44C} is absent, and
R^{11C} and R^{12C}, R^{12C} and R^{13C}, R^{13C} and R^{14C}, R^{14C} and R^{34C}, R^{34C} and R^{33C}, R^{33C} and R^{32C}, R^{32C} and R^{31C}, R^{31C} and R^{41C}, R^{41C} and R^{42C}, R^{42C} and R^{43C}, R^{43C} and R^{44C}, R^{44C} and R^{24C}, R^{24C} and R^{23C}, R^{23C} and R^{22C}, R^{22C} and R^{21C}, and R^{21C} and R^{11C} each may be combined together to form a ring together with the carbon atoms to which they are attached.

5. The light emitting device according to Claim 4, wherein said compound represented by the formula (C-2) is a compound represented by the formula (C-3): 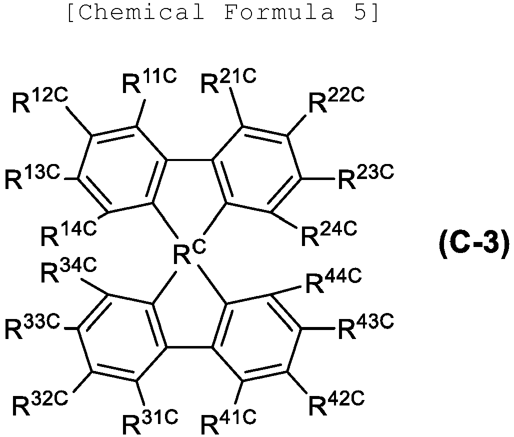 wherein, R^{C}, R^{11C}, R^{12C}, R^{13C}, R^{14C}, R^{21C}, R^{22C}, R^{23C}, R^{24C}, R^{31C}, R^{32C}, R^{33C}, R^{34C}, R^{41C}, R^{42C}, R^{43C} and R^{44C} represent the same meaning as described above.

6. The light emitting device according to Claim 4 or 5, wherein at least one of said R^{11C}, said R^{12C}, said R^{14C}, said R^{21C}, said R^{22C}, said R^{24C}, said R^{31C}, said R^{32C}, said R^{34C}, said R^{41C}, said R^{42C} and said R^{44C} is said group represented by the formula (D-1).

7. The light emitting device according to any one of Claims 1 to 6, wherein said first layer further contains a phosphorescent compound.

8. The light emitting device according to Claim 7, wherein said phosphorescent compound is a phosphorescent compound represented by the formula (1): 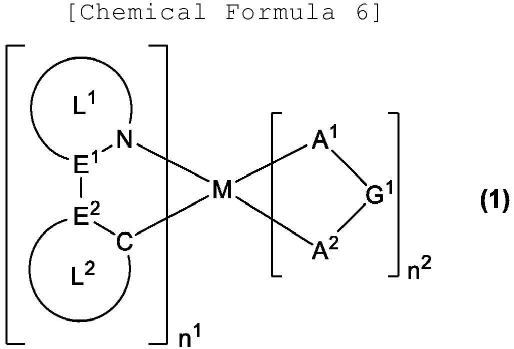 wherein,
M represents a ruthenium atom, a rhodium atom, a palladium atom, an iridium atom or a platinum atom,
n¹ represents an integer of 1 or more, and n² represents an integer of 0 or more, and, n¹+n² is 3 when M is a ruthenium atom, a rhodium atom or an iridium atom, while n¹+n² is 2 when M is a palladium atom or a platinum atom,
E¹ and E² each independently represent a carbon atom or a nitrogen atom, at least one of E¹ and E² is a carbon atom, and when a plurality of E¹ and E² are present, they may be the same or different at each occurrence,
Ring L¹ represents an aromatic hetero ring optionally having a substituent, and when a plurality of the substituents are present, they may be combined together to form a ring together with the atoms to which they are attached, and when a plurality of Ring L¹ are present, they may be the same or different,
Ring L² represents an aromatic hydrocarbon ring or an aromatic hetero ring, and the foregoing rings optionally have a substituent, and when a plurality of the substituents are present, they may be combined together to form a ring together with the atoms to which they are attached, and when a plurality of Ring L² are present, they may be the same or different,
the substituent which Ring L¹ optionally has and the substituent which Ring L² optionally has may be combined together to form a ring together with the atoms to which they are attached,
A¹-G¹-A² represents an anionic bidentate ligand, A¹ and A² each independently represent a carbon atom, an oxygen atom or a nitrogen atom, and these atoms may be a ring constituent atom, G¹ represents a single bond or an atomic group constituting a bidentate ligand together with A¹ and A², and when a plurality of A¹-G¹-A² are present, they may be the same or different.

9. The light emitting device according to Claim 8, wherein said phosphorescent compound represented by the formula (1) is a phosphorescent compound represented by the formula (1-A) or a phosphorescent compound represented by the formula (1-B): 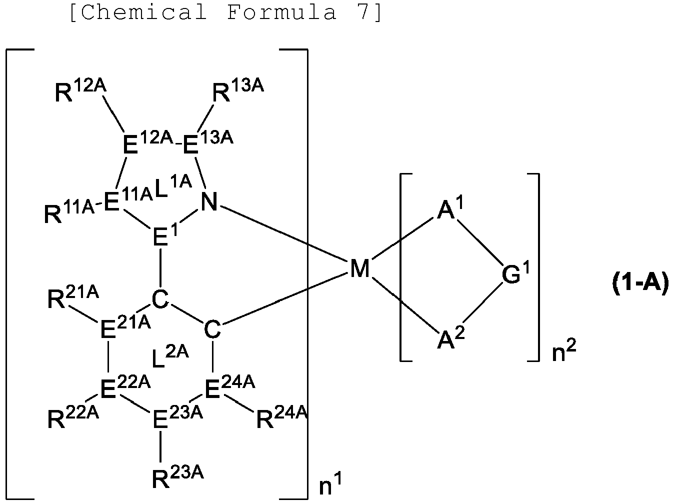 wherein,
M, n¹, n², E¹ and A¹-G¹-A² represent the same meaning as described above,
E^{11A}, E^{12A}, E^{13A}, E^{21A}, E^{22A}, E^{23A} and E^{24A} each independently represent a nitrogen atom or a carbon atom, and when a plurality of E^{11A}, E^{12A}, E^{13A}, E^{21A}, E^{22A}, E^{23A} and E^{24A} are present, they may be the same or different at each occurrence, and when E^{11A} is a nitrogen atom, R^{11A} may be present or absent, when E^{12A} is a nitrogen atom, R^{12A} may be present or absent, when E^{13A} is a nitrogen atom, R^{13A} may be present or absent, when E^{21A} is a nitrogen atom, R^{21A} is absent, when E^{22A} is a nitrogen atom, R^{22A} is absent, when E^{23A} is a nitrogen atom, R^{23A} is absent and when E^{24A} is a nitrogen atom, R^{24A} is absent,
R^{11A}, R^{12A}, R^{13A}, R^{21A}, R^{22A}, R^{23A} and R^{24A} each independently represent a hydrogen atom, an alkyl group, a cycloalkyl group, an alkoxy group, a cycloalkoxy group, an aryl group, an aryloxy group, an alkenyl group, a monovalent heterocyclic group, a substituted amino group or a halogen atom, and the foregoing groups optionally have a substituent, and when a plurality of R^{11A}, R^{12A}, R^{13A}, R^{21A}, R^{22A}, R^{23A} and R^{24A} are present, they may be the same or different at each occurrence, and, R^{11A} and R^{12A}, R^{12A} and R^{13A}, R^{11A} and R^{21A}, R^{21A} and R^{22A}, R^{22A} and R^{23A}, and R^{23A} and R^{24A} each may be combined together to form a ring together with the atoms to which they are attached,
Ring L^{1A} represents a triazole ring or a diazole ring,
Ring L^{2A} represents a benzene ring, a pyridine ring or a diazabenzene ring, 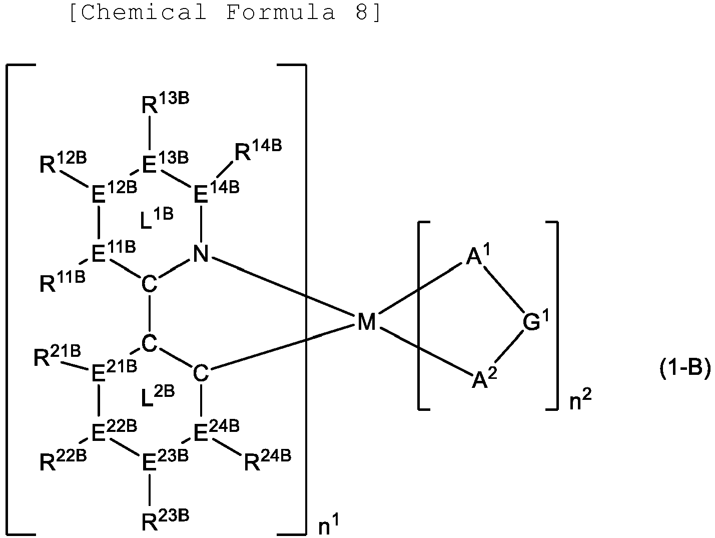
wherein,
M, n¹ , n² and A¹-G¹-A² represent the same meaning as described above,
E^{11B}, E^{12B}, E^{13B}, E^{14B}, E^{21B}, E^{22B}, E^{23B} and E^{24B} each independently represent a nitrogen atom or a carbon atom, and when a plurality of E^{11B}, E^{12B}, E^{13B}, E^{14B}, E^{21B}, E^{22B}, E^{23B} and E^{24B} are present, they may be the same or different at each occurrence, and when E^{11B} is a nitrogen atom, R^{11B} is absent, when E^{12B} is a nitrogen atom, R^{12B} is absent, when E^{13B} is a nitrogen atom, R^{13B} is absent, when E^{14B} is a nitrogen atom, R^{14B} is absent, when E^{21B} is a nitrogen atom, R^{21B} is absent, when E^{22B} is a nitrogen atom, R^{22B} is absent, when E^{23B} is a nitrogen atom, R^{23B} is absent and when E^{24B} is a nitrogen atom, R^{24B} is absent,
R^{11B}, R^{12B}, R^{13B}, R^{14B}, R^{21B}, R^{22B}, R^{23B} and R^{24B} each independently represent a hydrogen atom, an alkyl group, a cycloalkyl group, an alkoxy group, a cycloalkoxy group, an aryl group, an aryloxy group, an alkenyl group, a monovalent heterocyclic group, a substituted amino group or a halogen atom, and the foregoing groups optionally have a substituent, and when a plurality of R^{11B}, R^{12B}, R^{13B}, R^{14B}, R^{21B}, R^{22B}, R^{23B} and R^{24B} are present, they may be the same or different at each occurrence, and, R^{11B} and R^{12B}, R^{12B} and R^{13B}, R^{13B} and R^{14B}, R^{11B} and R^{21B}, R^{21B} and R^{22B}, R^{22B} and R^{23B}, and R^{23B} and R^{24B} each may be combined together to form a ring together with the carbon atoms to which they are attached,
Ring L^{1B} represents a pyridine ring or a diazabenzene ring, and
Ring L^{2B} represents a benzene ring, a pyridine ring or a diazabenzene ring.

10. The light emitting device according to any one of Claims 1 to 9, wherein said second layer is a layer containing at least one selected from the group consisting of said compound containing an alkali metal element and said compound containing a group 2 element.

11. The light emitting device according to any one of Claims 1 to 10, wherein said third layer is a layer containing at least one selected from the group consisting of said compound containing an alkali metal element and said simple substance composed only of a group 2 element.

12. The light emitting device according to any one of Claims 1 to 11, wherein said second layer and said third layer are adjacent.

13. The light emitting device according to any one of Claims 1 to 12, wherein said first layer and said second layer are adjacent.
